# EUROPEAN PATENT SPECIFICATION

(11) **EP 3 924 054 B1**
(45) Date of publication and mention of the grant of the patent: **02.04.2025**
(21) Application number: 20708198.5
(22) Date of filing: 13.02.2020
(51) Int. Cl.: A61P 35/00, C07D 401/04, C07D 401/14, A61K 31/454

(54) **3-(1-OXO-5-(PIPERIDIN-4-YL)ISOINDOLIN-2-YL)PIPERIDINE-2,6-DIONE DERIVATIVES AND USES THEREOF**
3-(1-OXO-5- (PIPERIDIN-4-YL)ISOINDOLIN-2-YL)PIPERIDIN-2,6-DIONDERIVATE UND IHRE VERWENDUNGEN
DÉRIVÉS DE 3-(1-OXO-5-(PIPÉRIDIN-4-YL)ISOINDOLIN-2-YL)PIPÉRIDINE-2,6-DIONE ET LEURS UTILISATIONS

(30) Priority: 15.02.2019 US 201962806140 P
(43) Date of publication of application: 22.12.2021
(73) Proprietor: Novartis AG, 4056 Basel (CH)
(72) Inventor: BECKWITH, Rohan Eric John, Cambridge, Massachusetts 02139 (US); BONAZZI, Simone, Cambridge, Massachusetts 02139 (US); CERNIJENKO, Artiom, Cambridge, Massachusetts 02139 (US); VISSER, Michael Scott, Cambridge, Massachusetts 02139 (US)
(74) Representative: Reid, Stephanie Marie
(86) International application number: PCT/IB2020/051205
(87) International publication number: WO 2020/165833

(56) References cited:
- WO-A1-2018/102725
- WO-A1-2018/140809
- WO-A1-2019/038717
- WO-A1-2019/079569
- US-A1- 2018 099 940
- PETER H SCHAFER ET AL: "Cereblon modulator iberdomide induces degradation of the transcription factors Ikaros and Aiolos: immunomodulation in healthy volunteers and relevance to systemic lupus erythematosus", ANNALS OF THE RHEUMATIC DISEASES, vol. 77, no. 10, 26 June 2018 (2018-06-26), GB, pages 1516 - 1523, XP055686777, ISSN: 0003-4967, DOI: 10.1136/annrheumdis-2017-212916

## Description

### FIELD OF THE INVENTION

The present invention relates to 3-(1-oxo-5-(piperidin-4-yl)isoindolin-2-yl)piperidine-2,6-dione compounds and compositions and their use for the treatment of IKAROS Family Zinc Finger 2 (IKZF2)-dependent diseases or disorders or where reduction of IKZF2 or IKZF4 protein levels can ameliorate a disease or disorder.

### BACKGROUND OF THE INVENTION

IKAROS Family Zinc Finger 2 (IKZF2) (also known as Helios) is one of the five members of the Ikaros family of transcription factors found in mammals. IKZF2 contains four zinc finger domains near the N-terminus, which are involved in DNA binding, and two zinc finger domains at the C-terminus, which are involved in protein dimerization. IKZF2 is about 50% identical with Ikaros family members, Ikaros (IKZF1), Aiolos (IKZF3), and Eos (IKZF4) with highest homology in the zinc finger regions (80%+ identity). These four Ikaros family transcription factors bind to the same DNA consensus site and can heterodimerize with each other when co-expressed in cells. The fifth Ikaros family protein, Pegasus (IKZF5), is only 25% identical to IKZF2, binds a different DNA site than other Ikaros family members and does not readily heterodimerize with the other Ikaros family proteins. IKZF2, IKZF1 and IKZF3 are expressed mainly in hematopoietic cells while IKZF4 and IKZF5 are expressed in a wide variety of tissues. (John, L.B., et al., (2011), Mol. Immunol. 48:1272-1278; Perdomo, J., et al., (2000), J. Biol. Chem. 275:38347-38354.)

IKZF2 is believed to have an important role in the function and stability of regulatory T cells (Tregs). IKZF2 is highly expressed at the mRNA and protein level by regulatory T-cell populations. Knockdown of IKZF2 by siRNA has been shown to result in downregulation of FoxP3 and to impair the ability of isolated human CD4+ CD25+ Tregs to block T-cell activation *in vitro.* Moreover, overexpression of IKZF2 in isolated murine Tregs has been shown to increase expression of Treg related markers such as CD103 and GITR and the IKZF2 overexpressing cells showed increased suppression of responder T-cells. IKZF2 has also been found to bind the promoter of FoxP3, the defining transcription factor of the regulatory T-cell lineage, and to affect FoxP3 expression.

Knockout of IKZF2 within FoxP3-expressing Tregs in mice has been shown to cause activated Tregs to lose their inhibitory properties, to express T-effector cytokines, and to take on T-effector functions. IKZF2 knockout mutant mice develop autoimmune disease by 6-8 months of age, with increased numbers of activated CD4 and CD8 T cells, follicular helper T cells and germinal center B cells. This observed effect is believed to be cell intrinsic, as Rag2-/- mice given bone marrow from IKZF2 knockout mice, but not bone marrow from IKZF2+/+ develop autoimmune disease. Direct evidence that IKZF2 affects regulatory T-cell function has been shown in the analysis of mice in which IKZF2 was deleted only in FoxP3 expressing cells (FoxP3-YFP-Cre Heliosfl/fl). The results showed that the mice also develop autoimmune disease with similar features as observed in the whole animal IKZF2 knockout. Moreover, pathway analysis of a CHIP-SEQ experiment has also suggested that IKZF2 is affecting expression of genes in the STAT5/IL-2Rα pathway in regulatory T-cells. This effect of IKZF2 loss was shown to be more apparent after an immune challenge (viral infection or injection with sheep's blood), and it was noted that after immune stimulation, the IKZF2 negative regulatory T cells began to take on features of effector T cells. (Getnet, D., et al., Mol. Immunol. (2010), 47:1595-1600; Bin Dhuban, K.., et al., (2015), J. Immunol. 194 :3687-96; Kim, H-J., et al., (2015), Science 350 :334-339; Nakawaga, H., et al., (2016) PNAS, 113: 6248-6253)

Overexpression of Ikaros isoforms which lack the DNA binding regions have been shown to be associated with multiple human haematological malignancies. Recently, mutations in the IKZF2 gene, which lead to abnormal splicing variants, have been identified in adult T-cell leukemias and low hypodiploid acute lymphoblastic leukemia. It has been proposed that these isoforms, which are capable of dimerization, have a dominant negative effect on Ikaros family transcription factors which primes the development of lymphomas. IKZF2 knockout mutants that survive into adulthood do not develop lymphomas, supporting this hypothesis (Asanuma, S., et al., (2013), Cancer Sci. 104:1097-1106; Zhang, Z., et al., (2007), Blood 109:2190-2197; Kataoka, D., et al., (2015), Nature Genetics 47:1304-1315.)

Iberdomide (CC-220) is an orally available immunomodulatory compound under development for the treatment of SLE and relapsed/refractory multiple myeloma (Schafer, P. H., et al., (2018), Annals of the Rheumatic Diseases 77: 1516-1523.)

Currently, anti-CTLA4 antibodies are used in the clinic to target Tregs in tumors. However, targeting CTLA4 often causes systemic activation of T-effector cells, resulting in excessive toxicity and limiting therapeutic utility. Up to 3/4 of patients treated with a combination of anti-PD1 and anti-CTLA4 have reported grade 3 or higher adverse events. Thus, a strong need exists to provide compounds that target Tregs in tumors without causing systemic activation of T-effector cells.

An IKZF2-specific degrader has the potential to focus the enhanced immune response to areas within or near tumors providing a potentially more tolerable and less toxic therapeutic agent for the treatment of cancer.

### SUMMARY OF THE INVENTION

The compounds of the invention have use as therapeutic agents, particularly for cancers and related diseases. In one aspect, the compounds of the invention have IKZF2 degrader activity, preferably having such activity at or below the 50 µM level, and more preferably having such activity at or below the 10 µM level. In another aspect, the compounds of the invention have degrader activity for IKZF2 that is selective over one or more of IKZF1, IKZF3, IKZF4, and/or IKZF5. In another aspect, the compounds of the invention have degrader activity for both IKZF2 and IKZF4. The compounds of the invention have usefulness in treating cancer and other diseases for which such degrader activity would be beneficial for the patient. For example, while not intending to be bound by any theory, the inventors believe that reducing levels of IKZF2 in Tregs in a tumor may allow the patient immune system to more effectively attack the disease. In summary, the present invention provides novel IKZF2 degraders useful for the treatment of cancer and other diseases.

A first aspect of the present invention relates to compounds of Formula (I) 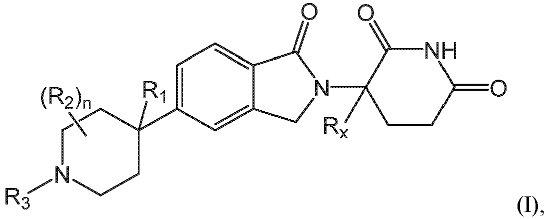 wherein:
R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, -C(O)NH₂, -C(O)OH or CN;
each R₂ is independently (C₁-C₆)alkyl, (C₁-C₆)haloalkyl, (C₁-C₆)hydroxyalkyl, CN, or halogen, or
R₁ and R₂ together with the carbon atoms to which they are attached form a (C₃-C₇)cycloalkyl or a 4- to 6- membered heterocycloalkyl ring comprising 1 to 3 heteroatoms selected from O, N, and S, or
two R₂ together with the carbon atoms to which they are attached form (C₃-C₇)cycloalkyl or a 4- to 6-membered heterocycloalkyl ring comprising 1 to 3 heteroatoms selected from O, N, and S;
R₃ is (C₁-C₆)alkyl, (C₆-C₁₀)aryl, 5- or 6-membered heteroaryl comprising 1 to 3 heteroatoms selected from O, N, and S, (C₃-C₈)cycloalkyl, or 4- to 7-membered heterocycloalkyl comprising 1 to 3 heteroatoms selected from O, N, and S, wherein the alkyl is optionally substituted with one or more R₄; and the aryl, heteroaryl, cycloalkyl, and heterocycloalkyl are optionally substituted with one or more R₅, or
R₂ and R₃, when on adjacent atoms, together with the atoms to which they are attached form a 5- or 6-membered heterocycloalkyl ring;
each R₄ is independently selected from -C(O)OR₆, -C(O)NR₆R_{6'}, -NR₆C(O)R_{6'}, halogen, -OH, -NH₂, CN, (C₆-C₁₀)aryl, 5- or 6-membered heteroaryl comprising 1 to 4 heteroatoms selected from O, N, and S, (C₃-C₈)cycloalkyl, and 4- to 7-membered heterocycloalkyl ring comprising 1 to 3 heteroatoms selected from O, N, and S, wherein the aryl, heteroaryl, cycloalkyl, and heterocycloalkyl groups are optionally substituted with one or more R₇;
each R₅ is independently selected from (C₁-C₆)alkyl, (C₂-C₆)alkenyl, (C₂-C₆)alkynyl, (C₁-C₆)alkoxy, (C₁-C₆)haloalkyl, (C₁-C₆)haloalkoxy, (C₁-C₆)hydroxyalkyl, halogen, -OH, -NH₂, CN, (C₃-C₇)cycloalkyl, 5- to 7-membered heterocycloalkyl comprising 1 to 3 heteroatoms selected from O, N, and S, (C₆-C₁₀)aryl, and 5- or 6-membered heteroaryl comprising 1 to 3 heteroatoms selected from O, N, and S, or
two R₅, when on adjacent atoms, together with the atoms to which they are attached form a (C₆-C₁₀)aryl ring or a 5- or 6-membered heteroaryl ring comprising 1 to 3 heteroatoms selected from O, N, and S, optionally substituted with one or more R₁₀, or
two R₅ together with the atoms to which they are attached form a (C₃-C₇)cycloalkyl ring or a 4- to 7-membered heterocycloalkyl ring comprising 1 to 3 heteroatoms selected from O, N, and S optionally substituted with one or more R₁₀;
R₆ and R_{6'} are each independently H, (C₁-C₆)alkyl, or (C₆-C₁₀)aryl;
each R₇ is independently selected from (C₁-C₆)alkyl, (C₂-C₆)alkenyl, (C₂-C₆)alkynyl, (C₁-C₆)alkoxy, (C₁-C₆)haloalkyl, (C₁-C₆)haloalkoxy, -C(O)R₈, -(CH₂)₀₋₃C(O)OR₈, -C(O)NR₈R₉, -NR₈C(O)R₉, - NR₈C(O)OR₉, -S(O)ₚNR₈R₉, -S(O)ₚR₁₂, (C₁-C₆)hydroxyalkyl, halogen, -OH, -O(CH₂)₁₋₃CN, -NH₂, CN, -O(CH₂)₀₋₃(C₆-C₁₀)aryl, adamantyl, -O(CH₂)₀₋₃-5- or 6-membered heteroaryl comprising 1 to 3 heteroatoms selected from O, N, and S, (C₆-C₁₀)aryl, monocyclic or bicyclic 5- to 10-membered heteroaryl comprising 1 to 3 heteroatoms selected from O, N, and S, (C₃-C₇)cycloalkyl, and 5- to 7-membered heterocycloalkyl comprising 1 to 3 heteroatoms selected from O, N, and S, wherein the alkyl is optionally substituted with one or more R₁₁, and the aryl, heteroaryl, and heterocycloalkyl are optionally substituted with one or more substituents each independently selected from halogen, (C₁-C₆)alkyl, (C₁-C₆)haloalkyl, and (C₁-C₆)alkoxy, or
two R₇ together with the carbon atom to which they are attached form a =(O), or
two R₇, when on adjacent atoms, together with the atoms to which they are attached form a (C₆-C₁₀)aryl ring or a 5- or 6-membered heteroaryl ring comprising 1 to 3 heteroatoms selected from O, N, and S, optionally substituted with one or more R₁₀, or
two R₇ together with the atoms to which they are attached form a (C₅-C₇) cycloalkyl ring or a 5- to 7-membered heterocycloalkyl ring comprising 1 to 3 heteroatoms selected from O, N, and S, optionally substituted with one or more R₁₀;
R₅ and R₉ are each independently H or (C₁-C₆)alkyl;
each R₁₀ is independently selected from (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -NH₂, and CN, or
two R₁₀ together with the carbon atom to which they are attached form a =(O);
each R₁₁ is independently selected from CN, (C₁-C₆)alkoxy, (C₆-C₁₀)aryl, and 5- to 7-membered heterocycloalkyl comprising 1 to 3 heteroatoms selected from O, N, and S, wherein the aryl and heterocycloalkyl are optionally substituted with one or more substituents each independently selected from (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -NH₂, and CN;
R₁₂ is (C₁-C₆)alkyl, (C₁-C₆)haloalkyl, (C₆-C₁₀)aryl, or 5- to 7-membered heterocycloalkyl comprising 1 to 3 heteroatoms selected from O, N, and S;
Rₓ is H or D; and
n is 0, 1, 2, or 3;
or pharmaceutically acceptable salts, hydrates, solvates, stereoisomers, and tautomers thereof.

In one aspect of the invention, the hydrogens in the compound of Formula (I) are present in their normal isotopic abundances. In a preferred aspect of the invention, the hydrogens are isotopically enriched in deuterium (D), and in a particularly preferred aspect of the invention the hydrogen at position Rₓ is enriched in D, as discussed in more detail concerning isotopes and isotopic enrichment below.

In another aspect, the present invention relates to a compound selected from: 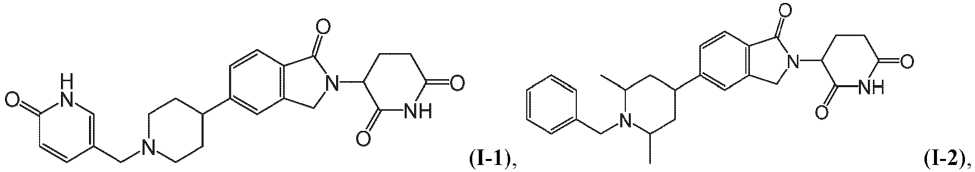 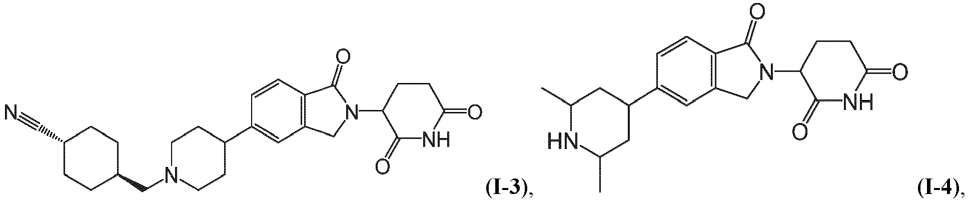 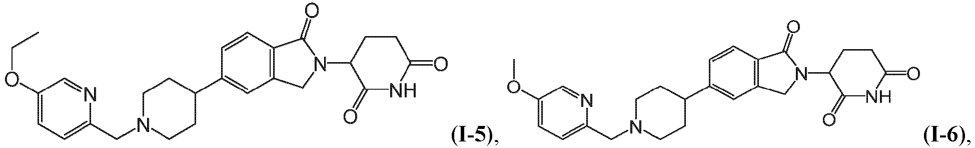 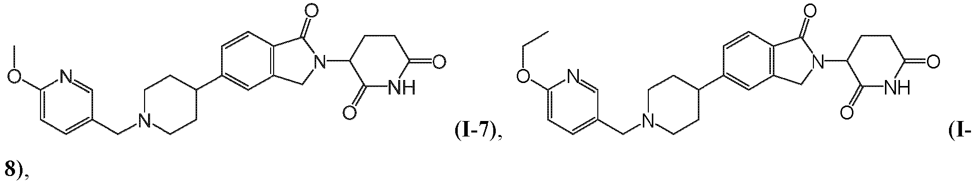 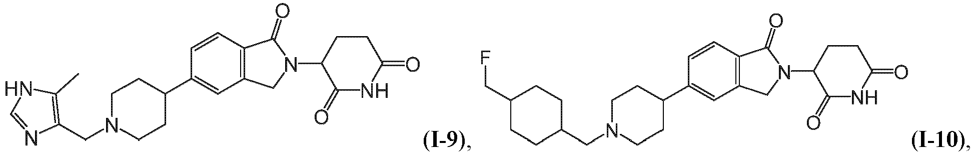 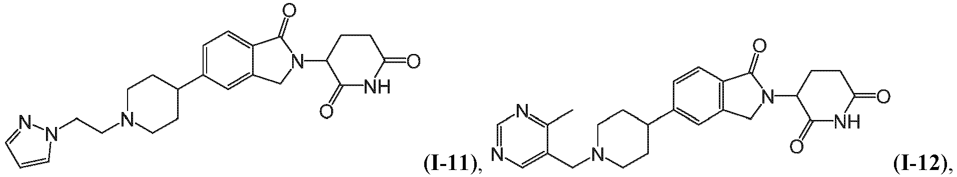 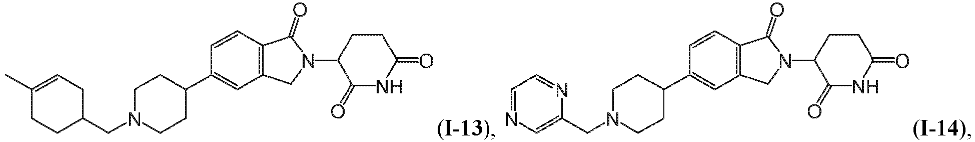 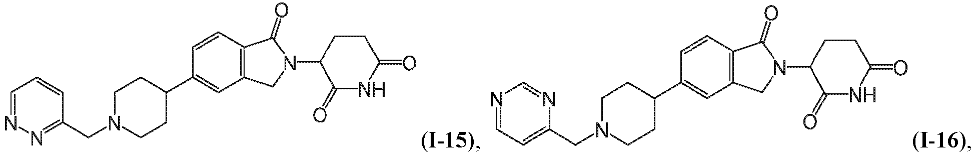 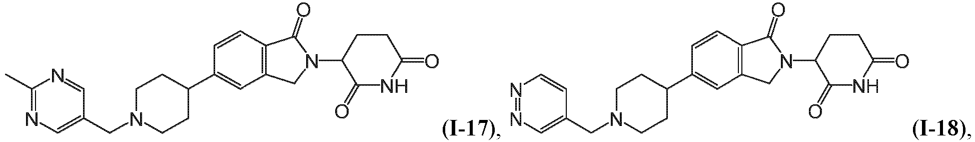 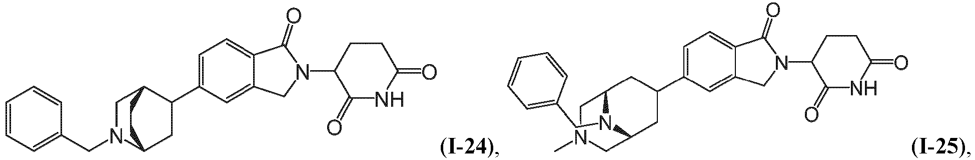 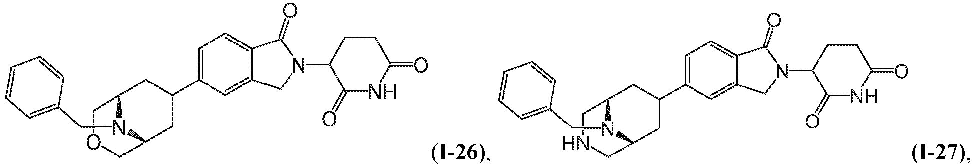 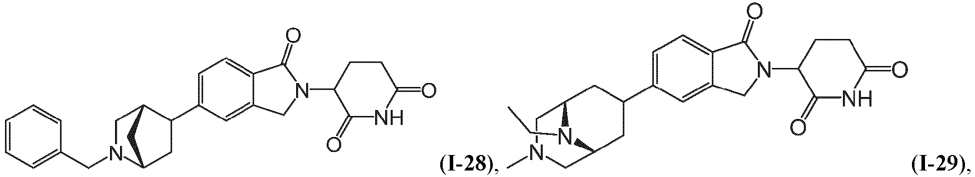 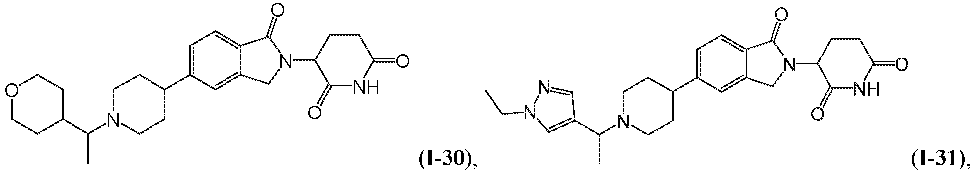 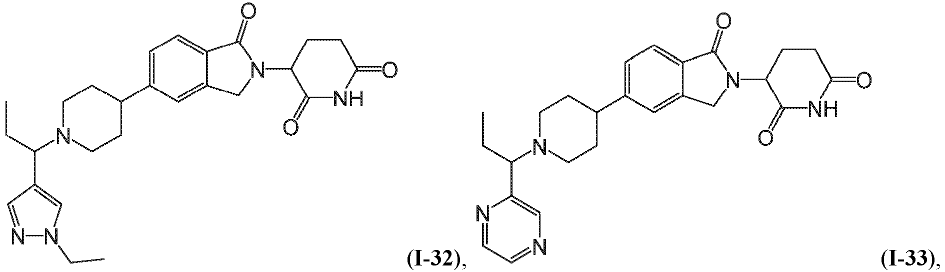 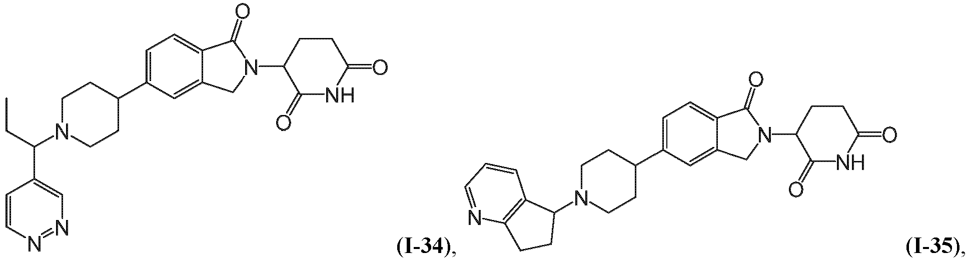 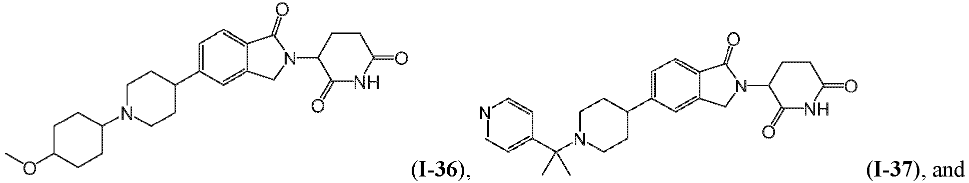 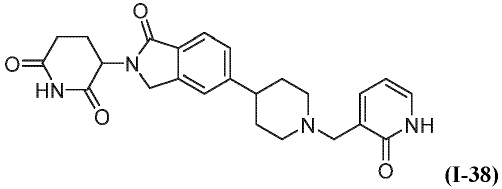 or pharmaceutically acceptable salts, hydrates, solvates, stereoisomers, and tautomers thereof.

Another aspect of the present invention relates to a pharmaceutical composition comprising a therapeutically effective amount of a compound of Formula (I), or a pharmaceutically acceptable salt, hydrate, solvate, stereoisomer, or tautomer thereof, and a pharmaceutically acceptable carrier or excipient. The pharmaceutical composition is useful in the treatment of IKZF2-dependent diseases or disorders. The pharmaceutical composition may further comprise at least one additional pharmaceutical agent.

In another aspect, the present invention relates to a pharmaceutical composition comprising a therapeutically effective amount of a compound of Formula (I), or a pharmaceutically acceptable salt, hydrate, solvate, stereoisomer, or tautomer thereof, and a pharmaceutically acceptable carrier or excipient for use in the treatment of an IKZF2-dependent disease or disorder by reducing IKZF2 protein levels wherein reduction of IKZF2 protein levels treats the IKZF2-dependent disease or disorder. The pharmaceutical composition is useful in the treatment of diseases or disorders affected by the reduction of IKZF2 protein levels. The pharmaceutical composition may further comprise at least one additional pharmaceutical agent.

Another aspect of the present invention relates to a pharmaceutical composition comprising a therapeutically effective amount of a compound selected from compound **(I-1), (I-2), (I-3), (I-4)**, **(I-5), (I-6), (I-7), (1-8), (I-9), (I-10), (I-11), (I-12), (I-13), (I-14), (I-15), (I-16), (I-17), (I-18), (I-24), (I-25), (I-26), (I-27), (I-28), (1-29), (I-30), (1-31), (I-32), (I-33), (I-34), (I-34), (I-36), (I-37),** and **(I-38),** or a pharmaceutically acceptable salt, hydrate, solvate, stereoisomer, or tautomer thereof, and a pharmaceutically acceptable carrier or excipient. The pharmaceutical composition is useful in the treatment of diseases or disorders affected by the reduction of IKZF2 protein levels. The pharmaceutical composition is useful in the treatment of IKZF2-dependent diseases or disorders. The pharmaceutical composition may further comprise at least one additional pharmaceutical agent.

The present invention provides degraders of IKZF2 that are therapeutic agents in the treatment of diseases such as cancer and metastasis, in the treatment of diseases affected by the modulation of IKZF2 protein levels, and in the treatment IKZF2-dependent diseases or disorders.

In one embodiment, the disease or disorder that can be treated by the compounds of the present invention is selected from non-small cell lung cancer (NSCLC), melanoma, triple-negative breast cancer (TNBC), nasopharyngeal cancer (NPC), microsatellite stable colorectal cancer (mssCRC), thymoma, carcinoid, gastrointestinal stromal tumor (GIST), prostate cancer, breast carcinoma, lymphomas, leukaemia, myeloma, bladder carcinoma, colon cancer, cutaneous melanoma, hepatocellular carcinoma, endometrial cancer, ovarian cancer, cervical cancer, lung cancer, renal cancer, glioblastoma multiform, glioma, thyroid cancer, parathyroid tumor, nasopharyngeal cancer, tongue cancer, pancreatic cancer, esophageal cancer, cholangiocarcinoma, gastric cancer, soft tissue sarcomas, rhabdomyosarcoma (RMS), synovial sarcoma, osteosarcoma, rhabdoid cancers, and Ewing's sarcoma. In another embodiment, the IKZF2-dependent disease or disorder is a cancer for which the immune response is deficient or an immunogenic cancer.

The present invention provides agents with novel mechanisms of action toward IKZF2 proteins in the treatment of various types of diseases including cancer and metastasis, in the treatment of diseases affected by the modulation of IKZF2 protein levels, and in the treatment IKZF2-dependent diseases or disorders. Ultimately the present invention provides the medical community with a novel pharmacological strategy for the treatment of diseases and disorders associated with IKZF2 proteins.

The present invention provides agents with novel mechanisms of action toward IKZF2 proteins in the treatment of various types of diseases including cancer and metastasis, in the treatment of diseases affected by the modulation of IKZF2 protein levels, and in the treatment IKZF2-dependent diseases or disorders. Ultimately, the present invention provides the medical community with a novel pharmacological strategy for the treatment of diseases and disorders associated with IKZF2 proteins.

### DETAILED DESCRIPTION OF THE INVENTION

The present invention relates to compounds and compositions that are capable of modulating IKZF2 protein levels. The invention features methods of treating, preventing, or ameliorating a disease or disorder in which IKZF2 plays a role by administering to a patient in need thereof a therapeutically effective amount of a compound of Formula (I), or a pharmaceutically acceptable salt, hydrate, solvate, stereoisomer, or tautomer thereof. The compounds of the present invention can be used in the treatment of a variety of IKZF2-dependent diseases and disorders by modulating IKZF2 protein levels. Modulation of IKZF2 protein levels through degradation provides a novel approach to the treatment, prevention, or amelioration of diseases including, but not limited to, cancer and metathesis, and other IKZF2-dependent diseases or disorders.

In one aspect, the compounds of the invention have use as therapeutic agents, particularly for cancers and related diseases. In one aspect, the compounds of the invention have IKZF2 degradation activity, preferably having such activity at or below the 50 µM level, and more preferably having such activity at or below the 10 µM level. In another aspect, the compounds of the invention have degrader activity for IKZF2 that is selective over one or more of IKZF1, IKZF3, IKZF4, and/or IKZF5. In another aspect, the compounds of the invention have degrader activity for both IKZF2 and IKZF4. The compounds of the invention have usefulness in treating cancer and other diseases for which such degradation activity would be beneficial for the patient. For example, while not intending to be bound by any theory, the inventors believe that reducing levels of IKZF2 in Tregs in a tumor may allow the patient immune system to more effectively attack the disease. In summary, the present invention provides novel IKZF2 degraders useful for the treatment of cancer and other diseases.

In a first aspect of the invention, the compounds of Formula (I) are described: 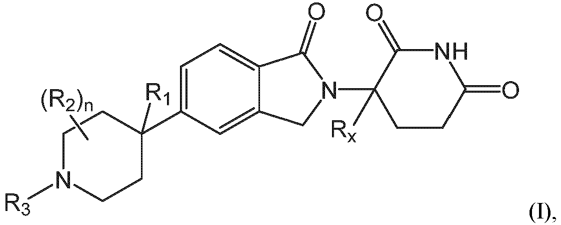 or pharmaceutically acceptable salts, hydrates, solvates, stereoisomers, and tautomers thereof, wherein R₁, R₂, R₃, Rₓ, and n are as described herein above.

The details of the invention are set forth in the accompanying description below. Although methods and materials similar or equivalent to those described herein can be used in the practice or testing of the present invention, illustrative methods and materials are now described. Other features, objects, and advantages of the invention will be apparent from the description and from the claims. In the specification and the appended claims, the singular forms also include the plural unless the context clearly dictates otherwise. Unless defined otherwise, all technical and scientific terms used herein have the same meaning as commonly understood by one of ordinary skill in the art to which this invention belongs.

### Definition of Terms and Conventions Used

Terms not specifically defined herein should be given the meanings that would be given to them by one of skill in the art in light of the invention and the context. As used in the specification and appended claims, however, unless specified to the contrary, the following terms have the meaning indicated and the following conventions are adhered to.

### A. Chemical Nomenclature, Terms, and Conventions

In the groups, radicals, or moieties defined below, the number of carbon atoms is often specified preceding the group, for example, (C₁-C₁₀)alkyl means an alkyl group or radical having 1 to 10 carbon atoms. In general, for groups comprising two or more subgroups, the last named group is the radical attachment point, for example, "alkylaryl" means a monovalent radical of the formula alkyl-aryl-, while "arylalkyl" means a monovalent radical of the formula aryl-alkyl-. Furthermore, the use of a term designating a monovalent radical where a divalent radical is appropriate shall be construed to designate the respective divalent radical and vice versa. Unless otherwise specified, conventional definitions of terms control and conventional stable atom valences are presumed and achieved in all formulas and groups. The articles "a" and "an" refer to one or more than one (e.g., to at least one) of the grammatical object of the article. By way of example, "an element" means one element or more than one element.

The term "and/or" means either "and" or "or" unless indicated otherwise.

The term "optionally substituted" means that a given chemical moiety (e.g., an alkyl group) can (but is not required to) be bonded other substituents (e.g., heteroatoms). For instance, an alkyl group that is optionally substituted can be a fully saturated alkyl chain (e.g., a pure hydrocarbon). Alternatively, the same optionally substituted alkyl group can have substituents different from hydrogen. For instance, it can, at any point along the chain be bounded to a halogen atom, a hydroxyl group, or any other substituent described herein. Thus, the term "optionally substituted" means that a given chemical moiety has the potential to contain other functional groups, but does not necessarily have any further functional groups. Suitable substituents used in the optional substitution of the described groups include, without limitation, halogen, oxo, -OH, -CN, -COOH, -CH₂CN, -O-(C₁-C₆)alkyl, (C₁-C₆)alkyl, (C₁-C₆)alkoxy, (C₁-C₆)haloalkyl, (C₁-C₆)haloalkoxy, -O-(C₂-C₆)alkenyl, -O-(C₂-C₆)alkynyl, (C₂-C₆)alkenyl, (C₂-C₆)alkynyl, -OH, - OP(O)(OH)₂, -OC(O)(C₁-C₆)alkyl, -C(O)(C₁-C₆)alkyl, -OC(O)O(C₁-C₆)alkyl, -NH₂, -NH((C₁-C₆)alkyl), - N((C₁-C₆)alkyl)₂, -NHC(O)(C₁-C₆)alkyl, -C(O)NH(C₁-C₆)alkyl, -S(O)₂(C₁-C₆)alkyl, -S(O)NH(C₁-C₆)alkyl, and S(O)N((C₁-C₆)alkyl)₂. The substituents can themselves be optionally substituted. "Optionally substituted" as used herein also refers to substituted or unsubstituted whose meaning is described below.

The term "substituted" means that the specified group or moiety bears one or more suitable substituents wherein the substituents may connect to the specified group or moiety at one or more positions. For example, an aryl substituted with a cycloalkyl may indicate that the cycloalkyl connects to one atom of the aryl with a bond or by fusing with the aryl and sharing two or more common atoms.

The term "unsubstituted" means that the specified group bears no substituents.

Unless otherwise specifically defined, "aryl" means a cyclic, aromatic hydrocarbon group having 1 to 3 aromatic rings, including monocyclic or bicyclic groups such as phenyl, biphenyl, or naphthyl. When containing two aromatic rings (bicyclic, etc.), the aromatic rings of the aryl group are optionally joined at a single point (e.g., biphenyl), or fused (e.g., naphthyl). The aryl group is optionally substituted by one or more substituents, e.g., 1 to 5 substituents, at any point of attachment. Exemplary substituents include, but are not limited to, -H, -halogen, -CN, -O-(C₁-C₆)alkyl, (C₁-C₆)alkyl, -O-(C₂-C₆)alkenyl, -O-(C₂-C₆)alkynyl, (C₂-C₆)alkenyl, (C₂-C₆)alkynyl, -OH, -OP(O)(OH)₂, -OC(O)(C₁-C₆)alkyl, -C(O)(C₁-C₆)alkyl, - OC(O)O(C₁-C₆) alkyl, NH₂, NH((C₁-C₆)alkyl), N((C₁-C₆)alkyl)₂, -S(O)₂-(C₁-C₆)alkyl, -S(O)NH(C₁-C₆)alkyl, and S(O)N((C₁-C₆)alkyl)₂. The substituents are themselves optionally substituted. Furthermore, when containing two fused rings, the aryl groups optionally have an unsaturated or partially saturated ring fused with a fully saturated ring. Exemplary ring systems of these aryl groups include, but are not limited to, phenyl, biphenyl, naphthyl, anthracenyl, phenalenyl, phenanthrenyl, indanyl, indenyl, tetrahydronaphthalenyl, tetrahydrobenzoannulenyl, and the like.

Unless otherwise specifically defined, "heteroaryl" means a monovalent monocyclic aromatic radical of 5 to 24 ring atoms or a polycyclic aromatic radical, containing one or more ring heteroatoms selected from N, O, or S, the remaining ring atoms being C. Heteroaryl as herein defined also means a bicyclic heteroaromatic group wherein the heteroatom is selected from N, O, or S. The aromatic radical is optionally substituted independently with one or more substituents described herein. Examples include, but are not limited to, furyl, thienyl, pyrrolyl, pyridyl, pyrazolyl, pyrimidinyl, imidazolyl, isoxazolyl, oxazolyl, oxadiazolyl, pyrazinyl, indolyl, thiophen-2-yl, quinolyl, benzopyranyl, isothiazolyl, thiazolyl, thiadiazole, indazole, benzimidazolyl, thieno[3,2-b]thiophene, triazolyl, triazinyl, imidazo[1,2-b]pyrazolyl, furo[2,3-c]pyridinyl, imidazo[1,2-a]pyridinyl, indazolyl, pyrrolo[2,3-c]pyridinyl, pyrrolo[3,2-c]pyridinyl, pyrazolo[3,4-c]pyridinyl, thieno[3,2-c]pyridinyl, thieno[2,3-c]pyridinyl, thieno[2,3-b]pyridinyl, benzothiazolyl, indolyl, indolinyl, indolinonyl, dihydrobenzothiophenyl, dihydrobenzofuranyl, benzofuran, chromanyl, thiochromanyl, tetrahydroquinolinyl, dihydrobenzothiazine, dihydrobenzoxanyl, quinolinyl, isoquinolinyl, 1,6-naphthyridinyl, benzo[de]isoquinolinyl, pyrido[4,3-b][1,6]naphthyridinyl, thieno[2,3-b]pyrazinyl, quinazolinyl, tetrazolo[1,5-a]pyridinyl, [1,2,4]triazolo[4,3-a]pyridinyl, isoindolyl, pyrrolo[2,3-b]pyridinyl, pyrrolo[3,4-b]pyridinyl, pyrrolo[3,2-b]pyridinyl, imidazo[5,4-b]pyridinyl, pyrrolo[1,2-a]pyrimidinyl, tetrahydropyrrolo[1,2-a]pyrimidinyl, 3,4-dihydro-2H-1Δ²-pyrrolo[2,1-b]pyrimidine, dibenzo[b,d]thiophene, pyridin-2-one, furo[3,2-c]pyridinyl, furo[2,3-c]pyridinyl, 1H-pyrido[3,4-b][1,4]thiazinyl, benzooxazolyl, benzoisoxazolyl, furo[2,3-b]pyridinyl, benzothiophenyl, 1,5-naphthyridinyl, furo[3,2-b]pyridine, [1,2,4]triazolo[1,5-a]pyridinyl, benzo[1,2,3]triazolyl, imidazo[1,2-a]pyrimidinyl, [1,2,4]triazolo[4,3-b]pyridazinyl, benzo[c][1,2,5]thiadiazolyl, benzo[c][1,2,5]oxadiazole, 1,3-dihydro-2H-benzo[d]imidazol-2-one, 3,4-dihydro-2H-pyrazolo[1,5-b][1,2]oxazinyl, 4,5,6,7-tetrahydropyrazolo[1,5-a]pyridinyl, thiazolo[5,4 d]thiazolyl, imidazo[2,1-b][1,3,4]thiadiazolyl, thieno[2,3-b]pyrrolyl, 3H-indolyl, and derivatives thereof. Furthermore, when containing two fused rings the aryl groups herein defined may have an unsaturated or partially saturated ring fused with a fully saturated ring. Exemplary ring systems of these heteroaryl groups include indolinyl, indolinonyl, dihydrobenzothiophenyl, dihydrobenzofuran, chromanyl, thiochromanyl, tetrahydroquinolinyl, dihydrobenzothiazine,3,4-dihydro-lH-isoquinolinyl, 2,3-dihydrobenzofuran, indolinyl, indolyl, and dihydrobenzoxanyl.

Halogen or "halo" mean fluorine, chlorine, bromine, or iodine.

"Alkyl" means a straight or branched chain saturated hydrocarbon containing 1-12 carbon atoms. Examples of a (C₁-C₆)alkyl group include, but are not limited to, methyl, ethyl, propyl, butyl, pentyl, hexyl, isopropyl, isobutyl, *sec*-butyl, *tert*-butyl*,* isopentyl, neopentyl, and isohexyl.

"Alkoxy" means a straight or branched chain saturated hydrocarbon containing 1-12 carbon atoms containing a terminal "O" in the chain, e.g., -O(alkyl). Examples of alkoxy groups include, without limitation, methoxy, ethoxy, propoxy, butoxy, t-butoxy, or pentoxy groups.

"Alkenyl" means a straight or branched chain unsaturated hydrocarbon containing 2-12 carbon atoms. The "alkenyl" group contains at least one double bond in the chain. The double bond of an alkenyl group can be unconjugated or conjugated to another unsaturated group. Examples of alkenyl groups include ethenyl, propenyl, *n*-butenyl, iso-butenyl, pentenyl, or hexenyl. An alkenyl group can be unsubstituted or substituted and may be straight or branched.

"Alkynyl" means a straight or branched chain unsaturated hydrocarbon containing 2-12 carbon atoms. The "alkynyl" group contains at least one triple bond in the chain. Examples of alkenyl groups include ethynyl, propargyl, *n*-butynyl, iso-butynyl, pentynyl, or hexynyl. An alkynyl group can be unsubstituted or substituted.

"Alkylene" or "alkylenyl" means a divalent alkyl radical. Any of the above mentioned monovalent alkyl groups may be an alkylene by abstraction of a second hydrogen atom from the alkyl. As herein defined, alkylene may also be a (C₁-C₆)alkylene. An alkylene may further be a (C₁-C₄)alkylene. Typical alkylene groups include, but are not limited to, -CH₂-, -CH(CH₃)-, -C(CH₃)₂-, -CH₂CH₂-, -CH₂CH(CH₃)-, - CH₂C(CH₃)₂-, -CH₂CH₂CH₂-, -CH₂CH₂CH₂CH-, and the like.

"Cycloalkyl" or "carbocyclyl" means a monocyclic or polycyclic saturated or partially unsaturated non-aromatic carbon ring containing 3-18 carbon atoms. Examples of cycloalkyl groups include, without limitations, cyclopropyl, cyclobutyl, cyclopentyl, cyclohexyl, cycloheptanyl, cyclooctanyl, norboranyl, norborenyl, bicyclo[2.2.2]octanyl, or bicyclo[2.2.2]octenyl and derivatives thereof. A (C₃-C₈)cycloalkyl is a cycloalkyl group containing between 3 and 8 carbon atoms. A cycloalkyl group can be fused (e.g., decalin) or bridged (e.g., norbornane).

"Heterocyclyl" or "heterocycloalkyl" means a saturated or partially saturated monocyclic or polycyclic ring containing carbon and at least one heteroatom selected from oxygen, nitrogen, or sulfur (O, N, or S) and wherein there is not delocalized n electrons (aromaticity) shared among the ring carbon or heteroatoms. The heterocycloalkyl ring structure may be substituted by one or more substituents. The substituents can themselves be optionally substituted. Examples of heterocyclyl rings include, but are not limited to, oxetanyl, azetadinyl, tetrahydrofuranyl, tetrahydropyranyl, pyrrolidinyl, oxazolinyl, oxazolidinyl, thiazolinyl, thiazolidinyl, pyranyl, thiopyranyl, tetrahydropyranyl, dioxalinyl, piperidinyl, morpholinyl, thiomorpholinyl, thiomorpholinyl S-oxide, thiomorpholinyl S-dioxide, piperazinyl, azepinyl, oxepinyl, diazepinyl, tropanyl, oxazolidinonyl, 1,4-dioxanyl, dihydrofuranyl, 1,3-dioxolanyl, imidazolidinyl, imidazolinyl, dithiolanyl, and homotropanyl.

"Hydroxyalkyl" means an alkyl group substituted with one or more -OH groups. Examples of hydroxyalkyl groups include HO-CH₂-, HO-CH₂CH₂-, and CH₂-CH(OH)-.

"Haloalkyl" means an alkyl group substituted with one or more halogens. Examples of haloalkyl groups include, but are not limited to, trifluoromethyl, difluoromethyl, pentafluoroethyl, trichloromethyl, etc.

"Haloalkoxy" means an alkoxy group substituted with one or more halogens. Examples of haloalkyl groups include, but are not limited to, trifluoromethoxy, difluoromethoxy, pentafluoroethoxy, trichloromethoxy, etc.

"Cyano" means a substituent having a carbon atom joined to a nitrogen atom by a triple bond, e.g., C≡N.

"Amino" means a substituent containing at least one nitrogen atom (e.g., NH₂).

"Pomalidomide" or 4-amino-2-(2,6-dioxopiperidin-3-yl)isoindoline-1,3-dione has the following structure:

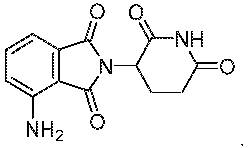

### B. Salt, Derivative, and Solvate Terms and Conventions

"Salt" means an ionic form of the parent compound or the product of the reaction between the parent compound with a suitable acid or base to make the acid salt or base salt of the parent compound. Salts of the compounds of the present invention can be synthesized from the parent compounds which contain a basic or acidic moiety by conventional chemical methods. Generally, the salts are prepared by reacting the free base or acid parent compound with stoichiometric amounts or with an excess of the desired salt-forming inorganic or organic acid or base in a suitable solvent or various combinations of solvents.

"Pharmaceutically acceptable salt" means a salt of a compound of the invention which is, within the scope of sound medical judgment, suitable for use in contact with the tissues of humans and lower animals without undue toxicity, irritation, allergic response, and the like, commensurate with a reasonable benefit/risk ratio, generally water or oil-soluble or dispersible, and effective for their intended use. The term includes pharmaceutically-acceptable acid addition salts and pharmaceutically-acceptable base addition salts. As the compounds of the present invention are useful in both free base and salt form, in practice, the use of the salt form amounts to use of the base form. Lists of suitable salts are found in, e.g., S.M. Birge et al., J. Pharm. Sci., 1977, 66, pp. 1-19.

"Pharmaceutically-acceptable acid addition salt" means those salts which retain the biological effectiveness and properties of the free bases and which are not biologically or otherwise undesirable, formed with inorganic acids such as hydrochloric acid, hydrobromic acid, hydroiodic acid, sulfuric acid, sulfamic acid, nitric acid, phosphoric acid, and the like, and organic acids such as acetic acid, trichloroacetic acid, trifluoroacetic acid, adipic acid, alginic acid, ascorbic acid, aspartic acid, benzenesulfonic acid, benzoic acid, 2-acetoxybenzoic acid, butyric acid, camphoric acid, camphorsulfonic acid, cinnamic acid, citric acid, digluconic acid, ethanesulfonic acid, glutamic acid, glycolic acid, glycerophosphoric acid, hemisulfic acid, heptanoic acid, hexanoic acid, formic acid, fumaric acid, 2-hydroxyethanesulfonic acid (isethionic acid), lactic acid, maleic acid, hydroxymaleic acid, malic acid, malonic acid, mandelic acid, mesitylenesulfonic acid, methanesulfonic acid, naphthalenesulfonic acid, nicotinic acid, 2-naphthalenesulfonic acid, oxalic acid, pamoic acid, pectinic acid, phenylacetic acid, 3-phenylpropionic acid, picric acid, pivalic acid, propionic acid, pyruvic acid, pyruvic acid, salicylic acid, stearic acid, succinic acid, sulfanilic acid, tartaric acid, p-toluenesulfonic acid, undecanoic acid, and the like.

"Pharmaceutically-acceptable base addition salt" means those salts which retain the biological effectiveness and properties of the free acids and which are not biologically or otherwise undesirable, formed with inorganic bases such as ammonia or hydroxide, carbonate, or bicarbonate of ammonium or a metal cation such as sodium, potassium, lithium, calcium, magnesium, iron, zinc, copper, manganese, aluminum, and the like. Particularly preferred are the ammonium, potassium, sodium, calcium, and magnesium salts. Salts derived from pharmaceutically-acceptable organic nontoxic bases include salts of primary, secondary, and tertiary amines, quaternary amine compounds, substituted amines including naturally occurring substituted amines, cyclic amines and basic ion-exchange resins, such as methylamine, dimethylamine, trimethylamine, ethylamine, diethylamine, triethylamine, isopropylamine, tripropylamine, tributylamine, ethanolamine, diethanolamine, 2-dimethylaminoethanol, 2-diethylaminoethanol, dicyclohexylamine, lysine, arginine, histidine, caffeine, hydrabamine, choline, betaine, ethylenediamine, glucosamine, methylglucamine, theobromine, purines, piperazine, piperidine, N-ethylpiperidine, tetramethylammonium compounds, tetraethylammonium compounds, pyridine, N,N-dimethylaniline, N-methylpiperidine, N-methylmorpholine, dicyclohexylamine, dibenzylamine, N,N-dibenzylphenethylamine, 1-ephenamine, N,N'-dibenzylethylenediamine, polyamine resins, and the like. Particularly preferred organic nontoxic bases are isopropylamine, diethylamine, ethanolamine, trimethylamine, dicyclohexylamine, choline, and caffeine.

"Solvate" means a complex of variable stoichiometry formed by a solute, for example, a compound of Formula (I)) and solvent, for example, water, ethanol, or acetic acid. This physical association may involve varying degrees of ionic and covalent bonding, including hydrogen bonding. In certain instances, the solvate will be capable of isolation, for example, when one or more solvent molecules are incorporated in the crystal lattice of the crystalline solid. In general, such solvents selected for the purpose of the invention do not interfere with the biological activity of the solute. Solvates encompasses both solution-phase and isolatable solvates. Representative solvates include hydrates, ethanolates, methanolates, and the like.

"Hydrate" means a solvate wherein the solvent molecule(s) is/are water.

The compounds of the present invention as discussed below include the free base or acid thereof, their salts, and solvates, and may include oxidized sulfur atoms or quaternized nitrogen atoms in their structure, although not explicitly stated or shown, particularly the pharmaceutically acceptable forms thereof. Such forms, particularly the pharmaceutically acceptable forms, are intended to be embraced by the appended claims.

### C. Isomer Terms and Conventions

"Isomers" means compounds having the same number and kind of atoms, and hence the same molecular weight, but differing with respect to the arrangement or configuration of the atoms in space. The term includes stereoisomers and geometric isomers.

"Stereoisomer" or "optical isomer" mean a stable isomer that has at least one chiral atom or restricted rotation giving rise to perpendicular dissymmetric planes (e.g., certain biphenyls, allenes, and spiro compounds) and can rotate plane-polarized light. Because asymmetric centers and other chemical structure exist in the compounds of the invention, which may give rise to stereoisomerism, the invention contemplates stereoisomers and mixtures thereof. The compounds of the invention and their salts include asymmetric carbon atoms and may therefore exist as single stereoisomers, racemates, and as mixtures of enantiomers and diastereomers. Typically, such compounds will be prepared as a racemic mixture. If desired, however, such compounds can be prepared or isolated as pure stereoisomers, *i.e.,* as individual enantiomers or diastereomers, or as stereoisomer-enriched mixtures. As discussed in more detail below, individual stereoisomers of compounds are prepared by synthesis from optically active starting materials containing the desired chiral centers or by preparation of mixtures of enantiomeric products followed by separation or resolution, such as conversion to a mixture of diastereomers followed by separation or recrystallization, chromatographic techniques, use of chiral resolving agents, or direct separation of the enantiomers on chiral chromatographic columns. Starting compounds of particular stereochemistry are either commercially available or are made by the methods described below and resolved by techniques well-known in the art.

"Enantiomers" means a pair of stereoisomers that are non-superimposable mirror images of each other.

"Diastereoisomers" or "diastereomers" mean optical isomers which are not mirror images of each other.

"Racemic mixture" or "racemate" mean a mixture containing equal parts of individual enantiomers.

"Non-racemic mixture" means a mixture containing unequal parts of individual enantiomers.

"Geometrical isomer" means a stable isomer, which results from restricted freedom of rotation about double bonds (e.g., cis-2-butene and trans-2-butene) or in a cyclic structure (e.g., cis-1,3-dichlorocyclobutane and trans-1,3-dichlorocyclobutane). Because carbon-carbon double (olefinic) bonds, C=N double bonds, cyclic structures, and the like may be present in the compounds of the invention, the invention contemplates each of the various stable geometric isomers and mixtures thereof resulting from the arrangement of substituents around these double bonds and in these cyclic structures. The substituents and the isomers are designated using the cis/trans convention or using the E or Z system, wherein the term "E" means higher order substituents on opposite sides of the double bond, and the term "Z" means higher order substituents on the same side of the double bond. A thorough discussion of E and Z isomerism is provided in J. March, Advanced Organic Chemistry: Reactions, Mechanisms, and Structure, 4th ed., John Wiley & Sons, 1992. Several of the following examples represent single E isomers, single Z isomers, and mixtures of E/Z isomers. Determination of the E and Z isomers can be done by analytical methods such as x-ray crystallography, ¹H NMR, and ¹³C NMR.

Some of the compounds of the invention can exist in more than one tautomeric form. As mentioned above, the compounds of the invention include all such tautomers.

It is well-known in the art that the biological and pharmacological activity of a compound is sensitive to the stereochemistry of the compound. Thus, for example, enantiomers often exhibit strikingly different biological activity including differences in pharmacokinetic properties, including metabolism, protein binding, and the like, and pharmacological properties, including the type of activity displayed, the degree of activity, toxicity, and the like. Thus, one skilled in the art will appreciate that one enantiomer may be more active or may exhibit beneficial effects when enriched relative to the other enantiomer or when separated from the other enantiomer. Additionally, one skilled in the art would know how to separate, enrich, or selectively prepare the enantiomers of the compounds of the invention from this invention and the knowledge of the prior art.

Thus, although the racemic form of drug may be used, it is often less effective than administering an equal amount of enantiomerically pure drug; indeed, in some cases, one enantiomer may be pharmacologically inactive and would merely serve as a simple diluent. For example, although ibuprofen had been previously administered as a racemate, it has been shown that only the S-isomer of ibuprofen is effective as an anti-inflammatory agent (in the case of ibuprofen, however, although the R-isomer is inactive, it is converted in vivo to the S-isomer, thus, the rapidity of action of the racemic form of the drug is less than that of the pure S-isomer). Furthermore, the pharmacological activities of enantiomers may have distinct biological activity. For example, S-penicillamine is a therapeutic agent for chronic arthritis, while R-penicillamine is toxic. Indeed, some purified enantiomers have advantages over the racemates, as it has been reported that purified individual isomers have faster transdermal penetration rates compared to the racemic mixture. See U.S. Pat. Nos. 5,114,946 and 4,818,541.

Thus, if one enantiomer is pharmacologically more active, less toxic, or has a preferred disposition in the body than the other enantiomer, it would be therapeutically more beneficial to administer that enantiomer preferentially. In this way, the patient undergoing treatment would be exposed to a lower total dose of the drug and to a lower dose of an enantiomer that is possibly toxic or an inhibitor of the other enantiomer.

Preparation of pure enantiomers or mixtures of desired enantiomeric excess (ee) or enantiomeric purity are accomplished by one or more of the many methods of (a) separation or resolution of enantiomers, or (b) enantioselective synthesis known to those of skill in the art, or a combination thereof. These resolution methods generally rely on chiral recognition and include, for example, chromatography using chiral stationary phases, enantioselective host-guest complexation, resolution or synthesis using chiral auxiliaries, enantioselective synthesis, enzymatic and nonenzymatic kinetic resolution, or spontaneous enantioselective crystallization. Such methods are disclosed generally in Chiral Separation Techniques: A Practical Approach (2nd Ed.), G. Subramanian (ed.), Wiley-VCH, 2000; T.E. Beesley and R.P.W. Scott, Chiral Chromatography, John Wiley & Sons, 1999; and Satinder Ahuja, Chiral Separations by Chromatography, Am. Chem. Soc., 2000. Furthermore, there are equally well-known methods for the quantitation of enantiomeric excess or purity, for example, GC, HPLC, CE, or NMR, and assignment of absolute configuration and conformation, for example, CD ORD, X-ray crystallography, or NMR.

In general, all tautomeric forms and isomeric forms and mixtures, whether individual geometric isomers or stereoisomers or racemic or non-racemic mixtures, of a chemical structure or compound is intended, unless the specific stereochemistry or isomeric form is specifically indicated in the compound name or structure.

### D. Pharmaceutical Administration and Treatment Terms and Conventions

A "patient" or "subject" is a mammal, e.g., a human, mouse, rat, guinea pig, dog, cat, horse, cow, pig, or nonhuman primate, such as a monkey, chimpanzee, baboon or, rhesus. In certain embodiments, the subject is a primate. In yet other embodiments, the subject is a human.

An "effective amount" or "therapeutically effective amount" when used in connection with a compound means an amount of a compound of the present invention that (i) treats or prevents the particular disease, condition, or disorder, (ii) attenuates, ameliorates, or eliminates one or more symptoms of the particular disease, condition, or disorder, or (iii) prevents or delays the onset of one or more symptoms of the particular disease, condition, or disorder described herein.

The terms "pharmaceutically effective amount" or "therapeutically effective amount" means an amount of a compound according to the invention which, when administered to a patient in need thereof, is sufficient to effect treatment for disease-states, conditions, or disorders for which the compounds have utility. Such an amount would be sufficient to elicit the biological or medical response of a tissue, system, or patient that is sought by a researcher or clinician. The amount of a compound of according to the invention which constitutes a therapeutically effective amount will vary depending on such factors as the compound and its biological activity, the composition used for administration, the time of administration, the route of administration, the rate of excretion of the compound, the duration of treatment, the type of disease-state or disorder being treated and its severity, drugs used in combination with or coincidentally with the compounds of the invention, and the age, body weight, general health, sex, and diet of the patient. Such a therapeutically effective amount can be determined routinely by one of ordinary skill in the art having regard to their own knowledge, the prior art, and this invention.

As used herein, the term "pharmaceutical composition" refers to a compound of the invention, or a pharmaceutically acceptable salt, hydrate, solvate, stereoisomer, or tautomer thereof, together with at least one pharmaceutically acceptable carrier, in a form suitable for oral or parenteral administration.

"Carrier" encompasses carriers, excipients, and diluents and means a material, composition or vehicle, such as a liquid or solid filler, diluent, excipient, solvent or encapsulating material, involved in carrying or transporting a pharmaceutical agent from one organ, or portion of the body, to another organ, or portion of the body of a subject.

A subject is "in need of" a treatment if such subject would benefit biologically, medically, or in quality of life from such treatment (preferably, a human).

As used herein, the term "inhibit", "inhibition", or "inhibiting" refers to the reduction or suppression of a given condition, symptom, or disorder, or disease, or a significant decrease in the baseline activity of a biological activity or process.

As used herein, the term "treat", "treating", or "treatment" of any disease or disorder refers to alleviating or ameliorating the disease or disorder (i.e., slowing or arresting the development of the disease or at least one of the clinical symptoms thereof); or alleviating or ameliorating at least one physical parameter or biomarker associated with the disease or disorder, including those which may not be discernible to the patient.

As used herein, the term "prevent", "preventing", or "prevention" of any disease or disorder refers to the prophylactic treatment of the disease or disorder; or delaying the onset or progression of the disease or disorder.

"Pharmaceutically acceptable" means that the substance or composition must be compatible chemically and/or toxicologically, with the other ingredients comprising a formulation, and/or the mammal being treated therewith.

"Disorder" means, and is used interchangeably with, the terms disease, condition, or illness, unless otherwise indicated.

"Administer", "administering", or "administration" means to either directly administering a disclosed compound or pharmaceutically acceptable salt of the disclosed compound or a composition to a subject, which can form an equivalent amount of active compound within the subject's body.

"Compounds of the present invention", "compounds of the invention", and equivalent expressions (unless specifically identified otherwise) refer to compounds of Formulae (I), (Ia), and (Ib), and compounds **(I-1), (I-2), (I-3), (I-4), (I-5), (I-6), (I-7), (I-8), (I-9), (I-10), (I-11), (I-12), (I-13), (I-14), (I-15), (I-16), (I-17), (I-18), (I-24), (I-25), (I-26), (I-27), (I-28), (I-29), (I-30), (I-31), (I-32), (I-33), (I-34), (I-34), (I-36), (I-37),** and **(I-38),** as herein described including the tautomers, salts particularly the pharmaceutically acceptable salts, and the solvates and hydrates thereof, where the context so permits thereof, as well as all stereoisomers (including diastereoisomers and enantiomers), rotamers, tautomers, and isotopically labelled compounds (including deuterium substitutions), as well as inherently formed moieties (e.g., polymorphs, solvates and/or hydrates). For purposes of this invention, solvates and hydrates are generally considered compositions. In general and preferably, the compounds of the invention and the formulas designating the compounds of the invention are understood to only include the stable compounds thereof and exclude unstable compounds, even if an unstable compound might be considered to be literally embraced by the compound formula. Similarly, reference to intermediates, whether or not they themselves are claimed, is meant to embrace their salts and solvates, where the context so permits. For the sake of clarity, particular instances when the context so permits are sometimes indicated in the text, but these instances are purely illustrative and it is not intended to exclude other instances when the context so permits.

"Stable compound" or "stable structure" means a compound that is sufficiently robust to survive isolation to a useful degree of purity from a reaction mixture, and formulation into an efficacious therapeutic or diagnostic agent. For example, a compound, which would have a "dangling valency" or is a carbanion is not a compound contemplated by the invention.

In a specific embodiment, the term "about" or "approximately" means within 20%, preferably within 10%, and more preferably within 5% of a given value or range.

The yield of each of the reactions described herein is expressed as a percentage of the theoretical yield. "Cancer" means any cancer caused by the proliferation of malignant neoplastic cells, such as tumors, neoplasms, carcinomas, sarcomas, leukemias, lymphomas, and the like. For example, cancers include, but are not limited to, mesothelioma, leukemias, and lymphomas such as cutaneous T-cell lymphomas (CTCL), noncutaneous peripheral T-cell lymphomas, lymphomas associated with human T-cell lymphotrophic virus (HTLV) such as adult T-cell leukemia/lymphoma (ATLL), B-cell lymphoma, acute nonlymphocytic leukemias, chronic lymphocytic leukemia, chronic myelogenous leukemia, acute myelogenous leukemia, lymphomas, and multiple myeloma, non-Hodgkin lymphoma, acute lymphatic leukemia (ALL), chronic lymphatic leukemia (CLL), Hodgkin's lymphoma, Burkitt lymphoma, adult T-cell leukemia lymphoma, acute-myeloid leukemia (AML), chronic myeloid leukemia (CML), or hepatocellular carcinoma. Further examples include myelodisplastic syndrome, childhood solid tumors such as brain tumors, neuroblastoma, retinoblastoma, Wilms' tumor, bone tumors, and soft-tissue sarcomas, common solid tumors of adults such as head and neck cancers (e.g., oral, laryngeal, and nasopharyngeal), esophageal cancer, genitourinary cancers (e.g., prostate, bladder, renal, uterine, ovarian, testicular), lung cancer (e.g., small-cell and non-small cell), breast cancer, pancreatic cancer, melanoma, and other skin cancers, stomach cancer, brain tumors, tumors related to Gorlin's syndrome (e.g., medulloblastoma, meningioma, etc.), and liver cancer. Additional exemplary forms of cancer which may be treated by the subject compounds include, but are not limited to, cancer of skeletal or smooth muscle, stomach cancer, cancer of the small intestine, rectum carcinoma, cancer of the salivary gland, endometrial cancer, adrenal cancer, anal cancer, rectal cancer, parathyroid cancer, and pituitary cancer.

Additional cancers that the compounds described herein may be useful in preventing, treating, and studying are, for example, colon carcinoma, familiary adenomatous polyposis carcinoma, and hereditary non-polyposis colorectal cancer, or melanoma. Further, cancers include, but are not limited to, labial carcinoma, larynx carcinoma, hypopharynx carcinoma, tongue carcinoma, salivary gland carcinoma, gastric carcinoma, adenocarcinoma, thyroid cancer (medullary and papillary thyroid carcinoma), renal carcinoma, kidney parenchyma carcinoma, cervix carcinoma, uterine corpus carcinoma, endometrium carcinoma, chorion carcinoma, testis carcinoma, urinary carcinoma, melanoma, brain tumors such as glioblastoma, astrocytoma, meningioma, medulloblastoma and peripheral neuroectodermal tumors, gall bladder carcinoma, bronchial carcinoma, multiple myeloma, basalioma, teratoma, retinoblastoma, choroidea melanoma, seminoma, rhabdomyosarcoma, craniopharyngeoma, osteosarcoma, chondrosarcoma, myosarcoma, liposarcoma, fibrosarcoma, Ewing's sarcoma, and plasmocytoma.

"Simultaneously" or "simultaneous" when referring to a method of treating or a therapeutic use means with a combination of a compound of Formula (I) or Embodiment 16, 17, or 35, or a pharmaceutically acceptable salt, hydrate, solvate, stereoisomer, or tautomer thereof, and one or more second agent(s) means administration of the compound and the one or more second agent(s) by the same route and at the same time.

"Separately" or "separate" when referring to a method of treating or a therapeutic use means with a combination of a compound of Formula (I) or Embodiment 16, 17, or 35, or a pharmaceutically acceptable salt, hydrate, solvate, stereoisomer, or tautomer thereof, and one or more second agent(s) means administration of the compound and the one or more second agent(s) by different routes and at approximately the same time.

By therapeutic administration "over a period of time" means, when referring to a method of treating or a therapeutic use with a combination of a compound of Formula (I) or Embodiment 16, 17, or 35, or a pharmaceutically acceptable salt, hydrate, solvate, stereoisomer, or tautomer thereof, and one or more second agent(s), administration of the compound and the one or more second agent(s) by the same or different routes and at different times. In some embodiments, the administration of the compound or the one or more second agent(s) occurs before the administration of the other begins. In this way, it is possible to administer a one of the active ingredients (i.e., a compound of Formula (I) or Embodiment 16, 17, or 35, or a pharmaceutically acceptable salt, hydrate, solvate, stereoisomer, or tautomer thereof, or one or more second agent(s)) for several months before administering the other active ingredient or ingredients. In this case, no simultaneous administration occurs. Another therapeutic administration over a period of time consists of the administration over time of the two or more active ingredients of the combination using different frequencies of administration for each of the active ingredients, whereby at certain time points in time simultaneous administration of all of the active ingredients takes place whereas at other time points in time only a part of the active ingredients of the combination may be administered (e.g., for example. a compound of Formula (I) or a compound of formula **(I-1), (I-2), (I-3), (I-4), (I-5), (I-6), (I-7), (I-8), (I-9), (I-10), (I-11), (I-12), (I-13), (I-14), (I-15), (I-16), (I-17), (I-18), (I-24), (I-25), (I-26), (I-27), (I-28), (I-29), (I-30), (I-31), (I-32), (I-33), (I-34), (I-34), (I-36), (I-37),** or **(I-38),** or a pharmaceutically acceptable salt, hydrate, solvate, stereoisomer, or tautomer thereof, and the one or more second agents the therapeutic administration over a period of time could be such that a compound of Formula (I) or **(I-1), (I-2), (I-3), (I-4), (I-5), (I-6), (I-7), (I-8), (I-9), (I-10), (I-11), (I-12), (I-13), (I-14), (I-15), (I-16), (I-17), (I-18), (I-24), (1-25), (I-26), (1-27), (1-28), (I-29), (1-30), (1-31), (I-32), (I-33), (I-34), (I-34), (I-36), (I-37),** or **(I-38),** , or a pharmaceutically acceptable salt, hydrate, solvate, stereoisomer, or tautomer thereof, is administered once a day and the one or more second agent(s) is administered once every four weeks.)

"IKZF2-dependent disease or disorder" means any disease or disorder which is directly or indirectly affected by the modulation of IKZF2 protein levels.

"IKZF4-dependent disease or disorder" means any disease or disorder which is directly or indirectly affected by the modulation of IKZF4 protein levels.

### D. Specific Embodiments and Methods for Testing Compounds of Formula (I)

The present invention relates to compounds or pharmaceutically acceptable salts, hydrates, solvates, stereoisomers, or tautomers thereof, capable of modulating IKZF2 protein levels, which are useful for the treatment of diseases and disorders associated with modulation of IKZF2 protein levels. The invention further relates to compounds, or pharmaceutically acceptable salts, hydrates, solvates, stereoisomers, or tautomers thereof, which are useful for reducing or decreasing IKZF2 protein levels.

In one embodiment, the compounds of Formula (I) have the structure of Formula (Ia): 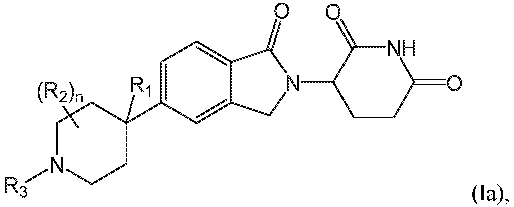 or pharmaceutically acceptable salts, hydrates, solvates, stereoisomers, and tautomers thereof.

In another embodiment, the compounds of Formula (I) have the structure of Formula (Ib): 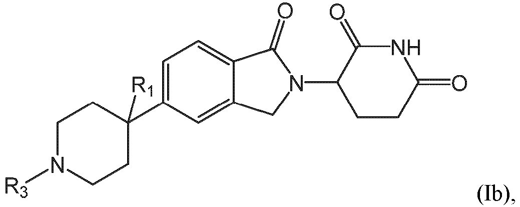 or pharmaceutically acceptable salts, hydrates, solvates, stereoisomers, and tautomers thereof.

In some embodiments of the formulae above (i.e., Formula (I), Formula (Ia), and/or Formula (Ib)),
R₃ is (C₁-C₆)alkyl, (C₆-C₁₀)aryl, 5- or 6-membered heteroaryl comprising 1 to 3 heteroatoms selected from O, N, and S, (C₃-C₈)cycloalkyl, or 4- to 7-membered heterocycloalkyl comprising 1 to 3 heteroatoms selected from O, N, and S, wherein the alkyl is optionally substituted with one to four R₄; and the aryl, heteroaryl, cycloalkyl, and heterocycloalkyl are optionally substituted with one to four R₅, or
each R₄ is independently selected from -C(O)OR₆, -C(O)NR₆R_{6'}, -NR₆C(O)R_{6'}, halogen, -OH, - NH₂, CN, (C₆-C₁₀)aryl, 5- or 6-membered heteroaryl comprising 1 to 4 heteroatoms selected from O, N, and S, (C₃-C₈)cycloalkyl, and 4- to 7-membered heterocycloalkyl ring comprising 1 to 3 heteroatoms selected from O, N, and S, wherein the aryl, heteroaryl, cycloalkyl, and heterocycloalkyl groups are optionally substituted with one to four R₇;
each R₅ is independently selected from (C₁-C₆)alkyl, (C₂-C₆)alkenyl, (C₂-C₆)alkynyl, (C₁-C₆)alkoxy, (C₁-C₆)haloalkyl, (C₁-C₆)haloalkoxy, (C₁-C₆)hydroxyalkyl, halogen, -OH, -NH₂, CN, (C₃-C₇)cycloalkyl, 5- to 7-membered heterocycloalkyl comprising 1 to 3 heteroatoms selected from O, N, and S, (C₆-C₁₀)aryl, and 5- or 6-membered heteroaryl comprising 1 to 3 heteroatoms selected from O, N, and S, or
two R₅, when on adjacent atoms, together with the atoms to which they are attached form a (C₆-C₁₀)aryl ring or a 5- or 6-membered heteroaryl ring comprising 1 to 3 heteroatoms selected from O, N, and S, optionally substituted with one to four R₁₀, or
two R₅ together with the atoms to which they are attached form a (C₃-C₇)cycloalkyl ring or a 4- to 7-membered heterocycloalkyl ring comprising 1 to 3 heteroatoms selected from O, N, and S optionally substituted with one to four R₁₀;
each R₇ is independently selected from (C₁-C₆)alkyl, (C₂-C₆)alkenyl, (C₂-C₆)alkynyl, (C₁-C₆)alkoxy, (C₁-C₆)haloalkyl, (C₁-C₆)haloalkoxy, -C(O)R₈, -(CH₂)₀₋₃C(O)OR₈, -C(O)NR₈R₉, -NR₈C(O)R₉, -NR₈C(O)OR₉, -S(O)ₚNR₈R₉, -S(O)ₚR₁₂, (C₁-C₆)hydroxyalkyl, halogen, -OH, -O(CH₂)₁₋₃CN, -NH₂, CN, - O(CH₂)₀₋₃(C₆-C₁₀)aryl, adamantyl, -O(CH₂)₀₋₃-5- or 6-membered heteroaryl comprising 1 to 3 heteroatoms selected from O, N, and S, (C₆-C₁₀)aryl, monocyclic or bicyclic 5- to 10-membered heteroaryl comprising 1 to 3 heteroatoms selected from O, N, and S, (C₃-C₇)cycloalkyl, and 5- to 7-membered heterocycloalkyl comprising 1 to 3 heteroatoms selected from O, N, and S, wherein the alkyl is optionally substituted with one to four R₁₁, and the aryl, heteroaryl, and heterocycloalkyl are optionally substituted with one to four substituents each independently selected from halogen, (C₁-C₆)alkyl, (C₁-C₆)haloalkyl, and (C₁-C₆)alkoxy, or
two R₇ together with the carbon atom to which they are attached form a =(O), or
two R₇, when on adjacent atoms, together with the atoms to which they are attached form a (C₆-C₁₀)aryl ring or a 5- or 6-membered heteroaryl ring comprising 1 to 3 heteroatoms selected from O, N, and S, optionally substituted with one to four R₁₀, or
two R₇ together with the atoms to which they are attached form a (C₅-C₇) cycloalkyl ring or a 5-to 7-membered heterocycloalkyl ring comprising 1 to 3 heteroatoms selected from O, N, and S, optionally substituted with one to four R₁₀;
each R₁₁ is independently selected from CN, (C₁-C₆)alkoxy, (C₆-C₁₀)aryl, and 5- to 7-membered heterocycloalkyl comprising 1 to 3 heteroatoms selected from O, N, and S, wherein the aryl and heterocycloalkyl are optionally substituted with one to four substituents each independently selected from (C₁-C₆)alkyl, (C₁-C₆)alkoxy, (C₁-C₆)haloalkyl, (C₁-C₆)haloalkoxy, (C₁-C₆)hydroxyalkyl, halogen, -OH, - NH₂, and CN;
or a pharmaceutically acceptable salt, hydrate, solvate, stereoisomer, or tautomer thereof.

In some embodiments of the formulae above, Rₓ is D. In another embodiment, Rₓ is H.

In some embodiments of the formulae above, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN. In another embodiment, R₁ is -C(O)NH₂, -C(O)OH, or CN. In yet another embodiment, R₁ is (C₁-C₆)alkyl, (C₁-C₆)alkoxy, (C₁-C₆)haloalkyl, (C₁-C₆)haloalkoxy, (C₁-C₆)hydroxyalkyl, or halogen. In another embodiment, R₁ is -OH, -(CH₂)₀₋₂NH₂, -(CH₂)₀₋₂NH(C₁-C₆)alkyl, -(CH₂)₀₋₂N((C₁-C₆)alkyl)₂, -C(O)NH₂, -C(O)OH, or CN. In yet another embodiment, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, or CN. In another embodiment, R₁ is (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, or CN. In yet another embodiment, R₁ is (C₁-C₆)alkoxy, (C₁-C₆)hydroxyalkyl, halogen, -OH, -(CH₂)₀₋₂NH₂, or CN. In another embodiment, R₁ is (C₁-C₆)alkoxy, halogen, -OH, -(CH₂)₀₋₂NH₂, or CN. In yet another embodiment, R₁ is (C₁-C₆)alkoxy, halogen, -OH, - NH₂, or CN.

In some embodiments of the formulae above, each R₂ is independently (C₁-C₆)haloalkyl, (C₁-C₆)hydroxyalkyl, CN, or halogen. In another embodiment, each R₂ is independently (C₁-C₆)alkyl, (C₁-C₆)haloalkyl, CN, or halogen. In yet another embodiment, each R₂ is independently (C₁-C₆)alkyl, (C₁-C₆)hydroxyalkyl, CN, or halogen. In another embodiment, each R₂ is independently (C₁-C₆)alkyl, (C₁-C₆)haloalkyl, CN, or halogen. In yet another embodiment, each R₂ is independently (C₁-C₆)alkyl or (C₁-C₆)haloalkyl.

In another embodiment, each R₂ is independently (C₁-C₆)haloalkyl, (C₁-C₆)hydroxyalkyl, or halogen. In another embodiment, each R₂ is independently (C₁-C₆)alkyl, (C₁-C₆)haloalkyl, or halogen. In yet another embodiment, each R₂ is independently (C₁-C₆)alkyl, (C₁-C₆)hydroxyalkyl, or halogen. In another embodiment, each R₂ is independently (C₁-C₆)alkyl, (C₁-C₆)haloalkyl, or halogen. In yet another embodiment, each R₂ is independently (C₁-C₆)alkyl or (C₁-C₆)haloalkyl. In another embodiment, each R₂ is independently (C₁-C₆)alkyl or halogen. In yet another embodiment, each R₂ is independently (C₁-C₆)haloalkyl or halogen. In another embodiment, each R₂ is independently (C₁-C₆)alkyl.

In some embodiments of the formulae above, R₁ and R₂ together with the carbon atoms to which they are attached form a (C₃-C₇)cycloalkyl or a 4- to 6- membered heterocycloalkyl ring comprising 1 to 3 heteroatoms selected from O, N, and S. In another embodiment, R₁ and R₂ together with the carbon atoms to which they are attached form a (C₃-C₇)cycloalkyl or a 5- or 6- membered heterocycloalkyl ring comprising 1 to 3 heteroatoms selected from O, N, and S. In yet another embodiment, R₁ and R₂ together with the carbon atoms to which they are attached form a (C₃-C₇)cycloalkyl or a 4- or 5- membered heterocycloalkyl ring comprising 1 to 3 heteroatoms selected from O, N, and S. In another embodiment, R₁ and R₂ together with the carbon atoms to which they are attached form a (C₄-C₇)cycloalkyl or a 4- to 6-membered heterocycloalkyl ring comprising 1 to 3 heteroatoms selected from O, N, and S. In yet another embodiment, R₁ and R₂ together with the carbon atoms to which they are attached form a (C₄-C₆)cycloalkyl or a 4- to 6- membered heterocycloalkyl ring comprising 1 to 3 heteroatoms selected from O, N, and S.

In another embodiment, R₁ and R₂ together with the carbon atoms to which they are attached form a (C₃-C₇)cycloalkyl. In yet another embodiment, R₁ and R₂ together with the carbon atoms to which they are attached form a (C₃-C₆)cycloalkyl. In another embodiment, R₁ and R₂ together with the carbon atoms to which they are attached form a (C₄-C₇)cycloalkyl. In yet another embodiment, R₁ and R₂ together with the carbon atoms to which they are attached form a (C₅-C₇)cycloalkyl. In another embodiment, R₁ and R₂ together with the carbon atoms to which they are attached form a (C₆-C₇)cycloalkyl. In yet another embodiment, R₁ and R₂ together with the carbon atoms to which they are attached form a (C₅-C₆)cycloalkyl. In another embodiment, R₁ and R₂ together with the carbon atoms to which they are attached form a (C₄-C₆)cycloalkyl. In yet another embodiment, R₁ and R₂ together with the carbon atoms to which they are attached form a (C₃-C₆)cycloalkyl. In another embodiment, R₁ and R₂ together with the carbon atoms to which they are attached form a (C₃-C₅)cycloalkyl. In yet another embodiment, R₁ and R₂ together with the carbon atoms to which they are attached form a 4- to 6- membered heterocycloalkyl ring comprising 1 to 3 heteroatoms selected from O, N, and S. In another embodiment, R₁ and R₂ together with the carbon atoms to which they are attached form a 5- or 6- membered heterocycloalkyl ring comprising 1 to 3 heteroatoms selected from O, N, and S. In yet another embodiment, R₁ and R₂ together with the carbon atoms to which they are attached form a 4- or 5- membered heterocycloalkyl ring comprising 1 to 3 heteroatoms selected from O, N, and S.

In some embodiments of the formulae above, two R₂ together with the carbon atoms to which they are attached form a (C₃-C₇)cycloalkyl or a 4- to 6- membered heterocycloalkyl ring comprising 1 to 3 heteroatoms selected from O, N, and S. In another embodiment, two R₂ together with the carbon atoms to which they are attached form a (C₃-C₇)cycloalkyl or a 5- or 6- membered heterocycloalkyl ring comprising 1 to 3 heteroatoms selected from O, N, and S. In yet another embodiment, two R₂ together with the carbon atoms to which they are attached form a (C₃-C₇)cycloalkyl or a 4- or 5- membered heterocycloalkyl ring comprising 1 to 3 heteroatoms selected from O, N, and S. In another embodiment, two R₂ together with the carbon atoms to which they are attached form a (C₄-C₇)cycloalkyl or a 4- to 6- membered heterocycloalkyl ring comprising 1 to 3 heteroatoms selected from O, N, and S. In yet another embodiment, two R₂ together with the carbon atoms to which they are attached form a (C₄-C₆)cycloalkyl or a 4- to 6- membered heterocycloalkyl ring comprising 1 to 3 heteroatoms selected from O, N, and S.

In another embodiment, two R₂ together with the carbon atoms to which they are attached form a (C₃-C₇)cycloalkyl. In yet another embodiment, two R₂ together with the carbon atoms to which they are attached form a (C₃-C₆)cycloalkyl. In another embodiment, two R₂ together with the carbon atoms to which they are attached form a (C₄-C₇)cycloalkyl. In yet another embodiment, two R₂ together with the carbon atoms to which they are attached form a (C₅-C₇)cycloalkyl. In another embodiment, two R₂ together with the carbon atoms to which they are attached form a (C₆-C₇)cycloalkyl. In yet another embodiment, two R₂ together with the carbon atoms to which they are attached form a (C₅-C₆)cycloalkyl. In another embodiment, two R₂ together with the carbon atoms to which they are attached form a (C₄-C₆)cycloalkyl. In yet another embodiment, two R₂ together with the carbon atoms to which they are attached form a (C₃-C₆)cycloalkyl. In another embodiment, two R₂ together with the carbon atoms to which they are attached form a (C₃-C₅)cycloalkyl. In yet another embodiment, two R₂ together with the carbon atoms to which they are attached form a 4- to 6- membered heterocycloalkyl ring comprising 1 to 3 heteroatoms selected from O, N, and S. In another embodiment, two R₂ together with the carbon atoms to which they are attached form a 5- or 6-membered heterocycloalkyl ring comprising 1 to 3 heteroatoms selected from O, N, and S. In yet another embodiment, two R₂ together with the carbon atoms to which they are attached form a 4- or 5- membered heterocycloalkyl ring comprising 1 to 3 heteroatoms selected from O, N, and S.

In some embodiments of the formulae above, R₃ is (C₁-C₆)alkyl, (C₆-C₁₀)aryl, 5- or 6-membered heteroaryl comprising 1 to 3 heteroatoms selected from O, N, and S, (C₃-C₈)cycloalkyl, or 4- to 7-membered heterocycloalkyl comprising 1 to 3 heteroatoms selected from O, N, and S, wherein the alkyl is optionally substituted with one to four R₄; and the aryl, heteroaryl, cycloalkyl, and heterocycloalkyl are optionally substituted with one to four R₅. In another embodiment, R₃ is (C₁-C₆)alkyl, (C₆-C₁₀)aryl, 5- or 6-membered heteroaryl comprising 1 to 3 heteroatoms selected from O, N, and S, (C₃-C₈)cycloalkyl, or 5- to 7-membered heterocycloalkyl comprising 1 to 3 heteroatoms selected from O, N, and S, wherein the alkyl is optionally substituted with one to four R₄; and the aryl, heteroaryl, cycloalkyl, and heterocycloalkyl are optionally substituted with one to four R₅.

In another embodiment, R₃ is (C₁-C₄)alkyl, (C₆-C₁₀)aryl, (C₃-C₈)cycloalkyl, or 5- to 7-membered heterocycloalkyl comprising 1 to 3 heteroatoms selected from O, N, and S, wherein the alkyl is optionally substituted with one to three R₄; and wherein the aryl, cycloalkyl, and heterocycloalkyl are optionally substituted with one to three R₅. In yet another embodiment, R₃ is (C₁-C₄)alkyl, 5- or 6-membered heteroaryl comprising 1 to 3 heteroatoms selected from O, N, and S, (C₃-C₈)cycloalkyl, or 5- to 7-membered heterocycloalkyl comprising 1 to 3 heteroatoms selected from O, N, and S, wherein the alkyl is optionally substituted with one to three R₄; and wherein the heteroaryl, cycloalkyl, and heterocycloalkyl are optionally substituted with one to three R₅. In another embodiment, R₃ is (C₁-C₄)alkyl, (C₆-C₁₀)aryl, 5- or 6-membered heteroaryl comprising 1 to 3 heteroatoms selected from O, N, and S, or (C₃-C₈)cycloalkyl, wherein the alkyl is optionally substituted with one to three R₄; and wherein the aryl, heteroaryl, and cycloalkyl, are optionally substituted with one to three R₅. In another embodiment, R₃ is (C₁-C₄)alkyl, (C₆-C₁₀)aryl, 5- or 6-membered heteroaryl comprising 1 to 3 heteroatoms selected from O, N, and S, or 5- to 7-membered heterocycloalkyl comprising 1 to 3 heteroatoms selected from O, N, and S, wherein the alkyl is optionally substituted with one to three R₄; and wherein the aryl, heteroaryl, and heterocycloalkyl are optionally substituted with one to three R₅.

In another embodiment, R₃ is (C₆-C₁₀)aryl, 5- or 6-membered heteroaryl comprising 1 to 3 heteroatoms selected from O, N, and S, (C₃-C₈)cycloalkyl, or 5- to 7-membered heterocycloalkyl comprising 1 to 3 heteroatoms selected from O, N, and S, wherein the aryl, heteroaryl, cycloalkyl, and heterocycloalkyl are optionally substituted with one to three R₅. In another embodiment, R₃ is (C₆-C₁₀)aryl, (C₃-C₈)cycloalkyl, or 5- to 7-membered heterocycloalkyl comprising 1 to 3 heteroatoms selected from O, N, and S, wherein the aryl, cycloalkyl, and heterocycloalkyl are optionally substituted with one to three R₅. In yet another embodiment, R₃ is phenyl, (C₃-C₈)cycloalkyl, or 5- to 7-membered heterocycloalkyl comprising 1 to 3 heteroatoms selected from O, N, and S, wherein the phenyl, cycloalkyl, and heterocycloalkyl are optionally substituted with one to three R₅. In another embodiment, R₃ is (C₁-C₃)alkyl optionally substituted with one to three R₄. In yet another embodiment, R₃ is (C₁-C₃)alkyl substituted with one to three R₄.

In another embodiment, R₃ is (C₃-C₈)cycloalkyl or 5- to 7-membered heterocycloalkyl comprising 1 to 3 heteroatoms selected from O, N, and S, wherein the cycloalkyl and heterocycloalkyl are optionally substituted with one to three R₅. In yet another embodiment, R₃ is (C₆-C₁₀)aryl or 5- or 6-membered heteroaryl comprising 1 to 3 heteroatoms selected from O, N, and S, wherein the aryl and heteroaryl are optionally substituted with one to three R₅. In another embodiment, R₃ is (C₃-C₈)cycloalkyl or (C₆-C₁₀)aryl, wherein the cycloalkyl and aryl are optionally substituted with one to three R₅. In yet another embodiment, R₃ is 5- or 6-membered heteroaryl comprising 1 to 3 heteroatoms selected from O, N, and S, or 5- to 7-membered heterocycloalkyl comprising 1 to 3 heteroatoms selected from O, N, and S, wherein the heteroaryl and heterocycloalkyl are optionally substituted with one to three R₅. In another embodiment, R₃ is (C₆-C₁₀)aryl optionally substituted with one to three R₅. In yet another embodiment, R₃ is 5- or 6-membered heteroaryl comprising 1 to 3 heteroatoms selected from O, N, and S, optionally substituted with one to three R₅. In another embodiment, R₃ is (C₃-C₈)cycloalkyl optionally substituted with one to three R₅. In yet another embodiment, R₃ is 5- to 7-membered heterocycloalkyl comprising 1 to 3 heteroatoms selected from O, N, and S, optionally substituted with one to three R₅.

In some embodiments of the formulae above, each R₄ is independently selected from -C(O)OR₆, - C(O)NR₆R_{6'}, -NR₆C(O)R_{6'}, halogen, -OH, -NH₂, CN, (C₆-C₁₀)aryl, 5- or 6-membered heteroaryl comprising 1 to 4 heteroatoms selected from O, N, and S, (C₃-C₈)cycloalkyl, and 4- to 7-membered heterocycloalkyl ring comprising 1 to 3 heteroatoms selected from O, N, and S, wherein the aryl, heteroaryl, cycloalkyl, and heterocycloalkyl groups are optionally substituted with one to four R₇. In another embodiment, each R₄ is independently selected from -C(O)OR₆, -C(O)NR₆R_{6'}, -NR₆C(O)R_{6'}, halogen, -OH, -NH₂, CN, (C₆-C₁₀)aryl, 5- or 6-membered heteroaryl comprising 1 to 4 heteroatoms selected from O, N, and S, (C₃-C₈)cycloalkyl, and 5- to 7-membered heterocycloalkyl ring comprising 1 to 3 heteroatoms selected from O, N, and S, wherein the aryl, heteroaryl, cycloalkyl, and heterocycloalkyl groups are optionally substituted with one to four R₇.

In another embodiment, each R₄ is independently selected from -C(O)OR₆, -C(O)NR₆R_{6'}, - NR₆C(O)R_{6'}, halogen, -OH, -NH₂, or CN. In another embodiment, each R₄ is independently selected from -C(O)OR₆, -C(O)NR₆R_{6'}, -NR₆C(O)R_{6'}, halogen, or -OH. In another embodiment, each R₄ is independently selected from halogen, -OH, (C₆-C₁₀)aryl, 5- or 6-membered heteroaryl comprising 1 to 4 heteroatoms selected from O, N, and S, (C₃-C₈)cycloalkyl, and 5- to 7-membered heterocycloalkyl ring comprising 1 to 3 heteroatoms selected from O, N, and S, wherein the aryl, heteroaryl, cycloalkyl, and heterocycloalkyl groups are optionally substituted with one to four R₇. In another embodiment, each R₄ is independently selected from halogen, -OH, (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl ring comprising 1 to 3 heteroatoms selected from O, N, and S, wherein the aryl, heteroaryl, cycloalkyl, and heterocycloalkyl groups are optionally substituted with one to four R₇.

In another embodiment, each R₄ is independently selected from -C(O)OR₆, -C(O)NR₆R_{6'}, and - NR₆C(O)R_{6'}. In another embodiment, each R₄ is independently selected from -C(O)OR₆, (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to four R₇. In yet another embodiment, each R₄ is independently selected from (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to four R₇. In another embodiment, each R₄ is independently selected from (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇.

In another embodiment, each R₄ is independently selected from (C₆-C₁₀)aryl and 5- or 6-membered heteroaryl comprising 1 to 3 heteroatoms selected from O, N, and S, wherein the aryl and heteroaryl are optionally substituted with one to three R₇. In yet another embodiment, each R₄ is independently selected from (C₆-C₁₀)aryl and 5- or 6-membered heteroaryl comprising 1 to 3 heteroatoms selected from O, N, and S, wherein the aryl and heteroaryl are substituted with one to three R₇.

In another embodiment, each R₄ is independently selected from (C₃-C₈)cycloalkyl and 5- to 7-membered heterocycloalkyl comprising 1 to 3 heteroatoms selected from O, N, and S, wherein the cycloalkyl and heterocycloalkyl groups are optionally substituted with one to three R₇. In another embodiment, each R₄ is independently selected from (C₃-C₈)cycloalkyl and 5- to 7-membered heterocycloalkyl comprising 1 to 3 heteroatoms selected from O, N, and S, wherein the cycloalkyl and heterocycloalkyl groups are substituted with one to three R₇.

In another embodiment, each R₄ is independently (C₆-C₁₀)aryl optionally substituted with one to three R₇. In yet another embodiment, each R₄ is independently 5- or 6-membered heteroaryl comprising 1 to 3 heteroatoms selected from O, N, and S, optionally substituted with one to three R₇.

In another embodiment, each R₄ is (C₃-C₈)cycloalkyl optionally substituted with one to three R₇. In another embodiment, each R₄ is independently 5- to 7-membered heterocycloalkyl comprising 1 to 3 heteroatoms selected from O, N, and S, optionally substituted with one to three R₇.

In some embodiments of the formulae above, each R₅ is independently selected from (C₁-C₆)alkyl, (C₂-C₆)alkenyl, (C₂-C₆)alkynyl, (C₁-C₆)alkoxy, (C₁-C₆)haloalkyl, (C₁-C₆)haloalkoxy, (C₁-C₆)hydroxyalkyl, halogen, -OH, -NH₂, CN, (C₃-C₇)cycloalkyl, 5- to 7-membered heterocycloalkyl comprising 1 to 3 heteroatoms selected from O, N, and S, (C₆-C₁₀)aryl, and 5- or 6-membered heteroaryl comprising 1 to 3 heteroatoms selected from O, N, and S. In another embodiment, each R₅ is independently selected from (C₁-C₆)alkyl, (C₂-C₆)alkenyl, (C₂-C₆)alkynyl, (C₁-C₆)alkoxy, (C₁-C₆)haloalkyl, (C₁-C₆)haloalkoxy, (C₁-C₆)hydroxyalkyl, halogen, -OH, -NH₂, and CN. In yet another embodiment, each R₅ is independently selected from (C₃-C₇)cycloalkyl, 5- to 7-membered heterocycloalkyl comprising 1 to 3 heteroatoms selected from O, N, and S, (C₆-C₁₀)aryl, and 5- or 6-membered heteroaryl comprising 1 to 3 heteroatoms selected from O, N, and S.

In another embodiment, each R₅ is independently selected from (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -NH₂, CN, (C₃-C₇)cycloalkyl, 5- to 7-membered heterocycloalkyl comprising 1 to 3 heteroatoms selected from O, N, and S, (C₆-C₁₀)aryl, and 5- or 6-membered heteroaryl comprising 1 to 3 heteroatoms selected from O, N, and S.

In another embodiment, each R₅ is independently selected from (C₁-C₆)alkyl, (C₁-C₆)alkoxy, (C₁-C₆)haloalkyl, and (C₁-C₆)haloalkoxy. In yet another embodiment, each R₅ is independently selected from (C₁-C₆)hydroxyalkyl, halogen, -OH, -NH₂, and CN. In another embodiment, each R₅ is independently selected from (C₁-C₆)alkyl, (C₁-C₆)alkoxy, (C₁-C₆)haloalkyl, (C₁-C₆)haloalkoxy, (C₁-C₆)hydroxyalkyl, halogen, -OH, and CN.

In some embodiments of the formulae above, two R₅, when on adjacent atoms, together with the atoms to which they are attached form a (C₆-C₁₀)aryl ring or a 5- or 6-membered heteroaryl ring comprising 1 to 3 heteroatoms selected from O, N, and S, optionally substituted with one to four R₁₀. In another embodiment, two R₅, when on adjacent atoms, together with the atoms to which they are attached form a (C₆-C₁₀)aryl ring or a 5- or 6-membered heteroaryl ring comprising 1 to 3 heteroatoms selected from O, N, and S, optionally substituted with one to three R₁₀. In another embodiment, two R₅, when on adjacent atoms, together with the atoms to which they are attached form a (C₆-C₁₀)aryl ring or a 5-membered heteroaryl ring comprising 1 to 3 heteroatoms selected from O, N, and S, optionally substituted with one to three R₁₀. In another embodiment, two R₅, when on adjacent atoms, together with the atoms to which they are attached form a (C₆-C₁₀)aryl ring or a 6-membered heteroaryl ring comprising 1 to 3 heteroatoms selected from O, N, and S, optionally substituted with one to three R₁₀.

In another embodiment, two R₅, when on adjacent atoms, together with the atoms to which they are attached form a phenyl ring or a 5- or 6-membered heteroaryl ring comprising 1 to 3 heteroatoms selected from O, N, and S, optionally substituted with one to three R₁₀. In yet another embodiment, two R₅, when on adjacent atoms, together with the atoms to which they are attached form a phenyl ring or a 5-membered heteroaryl ring comprising 1 to 3 heteroatoms selected from O, N, and S, optionally substituted with one to three R₁₀. In another embodiment, two R₅, when on adjacent atoms, together with the atoms to which they are attached form a phenyl ring or a 6-membered heteroaryl ring comprising 1 to 3 heteroatoms selected from O, N, and S, optionally substituted with one to three R₁₀.

In another embodiment, two R₅, when on adjacent atoms, together with the atoms to which they are attached form a (C₆-C₁₀)aryl ring optionally substituted with one to three R₁₀. In yet another embodiment, two R₅, when on adjacent atoms, together with the atoms to which they are attached form a phenyl ring optionally substituted with one to three R₁₀. In another embodiment, two R₅, when on adjacent atoms, together with the atoms to which they are attached form a 5- or 6-membered heteroaryl ring comprising 1 to 3 heteroatoms selected from O, N, and S, optionally substituted with one to three R₁₀. In yet another embodiment, two R₅, when on adjacent atoms, together with the atoms to which they are attached form a 5-membered heteroaryl ring comprising 1 to 3 heteroatoms selected from O, N, and S, optionally substituted with one to three R₁₀. In another embodiment, two R₅, when on adjacent atoms, together with the atoms to which they are attached form a 6-membered heteroaryl ring comprising 1 to 3 heteroatoms selected from O, N, and S, optionally substituted with one to three R₁₀.

In some embodiments of the formulae above, two R₅ together with the atoms to which they are attached form a (C₃-C₇)cycloalkyl ring or a 4- to 7-membered heterocycloalkyl ring comprising 1 to 3 heteroatoms selected from O, N, and S optionally substituted with one to four R₁₀. In another embodiment, two R₅ together with the atoms to which they are attached form a (C₃-C₇)cycloalkyl ring or a 4- to 7-membered heterocycloalkyl ring comprising 1 to 3 heteroatoms selected from O, N, and S optionally substituted with one to three R₁₀. In yet another embodiment, two R₅ together with the atoms to which they are attached form a (C₃-C₇)cycloalkyl ring or a 5- to 7-membered heterocycloalkyl ring comprising 1 to 3 heteroatoms selected from O, N, and S optionally substituted with one to three R₁₀. In another embodiment, two R₅ together with the atoms to which they are attached form a (C₃-C₇)cycloalkyl ring or a 6- or 7-membered heterocycloalkyl ring comprising 1 to 3 heteroatoms selected from O, N, and S optionally substituted with one to three R₁₀.

In another embodiment, two R₅ together with the atoms to which they are attached form a (C₃-C₇)cycloalkyl ring or a 5- or 6-membered heterocycloalkyl ring comprising 1 to 3 heteroatoms selected from O, N, and S optionally substituted with one to three R₁₀. In yet another embodiment, two R₅ together with the atoms to which they are attached form a (C₃-C₇)cycloalkyl ring or a 4- to 6-membered heterocycloalkyl ring comprising 1 to 3 heteroatoms selected from O, N, and S optionally substituted with one to three R₁₀. In another embodiment, two R₅ together with the atoms to which they are attached form a (C₃-C₇)cycloalkyl ring or a 4- or 5-membered heterocycloalkyl ring comprising 1 to 3 heteroatoms selected from O, N, and S optionally substituted with one to three R₁₀. In yet another embodiment, two R₅ together with the atoms to which they are attached form a (C₃-C₇)cycloalkyl ring or a 5- to 7-membered heterocycloalkyl ring comprising 1 to 3 heteroatoms selected from O, N, and S optionally substituted with one three R₁₀. In another embodiment, two R₅ together with the atoms to which they are attached form a (C₄-C₇)cycloalkyl ring or a 5- to 7-membered heterocycloalkyl ring comprising 1 to 3 heteroatoms selected from O, N, and S optionally substituted with one three R₁₀.

In another embodiment, two R₅ together with the atoms to which they are attached form a (C₅-C₇)cycloalkyl ring or a 5- to 7-membered heterocycloalkyl ring comprising 1 to 3 heteroatoms selected from O, N, and S optionally substituted with one three R₁₀. In another embodiment, two R₅ together with the atoms to which they are attached form a (C₆-C₇)cycloalkyl ring or a 5- to 7-membered heterocycloalkyl ring comprising 1 to 3 heteroatoms selected from O, N, and S optionally substituted with one three R₁₀. In another embodiment, two R₅ together with the atoms to which they are attached form a (C₅-C₆)cycloalkyl ring or a 5- to 7-membered heterocycloalkyl ring comprising 1 to 3 heteroatoms selected from O, N, and S optionally substituted with one three R₁₀. In another embodiment, two R₅ together with the atoms to which they are attached form a (C₄-C₆)cycloalkyl ring or a 5- to 7-membered heterocycloalkyl ring comprising 1 to 3 heteroatoms selected from O, N, and S optionally substituted with one three R₁₀. In another embodiment, two R₅ together with the atoms to which they are attached form a (C₃-C₆)cycloalkyl ring or a 5- to 7-membered heterocycloalkyl ring comprising 1 to 3 heteroatoms selected from O, N, and S optionally substituted with one three R₁₀. In another embodiment, two R₅ together with the atoms to which they are attached form a (C₃-C₅)cycloalkyl ring or a 5- to 7-membered heterocycloalkyl ring comprising 1 to 3 heteroatoms selected from O, N, and S optionally substituted with one three R₁₀. In another embodiment, two R₅ together with the atoms to which they are attached form a (C₃-C₄)cycloalkyl ring or a 5- to 7-membered heterocycloalkyl ring comprising 1 to 3 heteroatoms selected from O, N, and S optionally substituted with one three R₁₀.

In another embodiment, two R₅, together with the atoms to which they are attached form a (C₃-C₇)cycloalkyl ring optionally substituted with one three R₁₀. In another embodiment, two R₅, together with the atoms to which they are attached form a (C₄-C₇)cycloalkyl ring optionally substituted with one three R₁₀. In another embodiment, two R₅, together with the atoms to which they are attached form a (C₅-C₇)cycloalkyl ring optionally substituted with one three R₁₀. In yet another embodiment, two R₅, together with the atoms to which they are attached form a (C₆-C₇)cycloalkyl ring optionally substituted with one three R₁₀. In another embodiment, two R₅, together with the atoms to which they are attached form a (C₃-C₆)cycloalkyl ring optionally substituted with one three R₁₀. In yet another embodiment, two R₅, together with the atoms to which they are attached form a (C₃-C₅)cycloalkyl ring optionally substituted with one three R₁₀. In another embodiment, two R₅, together with the atoms to which they are attached form a (C₃-C₄)cycloalkyl ring optionally substituted with one three R₁₀.

In another embodiment, two R₅ together with the atoms to which they are attached form a 4- to 7-membered heterocycloalkyl ring comprising 1 to 3 heteroatoms selected from O, N, and S optionally substituted with one three R₁₀. In yet another embodiment, two R₅ together with the atoms to which they are attached form a 5- to 7-membered heterocycloalkyl ring comprising 1 to 3 heteroatoms selected from O, N, and S optionally substituted with one three R₁₀. In another embodiment, two R₅ together with the atoms to which they are attached form a 6- or 7-membered heterocycloalkyl ring comprising 1 to 3 heteroatoms selected from O, N, and S optionally substituted with one three R₁₀. In yet another embodiment, two R₅ together with the atoms to which they are attached form a 5- or 6-membered heterocycloalkyl ring comprising 1 to 3 heteroatoms selected from O, N, and S optionally substituted with one three R₁₀. In another embodiment, two R₅ together with the atoms to which they are attached form a 4- or 5-membered heterocycloalkyl ring comprising 1 to 3 heteroatoms selected from O, N, and S optionally substituted with one three R₁₀. In another embodiment, two R₅ together with the atoms to which they are attached form a 4-to 6-membered heterocycloalkyl ring comprising 1 to 3 heteroatoms selected from O, N, and S optionally substituted with one three R₁₀.

In some embodiments of the formulae above, R₆ is H or (C₁-C₃)alkyl. In another embodiment, R₆ is H or (C₆-C₁₀)aryl. In yet another embodiment, R₆ is (C₁-C₃)alkyl or (C₆-C₁₀)aryl. In another embodiment, R₆ is H, methyl, ethyl, n-propyl, or isopropyl. In another embodiment, R₆ is H, methyl or ethyl. In yet another embodiment, R₆ is H or methyl. In another embodiment, R₆ is H.

In some embodiments of the formulae above, R_{6'} is H or (C₁-C₃)alkyl. In another embodiment, R_{6'} is H or (C₆-C₁₀)aryl. In yet another embodiment, R_{6'} is (C₁-C₃)alkyl or (C₆-C₁₀)aryl. In another embodiment, R_{6'} is H, methyl, ethyl, n-propyl, or isopropyl. In another embodiment, R_{6'} is H, methyl or ethyl. In yet another embodiment, R_{6'} is H or methyl. In another embodiment, R_{6'} is H.

In some embodiments of the formulae above, each R₇ is independently selected from (C₁-C₆)alkyl, (C₂-C₆)alkenyl, (C₂-C₆)alkynyl, (C₁-C₆)alkoxy, (C₁-C₆)haloalkyl, (C₁-C₆)haloalkoxy, -C(O)R₈, -(CH₂)₀₋₃C(O)OR₈, -C(O)NR₈R₉, -NR₈C(O)R₉, -NR₈C(O)OR₉, -S(O)ₚNR₈R₉, -S(O)ₚR₁₂, (C₁-C₆)hydroxyalkyl, halogen, -OH, -O(CH₂)₁₋₃CN, -NH₂, CN, -O(CH₂)₀₋₃(C₆-C₁₀)aryl, adamantyl, -O(CH₂)₀₋₃-5- or 6-membered heteroaryl comprising 1 to 3 heteroatoms selected from O, N, and S, (C₆-C₁₀)aryl, monocyclic or bicyclic 5- to 10-membered heteroaryl comprising 1 to 3 heteroatoms selected from O, N, and S, (C₃-C₇)cycloalkyl, and 5- to 7-membered heterocycloalkyl comprising 1 to 3 heteroatoms selected from O, N, and S, wherein the alkyl is optionally substituted with one to four R₁₁, and the aryl, heteroaryl, and heterocycloalkyl are optionally substituted with one to four substituent each independently selected from halogen, (C₁-C₆)alkyl, (C₁-C₆)haloalkyl, and (C₁-C₆)alkoxy. In another embodiment, each R₇ is independently selected from (C₁-C₆)alkyl, (C₂-C₆)alkenyl, (C₂-C₆)alkynyl, (C₁-C₆)alkoxy, (C₁-C₆)haloalkyl, (C₁-C₆)haloalkoxy, -C(O)R₈, - (CH₂)₀₋₃C(O)OR₈, -C(O)NR₈R₉, -NR₈C(O)R₉, -NR₈C(O)OR₉, -S(O)ₚNR₈R₉, -S(O)ₚR₁₂, (C₁-C₆)hydroxyalkyl, halogen, -OH, -O(CH₂)₁₋₃CN, -NH₂, CN, -O(CH₂)₀₋₃(C₆-C₁₀)aryl, -O(CH₂)₀₋₃-5- or 6-membered heteroaryl comprising 1 to 3 heteroatoms selected from O, N, and S, (C₆-C₁₀)aryl, monocyclic or bicyclic 5- to 10-membered heteroaryl comprising 1 to 3 heteroatoms selected from O, N, and S, (C₃-C₇)cycloalkyl, and 5- to 7-membered heterocycloalkyl comprising 1 to 3 heteroatoms selected from O, N, and S, wherein the alkyl is optionally substituted with one to four R₁₁, and the aryl, heteroaryl, and heterocycloalkyl are optionally substituted with one to four substituent each independently selected from halogen, (C₁-C₆)alkyl, (C₁-C₆)haloalkyl, and (C₁-C₆)alkoxy.

In another embodiment, each R₇ is independently selected from (C₁-C₆)alkyl, (C₁-C₆)alkoxy, (C₁-C₆)haloalkyl, (C₁-C₆)haloalkoxy, -C(O)R₈, -(CH₂)₀₋₃C(O)OR₈, -C(O)NR₈R₉, -NR₈C(O)R₉, -NR₈C(O)OR₉, -S(O)ₚNR₈R₉, -S(O)ₚR₁₂, (C₁-C₆)hydroxyalkyl, halogen, -OH, -O(CH₂)₁₋₃CN, -NH₂, CN, -O(CH₂)₀₋₃(C₆-C₁₀)aryl, -O(CH₂)₀₋₃-5- or 6-membered heteroaryl comprising 1 to 3 heteroatoms selected from O, N, and S, (C₆-C₁₀)aryl, monocyclic or bicyclic 5- to 10-membered heteroaryl comprising 1 to 3 heteroatoms selected from O, N, and S, (C₃-C₇)cycloalkyl, and 5- to 7-membered heterocycloalkyl comprising 1 to 3 heteroatoms selected from O, N, and S, wherein the alkyl is optionally substituted with one to four R₁₁, and the aryl, heteroaryl, and heterocycloalkyl are optionally substituted with one to four substituent each independently selected from halogen, (C₁-C₆)alkyl, (C₁-C₆)haloalkyl, and (C₁-C₆)alkoxy.

In another embodiment, each R₇ is independently selected from -(CH₂)₀₋₃C(O)OR₈, -NR₈C(O)OR₉, -S(O)ₚNR₈R₉, -S(O)ₚR₁₂, (C₁-C₆)hydroxyalkyl, halogen, -OH, -O(CH₂)₁₋₃CN, -NH₂, CN, -O(CH₂)₀₋₃(C₆-C₁₀)aryl, -O(CH₂)₀₋₃-5- or 6-membered heteroaryl comprising 1 to 3 heteroatoms selected from O, N, and S, bicyclic 9- or 10-membered heteroaryl comprising 1 to 3 heteroatoms selected from O, N, and S, wherein the aryl and heteroaryl and heterocycloalkyl are optionally substituted with one or more substituent each independently selected from halogen, (C₁-C₆)alkyl, (C₁-C₆)haloalkyl, and (C₁-C₆)alkoxy.

In another embodiment, each R₇ is independently selected from (C₁-C₆)alkyl, (C₂-C₆)alkenyl, (C₂-C₆)alkynyl, (C₁-C₆)alkoxy, (C₁-C₆)haloalkyl, (C₁-C₆)haloalkoxy, -C(O)R₈, -C(O)OR₈, -C(O)NR₈R₉, - NR₈C(O)R₉, (C₁-C₆)hydroxyalkyl, halogen, -OH, -NH₂, CN, (C₆-C₁₀)aryl, 5- or 6-membered heteroaryl comprising 1 to 3 heteroatoms selected from O, N, and S, (C₃-C₇)cycloalkyl, and 5- to 7-membered heterocycloalkyl comprising 1 to 3 heteroatoms selected from O, N, and S. In another embodiment, each R₇ is independently selected from (C₁-C₆)alkyl, (C₂-C₆)alkenyl, (C₂-C₆)alkynyl, (C₁-C₆)alkoxy, (C₁-C₆)haloalkyl, (C₁-C₆)haloalkoxy, -C(O)R₈, -C(O)OR₈, -C(O)NR₈R₉, -NR₈C(O)R₉, (C₁-C₆)hydroxyalkyl, halogen, -OH, -NH₂, and CN.

In another embodiment, each R₇ is independently selected from (C₁-C₆)alkyl, (C₁-C₆)alkoxy, (C₁-C₆)haloalkyl, (C₁-C₆)haloalkoxy, -C(O)R₈, -C(O)OR₈, -C(O)NR₈R₉, -NR₈C(O)R₉, (C₁-C₆)hydroxyalkyl, halogen, -OH, -NH₂, and CN. In yet another embodiment, each R₇ is independently selected from (C₁-C₆)alkyl, (C₁-C₆)alkoxy, (C₁-C₆)haloalkyl, (C₁-C₆)haloalkoxy. In another embodiment, each R₇ is independently selected from -C(O)R₈, -C(O)OR₈, -C(O)NR₈R₉, -NR₈C(O)R₉, (C₁-C₆)hydroxyalkyl, halogen, -OH, -NH₂, and CN. In another embodiment, each R₇ is independently selected from (C₆-C₁₀)aryl, 5- or 6-membered heteroaryl comprising 1 to 3 heteroatoms selected from O, N, and S, (C₃-C₇)cycloalkyl, and 5- to 7-membered heterocycloalkyl comprising 1 to 3 heteroatoms selected from O, N, and S.

In another embodiment, each R₇ is independently selected from (C₁-C₆)alkyl, (C₁-C₆)alkoxy, (C₁-C₆)haloalkyl, (C₁-C₆)haloalkoxy, -C(O)R₈, -C(O)OR₈, -C(O)NR₈R₉, -NR₈C(O)R₉, (C₁-C₆)hydroxyalkyl, halogen, -OH, -NH₂, CN, (C₆-C₁₀)aryl, 5- or 6-membered heteroaryl comprising 1 to 3 heteroatoms selected from O, N, and S, (C₃-C₇)cycloalkyl, and 5- to 7-membered heterocycloalkyl comprising 1 to 3 heteroatoms selected from O, N, and S. In yet another embodiment, each R₇ is independently selected from (C₁-C₆)alkyl, (C₁-C₆)alkoxy, halogen, -OH, CN, and (C₆-C₁₀)aryl.

In some embodiments of the formulae above, two R₇, when on adjacent atoms, together with the atoms to which they are attached form a (C₆-C₁₀)aryl ring or a 5- or 6-membered heteroaryl ring comprising 1 to 3 heteroatoms selected from O, N, and S, optionally substituted with one or more R₁₀. In another embodiment, two R₇, when on adjacent atoms, together with the atoms to which they are attached form a (C₆-C₁₀)aryl ring optionally substituted with one or more R₁₀. In another embodiment, two R₇, when on adjacent atoms, together with the atoms to which they are attached form a 5- or 6-membered heteroaryl ring comprising 1 to 3 heteroatoms selected from O, N, and S, optionally substituted with one or more R₁₀. In another embodiment, two R₇ together with the atoms to which they are attached form a (C₅-C₇) cycloalkyl ring optionally substituted with one or more R₁₀. In another embodiment, two R₇ together with the atoms to which they are attached form a 5- to 7-membered heterocycloalkyl ring comprising 1 to 3 heteroatoms selected from O, N, and S, optionally substituted with one or more R₁₀.

In another embodiment, two R₇, when on adjacent atoms, together with the atoms to which they are attached form a (C₆-C₁₀)aryl ring or a 5- or 6-membered heteroaryl ring comprising 1 to 3 heteroatoms selected from O, N, and S, optionally substituted with one to four R₁₀, or two R₇, when on adjacent atoms, together with the atoms to which they are attached form a (C₅-C₇)cycloalkyl ring or a 5- to 7-membered heterocycloalkyl ring comprising 1 to 3 heteroatoms selected from O, N, and S, optionally substituted with one to four R₁₀.

In another embodiment, two R₇, when on adjacent atoms, together with the atoms to which they are attached form a (C₅-C₇)cycloalkyl ring or a 5- to 7-membered heterocycloalkyl ring comprising 1 to 3 heteroatoms selected from O, N, and S, optionally substituted with one to four R₁₀. In another embodiment, two R₇, when on adjacent atoms, together with the atoms to which they are attached form a (C₅-C₇)cycloalkyl ring optionally substituted with one to four R₁₀.In another embodiment, two R₇, when on adjacent atoms, together with the atoms to which they are attached form a 5- to 7-membered heterocycloalkyl ring comprising 1 to 3 heteroatoms selected from O, N, and S, optionally substituted with one to four R₁₀.

In some embodiments of the formulae above, R₈ is H or (C₁-C₃)alkyl. In another embodiment, R₈ is H, methyl, ethyl, n-propyl, or isopropyl. In another embodiment, R₈ is H, methyl or ethyl. In yet another embodiment, R₈ is H or methyl. In another embodiment, R₈ is H

In some embodiments of the formulae above, R₉ is H or (C₁-C₃)alkyl. In another embodiment, R₉ is H, methyl, ethyl, n-propyl, or isopropyl. In another embodiment, R₉ is H, methyl or ethyl. In yet another embodiment, R₉ is H or methyl. In another embodiment, R₉ is H.

In some embodiments of the formulae above, each R₁₀ is independently selected from (C₁-C₆)alkyl, (C₁-C₆)alkoxy, (C₁-C₆)haloalkyl, (C₁-C₆)haloalkoxy, (C₁-C₆)hydroxyalkyl, and halogen. In another embodiment, each R₁₀ is independently selected from -OH, -NH₂, and CN. In yet another embodiment, each R₁₀ is independently selected from (C₁-C₆)alkyl, (C₁-C₆)alkoxy, (C₁-C₆)haloalkyl, (C₁-C₆)haloalkoxy, and halogen. In another embodiment, each R₁₀ is independently selected from (C₁-C₆)alkyl, (C₁-C₆)haloalkyl, and halogen. In yet another embodiment, each R₁₀ is independently selected from (C₁-C₆)alkyl and halogen.

In some embodiments of the formulae above, two R₁₀ together with the carbon atom to which they are attached form a =(O).

In some embodiments of the formulae above, each R₁₁ is independently selected from CN, (C₁-C₆)alkoxy, (C₆-C₁₀)aryl, and 5- to 7-membered heterocycloalkyl comprising 1 to 3 heteroatoms selected from O, N, and S, wherein the aryl and heterocycloalkyl are optionally substituted with one to four substituents each independently selected from (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -NH₂, and CN. In another embodiment, each R₁₁ is independently selected from CN, (C₁-C₆)alkoxy, (C₆-C₁₀)aryl, and 5- to 7-membered heterocycloalkyl comprising 1 to 3 heteroatoms selected from O, N, and S, wherein the aryl and heterocycloalkyl are optionally substituted with one to three substituents each independently selected from (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -NH₂, and CN. In yet another embodiment, each R₁₁ is independently selected from CN, (C₁-C₆)alkoxy, and (C₆-C₁₀)aryl, wherein the aryl is optionally substituted with one to three substituents each independently selected from (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -NH₂, and CN.

In another embodiment, each R₁₁ is independently selected from CN, (C₁-C₆)alkoxy, and 5- to 7-membered heterocycloalkyl comprising 1 to 3 heteroatoms selected from O, N, and S, wherein the heterocycloalkyl is optionally substituted with one to four substituents each independently selected from (C₁-C₆)alkyl, (C₁-C₆)alkoxy, (C₁-C₆)haloalkyl, (C₁-C₆)haloalkoxy, (C₁-C₆)hydroxyalkyl, halogen, -OH, - NH₂, and CN. In another embodiment, each R₁₁ is independently selected from CN and (C₁-C₆)alkoxy. In yet another embodiment, each R₁₁ is independently selected from (C₆-C₁₀)aryl and 5- to 7-membered heterocycloalkyl comprising 1 to 3 heteroatoms selected from O, N, and S, wherein the aryl and heterocycloalkyl are optionally substituted with one to four substituents each independently selected from (C₁-C₆)alkyl, (C₁-C₆)alkoxy, (C₁-C₆)haloalkyl, (C₁-C₆)haloalkoxy, (C₁-C₆)hydroxyalkyl, halogen, -OH, - NH₂, and CN.

In some embodiments of the formulae above, R₁₂ is (C₁-C₆)alkyl, (C₁-C₆)haloalkyl, (C₆-C₁₀)aryl, or 5- or 6-membered heterocycloalkyl comprising 1 to 3 heteroatoms selected from O, N, and S. In another embodiment, R₁₂ is (C₁-C₆)alkyl, (C₁-C₆)haloalkyl, phenyl, or 5- or 6-membered heterocycloalkyl comprising 1 to 3 heteroatoms selected from O, N, and S. In another embodiment, R₁₂ is (C₁-C₄)alkyl, (C₁-C₄)haloalkyl, phenyl, or 5- or 6-membered heterocycloalkyl comprising 1 to 3 heteroatoms selected from O, N, and S.

In some embodiments of the formulae above, n is 0, 1, or 2. In another embodiment, n is 1, 2, or 3. In another embodiment, n is 0 or 1. In yet another embodiment, n is 1 or 2. In another embodiment, n is 2 or 3. In yet another embodiment, n is 0. In yet another embodiment, n is 1. In another embodiment, n is 2. In yet another embodiment, n is 3.

In some embodiments of the formulae above, Rₓ is H and n is 0. In another embodiment, Rₓ is H and n is 1. In another embodiment, Rₓ is H and n is 2.

In some embodiments of the formulae above, Rₓ is H, n is 0, and R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, -C(O)NH₂, -C(O)OH or CN. In another embodiment, Rₓ is H, n is 0, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, - OH, -(CH₂)₀₋₂NH₂, -(CH₂)₀₋₂NH(C₁-C₆)alkyl, -(CH₂)₀₋₂N((C₁-C₆)alkyl)₂, -C(O)NH₂, -C(O)OH or CN, and R₃ is (C₁-C₆)alkyl optionally substituted with one to three R₄. In another embodiment, Rₓ is H, n is 0, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, - (CH₂)₀₋₂NH₂, -(CH₂)₀₋₂NH(C₁-C₆)alkyl, -(CH₂)₀₋₂N((C₁-C₆)alkyl)₂, -C(O)NH₂, -C(O)OH or CN, and R₃ is (C₁-C₆)alkyl substituted with one to three R₄.

In some embodiments of the formulae above, Rₓ is H, n is 0, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, -C(O)NH₂, -C(O)OH or CN, R₃ is (C₁-C₆)alkyl optionally substituted with one to three R₄, and each R₄ is independently selected from -C(O)OR₆, (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇. In another embodiment, Rₓ is H, n is 0, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, - C(O)NH₂, -C(O)OH or CN, R₃ is (C₁-C₆)alkyl substituted with one to three R₄, and each R₄ is independently selected from -C(O)OR₆, (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇.

In some embodiments of the formulae above, Rₓ is H, n is 0, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, -C(O)NH₂, -C(O)OH or CN, R₃ is (C₁-C₆)alkyl optionally substituted with one to three R₄, and each R₄ is independently selected from (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇. In another embodiment, Rₓ is H, n is 0, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, - C(O)NH₂, -C(O)OH or CN, R₃ is (C₁-C₆)alkyl substituted with one to three R₄, and each R₄ is independently selected from (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇.

In some embodiments of the formulae above, Rₓ is H, n is 0, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, -C(O)NH₂, -C(O)OH or CN, R₃ is (C₁-C₆)alkyl optionally substituted with one to three R₄, and each R₄ is independently selected from halogen, -OH, (C₆-C₁₀)aryl, 5- or 6-membered heteroaryl comprising 1 to 3 heteroatoms selected from O, N, and S, (C₃-C₈)cycloalkyl, and 5-to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇. In another embodiment, Rₓ is H, n is 0, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, - C(O)NH₂, -C(O)OH or CN, R₃ is (C₁-C₆)alkyl substituted with one to three R₄, and each R₄ is independently selected from halogen, -OH, (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇.

In some embodiments of the formulae above, Rₓ is H, n is 0, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, -C(O)NH₂, -C(O)OH or CN, R₃ is (C₁-C₆)alkyl optionally substituted with one to three R₄, and each R₄ is independently selected from halogen, -OH, phenyl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the phenyl, heteroaryl, cycloalkyl, and heterocycloalkyl groups are optionally substituted with one to three R₇. In another embodiment, Rₓ is H, n is 0, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, - C(O)NH₂, -C(O)OH or CN, R₃ is (C₁-C₆)alkyl substituted with one to three R₄, and each R₄ is independently selected from halogen, -OH, phenyl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the phenyl, heteroaryl, cycloalkyl, and heterocycloalkyl groups are optionally substituted with one to three R₇.

In some embodiments of the formulae above, Rₓ is H, n is 0, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, -C(O)NH₂, -C(O)OH or CN, R₃ is (C₁-C₆)alkyl optionally substituted with one to three R₄, and each R₄ is independently selected from phenyl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the phenyl, heteroaryl, cycloalkyl, and heterocycloalkyl groups are optionally substituted with one to three R₇. In another embodiment, Rₓ is H, n is 0, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, - C(O)NH₂, -C(O)OH or CN, R₃ is (C₁-C₆)alkyl substituted with one to three R₄, and each R₄ is independently selected from phenyl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the phenyl, heteroaryl, cycloalkyl, and heterocycloalkyl groups are optionally substituted with one to three R₇.

In some embodiments of the formulae above, Rₓ is H, n is 0, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, -C(O)NH₂, -C(O)OH or CN, R₃ is (C₁-C₆)alkyl optionally substituted with one to three R₄, and each R₄ is independently selected from phenyl and 5- or 6-membered heteroaryl comprising 1 to 3 heteroatoms selected from O, N, and S, wherein the phenyl and heteroaryl groups are optionally substituted with one to three R₇. In another embodiment, Rₓ is H, n is 0, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, - (CH₂)₀₋₂NH(C₁-C₆)alkyl, -(CH₂)₀₋₂N((C₁-C₆)alkyl)₂, -C(O)NH₂, -C(O)OH or CN, R₃ is (C₁-C₆)alkyl substituted with one to three R₄, and each R₄ is independently selected from phenyl and 5- or 6-membered heteroaryl comprising 1 to 3 heteroatoms selected from O, N, and S, wherein the phenyl and heteroaryl groups are optionally substituted with one to three R₇.

In some embodiments of the formulae above, Rₓ is H, n is 0, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, -C(O)NH₂, -C(O)OH or CN, R₃ is (C₁-C₆)alkyl optionally substituted with one to three R₄, and each R₄ is phenyl optionally substituted with one to three R₇. In another embodiment, Rₓ is H, n is 0, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, - C(O)NH₂, -C(O)OH or CN, R₃ is (C₁-C₆)alkyl substituted with one to three R₄, and each R₄ is phenyl optionally substituted with one to three R₇.

In some embodiments of the formulae above, Rₓ is H, n is 0, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, -C(O)NH₂, -C(O)OH or CN, R₃ is (C₁-C₆)alkyl optionally substituted with one to three R₄, and each R₄ is 5- or 6-membered heteroaryl comprising 1 to 3 heteroatoms selected from O, N, and S, optionally substituted with one to three R₇. In another embodiment, Rₓ is H, n is 0, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, - (CH₂)₀₋₂NH₂, -(CH₂)₀₋₂NH(C₁-C₆)alkyl, -(CH₂)₀₋₂N((C₁-C₆)alkyl)₂, -C(O)NH₂, -C(O)OH or CN, R₃ is (C₁-C₆)alkyl substituted with one to three R₄, and each R₄ is 5- or 6-membered heteroaryl comprising 1 to 3 heteroatoms selected from O, N, and S, optionally substituted with one to three R₇.

In some embodiments of the formulae above, Rₓ is H, n is 0, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, -C(O)NH₂, -C(O)OH or CN, R₃ is (C₁-C₆)alkyl optionally substituted with one to three R₄, and each R₄ is (C₃-C₈)cycloalkyl optionally substituted with one to three R₇. In another embodiment, Rₓ is H, n is 0, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, - C(O)NH₂, -C(O)OH or CN, R₃ is (C₁-C₆)alkyl substituted with one to three R₄, and each R₄ is (C₃-C₈)cycloalkyl optionally substituted with one to three R₇.

In some embodiments of the formulae above, Rₓ is H, n is 0, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, -C(O)NH₂, -C(O)OH or CN, R₃ is (C₁-C₆)alkyl optionally substituted with one to three R₄, and each R₄ is 5- to 7-membered heterocycloalkyl comprising 1 to 3 heteroatoms selected from O, N, and S, optionally substituted with one to three R₇.

In another embodiment, Rₓ is H, n is 0, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, -C(O)NH₂, -C(O)OH or CN, R₃ is (C₁-C₆)alkyl substituted with one to three R₄, and each R₄ is 5- to 7-membered heterocycloalkyl comprising 1 to 3 heteroatoms selected from O, N, and S, optionally substituted with one to three R₇.

In some embodiments of the formulae above, Rₓ is H, n is 0, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, -C(O)NH₂, -C(O)OH or CN, and R₃ is (C₆-C₁₀)aryl, (C₃-C₈)cycloalkyl, or 5- to 7-membered heterocycloalkyl comprising 1 to 3 heteroatoms selected from O, N, and S, wherein the aryl, cycloalkyl, and heterocycloalkyl are optionally substituted with one to three R₅.

In some embodiments of the formulae above, Rₓ is H, n is 0, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, -C(O)NH₂, -C(O)OH or CN, and R₃ is (C₆-C₁₀)aryl, (C₃-C₈)cycloalkyl, or 5- to 7-membered heterocycloalkyl comprising 1 to 3 heteroatoms selected from O, N, and S.

In some embodiments of the formulae above, Rₓ is H, n is 0, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, -C(O)NH₂, -C(O)OH or CN, and R₃ is (C₆-C₁₀)aryl, 5- or 6-membered heteroaryl comprising 1 to 3 heteroatoms selected from O, N, and S, or (C₃-C₈)cycloalkyl, wherein the aryl, heteroaryl and cycloalkyl are optionally substituted with one to three R₅.

In some embodiments of the formulae above, Rₓ is H, n is 0, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, -C(O)NH₂, -C(O)OH or CN, and R₃ is (C₆-C₁₀)aryl, 5- or 6-membered heteroaryl comprising 1 to 3 heteroatoms selected from O, N, and S, or (C₃-C₈)cycloalkyl.

In some embodiments of the formulae above, Rₓ is H, n is 0, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, -C(O)NH₂, -C(O)OH or CN, and R₃ is (C₆-C₁₀)aryl optionally substituted with one to three R₅.

In some embodiments of the formulae above, Rₓ is H, n is 0, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, -C(O)NH₂, -C(O)OH or CN, and R₃ is 5- or 6-membered heteroaryl comprising 1 to 3 heteroatoms selected from O, N, and S optionally substituted with one to three R₅.

In some embodiments of the formulae above, Rₓ is H, n is 0, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, -C(O)NH₂, -C(O)OH or CN, and R₃ is (C₃-C₈)cycloalkyl optionally substituted with one to three R₅.

In some embodiments of the formulae above, Rₓ is H, n is 0, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, -C(O)NH₂, -C(O)OH or CN, and R₃ is 5- to 7-membered heterocycloalkyl comprising 1 to 3 heteroatoms selected from O, N, and S, optionally substituted with one to three R₅.

In some embodiments of the formulae above, Rₓ is H, n is 1, and R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, -C(O)NH₂, -C(O)OH or CN. In another embodiment, Rₓ is H, n is 1, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, - OH, -(CH₂)₀₋₂NH₂, -(CH₂)₀₋₂NH(C₁-C₆)alkyl, -(CH₂)₀₋₂N((C₁-C₆)alkyl)₂, -C(O)NH₂, -C(O)OH or CN, and R₃ is (C₁-C₆)alkyl optionally substituted with one to three R₄. In another embodiment, Rₓ is H, n is 1, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, - (CH₂)₀₋₂NH₂, -(CH₂)₀₋₂NH(C₁-C₆)alkyl, -(CH₂)₀₋₂N((C₁-C₆)alkyl)₂, -C(O)NH₂, -C(O)OH or CN, and R₃ is (C₁-C₆)alkyl substituted with one to three R₄.

In some embodiments of the formulae above, Rₓ is H, n is 1, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, -C(O)NH₂, -C(O)OH or CN, R₃ is (C₁-C₆)alkyl optionally substituted with one to three R₄, and each R₄ is independently selected from -C(O)OR₆, (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇. In another embodiment, Rₓ is H, n is 1, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, - C(O)NH₂, -C(O)OH or CN, R₃ is (C₁-C₆)alkyl substituted with one to three R₄, and each R₄ is independently selected from -C(O)OR₆, (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇.

In some embodiments of the formulae above, Rₓ is H, n is 1, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, -C(O)NH₂, -C(O)OH or CN, R₃ is (C₁-C₆)alkyl optionally substituted with one to three R₄, and each R₄ is independently selected from (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇. In another embodiment, Rₓ is H, n is 1, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, - C(O)NH₂, -C(O)OH or CN, R₃ is (C₁-C₆)alkyl substituted with one to three R₄, and each R₄ is independently selected from (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇.

In some embodiments of the formulae above, Rₓ is H, n is 1, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, -C(O)NH₂, -C(O)OH or CN, R₃ is (C₁-C₆)alkyl optionally substituted with one to three R₄, and each R₄ is independently selected from halogen, -OH, (C₆-C₁₀)aryl, 5- or 6-membered heteroaryl comprising 1 to 3 heteroatoms selected from O, N, and S, (C₃-C₈)cycloalkyl, and 5-to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇. In another embodiment, Rₓ is H, n is 1, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, - C(O)NH₂, -C(O)OH or CN, R₃ is (C₁-C₆)alkyl substituted with one to three R₄, and each R₄ is independently selected from halogen, -OH, (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇.

In some embodiments of the formulae above, Rₓ is H, n is 1, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, -C(O)NH₂, -C(O)OH or CN, R₃ is (C₁-C₆)alkyl optionally substituted with one to three R₄, and each R₄ is independently selected from halogen, -OH, phenyl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the phenyl, heteroaryl, cycloalkyl, and heterocycloalkyl groups are optionally substituted with one to three R₇. In another embodiment, Rₓ is H, n is 1, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, - C(O)NH₂, -C(O)OH or CN, R₃ is (C₁-C₆)alkyl substituted with one to three R₄, and each R₄ is independently selected from halogen, -OH, phenyl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the phenyl, heteroaryl, cycloalkyl, and heterocycloalkyl groups are optionally substituted with one to three R₇.

In some embodiments of the formulae above, Rₓ is H, n is 1, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, -C(O)NH₂, -C(O)OH or CN, R₃ is (C₁-C₆)alkyl optionally substituted with one to three R₄, and each R₄ is independently selected from phenyl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the phenyl, heteroaryl, cycloalkyl, and heterocycloalkyl groups are optionally substituted with one to three R₇. In another embodiment, Rₓ is H, n is 1, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, - C(O)NH₂, -C(O)OH or CN, R₃ is (C₁-C₆)alkyl substituted with one to three R₄, and each R₄ is independently selected from phenyl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the phenyl, heteroaryl, cycloalkyl, and heterocycloalkyl groups are optionally substituted with one to three R₇.

In some embodiments of the formulae above, Rₓ is H, n is 1, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, -C(O)NH₂, -C(O)OH or CN, R₃ is (C₁-C₆)alkyl optionally substituted with one to three R₄, and each R₄ is independently selected from phenyl and 5- or 6-membered heteroaryl comprising 1 to 3 heteroatoms selected from O, N, and S, wherein the phenyl and heteroaryl groups are optionally substituted with one to three R₇. In another embodiment, Rₓ is H, n is 1, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, - (CH₂)₀₋₂NH(C₁-C₆)alkyl, -(CH₂)₀₋₂N((C₁-C₆)alkyl)₂, -C(O)NH₂, -C(O)OH or CN, R₃ is (C₁-C₆)alkyl substituted with one to three R₄, and each R₄ is independently selected from phenyl and 5- or 6-membered heteroaryl comprising 1 to 3 heteroatoms selected from O, N, and S, wherein the phenyl and heteroaryl groups are optionally substituted with one to three R₇.

In some embodiments of the formulae above, Rₓ is H, n is 1, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, -C(O)NH₂, -C(O)OH or CN, R₃ is (C₁-C₆)alkyl optionally substituted with one to three R₄, and each R₄ is phenyl optionally substituted with one to three R₇. In another embodiment, Rₓ is H, n is 1, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, - C(O)NH₂, -C(O)OH or CN, R₃ is (C₁-C₆)alkyl substituted with one to three R₄, and each R₄ is phenyl optionally substituted with one to three R₇.

In some embodiments of the formulae above, Rₓ is H, n is 1, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, -C(O)NH₂, -C(O)OH or CN, R₃ is (C₁-C₆)alkyl optionally substituted with one to three R₄, and each R₄ is 5- or 6-membered heteroaryl comprising 1 to 3 heteroatoms selected from O, N, and S, optionally substituted with one to three R₇. In another embodiment, Rₓ is H, n is 1, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, - (CH₂)₀₋₂NH₂, -(CH₂)₀₋₂NH(C₁-C₆)alkyl, -(CH₂)₀₋₂N((C₁-C₆)alkyl)₂, -C(O)NH₂, -C(O)OH or CN, R₃ is (C₁-C₆)alkyl substituted with one to three R₄, and each R₄ is 5- or 6-membered heteroaryl comprising 1 to 3 heteroatoms selected from O, N, and S, optionally substituted with one to three R₇.

In some embodiments of the formulae above, Rₓ is H, n is 1, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, -C(O)NH₂, -C(O)OH or CN, R₃ is (C₁-C₆)alkyl optionally substituted with one to three R₄, and each R₄ is (C₃-C₈)cycloalkyl optionally substituted with one to three R₇.

In another embodiment, Rₓ is H, n is 1, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, -C(O)NH₂, -C(O)OH or CN, R₃ is (C₁-C₆)alkyl substituted with one to three R₄, and each R₄ is (C₃-C₈)cycloalkyl optionally substituted with one to three R₇.

In some embodiments of the formulae above, Rₓ is H, n is 1, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, -C(O)NH₂, -C(O)OH or CN, R₃ is (C₁-C₆)alkyl optionally substituted with one to three R₄, and each R₄ is 5- to 7-membered heterocycloalkyl comprising 1 to 3 heteroatoms selected from O, N, and S, optionally substituted with one to three R₇.

In another embodiment, Rₓ is H, n is 1, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, -C(O)NH₂, -C(O)OH or CN, R₃ is (C₁-C₆)alkyl substituted with one to three R₄, and each R₄ is 5- to 7-membered heterocycloalkyl comprising 1 to 3 heteroatoms selected from O, N, and S, optionally substituted with one to three R₇.

In some embodiments of the formulae above, Rₓ is H, n is 1, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, -C(O)NH₂, -C(O)OH or CN, and R₃ is (C₆-C₁₀)aryl, (C₃-C₈)cycloalkyl, or 5- to 7-membered heterocycloalkyl comprising 1 to 3 heteroatoms selected from O, N, and S, wherein the aryl, cycloalkyl, and heterocycloalkyl are optionally substituted with one to three R₅.

In some embodiments of the formulae above, Rₓ is H, n is 1, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, -C(O)NH₂, -C(O)OH or CN, and R₃ is (C₆-C₁₀)aryl, (C₃-C₈)cycloalkyl, or 5- to 7-membered heterocycloalkyl comprising 1 to 3 heteroatoms selected from O, N, and S.

In some embodiments of the formulae above, Rₓ is H, n is 1, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, -C(O)NH₂, -C(O)OH or CN, and R₃ is (C₆-C₁₀)aryl, 5- or 6-membered heteroaryl comprising 1 to 3 heteroatoms selected from O, N, and S, or (C₃-C₈)cycloalkyl, wherein the aryl, heteroaryl and cycloalkyl are optionally substituted with one to three R₅.

In some embodiments of the formulae above, Rₓ is H, n is 1, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, -C(O)NH₂, -C(O)OH or CN, and R₃ is (C₆-C₁₀)aryl, 5- or 6-membered heteroaryl comprising 1 to 3 heteroatoms selected from O, N, and S, or (C₃-C₈)cycloalkyl.

In some embodiments of the formulae above, Rₓ is H, n is 1, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, -C(O)NH₂, -C(O)OH or CN, and R₃ is (C₆-C₁₀)aryl optionally substituted with one to three R₅.

In some embodiments of the formulae above, Rₓ is H, n is 1, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, -C(O)NH₂, -C(O)OH or CN, and R₃ is 5- or 6-membered heteroaryl comprising 1 to 3 heteroatoms selected from O, N, and S optionally substituted with one to three R₅.

In some embodiments of the formulae above, Rₓ is H, n is 1, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, -C(O)NH₂, -C(O)OH or CN, and R₃ is (C₃-C₈)cycloalkyl optionally substituted with one to three R₅.

In some embodiments of the formulae above, Rₓ is H, n is 1, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, -C(O)NH₂, -C(O)OH or CN, and R₃ is 5- to 7-membered heterocycloalkyl comprising 1 to 3 heteroatoms selected from O, N, and S, optionally substituted with one to three R₅.

In some embodiments of the formulae above, Rₓ is H, n is 2, and R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, -C(O)NH₂, -C(O)OH or CN.

In some embodiments of the formulae above, Rₓ is H, n is 0, and R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN. In another embodiment, Rₓ is H, n is 0, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, - (CH₂)₀₋₂NH(C₁-C₆)alkyl, -(CH₂)₀₋₂N((C₁-C₆)alkyl)₂, or CN, and R₃ is (C₁-C₆)alkyl optionally substituted with one to three R₄. In another embodiment, Rₓ is H, n is 0, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, and R₃ is (C₁-C₆)alkyl substituted with one to three R₄.

In some embodiments of the formulae above, Rₓ is H, n is 0, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, R₃ is (C₁-C₆)alkyl optionally substituted with one to three R₄, and each R₄ is independently selected from -C(O)OR₆, (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇. In another embodiment, Rₓ is H, n is 0, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, R₃ is (C₁-C₆)alkyl substituted with one to three R₄, and each R₄ is independently selected from -C(O)OR₆, (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇.

In some embodiments of the formulae above, Rₓ is H, n is 0, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, R₃ is (C₁-C₆)alkyl optionally substituted with one to three R₄, and each R₄ is independently selected from (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇. In another embodiment, Rₓ is H, n is 0, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, R₃ is (C₁-C₆)alkyl substituted with one to three R₄, and each R₄ is independently selected from (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇.

In some embodiments of the formulae above, Rₓ is H, n is 0, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, R₃ is (C₁-C₆)alkyl optionally substituted with one to three R₄, and each R₄ is independently selected from halogen, -OH, (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇. In another embodiment, Rₓ is H, n is 0, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, R₃ is (C₁-C₆)alkyl substituted with one to three R₄, and each R₄ is independently selected from halogen, - OH, (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇.

In some embodiments of the formulae above, Rₓ is H, n is 0, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, R₃ is (C₁-C₆)alkyl optionally substituted with one to three R₄, and each R₄ is independently selected from halogen, -OH, phenyl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the phenyl, heteroaryl, cycloalkyl, and heterocycloalkyl groups are optionally substituted with one to three R₇. In another embodiment, Rₓ is H, n is 0, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, R₃ is (C₁-C₆)alkyl substituted with one to three R₄, and each R₄ is independently selected from halogen, -OH, phenyl, 5- or 6-membered heteroaryl comprising 1 to 3 heteroatoms selected from O, N, and S, (C₃-C₈)cycloalkyl, and 5-to 7-membered heterocycloalkyl comprising 1 to 3 heteroatoms selected from O, N, and S, wherein the phenyl, heteroaryl, cycloalkyl, and heterocycloalkyl groups are optionally substituted with one to three R₇.

In some embodiments of the formulae above, Rₓ is H, n is 0, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, R₃ is (C₁-C₆)alkyl optionally substituted with one to three R₄, and each R₄ is independently selected from phenyl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the phenyl, heteroaryl, cycloalkyl, and heterocycloalkyl groups are optionally substituted with one to three R₇. In another embodiment, Rₓ is H, n is 0, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, R₃ is (C₁-C₆)alkyl substituted with one to three R₄, and each R₄ is independently selected from phenyl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the phenyl, heteroaryl, cycloalkyl, and heterocycloalkyl groups are optionally substituted with one to three R₇.

In some embodiments of the formulae above, Rₓ is H, n is 0, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, R₃ is (C₁-C₆)alkyl optionally substituted with one to three R₄, and each R₄ is independently selected from phenyl and 5- or 6-membered heteroaryl comprising 1 to 3 heteroatoms selected from O, N, and S, wherein the phenyl and heteroaryl groups are optionally substituted with one to three R₇. In another embodiment, Rₓ is H, n is 0, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, R₃ is (C₁-C₆)alkyl substituted with one to three R₄, and each R₄ is independently selected from phenyl and 5- or 6-membered heteroaryl comprising 1 to 3 heteroatoms selected from O, N, and S, wherein the phenyl and heteroaryl groups are optionally substituted with one to three R₇.

In some embodiments of the formulae above, Rₓ is H, n is 0, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, R₃ is (C₁-C₆)alkyl optionally substituted with one to three R₄, and each R₄ is phenyl optionally substituted with one to three R₇. In another embodiment, Rₓ is H, n is 0, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, - OH, -(CH₂)₀₋₂NH₂, -(CH₂)₀₋₂NH(C₁-C₆)alkyl, -(CH₂)₀₋₂N((C₁-C₆)alkyl)₂, or CN, R₃ is (C₁-C₆)alkyl substituted with one to three R₄, and each R₄ is phenyl optionally substituted with one to three R₇.

In some embodiments of the formulae above, Rₓ is H, n is 0, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, R₃ is (C₁-C₆)alkyl optionally substituted with one to three R₄, and each R₄ is 5- or 6-membered heteroaryl comprising 1 to 3 heteroatoms selected from O, N, and S, optionally substituted with one to three R₇. In another embodiment, Rₓ is H, n is 0, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, - (CH₂)₀₋₂NH(C₁-C₆)alkyl, -(CH₂)₀₋₂N((C₁-C₆)alkyl)₂, or CN, R₃ is (C₁-C₆)alkyl substituted with one to three R₄, and each R₄ is 5- or 6-membered heteroaryl comprising 1 to 3 heteroatoms selected from O, N, and S, optionally substituted with one to three R₇.

In some embodiments of the formulae above, Rₓ is H, n is 0, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, R₃ is (C₁-C₆)alkyl optionally substituted with one to three R₄, and each R₄ is (C₃-C₈)cycloalkyl optionally substituted with one to three R₇. In another embodiment, Rₓ is H, n is 0, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, R₃ is (C₁-C₆)alkyl substituted with one to three R₄, and each R₄ is (C₃-C₈)cycloalkyl optionally substituted with one to three R₇.

In some embodiments of the formulae above, Rₓ is H, n is 0, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, R₃ is (C₁-C₆)alkyl optionally substituted with one to three R₄, and each R₄ is 5- to 7-membered heterocycloalkyl comprising 1 to 3 heteroatoms selected from O, N, and S, optionally substituted with one to three R₇. In another embodiment, Rₓ is H, n is 0, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, - (CH₂)₀₋₂NH(C₁-C₆)alkyl, -(CH₂)₀₋₂N((C₁-C₆)alkyl)₂, or CN, R₃ is (C₁-C₆)alkyl substituted with one to three R₄, and each R₄ is 5- to 7-membered heterocycloalkyl comprising 1 to 3 heteroatoms selected from O, N, and S, optionally substituted with one to three R₇.

In some embodiments of the formulae above, Rₓ is H, n is 0, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, and R₃ is (C₆-C₁₀)aryl, (C₃-C₈)cycloalkyl, or 5- to 7-membered heterocycloalkyl comprising 1 to 3 heteroatoms selected from O, N, and S, wherein the aryl, cycloalkyl, and heterocycloalkyl are optionally substituted with one to three R₅. In another embodiment, Rₓ is H, n is 0, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, - OH, -(CH₂)₀₋₂NH₂, -(CH₂)₀₋₂NH(C₁-C₆)alkyl, -(CH₂)₀₋₂N((C₁-C₆)alkyl)₂, or CN, and R₃ is (C₆-C₁₀)aryl, (C₃-C₈)cycloalkyl, or 5- to 7-membered heterocycloalkyl comprising 1 to 3 heteroatoms selected from O, N, and S, wherein the aryl, cycloalkyl, and heterocycloalkyl are optionally substituted with one to three R₅.

In some embodiments of the formulae above, Rₓ is H, n is 0, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, and R₃ is (C₆-C₁₀)aryl, (C₃-C₈)cycloalkyl, or 5- to 7-membered heterocycloalkyl comprising 1 to 3 heteroatoms selected from O, N, and S. In another embodiment, Rₓ is H, n is 0, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, and R₃ is (C₆-C₁₀)aryl, (C₃-C₈)cycloalkyl, or 5- to 7-membered heterocycloalkyl comprising 1 to 3 heteroatoms selected from O, N, and S.

In some embodiments of the formulae above, Rₓ is H, n is 0, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, and R₃ is (C₆-C₁₀)aryl, 5- or 6-membered heteroaryl comprising 1 to 3 heteroatoms selected from O, N, and S, or (C₃-C₈)cycloalkyl, wherein the aryl, heteroaryl and cycloalkyl are optionally substituted with one to three R₅. In another embodiment, Rₓ is H, n is 0, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, - (CH₂)₀₋₂NH₂, -(CH₂)₀₋₂NH(C₁-C₆)alkyl, -(CH₂)₀₋₂N((C₁-C₆)alkyl)₂, or CN, and R₃ is (C₆-C₁₀)aryl, 5- or 6-membered heteroaryl comprising 1 to 3 heteroatoms selected from O, N, and S, or (C₃-C₈)cycloalkyl, wherein the aryl, heteroaryl and cycloalkyl are optionally substituted with one to three R₅.

In some embodiments of the formulae above, Rₓ is H, n is 0, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, and R₃ is (C₆-C₁₀)aryl, 5- or 6-membered heteroaryl comprising 1 to 3 heteroatoms selected from O, N, and S, or (C₃-C₈)cycloalkyl. In another embodiment, Rₓ is H, n is 0, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, - OH, -(CH₂)₀₋₂NH₂, -(CH₂)₀₋₂NH(C₁-C₆)alkyl, -(CH₂)₀₋₂N((C₁-C₆)alkyl)₂, or CN, and R₃ is (C₆-C₁₀)aryl, 5-or 6-membered heteroaryl comprising 1 to 3 heteroatoms selected from O, N, and S, or (C₃-C₈)cycloalkyl.

In some embodiments of the formulae above, Rₓ is H, n is 0, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, and R₃ is (C₆-C₁₀)aryl optionally substituted with one to three R₅. In another embodiment, Rₓ is H, n is 0, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, and R₃ is (C₆-C₁₀)aryl optionally substituted with one to three R₅.

In some embodiments of the formulae above, Rₓ is H, n is 0, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, and R₃ is 5- or 6-membered heteroaryl comprising 1 to 3 heteroatoms selected from O, N, and S optionally substituted with one to three R₅. In another embodiment, Rₓ is H, n is 0, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, and R₃ is 5- or 6-membered heteroaryl comprising 1 to 3 heteroatoms selected from O, N, and S optionally substituted with one to three R₅.

In some embodiments of the formulae above, Rₓ is H, n is 0, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, and R₃ is (C₃-C₈)cycloalkyl optionally substituted with one to three R₅. In another embodiment, Rₓ is H, n is 0, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, and R₃ is (C₃-C₈)cycloalkyl optionally substituted with one to three R₅.

In some embodiments of the formulae above, Rₓ is H, n is 0, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, and R₃ is 5- to 7-membered heterocycloalkyl comprising 1 to 3 heteroatoms selected from O, N, and S, optionally substituted with one to three R₅. In another embodiment, Rₓ is H, n is 0, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, and R₃ is 5- to 7-membered heterocycloalkyl comprising 1 to 3 heteroatoms selected from O, N, and S, optionally substituted with one to three R₅.

In some embodiments of the formulae above, Rₓ is H, n is 1, and R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN. In another embodiment, Rₓ is H, n is 1, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, - (CH₂)₀₋₂NH(C₁-C₆)alkyl, -(CH₂)₀₋₂N((C₁-C₆)alkyl)₂, or CN, and each R₂ is independently (C₁-C₆)alkyl. In yet another embodiment, Rₓ is H, n is 1, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, and R₃ is (C₁-C₆)alkyl optionally substituted with one to three R₄. In another embodiment, Rₓ is H, n is 1, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, and R₃ is (C₁-C₆)alkyl substituted with one to three R₄.

In some embodiments of the formulae above, Rₓ is H, n is 1, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, R₃ is (C₁-C₆)alkyl optionally substituted with one to three R₄, and each R₄ is independently selected from -C(O)OR₆, (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇. In another embodiment, Rₓ is H, n is 1, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, R₃ is (C₁-C₆)alkyl substituted with one to three R₄, and each R₄ is independently selected from -C(O)OR₆, (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇.

In some embodiments of the formulae above, Rₓ is H, n is 1, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, R₃ is (C₁-C₆)alkyl optionally substituted with one to three R₄, and each R₄ is independently selected from (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇. In another embodiment, Rₓ is H, n is 1, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, R₃ is (C₁-C₆)alkyl substituted with one to three R₄, and each R₄ is independently selected from (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇.

In some embodiments of the formulae above, Rₓ is H, n is 1, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, R₃ is (C₁-C₆)alkyl optionally substituted with one to three R₄, and each R₄ is independently selected from halogen, -OH, (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇. In another embodiment, Rₓ is H, n is 1, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, R₃ is (C₁-C₆)alkyl substituted with one to three R₄, and each R₄ is independently selected from halogen, -OH, (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇.

In some embodiments of the formulae above, Rₓ is H, n is 1, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, R₃ is (C₁-C₆)alkyl optionally substituted with one to three R₄, and each R₄ is independently selected from halogen, -OH, phenyl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the phenyl, heteroaryl, cycloalkyl, and heterocycloalkyl groups are optionally substituted with one to three R₇. In another embodiment, Rₓ is H, n is 1, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, R₃ is (C₁-C₆)alkyl substituted with one to three R₄, and each R₄ is independently selected from halogen, -OH, phenyl, 5- or 6-membered heteroaryl comprising 1 to 3 heteroatoms selected from O, N, and S, (C₃-C₈)cycloalkyl, and 5-to 7-membered heterocycloalkyl comprising 1 to 3 heteroatoms selected from O, N, and S, wherein the phenyl, heteroaryl, cycloalkyl, and heterocycloalkyl groups are optionally substituted with one to three R₇.

In some embodiments of the formulae above, Rₓ is H, n is 1, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, R₃ is (C₁-C₆)alkyl optionally substituted with one to three R₄, and each R₄ is independently selected from phenyl, 5- or 6-membered heteroaryl comprising 1 to 3 heteroatoms selected from O, N, and S, (C₃-C₈)cycloalkyl, and 5-to 7-membered heterocycloalkyl comprising 1 to 3 heteroatoms selected from O, N, and S, wherein the phenyl, heteroaryl, cycloalkyl, and heterocycloalkyl groups are optionally substituted with one to three R₇. In another embodiment, Rₓ is H, n is 1, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, R₃ is (C₁-C₆)alkyl substituted with one to three R₄, and each R₄ is independently selected from phenyl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the phenyl, heteroaryl, cycloalkyl, and heterocycloalkyl groups are optionally substituted with one to three R₇.

In some embodiments of the formulae above, Rₓ is H, n is 1, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, R₃ is (C₁-C₆)alkyl optionally substituted with one to three R₄, and each R₄ is independently selected from phenyl and 5- or 6-membered heteroaryl comprising 1 to 3 heteroatoms selected from O, N, and S, wherein the phenyl and heteroaryl groups are optionally substituted with one to three R₇. In another embodiment, Rₓ is H, n is 1, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, - OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, R₃ is (C₁-C₆)alkyl substituted with one to three R₄, and each R₄ is independently selected from phenyl and 5- or 6-membered heteroaryl comprising 1 to 3 heteroatoms selected from O, N, and S, wherein the phenyl and heteroaryl groups are optionally substituted with one to three R₇.

In some embodiments of the formulae above, Rₓ is H, n is 1, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, R₃ is (C₁-C₆)alkyl optionally substituted with one to three R₄, and each R₄ is phenyl optionally substituted with one to three R₇. In another embodiment, Rₓ is H, n is 1, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, R₃ is (C₁-C₆)alkyl substituted with one to three R₄, and each R₄ is phenyl optionally substituted with one to three R₇.

In some embodiments of the formulae above, Rₓ is H, n is 1, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, R₃ is (C₁-C₆)alkyl optionally substituted with one to three R₄, and each R₄ is 5- or 6-membered heteroaryl comprising 1 to 3 heteroatoms selected from O, N, and S, optionally substituted with one to three R₇. In another embodiment, Rₓ is H, n is 1, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, R₃ is (C₁-C₆)alkyl substituted with one to three R₄, and each R₄ is 5- or 6-membered heteroaryl comprising 1 to 3 heteroatoms selected from O, N, and S, optionally substituted with one to three R₇.

In some embodiments of the formulae above, Rₓ is H, n is 1, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, R₃ is (C₁-C₆)alkyl optionally substituted with one to three R₄, and each R₄ is (C₃-C₈)cycloalkyl optionally substituted with one to three R₇. In another embodiment, Rₓ is H, n is 1, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, R₃ is (C₁-C₆)alkyl substituted with one to three R₄, and each R₄ is (C₃-C₈)cycloalkyl optionally substituted with one to three R₇.

In some embodiments of the formulae above, Rₓ is H, n is 1, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, R₃ is (C₁-C₆)alkyl optionally substituted with one to three R₄, and each R₄ is 5- to 7-membered heterocycloalkyl comprising 1 to 3 heteroatoms selected from O, N, and S, optionally substituted with one to three R₇. In another embodiment, Rₓ is H, n is 1, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, R₃ is (C₁-C₆)alkyl substituted with one to three R₄, and each R₄ is 5-to 7-membered heterocycloalkyl comprising 1 to 3 heteroatoms selected from O, N, and S, optionally substituted with one to three R₇.

In some embodiments of the formulae above, Rₓ is H, n is 1, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, and R₃ is (C₆-C₁₀)aryl, (C₃-C₈)cycloalkyl, or 5- to 7-membered heterocycloalkyl comprising 1 to 3 heteroatoms selected from O, N, and S, wherein the aryl, cycloalkyl, and heterocycloalkyl are optionally substituted with one to three Rs.

In some embodiments of the formulae above, Rₓ is H, n is 1, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, and R₃ is (C₆-C₁₀)aryl, (C₃-C₈)cycloalkyl, or 5- to 7-membered heterocycloalkyl comprising 1 to 3 heteroatoms selected from O, N, and S.

In some embodiments of the formulae above, Rₓ is H, n is 1, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, and R₃ is (C₆-C₁₀)aryl, 5- or 6-membered heteroaryl comprising 1 to 3 heteroatoms selected from O, N, and S, or (C₃-C₈)cycloalkyl, wherein the aryl, heteroaryl and cycloalkyl are optionally substituted with one to three Rs.

In some embodiments of the formulae above, Rₓ is H, n is 1, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, and R₃ is (C₆-C₁₀)aryl, 5- or 6-membered heteroaryl comprising 1 to 3 heteroatoms selected from O, N, and S, or (C₃-C₈)cycloalkyl.

In some embodiments of the formulae above, Rₓ is H, n is 1, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, and R₃ is (C₆-C₁₀)aryl optionally substituted with one to three Rs.

In some embodiments of the formulae above, Rₓ is H, n is 1, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, and R₃ is 5- or 6-membered heteroaryl comprising 1 to 3 heteroatoms selected from O, N, and S optionally substituted with one to three R₅.

In some embodiments of the formulae above, Rₓ is H, n is 1, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, and R₃ is (C₃-C₈)cycloalkyl optionally substituted with one to three R₅.

In some embodiments of the formulae above, Rₓ is H, n is 1, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, and R₃ is 5- to 7-membered heterocycloalkyl comprising 1 to 3 heteroatoms selected from O, N, and S, optionally substituted with one to three R₅.

In some embodiments of the formulae above, Rₓ is H, n is 0 or 1, and R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, - (CH₂)₀₋₂NH(C₁-C₆)alkyl, -(CH₂)₀₋₂N((C₁-C₆)alkyl)₂, or CN. In another embodiment, Rₓ is H, n is 0 or 1, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, and each R₂ is independently (C₁-C₆)alkyl. In another embodiment, Rₓ is H, n is 0 or 1, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, and R₃ is (C₁-C₆)alkyl optionally substituted with one to three R₄. In another embodiment, Rₓ is H, n is 0 or 1, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, - (CH₂)₀₋₂NH(C₁-C₆)alkyl, -(CH₂)₀₋₂N((C₁-C₆)alkyl)₂, or CN, each R₂ is independently (C₁-C₆)alkyl, and R₃ is (C₁-C₆)alkyl substituted with one to three R₄.

In some embodiments of the formulae above, Rₓ is H, n is 0 or 1, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, R₃ is (C₁-C₆)alkyl optionally substituted with one to three R₄, and each R₄ is independently selected from -C(O)OR₆, (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇. In another embodiment, Rₓ is H, n is 0 or 1, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, R₃ is (C₁-C₆)alkyl substituted with one to three R₄, and each R₄ is independently selected from -C(O)OR₆, (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇.

In some embodiments of the formulae above, Rₓ is H, n is 0 or 1, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, R₃ is (C₁-C₆)alkyl optionally substituted with one to three R₄, and each R₄ is independently selected from (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇. In another embodiment, Rₓ is H, n is 0 or 1, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, R₃ is (C₁-C₆)alkyl substituted with one to three R₄, and each R₄ is independently selected from (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇.

In some embodiments of the formulae above, Rₓ is H, n is 0 or 1, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, R₃ is (C₁-C₆)alkyl optionally substituted with one to three R₄, and each R₄ is independently selected from halogen, -OH, (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇. In another embodiment, Rₓ is H, n is 0 or 1, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, R₃ is (C₁-C₆)alkyl substituted with one to three R₄, and each R₄ is independently selected from halogen, -OH, (C₆-C₁₀)aryl, 5-or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇.

In some embodiments of the formulae above, Rₓ is H, n is 0 or 1, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, R₃ is (C₁-C₆)alkyl optionally substituted with one to three R₄, and each R₄ is independently selected from halogen, -OH, phenyl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the phenyl, heteroaryl, cycloalkyl, and heterocycloalkyl groups are optionally substituted with one to three R₇. In another embodiment, Rₓ is H, n is 0 or 1, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, R₃ is (C₁-C₆)alkyl substituted with one to three R₄, and each R₄ is independently selected from halogen, -OH, phenyl, 5- or 6-membered heteroaryl comprising 1 to 3 heteroatoms selected from O, N, and S, (C₃-C₈)cycloalkyl, and 5-to 7-membered heterocycloalkyl comprising 1 to 3 heteroatoms selected from O, N, and S, wherein the phenyl, heteroaryl, cycloalkyl, and heterocycloalkyl groups are optionally substituted with one to three R₇.

In some embodiments of the formulae above, Rₓ is H, n is 0 or 1, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, R₃ is (C₁-C₆)alkyl optionally substituted with one to three R₄, and each R₄ is independently selected from phenyl, 5- or 6-membered heteroaryl comprising 1 to 3 heteroatoms selected from O, N, and S, (C₃-C₈)cycloalkyl, and 5-to 7-membered heterocycloalkyl comprising 1 to 3 heteroatoms selected from O, N, and S, wherein the phenyl, heteroaryl, cycloalkyl, and heterocycloalkyl groups are optionally substituted with one to three R₇. In another embodiment, Rₓ is H, n is 0 or 1, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, R₃ is (C₁-C₆)alkyl substituted with one to three R₄, and each R₄ is independently selected from phenyl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the phenyl, heteroaryl, cycloalkyl, and heterocycloalkyl groups are optionally substituted with one to three R₇.

In some embodiments of the formulae above, Rₓ is H, n is 0 or 1, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, R₃ is (C₁-C₆)alkyl optionally substituted with one to three R₄, and each R₄ is independently selected from phenyl and 5- or 6-membered heteroaryl comprising 1 to 3 heteroatoms selected from O, N, and S, wherein the phenyl and heteroaryl groups are optionally substituted with one to three R₇. In another embodiment, Rₓ is H, n is 0 or 1, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, - OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, R₃ is (C₁-C₆)alkyl substituted with one to three R₄, and each R₄ is independently selected from phenyl and 5- or 6-membered heteroaryl comprising 1 to 3 heteroatoms selected from O, N, and S, wherein the phenyl and heteroaryl groups are optionally substituted with one to three R₇.

In some embodiments of the formulae above, Rₓ is H, n is 0 or 1, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, R₃ is (C₁-C₆)alkyl optionally substituted with one to three R₄, and each R₄ is phenyl optionally substituted with one to three R₇. In another embodiment, Rₓ is H, n is 0 or 1, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, R₃ is (C₁-C₆)alkyl substituted with one to three R₄, and each R₄ is phenyl optionally substituted with one to three R₇.

In some embodiments of the formulae above, Rₓ is H, n is 0 or 1, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, R₃ is (C₁-C₆)alkyl optionally substituted with one to three R₄, and each R₄ is 5- or 6-membered heteroaryl comprising 1 to 3 heteroatoms selected from O, N, and S, optionally substituted with one to three R₇. In another embodiment, Rₓ is H, n is 0 or 1, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, R₃ is (C₁-C₆)alkyl substituted with one to three R₄, and each R₄ is 5- or 6-membered heteroaryl comprising 1 to 3 heteroatoms selected from O, N, and S, optionally substituted with one to three R₇.

In some embodiments of the formulae above, Rₓ is H, n is 0 or 1, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, R₃ is (C₁-C₆)alkyl optionally substituted with one to three R₄, and each R₄ is (C₃-C₈)cycloalkyl optionally substituted with one to three R₇. In another embodiment, Rₓ is H, n is 0 or 1, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, R₃ is (C₁-C₆)alkyl substituted with one to three R₄, and each R₄ is (C₃-C₈)cycloalkyl optionally substituted with one to three R₇.

In some embodiments of the formulae above, Rₓ is H, n is 0 or 1, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, R₃ is (C₁-C₆)alkyl optionally substituted with one to three R₄, and each R₄ is 5- to 7-membered heterocycloalkyl comprising 1 to 3 heteroatoms selected from O, N, and S, optionally substituted with one to three R₇. In another embodiment, Rₓ is H, n is 0 or 1, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, R₃ is (C₁-C₆)alkyl substituted with one to three R₄, and each R₄ is 5- to 7-membered heterocycloalkyl comprising 1 to 3 heteroatoms selected from O, N, and S, optionally substituted with one to three R₇.

In some embodiments of the formulae above, Rₓ is H, n is 0 or 1, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, and R₃ is (C₆-C₁₀)aryl, (C₃-C₈)cycloalkyl, or 5- to 7-membered heterocycloalkyl comprising 1 to 3 heteroatoms selected from O, N, and S, wherein the aryl, cycloalkyl, and heterocycloalkyl are optionally substituted with one to three Rs. In another embodiment, Rₓ is H, n is 0 or 1, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, and R₃ is (C₆-C₁₀)aryl, (C₃-C₈)cycloalkyl, or 5- to 7-membered heterocycloalkyl comprising 1 to 3 heteroatoms selected from O, N, and S, wherein the aryl, cycloalkyl, and heterocycloalkyl are optionally substituted with one to three R₅.

In some embodiments of the formulae above, Rₓ is H, n is 0 or 1, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, and R₃ is (C₆-C₁₀)aryl, (C₃-C₈)cycloalkyl, or 5- to 7-membered heterocycloalkyl comprising 1 to 3 heteroatoms selected from O, N, and S. In another embodiment, Rₓ is H, n is 0 or 1, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, and R₃ is (C₆-C₁₀)aryl, (C₃-C₈)cycloalkyl, or 5- to 7-membered heterocycloalkyl comprising 1 to 3 heteroatoms selected from O, N, and S.

In some embodiments of the formulae above, Rₓ is H, n is 0 or 1, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, and R₃ is (C₆-C₁₀)aryl, 5- or 6-membered heteroaryl comprising 1 to 3 heteroatoms selected from O, N, and S, or (C₃-C₈)cycloalkyl, wherein the aryl, heteroaryl and cycloalkyl are optionally substituted with one to three Rs. In another embodiment, Rₓ is H, n is 0 or 1, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, and R₃ is (C₆-C₁₀)aryl, 5- or 6-membered heteroaryl comprising 1 to 3 heteroatoms selected from O, N, and S, or (C₃-C₈)cycloalkyl, wherein the aryl, heteroaryl and cycloalkyl are optionally substituted with one to three R₅.

In some embodiments of the formulae above, Rₓ is H, n is 0 or 1, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, and R₃ is (C₆-C₁₀)aryl, 5- or 6-membered heteroaryl comprising 1 to 3 heteroatoms selected from O, N, and S, or (C₃-C₈)cycloalkyl. In another embodiment, Rₓ is H, n is 0 or 1, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, and R₃ is (C₆-C₁₀)aryl, 5- or 6-membered heteroaryl comprising 1 to 3 heteroatoms selected from O, N, and S, or (C₃-C₈)cycloalkyl.

In some embodiments of the formulae above, Rₓ is H, n is 0 or 1, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, and R₃ is (C₆-C₁₀)aryl optionally substituted with one to three Rs. In another embodiment, Rₓ is H, n is 0 or 1, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, - (CH₂)₀₋₂NH(C₁-C₆)alkyl, -(CH₂)₀₋₂N((C₁-C₆)alkyl)₂, or CN, each R₂ is independently (C₁-C₆)alkyl, and R₃ is (C₆-C₁₀)aryl optionally substituted with one to three R₅.

In some embodiments of the formulae above, Rₓ is H, n is 0 or 1, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, and R₃ is 5- or 6-membered heteroaryl comprising 1 to 3 heteroatoms selected from O, N, and S optionally substituted with one to three Rs. In another embodiment, Rₓ is H, n is 0 or 1, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, and R₃ is 5- or 6-membered heteroaryl comprising 1 to 3 heteroatoms selected from O, N, and S optionally substituted with one to three R₅.

In some embodiments of the formulae above, Rₓ is H, n is 0 or 1, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, and R₃ is (C₃-C₈)cycloalkyl optionally substituted with one to three Rs. In another embodiment, Rₓ is H, n is 0 or 1, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, - (CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, and R₃ is (C₃-C₈)cycloalkyl optionally substituted with one to three Rs.

In some embodiments of the formulae above, Rₓ is H, n is 0 or 1, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, and R₃ is 5- to 7-membered heterocycloalkyl comprising 1 to 3 heteroatoms selected from O, N, and S, optionally substituted with one to three R₅. In another embodiment, Rₓ is H, n is 0 or 1, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, and R₃ is 5- to 7-membered heterocycloalkyl comprising 1 to 3 heteroatoms selected from O, N, and S, optionally substituted with one to three R₅.

In some embodiments of the formulae above, Rₓ is H, n is 0, 1, or 2, and R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, - (CH₂)₀₋₂NH(C₁-C₆)alkyl, -(CH₂)₀₋₂N((C₁-C₆)alkyl)₂, or CN. In another embodiment, Rₓ is H, n is 0, 1, or 2, and R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, and each R₂ is independently (C₁-C₆)alkyl. In another embodiment, Rₓ is H, n is 0, 1, or 2, and R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, - (CH₂)₀₋₂NH(C₁-C₆)alkyl, -(CH₂)₀₋₂N((C₁-C₆)alkyl)₂, or CN, each R₂ is independently (C₁-C₆)alkyl, and R₃ is (C₁-C₆)alkyl optionally substituted with one to three R₄. In another embodiment, Rₓ is H, n is 0, 1, or 2, and R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, and R₃ is (C₁-C₆)alkyl substituted with one to three R₄.

In some embodiments of the formulae above, Rₓ is H, n is 0, 1, or 2, and R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, - (CH₂)₀₋₂NH(C₁-C₆)alkyl, -(CH₂)₀₋₂N((C₁-C₆)alkyl)₂, or CN, each R₂ is independently (C₁-C₆)alkyl, R₃ is (C₁-C₆)alkyl optionally substituted with one to three R₄, and each R₄ is independently selected from - C(O)OR₆, (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇. In another embodiment, Rₓ is H, n is 0, 1, or 2, and R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂,-(CH₂)₀₋₂NH(C₁-C₆)alkyl, -(CH₂)₀₋₂N((C₁-C₆)alkyl)₂, or CN, each R₂ is independently (C₁-C₆)alkyl, R₃ is (C₁-C₆)alkyl substituted with one to three R₄, and each R₄ is independently selected from -C(O)OR₆, (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇.

In some embodiments of the formulae above, Rₓ is H, n is 0, 1, or 2, and R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, - (CH₂)₀₋₂NH(C₁-C₆)alkyl, -(CH₂)₀₋₂N((C₁-C₆)alkyl)₂, or CN, each R₂ is independently (C₁-C₆)alkyl, R₃ is (C₁-C₆)alkyl optionally substituted with one to three R₄, and each R₄ is independently selected from (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇. In another embodiment, Rₓ is H, n is 0, 1, or 2, and R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, R₃ is (C₁-C₆)alkyl substituted with one to three R₄, and each R₄ is independently selected from (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇.

In some embodiments of the formulae above, Rₓ is H, n is 0, 1, or 2, and R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, - (CH₂)₀₋₂NH(C₁-C₆)alkyl, -(CH₂)₀₋₂N((C₁-C₆)alkyl)₂, or CN, each R₂ is independently (C₁-C₆)alkyl, R₃ is (C₁-C₆)alkyl optionally substituted with one to three R₄, and each R₄ is independently selected from halogen, -OH, (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇. In another embodiment, Rₓ is H, n is 0, 1, or 2, and R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, R₃ is (C₁-C₆)alkyl substituted with one to three R₄, and each R₄ is independently selected from halogen, -OH, (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇.

In some embodiments of the formulae above, Rₓ is H, n is 0, 1, or 2, and R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, - (CH₂)₀₋₂NH(C₁-C₆)alkyl, -(CH₂)₀₋₂N((C₁-C₆)alkyl)₂, or CN, each R₂ is independently (C₁-C₆)alkyl, R₃ is (C₁-C₆)alkyl optionally substituted with one to three R₄, and each R₄ is independently selected from halogen, -OH, phenyl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the phenyl, heteroaryl, cycloalkyl, and heterocycloalkyl groups are optionally substituted with one to three R₇. In another embodiment, Rₓ is H, n is 0, 1, or 2, and R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, R₃ is (C₁-C₆)alkyl substituted with one to three R₄, and each R₄ is independently selected from halogen, -OH, phenyl, 5- or 6-membered heteroaryl comprising 1 to 3 heteroatoms selected from O, N, and S, (C₃-C₈)cycloalkyl, and 5-to 7-membered heterocycloalkyl comprising 1 to 3 heteroatoms selected from O, N, and S, wherein the phenyl, heteroaryl, cycloalkyl, and heterocycloalkyl groups are optionally substituted with one to three R₇.

In some embodiments of the formulae above, Rₓ is H, n is 0, 1, or 2, and R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, - (CH₂)₀₋₂NH(C₁-C₆)alkyl, -(CH₂)₀₋₂N((C₁-C₆)alkyl)₂, or CN, each R₂ is independently (C₁-C₆)alkyl, R₃ is (C₁-C₆)alkyl optionally substituted with one to three R₄, and each R₄ is independently selected from phenyl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the phenyl, heteroaryl, cycloalkyl, and heterocycloalkyl groups are optionally substituted with one to three R₇. In another embodiment, Rₓ is H, n is 0, 1, or 2, and R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, R₃ is (C₁-C₆)alkyl substituted with one to three R₄, and each R₄ is independently selected from phenyl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the phenyl, heteroaryl, cycloalkyl, and heterocycloalkyl groups are optionally substituted with one to three R₇.

In some embodiments of the formulae above, Rₓ is H, n is 0, 1, or 2, and R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, - (CH₂)₀₋₂NH(C₁-C₆)alkyl, -(CH₂)₀₋₂N((C₁-C₆)alkyl)₂, or CN, each R₂ is independently (C₁-C₆)alkyl, R₃ is (C₁-C₆)alkyl optionally substituted with one to three R₄, and each R₄ is independently selected from phenyl and 5- or 6-membered heteroaryl comprising 1 to 3 heteroatoms selected from O, N, and S, wherein the phenyl and heteroaryl groups are optionally substituted with one to three R₇. In another embodiment, Rₓ is H, n is 0, 1, or 2, and R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, R₃ is (C₁-C₆)alkyl substituted with one to three R₄, and each R₄ is independently selected from phenyl and 5- or 6-membered heteroaryl comprising 1 to 3 heteroatoms selected from O, N, and S, wherein the phenyl and heteroaryl groups are optionally substituted with one to three R₇.

In some embodiments of the formulae above, Rₓ is H, n is 0, 1, or 2, and R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, - (CH₂)₀₋₂NH(C₁-C₆)alkyl, -(CH₂)₀₋₂N((C₁-C₆)alkyl)₂, or CN, each R₂ is independently (C₁-C₆)alkyl, R₃ is (C₁-C₆)alkyl optionally substituted with one to three R₄, and each R₄ is phenyl optionally substituted with one to three R₇. In another embodiment, Rₓ is H, n is 0, 1, or 2, and R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, R₃ is (C₁-C₆)alkyl substituted with one to three R₄, and each R₄ is phenyl optionally substituted with one to three R₇.

In some embodiments of the formulae above, Rₓ is H, n is 0, 1, or 2, and R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, - (CH₂)₀₋₂NH(C₁-C₆)alkyl, -(CH₂)₀₋₂N((C₁-C₆)alkyl)₂, or CN, each R₂ is independently (C₁-C₆)alkyl, R₃ is (C₁-C₆)alkyl optionally substituted with one to three R₄, and each R₄ is 5- or 6-membered heteroaryl comprising 1 to 3 heteroatoms selected from O, N, and S, optionally substituted with one to three R₇. In another embodiment, Rₓ is H, n is 0, 1, or 2, and R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, R₃ is (C₁-C₆)alkyl substituted with one to three R₄, and each R₄ is 5- or 6-membered heteroaryl comprising 1 to 3 heteroatoms selected from O, N, and S, optionally substituted with one to three R₇.

In some embodiments of the formulae above, Rₓ is H, n is 0, 1, or 2, and R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, - (CH₂)₀₋₂NH(C₁-C₆)alkyl, -(CH₂)₀₋₂N((C₁-C₆)alkyl)₂, or CN, each R₂ is independently (C₁-C₆)alkyl, R₃ is (C₁-C₆)alkyl optionally substituted with one to three R₄, and each R₄ is (C₃-C₈)cycloalkyl optionally substituted with one to three R₇. In another embodiment, Rₓ is H, n is 0, 1, or 2, and R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, - (CH₂)₀₋₂NH(C₁-C₆)alkyl, -(CH₂)₀₋₂N((C₁-C₆)alkyl)₂, or CN, each R₂ is independently (C₁-C₆)alkyl, R₃ is (C₁-C₆)alkyl substituted with one to three R₄, and each R₄ is (C₃-C₈)cycloalkyl optionally substituted with one to three R₇.

In some embodiments of the formulae above, Rₓ is H, n is 0, 1, or 2, and R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, - (CH₂)₀₋₂NH(C₁-C₆)alkyl, -(CH₂)₀₋₂N((C₁-C₆)alkyl)₂, or CN, each R₂ is independently (C₁-C₆)alkyl, R₃ is (C₁-C₆)alkyl optionally substituted with one to three R₄, and each R₄ is 5- to 7-membered heterocycloalkyl comprising 1 to 3 heteroatoms selected from O, N, and S, optionally substituted with one to three R₇. In another embodiment, Rₓ is H, n is 0, 1, or 2, and R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, R₃ is (C₁-C₆)alkyl substituted with one to three R₄, and each R₄ is 5- to 7-membered heterocycloalkyl comprising 1 to 3 heteroatoms selected from O, N, and S, optionally substituted with one to three R₇.

In some embodiments of the formulae above, Rₓ is H, n is 0, 1, or 2, and R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, - (CH₂)₀₋₂NH(C₁-C₆)alkyl, -(CH₂)₀₋₂N((C₁-C₆)alkyl)₂, or CN, each R₂ is independently (C₁-C₆)alkyl, and R₃ is (C₆-C₁₀)aryl, (C₃-C₈)cycloalkyl, or 5- to 7-membered heterocycloalkyl comprising 1 to 3 heteroatoms selected from O, N, and S, wherein the aryl, cycloalkyl, and heterocycloalkyl are optionally substituted with one to three R₅.

In some embodiments of the formulae above, Rₓ is H, n is 0, 1, or 2, and R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, - (CH₂)₀₋₂NH(C₁-C₆)alkyl, -(CH₂)₀₋₂N((C₁-C₆)alkyl)₂, or CN, each R₂ is independently (C₁-C₆)alkyl, and R₃ is (C₆-C₁₀)aryl, (C₃-C₈)cycloalkyl, or 5- to 7-membered heterocycloalkyl comprising 1 to 3 heteroatoms selected from O, N, and S.

In some embodiments of the formulae above, Rₓ is H, n is 0, 1, or 2, and R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, - (CH₂)₀₋₂NH(C₁-C₆)alkyl, -(CH₂)₀₋₂N((C₁-C₆)alkyl)₂, or CN, each R₂ is independently (C₁-C₆)alkyl, and R₃ is (C₆-C₁₀)aryl, 5- or 6-membered heteroaryl comprising 1 to 3 heteroatoms selected from O, N, and S, or (C₃-C₈)cycloalkyl, wherein the aryl, heteroaryl and cycloalkyl are optionally substituted with one to three R₅.

In some embodiments of the formulae above, Rₓ is H, n is 0, 1, or 2, and R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, - (CH₂)₀₋₂NH(C₁-C₆)alkyl, -(CH₂)₀₋₂N((C₁-C₆)alkyl)₂, or CN, each R₂ is independently (C₁-C₆)alkyl, and R₃ is (C₆-C₁₀)aryl, 5- or 6-membered heteroaryl comprising 1 to 3 heteroatoms selected from O, N, and S, or (C₃-C₈)cycloalkyl.

In some embodiments of the formulae above, Rₓ is H, n is 0, 1, or 2, and R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, - (CH₂)₀₋₂NH(C₁-C₆)alkyl, -(CH₂)₀₋₂N((C₁-C₆)alkyl)₂, or CN, each R₂ is independently (C₁-C₆)alkyl, and R₃ is (C₆-C₁₀)aryl optionally substituted with one to three R₅.

In some embodiments of the formulae above, Rₓ is H, n is 0, 1, or 2, and R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, - (CH₂)₀₋₂NH(C₁-C₆)alkyl, -(CH₂)₀₋₂N((C₁-C₆)alkyl)₂, or CN, each R₂ is independently (C₁-C₆)alkyl, and R₃ is 5- or 6-membered heteroaryl comprising 1 to 3 heteroatoms selected from O, N, and S optionally substituted with one to three R₅.

In some embodiments of the formulae above, Rₓ is H, n is 0, 1, or 2, and R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, - (CH₂)₀₋₂NH(C₁-C₆)alkyl, -(CH₂)₀₋₂N((C₁-C₆)alkyl)₂, or CN, each R₂ is independently (C₁-C₆)alkyl, and R₃ is (C₃-C₈)cycloalkyl optionally substituted with one to three R₅.

In some embodiments of the formulae above, Rₓ is H, n is 0, 1, or 2, and R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, - (CH₂)₀₋₂NH(C₁-C₆)alkyl, -(CH₂)₀₋₂N((C₁-C₆)alkyl)₂, or CN, each R₂ is independently (C₁-C₆)alkyl, and R₃ is 5- to 7-membered heterocycloalkyl comprising 1 to 3 heteroatoms selected from O, N, and S, optionally substituted with one to three R₅.

In some embodiments of the formulae above, Rₓ is H, n is 1 or 2, and R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, - (CH₂)₀₋₂NH(C₁-C₆)alkyl, -(CH₂)₀₋₂N((C₁-C₆)alkyl)₂, or CN. In another embodiment, Rₓ is H, n is 1 or 2, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, and each R₂ is independently (C₁-C₆)alkyl. In another embodiment, Rₓ is H, n is 1 or 2, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, and R₃ is (C₁-C₆)alkyl optionally substituted with one to three R₄. In yet another embodiment, Rₓ is H, n is 1 or 2, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, - (CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, and R₃ is (C₁-C₆)alkyl substituted with one to three R₄.

In some embodiments of the formulae above, Rₓ is H, n is 1 or 2, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, R₃ is (C₁-C₆)alkyl optionally substituted with one to three R₄, and each R₄ is independently selected from -C(O)OR₆, (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇. In another embodiment, Rₓ is H, n is 1 or 2, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, R₃ is (C₁-C₆)alkyl substituted with one to three R₄, and each R₄ is independently selected from -C(O)OR₆, (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇.

In some embodiments of the formulae above, Rₓ is H, n is 1 or 2, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, R₃ is (C₁-C₆)alkyl optionally substituted with one to three R₄, and each R₄ is independently selected from (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇. In another embodiment, Rₓ is H, n is 1 or 2, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, R₃ is (C₁-C₆)alkyl substituted with one to three R₄, and each R₄ is independently selected from (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇.

In some embodiments of the formulae above, Rₓ is H, n is 1 or 2, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, R₃ is (C₁-C₆)alkyl optionally substituted with one to three R₄, and each R₄ is independently selected from halogen, -OH, (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇. In another embodiment, Rₓ is H, n is 1 or 2, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, R₃ is (C₁-C₆)alkyl substituted with one to three R₄, and each R₄ is independently selected from halogen, -OH, (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇.

In some embodiments of the formulae above, Rₓ is H, n is 1 or 2, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, R₃ is (C₁-C₆)alkyl optionally substituted with one to three R₄, and each R₄ is independently selected from halogen, -OH, phenyl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the phenyl, heteroaryl, cycloalkyl, and heterocycloalkyl groups are optionally substituted with one to three R₇. In another embodiment, Rₓ is H, n is 1 or 2, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, R₃ is (C₁-C₆)alkyl substituted with one to three R₄, and each R₄ is independently selected from halogen, -OH, phenyl, 5- or 6-membered heteroaryl comprising 1 to 3 heteroatoms selected from O, N, and S, (C₃-C₈)cycloalkyl, and 5-to 7-membered heterocycloalkyl comprising 1 to 3 heteroatoms selected from O, N, and S, wherein the phenyl, heteroaryl, cycloalkyl, and heterocycloalkyl groups are optionally substituted with one to three R₇.

In some embodiments of the formulae above, Rₓ is H, n is 1 or 2, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, R₃ is (C₁-C₆)alkyl optionally substituted with one to three R₄, and each R₄ is independently selected from phenyl, 5- or 6-membered heteroaryl comprising 1 to 3 heteroatoms selected from O, N, and S, (C₃-C₈)cycloalkyl, and 5-to 7-membered heterocycloalkyl comprising 1 to 3 heteroatoms selected from O, N, and S, wherein the phenyl, heteroaryl, cycloalkyl, and heterocycloalkyl groups are optionally substituted with one to three R₇. In another embodiment, Rₓ is H, n is 1 or 2, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, R₃ is (C₁-C₆)alkyl substituted with one to three R₄, and each R₄ is independently selected from phenyl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the phenyl, heteroaryl, cycloalkyl, and heterocycloalkyl groups are optionally substituted with one to three R₇.

In some embodiments of the formulae above, Rₓ is H, n is 1 or 2, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, R₃ is (C₁-C₆)alkyl optionally substituted with one to three R₄, and each R₄ is independently selected from phenyl and 5- or 6-membered heteroaryl comprising 1 to 3 heteroatoms selected from O, N, and S, wherein the phenyl and heteroaryl groups are optionally substituted with one to three R₇. In another embodiment, Rₓ is H, n is 1 or 2, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, - OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, R₃ is (C₁-C₆)alkyl substituted with one to three R₄, and each R₄ is independently selected from phenyl and 5- or 6-membered heteroaryl comprising 1 to 3 heteroatoms selected from O, N, and S, wherein the phenyl and heteroaryl groups are optionally substituted with one to three R₇.

In some embodiments of the formulae above, Rₓ is H, n is 1 or 2, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, R₃ is (C₁-C₆)alkyl optionally substituted with one to three R₄, and each R₄ is phenyl optionally substituted with one to three R₇. In another embodiment, Rₓ is H, n is 1 or 2, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, R₃ is (C₁-C₆)alkyl substituted with one to three R₄, and each R₄ is phenyl optionally substituted with one to three R₇.

In some embodiments of the formulae above, Rₓ is H, n is 1 or 2, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, R₃ is (C₁-C₆)alkyl optionally substituted with one to three R₄, and each R₄ is 5- or 6-membered heteroaryl comprising 1 to 3 heteroatoms selected from O, N, and S, optionally substituted with one to three R₇. In another embodiment, Rₓ is H, n is 1 or 2, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, R₃ is (C₁-C₆)alkyl substituted with one to three R₄, and each R₄ is 5- or 6-membered heteroaryl comprising 1 to 3 heteroatoms selected from O, N, and S, optionally substituted with one to three R₇.

In some embodiments of the formulae above, Rₓ is H, n is 1 or 2, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, R₃ is (C₁-C₆)alkyl optionally substituted with one to three R₄, and each R₄ is (C₃-C₈)cycloalkyl optionally substituted with one to three R₇. In another embodiment, Rₓ is H, n is 1 or 2, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, R₃ is (C₁-C₆)alkyl substituted with one to three R₄, and each R₄ is (C₃-C₈)cycloalkyl optionally substituted with one to three R₇.

In some embodiments of the formulae above, Rₓ is H, n is 1 or 2, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, R₃ is (C₁-C₆)alkyl optionally substituted with one to three R₄, and each R₄ is 5- to 7-membered heterocycloalkyl comprising 1 to 3 heteroatoms selected from O, N, and S, optionally substituted with one to three R₇. In another embodiment, Rₓ is H, n is 1 or 2, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, R₃ is (C₁-C₆)alkyl substituted with one to three R₄, and each R₄ is 5- to 7-membered heterocycloalkyl comprising 1 to 3 heteroatoms selected from O, N, and S, optionally substituted with one to three R₇.

In some embodiments of the formulae above, Rₓ is H, n is 1 or 2, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, and R₃ is (C₆-C₁₀)aryl, (C₃-C₈)cycloalkyl, or 5- to 7-membered heterocycloalkyl comprising 1 to 3 heteroatoms selected from O, N, and S, wherein the aryl, cycloalkyl, and heterocycloalkyl are optionally substituted with one to three R₅.

In some embodiments of the formulae above, Rₓ is H, n is 1 or 2, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, and R₃ is (C₆-C₁₀)aryl, (C₃-C₈)cycloalkyl, or 5- to 7-membered heterocycloalkyl comprising 1 to 3 heteroatoms selected from O, N, and S.

In some embodiments of the formulae above, Rₓ is H, n is 1 or 2, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, and R₃ is (C₆-C₁₀)aryl, 5- or 6-membered heteroaryl comprising 1 to 3 heteroatoms selected from O, N, and S, or (C₃-C₈)cycloalkyl, wherein the aryl, heteroaryl and cycloalkyl are optionally substituted with one to three R₅.

In some embodiments of the formulae above, Rₓ is H, n is 1 or 2, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, and R₃ is (C₆-C₁₀)aryl, 5- or 6-membered heteroaryl comprising 1 to 3 heteroatoms selected from O, N, and S, or (C₃-C₈)cycloalkyl.

In some embodiments of the formulae above, Rₓ is H, n is 1 or 2, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, and R₃ is (C₆-C₁₀)aryl optionally substituted with one to three R₅.

In some embodiments of the formulae above, Rₓ is H, n is 1 or 2, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, and R₃ is 5- or 6-membered heteroaryl comprising 1 to 3 heteroatoms selected from O, N, and S optionally substituted with one to three R₅.

In some embodiments of the formulae above, Rₓ is H, n is 1 or 2, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, and R₃ is (C₃-C₈)cycloalkyl optionally substituted with one to three R₅.

In some embodiments of the formulae above, Rₓ is H, n is 1 or 2, R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, and R₃ is 5- to 7-membered heterocycloalkyl comprising 1 to 3 heteroatoms selected from O, N, and S, optionally substituted with one to three R₅.

In some embodiments of the formulae above, Rₓ is H, n is 2, and R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN. In another embodiment, Rₓ is H, n is 2, and R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, - (CH₂)₀₋₂NH(C₁-C₆)alkyl, -(CH₂)₀₋₂N((C₁-C₆)alkyl)₂, or CN, and each R₂ is independently (C₁-C₆)alkyl. In another embodiment, Rₓ is H, n is 2, and R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl. and R₃ is (C₁-C₆)alkyl optionally substituted with one to three R₄. In yet another embodiment, Rₓ is H, n is 2, and R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, - (CH₂)₀₋₂NH(C₁-C₆)alkyl, -(CH₂)₀₋₂N((C₁-C₆)alkyl)₂, or CN, each R₂ is independently (C₁-C₆)alkyl, and R₃ is (C₁-C₆)alkyl substituted with one to three R₄.

In some embodiments of the formulae above, Rₓ is H, n is 2, and R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl. R₃ is (C₁-C₆)alkyl optionally substituted with one to three R₄, and each R₄ is independently selected from -C(O)OR₆, (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇. In another embodiment, Rₓ is H, n is 2, and R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl. R₃ is (C₁-C₆)alkyl substituted with one to three R₄, and each R₄ is independently selected from -C(O)OR₆, (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇.

In some embodiments of the formulae above, Rₓ is H, n is 2, and R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl. R₃ is (C₁-C₆)alkyl optionally substituted with one to three R₄, and each R₄ is independently selected from (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇. In another embodiment, Rₓ is H, n is 2, and R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl. R₃ is (C₁-C₆)alkyl substituted with one to three R₄, and each R₄ is independently selected from (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇.

In some embodiments of the formulae above, Rₓ is H, n is 2, and R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl. R₃ is (C₁-C₆)alkyl optionally substituted with one to three R₄, and each R₄ is independently selected from halogen, -OH, (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇. In another embodiment, Rₓ is H, n is 2, and R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl. R₃ is (C₁-C₆)alkyl substituted with one to three R₄, and each R₄ is independently selected from halogen, -OH, (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇.

In some embodiments of the formulae above, Rₓ is H, n is 2, and R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl. R₃ is (C₁-C₆)alkyl optionally substituted with one to three R₄, and each R₄ is independently selected from halogen, -OH, phenyl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the phenyl, heteroaryl, cycloalkyl, and heterocycloalkyl groups are optionally substituted with one to three R₇. In another embodiment, Rₓ is H, n is 2, and R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl. R₃ is (C₁-C₆)alkyl substituted with one to three R₄, and each R₄ is independently selected from halogen, -OH, phenyl, 5- or 6-membered heteroaryl comprising 1 to 3 heteroatoms selected from O, N, and S, (C₃-C₈)cycloalkyl, and 5-to 7-membered heterocycloalkyl comprising 1 to 3 heteroatoms selected from O, N, and S, wherein the phenyl, heteroaryl, cycloalkyl, and heterocycloalkyl groups are optionally substituted with one to three R₇.

In some embodiments of the formulae above, Rₓ is H, n is 2, and R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl. R₃ is (C₁-C₆)alkyl optionally substituted with one to three R₄, and each R₄ is independently selected from phenyl, 5- or 6-membered heteroaryl comprising 1 to 3 heteroatoms selected from O, N, and S, (C₃-C₈)cycloalkyl, and 5-to 7-membered heterocycloalkyl comprising 1 to 3 heteroatoms selected from O, N, and S, wherein the phenyl, heteroaryl, cycloalkyl, and heterocycloalkyl groups are optionally substituted with one to three R₇. In another embodiment, Rₓ is H, n is 2, and R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl. R₃ is (C₁-C₆)alkyl substituted with one to three R₄, and each R₄ is independently selected from phenyl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the phenyl, heteroaryl, cycloalkyl, and heterocycloalkyl groups are optionally substituted with one to three R₇.

In some embodiments of the formulae above, Rₓ is H, n is 2, and R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl. R₃ is (C₁-C₆)alkyl optionally substituted with one to three R₄, and each R₄ is independently selected from phenyl and 5- or 6-membered heteroaryl comprising 1 to 3 heteroatoms selected from O, N, and S, wherein the phenyl and heteroaryl groups are optionally substituted with one to three R₇. In another embodiment, Rₓ is H, n is 2, and R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl. R₃ is (C₁-C₆)alkyl substituted with one to three R₄, and each R₄ is independently selected from phenyl and 5- or 6-membered heteroaryl comprising 1 to 3 heteroatoms selected from O, N, and S, wherein the phenyl and heteroaryl groups are optionally substituted with one to three R₇.

In some embodiments of the formulae above, Rₓ is H, n is 2, and R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl. R₃ is (C₁-C₆)alkyl optionally substituted with one to three R₄, and each R₄ is phenyl optionally substituted with one to three R₇. In another embodiment, Rₓ is H, n is 2, and R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl. R₃ is (C₁-C₆)alkyl substituted with one to three R₄, and each R₄ is phenyl optionally substituted with one to three R₇.

In some embodiments of the formulae above, Rₓ is H, n is 2, and R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl. R₃ is (C₁-C₆)alkyl optionally substituted with one to three R₄, and each R₄ is 5- or 6-membered heteroaryl comprising 1 to 3 heteroatoms selected from O, N, and S, optionally substituted with one to three R₇. In another embodiment, Rₓ is H, n is 2, and R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl. R₃ is (C₁-C₆)alkyl substituted with one to three R₄, and each R₄ is 5-or 6-membered heteroaryl comprising 1 to 3 heteroatoms selected from O, N, and S, optionally substituted with one to three R₇.

In some embodiments of the formulae above, Rₓ is H, n is 2, and R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl. R₃ is (C₁-C₆)alkyl optionally substituted with one to three R₄, and each R₄ is (C₃-C₈)cycloalkyl optionally substituted with one to three R₇. In another embodiment, Rₓ is H, n is 2, and R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl. R₃ is (C₁-C₆)alkyl substituted with one to three R₄, and each R₄ is (C₃-C₈)cycloalkyl optionally substituted with one to three R₇.

In some embodiments of the formulae above, Rₓ is H, n is 2, and R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl. R₃ is (C₁-C₆)alkyl optionally substituted with one to three R₄, and each R₄ is 5- to 7-membered heterocycloalkyl comprising 1 to 3 heteroatoms selected from O, N, and S, optionally substituted with one to three R₇. In another embodiment, Rₓ is H, n is 2, and R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl. R₃ is (C₁-C₆)alkyl substituted with one to three R₄, and each R₄ is 5- to 7-membered heterocycloalkyl comprising 1 to 3 heteroatoms selected from O, N, and S, optionally substituted with one to three R₇.

In some embodiments of the formulae above, Rₓ is H, n is 2, and R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl. and R₃ is (C₆-C₁₀)aryl, (C₃-C₈)cycloalkyl, or 5- to 7-membered heterocycloalkyl comprising 1 to 3 heteroatoms selected from O, N, and S, wherein the aryl, cycloalkyl, and heterocycloalkyl are optionally substituted with one to three R₅.

In some embodiments of the formulae above, Rₓ is H, n is 2, and R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl. and R₃ is (C₆-C₁₀)aryl, (C₃-C₈)cycloalkyl, or 5- to 7-membered heterocycloalkyl comprising 1 to 3 heteroatoms selected from O, N, and S.

In some embodiments of the formulae above, Rₓ is H, n is 2, and R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, and R₃ is (C₆-C₁₀)aryl, 5- or 6-membered heteroaryl comprising 1 to 3 heteroatoms selected from O, N, and S, or (C₃-C₈)cycloalkyl, wherein the aryl, heteroaryl and cycloalkyl are optionally substituted with one to three R₅.

In some embodiments of the formulae above, Rₓ is H, n is 2, and R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, and R₃ is (C₆-C₁₀)aryl, 5- or 6-membered heteroaryl comprising 1 to 3 heteroatoms selected from O, N, and S, or (C₃-C₈)cycloalkyl.

In some embodiments of the formulae above, Rₓ is H, n is 2, and R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, and R₃ is (C₆-C₁₀)aryl optionally substituted with one to three R₅.

In some embodiments of the formulae above, Rₓ is H, n is 2, and R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, and R₃ is 5- or 6-membered heteroaryl comprising 1 to 3 heteroatoms selected from O, N, and S optionally substituted with one to three R₅.

In some embodiments of the formulae above, Rₓ is H, n is 2, and R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, and R₃ is (C₃-C₈)cycloalkyl optionally substituted with one to three R₅.

In some embodiments of the formulae above, Rₓ is H, n is 2, and R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, each R₂ is independently (C₁-C₆)alkyl, and R₃ is 5- to 7-membered heterocycloalkyl comprising 1 to 3 heteroatoms selected from O, N, and S, optionally substituted with one to three R₅.

In some embodiments of the formulae above, Rₓ is H, n is 0, and R₁ is (C₁-C₆)alkoxy, halogen, - OH, -(CH₂)₀₋₂NH₂, -(CH₂)₀₋₂NH(C₁-C₆)alkyl, -(CH₂)₀₋₂N((C₁-C₆)alkyl)₂, or CN. In another embodiment, Rₓ is H, n is 0, R₁ is (C₁-C₆)alkoxy, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, and R₃ is (C₁-C₆)alkyl optionally substituted with one to three R₄. In another embodiment, Rₓ is H, n is 0, R₁ is (C₁-C₆)alkoxy, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, and R₃ is (C₁-C₆)alkyl substituted with one to three R₄.

In some embodiments of the formulae above, Rₓ is H, n is 0, R₁ is (C₁-C₆)alkoxy, halogen, -OH, - (CH₂)₀₋₂NH₂, -(CH₂)₀₋₂NH(C₁-C₆)alkyl, -(CH₂)₀₋₂N((C₁-C₆)alkyl)₂, or CN, R₃ is (C₁-C₆)alkyl optionally substituted with one to three R₄, and each R₄ is independently selected from -C(O)OR₆, (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇. In another embodiment, Rₓ is H, n is 0, R₁ is (C₁-C₆)alkoxy, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, R₃ is (C₁-C₆)alkyl substituted with one to three R₄, and each R₄ is independently selected from -C(O)OR₆, (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇.

In some embodiments of the formulae above, Rₓ is H, n is 0, R₁ is (C₁-C₆)alkoxy, halogen, -OH, - (CH₂)₀₋₂NH₂, -(CH₂)₀₋₂NH(C₁-C₆)alkyl, -(CH₂)₀₋₂N((C₁-C₆)alkyl)₂, or CN, R₃ is (C₁-C₆)alkyl optionally substituted with one to three R₄, and each R₄ is independently selected from (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇. In another embodiment, Rₓ is H, n is 0, R₁ is (C₁-C₆)alkoxy, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, R₃ is (C₁-C₆)alkyl substituted with one to three R₄, and each R₄ is independently selected from (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇.

In some embodiments of the formulae above, Rₓ is H, n is 0, R₁ is (C₁-C₆)alkoxy, halogen, -OH, - (CH₂)₀₋₂NH₂, -(CH₂)₀₋₂NH(C₁-C₆)alkyl, -(CH₂)₀₋₂N((C₁-C₆)alkyl)₂, or CN, R₃ is (C₁-C₆)alkyl optionally substituted with one to three R₄, and each R₄ is independently selected from halogen, -OH, (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇. In another embodiment, Rₓ is H, n is 0, R₁ is (C₁-C₆)alkoxy, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, R₃ is (C₁-C₆)alkyl substituted with one to three R₄, and each R₄ is independently selected from halogen, -OH, (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇.

In some embodiments of the formulae above, Rₓ is H, n is 0, R₁ is (C₁-C₆)alkoxy, halogen, -OH, - (CH₂)₀₋₂NH₂, -(CH₂)₀₋₂NH(C₁-C₆)alkyl, -(CH₂)₀₋₂N((C₁-C₆)alkyl)₂, or CN, R₃ is (C₁-C₆)alkyl optionally substituted with one to three R₄, and each R₄ is independently selected from halogen, -OH, phenyl, 5- or 6-membered heteroaryl comprising 1 to 3 heteroatoms selected from O, N, and S, (C₃-C₈)cycloalkyl, and 5-to 7-membered heterocycloalkyl comprising 1 to 3 heteroatoms selected from O, N, and S, wherein the phenyl, heteroaryl, cycloalkyl, and heterocycloalkyl groups are optionally substituted with one to three R₇. In another embodiment, Rₓ is H, n is 0, R₁ is (C₁-C₆)alkoxy, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, R₃ is (C₁-C₆)alkyl substituted with one to three R₄, and each R₄ is independently selected from halogen, -OH, phenyl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the phenyl, heteroaryl, cycloalkyl, and heterocycloalkyl groups are optionally substituted with one to three R₇.

In some embodiments of the formulae above, Rₓ is H, n is 0, R₁ is (C₁-C₆)alkoxy, halogen, -OH, - (CH₂)₀₋₂NH₂, -(CH₂)₀₋₂NH(C₁-C₆)alkyl, -(CH₂)₀₋₂N((C₁-C₆)alkyl)₂, or CN, R₃ is (C₁-C₆)alkyl optionally substituted with one to three R₄, and each R₄ is independently selected from phenyl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the phenyl, heteroaryl, cycloalkyl, and heterocycloalkyl groups are optionally substituted with one to three R₇. In another embodiment, Rₓ is H, n is 0, R₁ is (C₁-C₆)alkoxy, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, R₃ is (C₁-C₆)alkyl substituted with one to three R₄, and each R₄ is independently selected from phenyl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the phenyl, heteroaryl, cycloalkyl, and heterocycloalkyl groups are optionally substituted with one to three R₇.

In some embodiments of the formulae above, Rₓ is H, n is 0, R₁ is (C₁-C₆)alkoxy, halogen, -OH, - (CH₂)₀₋₂NH₂, -(CH₂)₀₋₂NH(C₁-C₆)alkyl, -(CH₂)₀₋₂N((C₁-C₆)alkyl)₂, or CN, R₃ is (C₁-C₆)alkyl optionally substituted with one to three R₄, and each R₄ is independently selected from phenyl and 5- or 6-membered heteroaryl comprising 1 to 3 heteroatoms selected from O, N, and S, wherein the phenyl and heteroaryl groups are optionally substituted with one to three R₇. In another embodiment, Rₓ is H, n is 0, R₁ is (C₁-C₆)alkoxy, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, R₃ is (C₁-C₆)alkyl substituted with one to three R₄, and each R₄ is independently selected from phenyl and 5- or 6-membered heteroaryl comprising 1 to 3 heteroatoms selected from O, N, and S, wherein the phenyl and heteroaryl groups are optionally substituted with one to three R₇.

In some embodiments of the formulae above, Rₓ is H, n is 0, R₁ is (C₁-C₆)alkoxy, halogen, -OH, - (CH₂)₀₋₂NH₂, -(CH₂)₀₋₂NH(C₁-C₆)alkyl, -(CH₂)₀₋₂N((C₁-C₆)alkyl)₂, or CN, R₃ is (C₁-C₆)alkyl optionally substituted with one to three R₄, and each R₄ is phenyl optionally substituted with one to three R₇. In another embodiment, Rₓ is H, n is 0, R₁ is (C₁-C₆)alkoxy, halogen, -OH, -(CH₂)₀₋₂NH₂, -(CH₂)₀₋₂NH(C₁-C₆)alkyl, - (CH₂)₀₋₂N((C₁-C₆)alkyl)₂, or CN, R₃ is (C₁-C₆)alkyl substituted with one to three R₄, and each R₄ is phenyl optionally substituted with one to three R₇.

In some embodiments of the formulae above, Rₓ is H, n is 0, R₁ is (C₁-C₆)alkoxy, halogen, -OH, - (CH₂)₀₋₂NH₂, -(CH₂)₀₋₂NH(C₁-C₆)alkyl, -(CH₂)₀₋₂N((C₁-C₆)alkyl)₂, or CN, R₃ is (C₁-C₆)alkyl optionally substituted with one to three R₄, and each R₄ is 5- or 6-membered heteroaryl comprising 1 to 3 heteroatoms selected from O, N, and S, optionally substituted with one to three R₇. In another embodiment, Rₓ is H, n is 0, R₁ is (C₁-C₆)alkoxy, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, R₃ is (C₁-C₆)alkyl substituted with one to three R₄, and each R₄ is 5- or 6-membered heteroaryl comprising 1 to 3 heteroatoms selected from O, N, and S, optionally substituted with one to three R₇.

In some embodiments of the formulae above, Rₓ is H, n is 0, R₁ is (C₁-C₆)alkoxy, halogen, -OH, - (CH₂)₀₋₂NH₂, -(CH₂)₀₋₂NH(C₁-C₆)alkyl, -(CH₂)₀₋₂N((C₁-C₆)alkyl)₂, or CN, R₃ is (C₁-C₆)alkyl optionally substituted with one to three R₄, and each R₄ is (C₃-C₈)cycloalkyl optionally substituted with one to three R₇. In another embodiment, Rₓ is H, n is 0, R₁ is (C₁-C₆)alkoxy, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, R₃ is (C₁-C₆)alkyl substituted with one to three R₄, and each R₄ is (C₃-C₈)cycloalkyl optionally substituted with one to three R₇.

In some embodiments of the formulae above, Rₓ is H, n is 0, R₁ is (C₁-C₆)alkoxy, halogen, -OH, - (CH₂)₀₋₂NH₂, -(CH₂)₀₋₂NH(C₁-C₆)alkyl, -(CH₂)₀₋₂N((C₁-C₆)alkyl)₂, or CN, R₃ is (C₁-C₆)alkyl optionally substituted with one to three R₄, and each R₄ is 5- to 7-membered heterocycloalkyl comprising 1 to 3 heteroatoms selected from O, N, and S, optionally substituted with one to three R₇. In another embodiment, Rₓ is H, n is 0, R₁ is (C₁-C₆)alkoxy, halogen, -OH, -(CH₂)₀₋₂NH₂, -(CH₂)₀₋₂NH(C₁-C₆)alkyl, -(CH₂)₀₋₂N((C₁-C₆)alkyl)₂, or CN, R₃ is (C₁-C₆)alkyl substituted with one to three R₄, and each R₄ is 5- to 7-membered heterocycloalkyl comprising 1 to 3 heteroatoms selected from O, N, and S, optionally substituted with one to three R₇.

In some embodiments of the formulae above, Rₓ is H, n is 0, R₁ is (C₁-C₆)alkoxy, halogen, -OH, - (CH₂)₀₋₂NH₂, -(CH₂)₀₋₂NH(C₁-C₆)alkyl, -(CH₂)₀₋₂N((C₁-C₆)alkyl)₂, or CN, and R₃ is (C₆-C₁₀)aryl, (C₃-C₈)cycloalkyl, or 5- to 7-membered heterocycloalkyl comprising 1 to 3 heteroatoms selected from O, N, and S, wherein the aryl, cycloalkyl, and heterocycloalkyl are optionally substituted with one to three R₅.

In some embodiments of the formulae above, Rₓ is H, n is 0, R₁ is (C₁-C₆)alkoxy, halogen, -OH, - (CH₂)₀₋₂NH₂, -(CH₂)₀₋₂NH(C₁-C₆)alkyl, -(CH₂)₀₋₂N((C₁-C₆)alkyl)₂, or CN, and R₃ is (C₆-C₁₀)aryl, (C₃-C₈)cycloalkyl, or 5- to 7-membered heterocycloalkyl comprising 1 to 3 heteroatoms selected from O, N, and S.

In some embodiments of the formulae above, Rₓ is H, n is 0, R₁ is (C₁-C₆)alkoxy, halogen, -OH, - (CH₂)₀₋₂NH₂, -(CH₂)₀₋₂NH(C₁-C₆)alkyl, -(CH₂)₀₋₂N((C₁-C₆)alkyl)₂, or CN, and R₃ is (C₆-C₁₀)aryl, 5- or 6-membered heteroaryl comprising 1 to 3 heteroatoms selected from O, N, and S, or (C₃-C₈)cycloalkyl, wherein the aryl, heteroaryl and cycloalkyl are optionally substituted with one to three R₅.

In some embodiments of the formulae above, Rₓ is H, n is 0, R₁ is (C₁-C₆)alkoxy, halogen, -OH, - (CH₂)₀₋₂NH₂, -(CH₂)₀₋₂NH(C₁-C₆)alkyl, -(CH₂)₀₋₂N((C₁-C₆)alkyl)₂, or CN, and R₃ is (C₆-C₁₀)aryl, 5- or 6-membered heteroaryl comprising 1 to 3 heteroatoms selected from O, N, and S, or (C₃-C₈)cycloalkyl.

In some embodiments of the formulae above, Rₓ is H, n is 0, R₁ is (C₁-C₆)alkoxy, halogen, -OH, - (CH₂)₀₋₂NH₂, -(CH₂)₀₋₂NH(C₁-C₆)alkyl, -(CH₂)₀₋₂N((C₁-C₆)alkyl)₂, or CN, and R₃ is (C₆-C₁₀)aryl optionally substituted with one to three R₅.

In some embodiments of the formulae above, Rₓ is H, n is 0, R₁ is (C₁-C₆)alkoxy, halogen, -OH, - (CH₂)₀₋₂NH₂, -(CH₂)₀₋₂NH(C₁-C₆)alkyl, -(CH₂)₀₋₂N((C₁-C₆)alkyl)₂, or CN, and R₃ is 5- or 6-membered heteroaryl comprising 1 to 3 heteroatoms selected from O, N, and S optionally substituted with one to three R₅.

In some embodiments of the formulae above, Rₓ is H, n is 0, R₁ is (C₁-C₆)alkoxy, halogen, -OH, - (CH₂)₀₋₂NH₂, -(CH₂)₀₋₂NH(C₁-C₆)alkyl, -(CH₂)₀₋₂N((C₁-C₆)alkyl)₂, or CN, and R₃ is (C₃-C₈)cycloalkyl optionally substituted with one to three R₅.

In some embodiments of the formulae above, Rₓ is H, n is 0, R₁ is (C₁-C₆)alkoxy, halogen, -OH, - (CH₂)₀₋₂NH₂, -(CH₂)₀₋₂NH(C₁-C₆)alkyl, -(CH₂)₀₋₂N((C₁-C₆)alkyl)₂, or CN, and R₃ is 5- to 7-membered heterocycloalkyl comprising 1 to 3 heteroatoms selected from O, N, and S, optionally substituted with one to three R₅.

In some embodiments of the formulae above, Rₓ is H, n is 0, and R₁ is (C₁-C₆)alkoxy, halogen, - OH, -(CH₂)₀₋₂NH₂, or CN. In another embodiment, Rₓ is H, n is 0, R₁ is (C₁-C₆)alkoxy, halogen, -OH, - (CH₂)₀₋₂NH₂, or CN, and R₃ is (C₁-C₆)alkyl optionally substituted with one to three R₄. In another embodiment, Rₓ is H, n is 0, R₁ is (C₁-C₆)alkoxy, halogen, -OH, -(CH₂)₀₋₂NH₂, or CN, and R₃ is (C₁-C₆)alkyl substituted with one to three R₄.

In some embodiments of the formulae above, Rₓ is H, n is 0, R₁ is (C₁-C₆)alkoxy, halogen, -OH, - (CH₂)₀₋₂NH₂, or CN, R₃ is (C₁-C₆)alkyl optionally substituted with one to three R₄, and each R₄ is independently selected from -C(O)OR₆, (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇. In another embodiment, Rₓ is H, n is 0, R₁ is (C₁-C₆)alkoxy, halogen, -OH, -(CH₂)₀₋₂NH₂, or CN, and R₃ is (C₁-C₆)alkyl substituted with one to three R₄, and each R₄ is independently selected from -C(O)OR₆, (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇.

In some embodiments of the formulae above, Rₓ is H, n is 0, R₁ is (C₁-C₆)alkoxy, halogen, -OH, - (CH₂)₀₋₂NH₂, or CN, R₃ is (C₁-C₆)alkyl optionally substituted with one to three R₄, and each R₄ is independently selected from (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇. In another embodiment, Rₓ is H, n is 0, R₁ is (C₁-C₆)alkoxy, halogen, -OH, -(CH₂)₀₋₂NH₂, or CN, and R₃ is (C₁-C₆)alkyl substituted with one to three R₄, and each R₄ is independently selected from (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇.

In some embodiments of the formulae above, Rₓ is H, n is 0, R₁ is (C₁-C₆)alkoxy, halogen, -OH, - (CH₂)₀₋₂NH₂, or CN, R₃ is (C₁-C₆)alkyl optionally substituted with one to three R₄, and each R₄ is independently selected from halogen, -OH, (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇. In another embodiment, Rₓ is H, n is 0, R₁ is (C₁-C₆)alkoxy, halogen, -OH, -(CH₂)₀₋₂NH₂, or CN, and R₃ is (C₁-C₆)alkyl substituted with one to three R₄, and each R₄ is independently selected from halogen, -OH, (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇.

In some embodiments of the formulae above, Rₓ is H, n is 0, R₁ is (C₁-C₆)alkoxy, halogen, -OH, - (CH₂)₀₋₂NH₂, or CN, R₃ is (C₁-C₆)alkyl optionally substituted with one to three R₄, and each R₄ is independently selected from halogen, -OH, phenyl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the phenyl, heteroaryl, cycloalkyl, and heterocycloalkyl groups are optionally substituted with one to three R₇. In another embodiment, Rₓ is H, n is 0, R₁ is (C₁-C₆)alkoxy, halogen, -OH, -(CH₂)₀₋₂NH₂, or CN, and R₃ is (C₁-C₆)alkyl substituted with one to three R₄, and each R₄ is independently selected from halogen, -OH, phenyl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the phenyl, heteroaryl, cycloalkyl, and heterocycloalkyl groups are optionally substituted with one to three R₇.

In some embodiments of the formulae above, Rₓ is H, n is 0, R₁ is (C₁-C₆)alkoxy, halogen, -OH, - (CH₂)₀₋₂NH₂, or CN, R₃ is (C₁-C₆)alkyl optionally substituted with one to three R₄, and each R₄ is independently selected from phenyl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the phenyl, heteroaryl, cycloalkyl, and heterocycloalkyl groups are optionally substituted with one to three R₇. In another embodiment, Rₓ is H, n is 0, R₁ is (C₁-C₆)alkoxy, halogen, -OH, -(CH₂)₀₋₂NH₂, or CN, and R₃ is (C₁-C₆)alkyl substituted with one to three R₄, and each R₄ is independently selected from phenyl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the phenyl, heteroaryl, cycloalkyl, and heterocycloalkyl groups are optionally substituted with one to three R₇.

In some embodiments of the formulae above, Rₓ is H, n is 0, R₁ is (C₁-C₆)alkoxy, halogen, -OH, - (CH₂)₀₋₂NH₂, or CN, R₃ is (C₁-C₆)alkyl optionally substituted with one to three R₄, and each R₄ is independently selected from phenyl and 5- or 6-membered heteroaryl comprising 1 to 3 heteroatoms selected from O, N, and S, wherein the phenyl and heteroaryl groups are optionally substituted with one to three R₇. In another embodiment, Rₓ is H, n is 0, R₁ is (C₁-C₆)alkoxy, halogen, -OH, -(CH₂)₀₋₂NH₂, or CN, and R₃ is (C₁-C₆)alkyl substituted with one to three R₄, and each R₄ is independently selected from phenyl and 5- or 6-membered heteroaryl comprising 1 to 3 heteroatoms selected from O, N, and S, wherein the phenyl and heteroaryl groups are optionally substituted with one to three R₇.

In some embodiments of the formulae above, Rₓ is H, n is 0, R₁ is (C₁-C₆)alkoxy, halogen, -OH, - (CH₂)₀₋₂NH₂, or CN, R₃ is (C₁-C₆)alkyl optionally substituted with one to three R₄, and each R₄ is phenyl optionally substituted with one to three R₇. In another embodiment, Rₓ is H, n is 0, R₁ is (C₁-C₆)alkoxy, halogen, -OH, -(CH₂)₀₋₂NH₂, or CN, and R₃ is (C₁-C₆)alkyl substituted with one to three R₄, and each R₄ is phenyl optionally substituted with one to three R₇.

In some embodiments of the formulae above, Rₓ is H, n is 0, R₁ is (C₁-C₆)alkoxy, halogen, -OH, - (CH₂)₀₋₂NH₂, or CN, R₃ is (C₁-C₆)alkyl optionally substituted with one to three R₄, and each R₄ is 5- or 6-membered heteroaryl comprising 1 to 3 heteroatoms selected from O, N, and S, optionally substituted with one to three R₇. In another embodiment, Rₓ is H, n is 0, R₁ is (C₁-C₆)alkoxy, halogen, -OH, -(CH₂)₀₋₂NH₂, or CN, and R₃ is (C₁-C₆)alkyl substituted with one to three R₄, and each R₄ is 5- or 6-membered heteroaryl comprising 1 to 3 heteroatoms selected from O, N, and S, optionally substituted with one to three R₇.

In some embodiments of the formulae above, Rₓ is H, n is 0, R₁ is (C₁-C₆)alkoxy, halogen, -OH, - (CH₂)₀₋₂NH₂, or CN, R₃ is (C₁-C₆)alkyl optionally substituted with one to three R₄, and each R₄ is (C₃-C₈)cycloalkyl optionally substituted with one to three R₇. In another embodiment, Rₓ is H, n is 0, R₁ is (C₁-C₆)alkoxy, halogen, -OH, -(CH₂)₀₋₂NH₂, or CN, and R₃ is (C₁-C₆)alkyl substituted with one to three R₄, and each R₄ is (C₃-C₈)cycloalkyl optionally substituted with one to three R₇.

In some embodiments of the formulae above, Rₓ is H, n is 0, R₁ is (C₁-C₆)alkoxy, halogen, -OH, - (CH₂)₀₋₂NH₂, or CN, R₃ is (C₁-C₆)alkyl optionally substituted with one to three R₄, and each R₄ is 5- to 7-membered heterocycloalkyl comprising 1 to 3 heteroatoms selected from O, N, and S, optionally substituted with one to three R₇. In another embodiment, Rₓ is H, n is 0, R₁ is (C₁-C₆)alkoxy, halogen, -OH, -(CH₂)₀₋₂NH₂, or CN, and R₃ is (C₁-C₆)alkyl substituted with one to three R₄, and each R₄ is 5- to 7-membered heterocycloalkyl comprising 1 to 3 heteroatoms selected from O, N, and S, optionally substituted with one to three R₇.

In some embodiments of the formulae above, Rₓ is H, n is 0, R₁ is (C₁-C₆)alkoxy, halogen, -OH, - (CH₂)₀₋₂NH₂, or CN, and R₃ is (C₆-C₁₀)aryl, (C₃-C₈)cycloalkyl, or 5- to 7-membered heterocycloalkyl comprising 1 to 3 heteroatoms selected from O, N, and S, wherein the aryl, cycloalkyl, and heterocycloalkyl are optionally substituted with one to three R₅.

In some embodiments of the formulae above, Rₓ is H, n is 0, R₁ is (C₁-C₆)alkoxy, halogen, -OH, - (CH₂)₀₋₂NH₂, or CN, and R₃ is (C₆-C₁₀)aryl, (C₃-C₈)cycloalkyl, or 5- to 7-membered heterocycloalkyl comprising 1 to 3 heteroatoms selected from O, N, and S.

In some embodiments of the formulae above, Rₓ is H, n is 0, R₁ is (C₁-C₆)alkoxy, halogen, -OH, - (CH₂)₀₋₂NH₂, or CN, and R₃ is (C₆-C₁₀)aryl, 5- or 6-membered heteroaryl comprising 1 to 3 heteroatoms selected from O, N, and S, or (C₃-C₈)cycloalkyl, wherein the aryl, heteroaryl and cycloalkyl are optionally substituted with one to three R₅.

In some embodiments of the formulae above, Rₓ is H, n is 0, R₁ is (C₁-C₆)alkoxy, halogen, -OH, - (CH₂)₀₋₂NH₂, or CN, and R₃ is (C₆-C₁₀)aryl, 5- or 6-membered heteroaryl comprising 1 to 3 heteroatoms selected from O, N, and S, or (C₃-C₈)cycloalkyl.

In some embodiments of the formulae above, Rₓ is H, n is 0, R₁ is (C₁-C₆)alkoxy, halogen, -OH, - (CH₂)₀₋₂NH₂, or CN, and R₃ is (C₆-C₁₀)aryl optionally substituted with one to three R₅.

In some embodiments of the formulae above, Rₓ is H, n is 0, R₁ is (C₁-C₆)alkoxy, halogen, -OH, - (CH₂)₀₋₂NH₂, or CN, and R₃ is 5- or 6-membered heteroaryl comprising 1 to 3 heteroatoms selected from O, N, and S optionally substituted with one to three R₅.

In some embodiments of the formulae above, Rₓ is H, n is 0, R₁ is (C₁-C₆)alkoxy, halogen, -OH, - (CH₂)₀₋₂NH₂, or CN, and R₃ is (C₃-C₈)cycloalkyl optionally substituted with one to three R₅.

In some embodiments of the formulae above, Rₓ is H, n is 0, R₁ is (C₁-C₆)alkoxy, halogen, -OH, - (CH₂)₀₋₂NH₂, or CN, and R₃ is 5- to 7-membered heterocycloalkyl comprising 1 to 3 heteroatoms selected from O, N, and S, optionally substituted with one to three R₅.

In some embodiments of the formulae above, Rₓ is H, n is 1, and each R₂ is independently (C₁-C₆)alkyl. In another embodiment, Rₓ is H, n is 1, each R₂ is independently (C₁-C₆)alkyl, and R₁ is (C₁-C₆)alkoxy, halogen, -OH, -(CH₂)₀₋₂NH₂, or CN. In another embodiment, Rₓ is H, n is 1, each R₂ is independently (C₁-C₆)alkyl, R₁ is (C₁-C₆)alkoxy, halogen, -OH, -(CH₂)₀₋₂NH₂, or CN, and R₃ is (C₁-C₆)alkyl optionally substituted with one to three R₄. In another embodiment, Rₓ is H, n is 1, each R₂ is independently (C₁-C₆)alkyl, R₁ is (C₁-C₆)alkoxy, halogen, -OH, -(CH₂)₀₋₂NH₂, or CN, and R₃ is (C₁-C₆)alkyl substituted with one to three R₄.

In some embodiments of the formulae above, Rₓ is H, n is 1, each R₂ is independently (C₁-C₆)alkyl, R₁ is (C₁-C₆)alkoxy, halogen, -OH, -(CH₂)₀₋₂NH₂, or CN, R₃ is (C₁-C₆)alkyl optionally substituted with one to three R₄, and each R₄ is independently selected from -C(O)OR₆, (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇. In another embodiment, Rₓ is H, n is 1, each R₂ is independently (C₁-C₆)alkyl, R₁ is (C₁-C₆)alkoxy, halogen, -OH, -(CH₂)₀₋₂NH₂, or CN, R₃ is (C₁-C₆)alkyl substituted with one to three R₄, and each R₄ is independently selected from -C(O)OR₆, (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇.

In some embodiments of the formulae above, Rₓ is H, n is 1, each R₂ is independently (C₁-C₆)alkyl, R₁ is (C₁-C₆)alkoxy, halogen, -OH, -(CH₂)₀₋₂NH₂, or CN, R₃ is (C₁-C₆)alkyl optionally substituted with one to three R₄, and each R₄ is independently selected from (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇. In another embodiment, Rₓ is H, n is 1, each R₂ is independently (C₁-C₆)alkyl, R₁ is (C₁-C₆)alkoxy, halogen, -OH, - (CH₂)₀₋₂NH₂, or CN, R₃ is (C₁-C₆)alkyl substituted with one to three R₄, and each R₄ is independently selected from (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇.

In some embodiments of the formulae above, Rₓ is H, n is 1, each R₂ is independently (C₁-C₆)alkyl, R₁ is (C₁-C₆)alkoxy, halogen, -OH, -(CH₂)₀₋₂NH₂, or CN, R₃ is (C₁-C₆)alkyl optionally substituted with one to three R₄, and each R₄ is independently selected from halogen, -OH, (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇. In another embodiment, Rₓ is H, n is 1, each R₂ is independently (C₁-C₆)alkyl, R₁ is (C₁-C₆)alkoxy, halogen, -OH, -(CH₂)₀₋₂NH₂, or CN, R₃ is (C₁-C₆)alkyl substituted with one to three R₄, and each R₄ is independently selected from halogen, -OH, (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇.

In some embodiments of the formulae above, Rₓ is H, n is 1, each R₂ is independently (C₁-C₆)alkyl, R₁ is (C₁-C₆)alkoxy, halogen, -OH, -(CH₂)₀₋₂NH₂, or CN, R₃ is (C₁-C₆)alkyl optionally substituted with one to three R₄, and each R₄ is independently selected from halogen, -OH, phenyl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the phenyl, heteroaryl, cycloalkyl, and heterocycloalkyl groups are optionally substituted with one to three R₇. In another embodiment, Rₓ is H, n is 1, each R₂ is independently (C₁-C₆)alkyl, R₁ is (C₁-C₆)alkoxy, halogen, -OH, - (CH₂)₀₋₂NH₂, or CN, R₃ is (C₁-C₆)alkyl substituted with one to three R₄, and each R₄ is independently selected from halogen, -OH, phenyl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the phenyl, heteroaryl, cycloalkyl, and heterocycloalkyl groups are optionally substituted with one to three R₇.

In some embodiments of the formulae above, Rₓ is H, n is 1, each R₂ is independently (C₁-C₆)alkyl, R₁ is (C₁-C₆)alkoxy, halogen, -OH, -(CH₂)₀₋₂NH₂, or CN, R₃ is (C₁-C₆)alkyl optionally substituted with one to three R₄, and each R₄ is independently selected from phenyl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the phenyl, heteroaryl, cycloalkyl, and heterocycloalkyl groups are optionally substituted with one to three R₇. In another embodiment, Rₓ is H, n is 1, each R₂ is independently (C₁-C₆)alkyl, R₁ is (C₁-C₆)alkoxy, halogen, -OH, -(CH₂)₀₋₂NH₂, or CN, R₃ is (C₁-C₆)alkyl substituted with one to three R₄, and each R₄ is independently selected from phenyl, 5- or 6-membered heteroaryl comprising 1 to 3 heteroatoms selected from O, N, and S, (C₃-C₈)cycloalkyl, and 5-to 7-membered heterocycloalkyl comprising 1 to 3 heteroatoms selected from O, N, and S, wherein the phenyl, heteroaryl, cycloalkyl, and heterocycloalkyl groups are optionally substituted with one to three R₇.

In some embodiments of the formulae above, Rₓ is H, n is 1, each R₂ is independently (C₁-C₆)alkyl, R₁ is (C₁-C₆)alkoxy, halogen, -OH, -(CH₂)₀₋₂NH₂, or CN, R₃ is (C₁-C₆)alkyl optionally substituted with one to three R₄, and each R₄ is independently selected from phenyl and 5- or 6-membered heteroaryl comprising 1 to 3 heteroatoms selected from O, N, and S, wherein the phenyl and heteroaryl groups are optionally substituted with one to three R₇. In another embodiment, Rₓ is H, n is 1, each R₂ is independently (C₁-C₆)alkyl, R₁ is (C₁-C₆)alkoxy, halogen, -OH, -(CH₂)₀₋₂NH₂, or CN, R₃ is (C₁-C₆)alkyl substituted with one to three R₄, and each R₄ is independently selected from phenyl and 5- or 6-membered heteroaryl comprising 1 to 3 heteroatoms selected from O, N, and S, wherein the phenyl and heteroaryl groups are optionally substituted with one to three R₇.

In some embodiments of the formulae above, Rₓ is H, n is 1, each R₂ is independently (C₁-C₆)alkyl, R₁ is (C₁-C₆)alkoxy, halogen, -OH, -(CH₂)₀₋₂NH₂, or CN, R₃ is (C₁-C₆)alkyl optionally substituted with one to three R₄, and each R₄ is phenyl optionally substituted with one to three R₇. In another embodiment, Rₓ is H, n is 1, each R₂ is independently (C₁-C₆)alkyl, R₁ is (C₁-C₆)alkoxy, halogen, -OH, -(CH₂)₀₋₂NH₂, or CN, R₃ is (C₁-C₆)alkyl substituted with one to three R₄, and each R₄ is phenyl optionally substituted with one to three R₇.

In some embodiments of the formulae above, Rₓ is H, n is 1, each R₂ is independently (C₁-C₆)alkyl, R₁ is (C₁-C₆)alkoxy, halogen, -OH, -(CH₂)₀₋₂NH₂, or CN, R₃ is (C₁-C₆)alkyl optionally substituted with one to three R₄, and each R₄ is 5- or 6-membered heteroaryl comprising 1 to 3 heteroatoms selected from O, N, and S, optionally substituted with one to three R₇. In another embodiment, Rₓ is H, n is 1, each R₂ is independently (C₁-C₆)alkyl, R₁ is (C₁-C₆)alkoxy, halogen, -OH, -(CH₂)₀₋₂NH₂, or CN, R₃ is (C₁-C₆)alkyl substituted with one to three R₄, and each R₄ is 5- or 6-membered heteroaryl comprising 1 to 3 heteroatoms selected from O, N, and S, optionally substituted with one to three R₇.

In some embodiments of the formulae above, Rₓ is H, n is 1, each R₂ is independently (C₁-C₆)alkyl, R₁ is (C₁-C₆)alkoxy, halogen, -OH, -(CH₂)₀₋₂NH₂, or CN, R₃ is (C₁-C₆)alkyl optionally substituted with one to three R₄, and each R₄ is (C₃-C₈)cycloalkyl optionally substituted with one to three R₇. In another embodiment, Rₓ is H, n is 1, each R₂ is independently (C₁-C₆)alkyl, R₁ is (C₁-C₆)alkoxy, halogen, -OH, - (CH₂)₀₋₂NH₂, or CN, R₃ is (C₁-C₆)alkyl substituted with one to three R₄, and each R₄ is (C₃-C₈)cycloalkyl optionally substituted with one to three R₇.

In some embodiments of the formulae above, Rₓ is H, n is 1, each R₂ is independently (C₁-C₆)alkyl, R₁ is (C₁-C₆)alkoxy, halogen, -OH, -(CH₂)₀₋₂NH₂, or CN, R₃ is (C₁-C₆)alkyl optionally substituted with one to three R₄, and each R₄ is 5- to 7-membered heterocycloalkyl comprising 1 to 3 heteroatoms selected from O, N, and S, optionally substituted with one to three R₇. In another embodiment, Rₓ is H, n is 1, each R₂ is independently (C₁-C₆)alkyl, R₁ is (C₁-C₆)alkoxy, halogen, -OH, -(CH₂)₀₋₂NH₂, or CN, R₃ is (C₁-C₆)alkyl substituted with one to three R₄, and each R₄ is 5- to 7-membered heterocycloalkyl comprising 1 to 3 heteroatoms selected from O, N, and S, optionally substituted with one to three R₇.

In some embodiments of the formulae above, Rₓ is H, n is 1, each R₂ is independently (C₁-C₆)alkyl, R₁ is (C₁-C₆)alkoxy, halogen, -OH, -(CH₂)₀₋₂NH₂, or CN, and R₃ is (C₆-C₁₀)aryl, (C₃-C₈)cycloalkyl, or 5- to 7-membered heterocycloalkyl comprising 1 to 3 heteroatoms selected from O, N, and S, wherein the aryl, cycloalkyl, and heterocycloalkyl are optionally substituted with one to three R₅.

In some embodiments of the formulae above, Rₓ is H, n is 1, each R₂ is independently (C₁-C₆)alkyl, R₁ is (C₁-C₆)alkoxy, halogen, -OH, -(CH₂)₀₋₂NH₂, or CN, and R₃ is (C₆-C₁₀)aryl, (C₃-C₈)cycloalkyl, or 5- to 7-membered heterocycloalkyl comprising 1 to 3 heteroatoms selected from O, N, and S.

In some embodiments of the formulae above, Rₓ is H, n is 1, each R₂ is independently (C₁-C₆)alkyl, R₁ is (C₁-C₆)alkoxy, halogen, -OH, -(CH₂)₀₋₂NH₂, or CN, and R₃ is (C₆-C₁₀)aryl, 5- or 6-membered heteroaryl comprising 1 to 3 heteroatoms selected from O, N, and S, or (C₃-C₈)cycloalkyl, wherein the aryl, heteroaryl and cycloalkyl are optionally substituted with one to three R₅.

In some embodiments of the formulae above, Rₓ is H, n is 1, each R₂ is independently (C₁-C₆)alkyl, R₁ is (C₁-C₆)alkoxy, halogen, -OH, -(CH₂)₀₋₂NH₂, or CN, and R₃ is (C₆-C₁₀)aryl, 5- or 6-membered heteroaryl comprising 1 to 3 heteroatoms selected from O, N, and S, or (C₃-C₈)cycloalkyl.

In some embodiments of the formulae above, Rₓ is H, n is 1, each R₂ is independently (C₁-C₆)alkyl, R₁ is (C₁-C₆)alkoxy, halogen, -OH, -(CH₂)₀₋₂NH₂, or CN, and R₃ is (C₆-C₁₀)aryl optionally substituted with one to three R₅.

In some embodiments of the formulae above, Rₓ is H, n is 1, each R₂ is independently (C₁-C₆)alkyl, R₁ is (C₁-C₆)alkoxy, halogen, -OH, -(CH₂)₀₋₂NH₂, or CN, and R₃ is 5- or 6-membered heteroaryl comprising 1 to 3 heteroatoms selected from O, N, and S optionally substituted with one to three R₅.

In some embodiments of the formulae above, Rₓ is H, n is 1, each R₂ is independently (C₁-C₆)alkyl, R₁ is (C₁-C₆)alkoxy, halogen, -OH, -(CH₂)₀₋₂NH₂, or CN, and R₃ is (C₃-C₈)cycloalkyl optionally substituted with one to three R₅.

In some embodiments of the formulae above, Rₓ is H, n is 1, each R₂ is independently (C₁-C₆)alkyl, R₁ is (C₁-C₆)alkoxy, halogen, -OH, -(CH₂)₀₋₂NH₂, or CN, and R₃ is 5- to 7-membered heterocycloalkyl comprising 1 to 3 heteroatoms selected from O, N, and S, optionally substituted with one to three R₅.

In some embodiments of the formulae above, Rₓ is H, n is 0 or 1, and each R₂ is independently (C₁-C₆)alkyl. In another embodiment, Rₓ is H, n is 0 or 1, each R₂ is independently (C₁-C₆)alkyl, and R₁ is (C₁-C₆)alkoxy, halogen, -OH, -(CH₂)₀₋₂NH₂, or CN. In another embodiment, Rₓ is H, n is 0 or 1, each R₂ is independently (C₁-C₆)alkyl, R₁ is (C₁-C₆)alkoxy, halogen, -OH, -(CH₂)₀₋₂NH₂, or CN, and R₃ is (C₁-C₆)alkyl optionally substituted with one to three R₄. In another embodiment, Rₓ is H, n is 0 or 1, each R₂ is independently (C₁-C₆)alkyl, R₁ is (C₁-C₆)alkoxy, halogen, -OH, -(CH₂)₀₋₂NH₂, or CN, and R₃ is (C₁-C₆)alkyl substituted with one to three R₄.

In some embodiments of the formulae above, Rₓ is H, n is 0 or 1, each R₂ is independently (C₁-C₆)alkyl, R₁ is (C₁-C₆)alkoxy, halogen, -OH, -(CH₂)₀₋₂NH₂, or CN, R₃ is (C₁-C₆)alkyl optionally substituted with one to three R₄, and each R₄ is independently selected from -C(O)OR₆, (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇. In another embodiment, Rₓ is H, n is 0 or 1, each R₂ is independently (C₁-C₆)alkyl, R₁ is (C₁-C₆)alkoxy, halogen, -OH, -(CH₂)₀₋₂NH₂, or CN, R₃ is (C₁-C₆)alkyl substituted with one to three R₄, and each R₄ is independently selected from -C(O)OR₆, (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇.

In some embodiments of the formulae above, Rₓ is H, n is 0 or 1, each R₂ is independently (C₁-C₆)alkyl, R₁ is (C₁-C₆)alkoxy, halogen, -OH, -(CH₂)₀₋₂NH₂, or CN, R₃ is (C₁-C₆)alkyl optionally substituted with one to three R₄, and each R₄ is independently selected from (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇. In another embodiment, Rₓ is H, n is 0 or 1, each R₂ is independently (C₁-C₆)alkyl, R₁ is (C₁-C₆)alkoxy, halogen, -OH, -(CH₂)₀₋₂NH₂, or CN, R₃ is (C₁-C₆)alkyl substituted with one to three R₄, and each R₄ is independently selected from (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇.

In some embodiments of the formulae above, Rₓ is H, n is 0 or 1, each R₂ is independently (C₁-C₆)alkyl, R₁ is (C₁-C₆)alkoxy, halogen, -OH, -(CH₂)₀₋₂NH₂, or CN, R₃ is (C₁-C₆)alkyl optionally substituted with one to three R₄, and each R₄ is independently selected from halogen, -OH, (C₆-C₁₀)aryl, 5- or 6-membered heteroaryl comprising 1 to 3 heteroatoms selected from O, N, and S, (C₃-C₈)cycloalkyl, and 5-to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇. In another embodiment, Rₓ is H, n is 0 or 1, each R₂ is independently (C₁-C₆)alkyl, R₁ is (C₁-C₆)alkoxy, halogen, -OH, -(CH₂)₀₋₂NH₂, or CN, R₃ is (C₁-C₆)alkyl substituted with one to three R₄, and each R₄ is independently selected from halogen, -OH, (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇.

In some embodiments of the formulae above, Rₓ is H, n is 0 or 1, each R₂ is independently (C₁-C₆)alkyl, R₁ is (C₁-C₆)alkoxy, halogen, -OH, -(CH₂)₀₋₂NH₂, or CN, R₃ is (C₁-C₆)alkyl optionally substituted with one to three R₄, and each R₄ is independently selected from halogen, -OH, phenyl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the phenyl, heteroaryl, cycloalkyl, and heterocycloalkyl groups are optionally substituted with one to three R₇. In another embodiment, Rₓ is H, n is 0 or 1, each R₂ is independently (C₁-C₆)alkyl, R₁ is (C₁-C₆)alkoxy, halogen, -OH, -(CH₂)₀₋₂NH₂, or CN, R₃ is (C₁-C₆)alkyl substituted with one to three R₄, and each R₄ is independently selected from halogen, -OH, phenyl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the phenyl, heteroaryl, cycloalkyl, and heterocycloalkyl groups are optionally substituted with one to three R₇.

In some embodiments of the formulae above, Rₓ is H, n is 0 or 1, each R₂ is independently (C₁-C₆)alkyl, R₁ is (C₁-C₆)alkoxy, halogen, -OH, -(CH₂)₀₋₂NH₂, or CN, R₃ is (C₁-C₆)alkyl optionally substituted with one to three R₄, and each R₄ is independently selected from phenyl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the phenyl, heteroaryl, cycloalkyl, and heterocycloalkyl groups are optionally substituted with one to three R₇. In another embodiment, Rₓ is H, n is 0 or 1, each R₂ is independently (C₁-C₆)alkyl, R₁ is (C₁-C₆)alkoxy, halogen, -OH, -(CH₂)₀₋₂NH₂, or CN, R₃ is (C₁-C₆)alkyl substituted with one to three R₄, and each R₄ is independently selected from phenyl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the phenyl, heteroaryl, cycloalkyl, and heterocycloalkyl groups are optionally substituted with one to three R₇.

In some embodiments of the formulae above, Rₓ is H, n is 0 or 1, each R₂ is independently (C₁-C₆)alkyl, R₁ is (C₁-C₆)alkoxy, halogen, -OH, -(CH₂)₀₋₂NH₂, or CN, R₃ is (C₁-C₆)alkyl optionally substituted with one to three R₄, and each R₄ is independently selected from phenyl and 5- or 6-membered heteroaryl comprising 1 to 3 heteroatoms selected from O, N, and S, wherein the phenyl and heteroaryl groups are optionally substituted with one to three R₇. In another embodiment, Rₓ is H, n is 0 or 1, each R₂ is independently (C₁-C₆)alkyl, R₁ is (C₁-C₆)alkoxy, halogen, -OH, -(CH₂)₀₋₂NH₂, or CN, R₃ is (C₁-C₆)alkyl substituted with one to three R₄, and each R₄ is independently selected from phenyl and 5- or 6-membered heteroaryl comprising 1 to 3 heteroatoms selected from O, N, and S, wherein the phenyl and heteroaryl groups are optionally substituted with one to three R₇.

In some embodiments of the formulae above, Rₓ is H, n is 0 or 1, each R₂ is independently (C₁-C₆)alkyl, R₁ is (C₁-C₆)alkoxy, halogen, -OH, -(CH₂)₀₋₂NH₂, or CN, R₃ is (C₁-C₆)alkyl optionally substituted with one to three R₄, and each R₄ is phenyl optionally substituted with one to three R₇. In another embodiment, Rₓ is H, n is 0 or 1, each R₂ is independently (C₁-C₆)alkyl, R₁ is (C₁-C₆)alkoxy, halogen, -OH, -(CH₂)₀₋₂NH₂, or CN, R₃ is (C₁-C₆)alkyl substituted with one to three R₄, and each R₄ is phenyl optionally substituted with one to three R₇.

In some embodiments of the formulae above, Rₓ is H, n is 0 or 1, each R₂ is independently (C₁-C₆)alkyl, R₁ is (C₁-C₆)alkoxy, halogen, -OH, -(CH₂)₀₋₂NH₂, or CN, R₃ is (C₁-C₆)alkyl optionally substituted with one to three R₄, and each R₄ is 5- or 6-membered heteroaryl comprising 1 to 3 heteroatoms selected from O, N, and S, optionally substituted with one to three R₇. In another embodiment, Rₓ is H, n is 0 or 1, each R₂ is independently (C₁-C₆)alkyl, R₁ is (C₁-C₆)alkoxy, halogen, -OH, -(CH₂)₀₋₂NH₂, or CN, R₃ is (C₁-C₆)alkyl substituted with one to three R₄, and each R₄ is 5- or 6-membered heteroaryl comprising 1 to 3 heteroatoms selected from O, N, and S, optionally substituted with one to three R₇.

In some embodiments of the formulae above, Rₓ is H, n is 0 or 1, each R₂ is independently (C₁-C₆)alkyl, R₁ is (C₁-C₆)alkoxy, halogen, -OH, -(CH₂)₀₋₂NH₂, or CN, R₃ is (C₁-C₆)alkyl optionally substituted with one to three R₄, and each R₄ is (C₃-C₈)cycloalkyl optionally substituted with one to three R₇. In another embodiment, Rₓ is H, n is 0 or 1, each R₂ is independently (C₁-C₆)alkyl, R₁ is (C₁-C₆)alkoxy, halogen, -OH, -(CH₂)₀₋₂NH₂, or CN, R₃ is (C₁-C₆)alkyl substituted with one to three R₄, and each R₄ is (C₃-C₈)cycloalkyl optionally substituted with one to three R₇.

In some embodiments of the formulae above, Rₓ is H, n is 0 or 1, each R₂ is independently (C₁-C₆)alkyl, R₁ is (C₁-C₆)alkoxy, halogen, -OH, -(CH₂)₀₋₂NH₂, or CN, R₃ is (C₁-C₆)alkyl optionally substituted with one to three R₄, and each R₄ is 5- to 7-membered heterocycloalkyl comprising 1 to 3 heteroatoms selected from O, N, and S, optionally substituted with one to three R₇. In another embodiment, Rₓ is H, n is 0 or 1, each R₂ is independently (C₁-C₆)alkyl, R₁ is (C₁-C₆)alkoxy, halogen, -OH, -(CH₂)₀₋₂NH₂, or CN, R₃ is (C₁-C₆)alkyl substituted with one to three R₄, and each R₄ is 5- to 7-membered heterocycloalkyl comprising 1 to 3 heteroatoms selected from O, N, and S, optionally substituted with one to three R₇.

In some embodiments of the formulae above, Rₓ is H, n is 0 or 1, each R₂ is independently (C₁-C₆)alkyl, R₁ is (C₁-C₆)alkoxy, halogen, -OH, -(CH₂)₀₋₂NH₂, or CN, and R₃ is (C₆-C₁₀)aryl, (C₃-C₈)cycloalkyl, or 5- to 7-membered heterocycloalkyl comprising 1 to 3 heteroatoms selected from O, N, and S, wherein the aryl, cycloalkyl, and heterocycloalkyl are optionally substituted with one to three R₅.

In some embodiments of the formulae above, Rₓ is H, n is 0 or 1, each R₂ is independently (C₁-C₆)alkyl, R₁ is (C₁-C₆)alkoxy, halogen, -OH, -(CH₂)₀₋₂NH₂, or CN, and R₃ is (C₆-C₁₀)aryl, (C₃-C₈)cycloalkyl, or 5- to 7-membered heterocycloalkyl comprising 1 to 3 heteroatoms selected from O, N, and S.

In some embodiments of the formulae above, Rₓ is H, n is 0 or 1, each R₂ is independently (C₁-C₆)alkyl, R₁ is (C₁-C₆)alkoxy, halogen, -OH, -(CH₂)₀₋₂NH₂, or CN, and R₃ is (C₆-C₁₀)aryl, 5- or 6-membered heteroaryl comprising 1 to 3 heteroatoms selected from O, N, and S, or (C₃-C₈)cycloalkyl, wherein the aryl, heteroaryl and cycloalkyl are optionally substituted with one to three R₅.

In some embodiments of the formulae above, Rₓ is H, n is 0 or 1, each R₂ is independently (C₁-C₆)alkyl, R₁ is (C₁-C₆)alkoxy, halogen, -OH, -(CH₂)₀₋₂NH₂, or CN, and R₃ is (C₆-C₁₀)aryl, 5- or 6-membered heteroaryl comprising 1 to 3 heteroatoms selected from O, N, and S, or (C₃-C₈)cycloalkyl.

In some embodiments of the formulae above, Rₓ is H, n is 0 or 1, each R₂ is independently (C₁-C₆)alkyl, R₁ is (C₁-C₆)alkoxy, halogen, -OH, -(CH₂)₀₋₂NH₂, or CN, and R₃ is (C₆-C₁₀)aryl optionally substituted with one to three R₅.

In some embodiments of the formulae above, Rₓ is H, n is 0 or 1, each R₂ is independently (C₁-C₆)alkyl, R₁ is (C₁-C₆)alkoxy, halogen, -OH, -(CH₂)₀₋₂NH₂, or CN, and R₃ is 5- or 6-membered heteroaryl comprising 1 to 3 heteroatoms selected from O, N, and S optionally substituted with one to three R₅.

In some embodiments of the formulae above, Rₓ is H, n is 0 or 1, each R₂ is independently (C₁-C₆)alkyl, R₁ is (C₁-C₆)alkoxy, halogen, -OH, -(CH₂)₀₋₂NH₂, or CN, and R₃ is (C₃-C₈)cycloalkyl optionally substituted with one to three R₅.

In some embodiments of the formulae above, Rₓ is H, n is 0 or 1, each R₂ is independently (C₁-C₆)alkyl, R₁ is (C₁-C₆)alkoxy, halogen, -OH, -(CH₂)₀₋₂NH₂, or CN, and R₃ is 5- to 7-membered heterocycloalkyl comprising 1 to 3 heteroatoms selected from O, N, and S, optionally substituted with one to three R₅.

In some embodiments of the formulae above, Rₓ is H, n is 1 or 2, and each R₂ is independently (C₁-C₆)alkyl. In another embodiment, Rₓ is H, n is 1, each R₂ is independently (C₁-C₆)alkyl, and R₁ is (C₁-C₆)alkoxy, halogen, -OH, -(CH₂)₀₋₂NH₂, or CN. In another embodiment, Rₓ is H, n is 1 or 2, each R₂ is independently (C₁-C₆)alkyl, R₁ is (C₁-C₆)alkoxy, halogen, -OH, -(CH₂)₀₋₂NH₂, or CN, and R₃ is (C₁-C₆)alkyl optionally substituted with one to three R₄. In another embodiment, Rₓ is H, n is 1 or 2, each R₂ is independently (C₁-C₆)alkyl, R₁ is (C₁-C₆)alkoxy, halogen, -OH, -(CH₂)₀₋₂NH₂, or CN, and R₃ is (C₁-C₆)alkyl substituted with one to three R₄.

In some embodiments of the formulae above, Rₓ is H, n is 1 or 2, each R₂ is independently (C₁-C₆)alkyl, R₁ is (C₁-C₆)alkoxy, halogen, -OH, -(CH₂)₀₋₂NH₂, or CN, R₃ is (C₁-C₆)alkyl optionally substituted with one to three R₄, and each R₄ is independently selected from -C(O)OR₆, (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇. In another embodiment, Rₓ is H, n is 1 or 2, each R₂ is independently (C₁-C₆)alkyl, R₁ is (C₁-C₆)alkoxy, halogen, -OH, -(CH₂)₀₋₂NH₂, or CN, R₃ is (C₁-C₆)alkyl substituted with one to three R₄, and each R₄ is independently selected from -C(O)OR₆, (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇.

In some embodiments of the formulae above, Rₓ is H, n is 1 or 2, each R₂ is independently (C₁-C₆)alkyl, R₁ is (C₁-C₆)alkoxy, halogen, -OH, -(CH₂)₀₋₂NH₂, or CN, R₃ is (C₁-C₆)alkyl optionally substituted with one to three R₄, and each R₄ is independently selected from (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇. In another embodiment, Rₓ is H, n is 1 or 2, each R₂ is independently (C₁-C₆)alkyl, R₁ is (C₁-C₆)alkoxy, halogen, -OH, -(CH₂)₀₋₂NH₂, or CN, R₃ is (C₁-C₆)alkyl substituted with one to three R₄, and each R₄ is independently selected from (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇.

In some embodiments of the formulae above, Rₓ is H, n is 1 or 2, each R₂ is independently (C₁-C₆)alkyl, R₁ is (C₁-C₆)alkoxy, halogen, -OH, -(CH₂)₀₋₂NH₂, or CN, R₃ is (C₁-C₆)alkyl optionally substituted with one to three R₄, and each R₄ is independently selected from halogen, -OH, (C₆-C₁₀)aryl, 5- or 6-membered heteroaryl comprising 1 to 3 heteroatoms selected from O, N, and S, (C₃-C₈)cycloalkyl, and 5-to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇. In another embodiment, Rₓ is H, n is 1 or 2, each R₂ is independently (C₁-C₆)alkyl, R₁ is (C₁-C₆)alkoxy, halogen, -OH, -(CH₂)₀₋₂NH₂, or CN, R₃ is (C₁-C₆)alkyl substituted with one to three R₄, and each R₄ is independently selected from halogen, -OH, (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇.

In some embodiments of the formulae above, Rₓ is H, n is 1 or 2, each R₂ is independently (C₁-C₆)alkyl, R₁ is (C₁-C₆)alkoxy, halogen, -OH, -(CH₂)₀₋₂NH₂, or CN, R₃ is (C₁-C₆)alkyl optionally substituted with one to three R₄, and each R₄ is independently selected from halogen, -OH, phenyl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the phenyl, heteroaryl, cycloalkyl, and heterocycloalkyl groups are optionally substituted with one to three R₇. In another embodiment, Rₓ is H, n is 1 or 2, each R₂ is independently (C₁-C₆)alkyl, R₁ is (C₁-C₆)alkoxy, halogen, -OH, -(CH₂)₀₋₂NH₂, or CN, R₃ is (C₁-C₆)alkyl substituted with one to three R₄, and each R₄ is independently selected from halogen, -OH, phenyl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the phenyl, heteroaryl, cycloalkyl, and heterocycloalkyl groups are optionally substituted with one to three R₇.

In some embodiments of the formulae above, Rₓ is H, n is 1 or 2, each R₂ is independently (C₁-C₆)alkyl, R₁ is (C₁-C₆)alkoxy, halogen, -OH, -(CH₂)₀₋₂NH₂, or CN, R₃ is (C₁-C₆)alkyl optionally substituted with one to three R₄, and each R₄ is independently selected from phenyl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the phenyl, heteroaryl, cycloalkyl, and heterocycloalkyl groups are optionally substituted with one to three R₇. In another embodiment, Rₓ is H, n is 1 or 2, each R₂ is independently (C₁-C₆)alkyl, R₁ is (C₁-C₆)alkoxy, halogen, -OH, -(CH₂)₀₋₂NH₂, or CN, R₃ is (C₁-C₆)alkyl substituted with one to three R₄, and each R₄ is independently selected from phenyl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the phenyl, heteroaryl, cycloalkyl, and heterocycloalkyl groups are optionally substituted with one to three R₇.

In some embodiments of the formulae above, Rₓ is H, n is 1 or 2, each R₂ is independently (C₁-C₆)alkyl, R₁ is (C₁-C₆)alkoxy, halogen, -OH, -(CH₂)₀₋₂NH₂, or CN, R₃ is (C₁-C₆)alkyl optionally substituted with one to three R₄, and each R₄ is independently selected from phenyl and 5- or 6-membered heteroaryl comprising 1 to 3 heteroatoms selected from O, N, and S, wherein the phenyl and heteroaryl groups are optionally substituted with one to three R₇. In another embodiment, Rₓ is H, n is 1 or 2, each R₂ is independently (C₁-C₆)alkyl, R₁ is (C₁-C₆)alkoxy, halogen, -OH, -(CH₂)₀₋₂NH₂, or CN, R₃ is (C₁-C₆)alkyl substituted with one to three R₄, and each R₄ is independently selected from phenyl and 5- or 6-membered heteroaryl comprising 1 to 3 heteroatoms selected from O, N, and S, wherein the phenyl and heteroaryl groups are optionally substituted with one to three R₇.

In some embodiments of the formulae above, Rₓ is H, n is 1 or 2, each R₂ is independently (C₁-C₆)alkyl, R₁ is (C₁-C₆)alkoxy, halogen, -OH, -(CH₂)₀₋₂NH₂, or CN, R₃ is (C₁-C₆)alkyl optionally substituted with one to three R₄, and each R₄ is phenyl optionally substituted with one to three R₇. In another embodiment, Rₓ is H, n is 1 or 2, each R₂ is independently (C₁-C₆)alkyl, R₁ is (C₁-C₆)alkoxy, halogen, -OH, -(CH₂)₀₋₂NH₂, or CN, R₃ is (C₁-C₆)alkyl substituted with one to three R₄, and each R₄ is phenyl optionally substituted with one to three R₇.

In some embodiments of the formulae above, Rₓ is H, n is 1 or 2, each R₂ is independently (C₁-C₆)alkyl, R₁ is (C₁-C₆)alkoxy, halogen, -OH, -(CH₂)₀₋₂NH₂, or CN, R₃ is (C₁-C₆)alkyl optionally substituted with one to three R₄, and each R₄ is 5- or 6-membered heteroaryl comprising 1 to 3 heteroatoms selected from O, N, and S, optionally substituted with one to three R₇. In another embodiment, Rₓ is H, n is 1 or 2, each R₂ is independently (C₁-C₆)alkyl, R₁ is (C₁-C₆)alkoxy, halogen, -OH, -(CH₂)₀₋₂NH₂, or CN, R₃ is (C₁-C₆)alkyl substituted with one to three R₄, and each R₄ is 5- or 6-membered heteroaryl comprising 1 to 3 heteroatoms selected from O, N, and S, optionally substituted with one to three R₇.

In some embodiments of the formulae above, Rₓ is H, n is 1 or 2, each R₂ is independently (C₁-C₆)alkyl, R₁ is (C₁-C₆)alkoxy, halogen, -OH, -(CH₂)₀₋₂NH₂, or CN, R₃ is (C₁-C₆)alkyl optionally substituted with one to three R₄, and each R₄ is (C₃-C₈)cycloalkyl optionally substituted with one to three R₇. In another embodiment, Rₓ is H, n is 1 or 2, each R₂ is independently (C₁-C₆)alkyl, R₁ is (C₁-C₆)alkoxy, halogen, -OH, -(CH₂)₀₋₂NH₂, or CN, R₃ is (C₁-C₆)alkyl substituted with one to three R₄, and each R₄ is (C₃-C₈)cycloalkyl optionally substituted with one to three R₇.

In some embodiments of the formulae above, Rₓ is H, n is 1 or 2, each R₂ is independently (C₁-C₆)alkyl, R₁ is (C₁-C₆)alkoxy, halogen, -OH, -(CH₂)₀₋₂NH₂, or CN, R₃ is (C₁-C₆)alkyl optionally substituted with one to three R₄, and each R₄ is 5- to 7-membered heterocycloalkyl comprising 1 to 3 heteroatoms selected from O, N, and S, optionally substituted with one to three R₇. In another embodiment, Rₓ is H, n is 1 or 2, each R₂ is independently (C₁-C₆)alkyl, R₁ is (C₁-C₆)alkoxy, halogen, -OH, -(CH₂)₀₋₂NH₂, or CN, R₃ is (C₁-C₆)alkyl substituted with one to three R₄, and each R₄ is 5- to 7-membered heterocycloalkyl comprising 1 to 3 heteroatoms selected from O, N, and S, optionally substituted with one to three R₇.

In some embodiments of the formulae above, Rₓ is H, n is 1 or 2, each R₂ is independently (C₁-C₆)alkyl, R₁ is (C₁-C₆)alkoxy, halogen, -OH, -(CH₂)₀₋₂NH₂, or CN, and R₃ is (C₆-C₁₀)aryl, (C₃-C₈)cycloalkyl, or 5- to 7-membered heterocycloalkyl comprising 1 to 3 heteroatoms selected from O, N, and S, wherein the aryl, cycloalkyl, and heterocycloalkyl are optionally substituted with one to three R₅.

In some embodiments of the formulae above, Rₓ is H, n is 1 or 2, each R₂ is independently (C₁-C₆)alkyl, R₁ is (C₁-C₆)alkoxy, halogen, -OH, -(CH₂)₀₋₂NH₂, or CN, and R₃ is (C₆-C₁₀)aryl, (C₃-C₈)cycloalkyl, or 5- to 7-membered heterocycloalkyl comprising 1 to 3 heteroatoms selected from O, N, and S.

In some embodiments of the formulae above, Rₓ is H, n is 1 or 2, each R₂ is independently (C₁-C₆)alkyl, R₁ is (C₁-C₆)alkoxy, halogen, -OH, -(CH₂)₀₋₂NH₂, or CN, and R₃ is (C₆-C₁₀)aryl, 5- or 6-membered heteroaryl comprising 1 to 3 heteroatoms selected from O, N, and S, or (C₃-C₈)cycloalkyl, wherein the aryl, heteroaryl and cycloalkyl are optionally substituted with one to three R₅.

In some embodiments of the formulae above, Rₓ is H, n is 1 or 2, each R₂ is independently (C₁-C₆)alkyl, R₁ is (C₁-C₆)alkoxy, halogen, -OH, -(CH₂)₀₋₂NH₂, or CN, and R₃ is (C₆-C₁₀)aryl, 5- or 6-membered heteroaryl comprising 1 to 3 heteroatoms selected from O, N, and S, or (C₃-C₈)cycloalkyl.

In some embodiments of the formulae above, Rₓ is H, n is 1 or 2, each R₂ is independently (C₁-C₆)alkyl, R₁ is (C₁-C₆)alkoxy, halogen, -OH, -(CH₂)₀₋₂NH₂, or CN, and R₃ is (C₆-C₁₀)aryl optionally substituted with one to three R₅.

In some embodiments of the formulae above, Rₓ is H, n is 1 or 2, each R₂ is independently (C₁-C₆)alkyl, R₁ is (C₁-C₆)alkoxy, halogen, -OH, -(CH₂)₀₋₂NH₂, or CN, and R₃ is 5- or 6-membered heteroaryl comprising 1 to 3 heteroatoms selected from O, N, and S optionally substituted with one to three R₅.

In some embodiments of the formulae above, Rₓ is H, n is 1 or 2, each R₂ is independently (C₁-C₆)alkyl, R₁ is (C₁-C₆)alkoxy, halogen, -OH, -(CH₂)₀₋₂NH₂, or CN, and R₃ is (C₃-C₈)cycloalkyl optionally substituted with one to three R₅.

In some embodiments of the formulae above, Rₓ is H, n is 1 or 2, each R₂ is independently (C₁-C₆)alkyl, R₁ is (C₁-C₆)alkoxy, halogen, -OH, -(CH₂)₀₋₂NH₂, or CN, and R₃ is 5- to 7-membered heterocycloalkyl comprising 1 to 3 heteroatoms selected from O, N, and S, optionally substituted with one to three R₅.

In some embodiments of the formulae above, Rₓ is H, n is 2, and each R₂ is independently (C₁-C₆)alkyl. In another embodiment, Rₓ is H, n is 2, each R₂ is independently (C₁-C₆)alkyl, and R₁ is (C₁-C₆)alkoxy, halogen, -OH, -(CH₂)₀₋₂NH₂, or CN. In another embodiment, Rₓ is H, n is 2, each R₂ is independently (C₁-C₆)alkyl, R₁ is (C₁-C₆)alkoxy, halogen, -OH, -(CH₂)₀₋₂NH₂, or CN, and R₃ is (C₁-C₆)alkyl optionally substituted with one to three R₄. In another embodiment, Rₓ is H, n is 2, each R₂ is independently (C₁-C₆)alkyl, R₁ is (C₁-C₆)alkoxy, halogen, -OH, -(CH₂)₀₋₂NH₂, or CN, and R₃ is (C₁-C₆)alkyl substituted with one to three R₄.

In some embodiments of the formulae above, Rₓ is H, n is 2, each R₂ is independently (C₁-C₆)alkyl, R₁ is (C₁-C₆)alkoxy, halogen, -OH, -(CH₂)₀₋₂NH₂, or CN, R₃ is (C₁-C₆)alkyl optionally substituted with one to three R₄, and each R₄ is independently selected from -C(O)OR₆, (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇. In another embodiment, Rₓ is H, n is 2, each R₂ is independently (C₁-C₆)alkyl, R₁ is (C₁-C₆)alkoxy, halogen, -OH, -(CH₂)₀₋₂NH₂, or CN, R₃ is (C₁-C₆)alkyl substituted with one to three R₄, and each R₄ is independently selected from -C(O)OR₆, (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇.

In some embodiments of the formulae above, Rₓ is H, n is 2, each R₂ is independently (C₁-C₆)alkyl, R₁ is (C₁-C₆)alkoxy, halogen, -OH, -(CH₂)₀₋₂NH₂, or CN, R₃ is (C₁-C₆)alkyl optionally substituted with one to three R₄, and each R₄ is independently selected from (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇. In another embodiment, Rₓ is H, n is 2, each R₂ is independently (C₁-C₆)alkyl, R₁ is (C₁-C₆)alkoxy, halogen, -OH, - (CH₂)₀₋₂NH₂, or CN, R₃ is (C₁-C₆)alkyl substituted with one to three R₄, and each R₄ is independently selected from (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇.

In some embodiments of the formulae above, Rₓ is H, n is 2, each R₂ is independently (C₁-C₆)alkyl, R₁ is (C₁-C₆)alkoxy, halogen, -OH, -(CH₂)₀₋₂NH₂, or CN, R₃ is (C₁-C₆)alkyl optionally substituted with one to three R₄, and each R₄ is independently selected from halogen, -OH, (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇. In another embodiment, Rₓ is H, n is 2, each R₂ is independently (C₁-C₆)alkyl, R₁ is (C₁-C₆)alkoxy, halogen, -OH, -(CH₂)₀₋₂NH₂, or CN, R₃ is (C₁-C₆)alkyl substituted with one to three R₄, and each R₄ is independently selected from halogen, -OH, (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇.

In some embodiments of the formulae above, Rₓ is H, n is 2, each R₂ is independently (C₁-C₆)alkyl, R₁ is (C₁-C₆)alkoxy, halogen, -OH, -(CH₂)₀₋₂NH₂, or CN, R₃ is (C₁-C₆)alkyl optionally substituted with one to three R₄, and each R₄ is independently selected from halogen, -OH, phenyl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇. In another embodiment, Rₓ is H, n is 2, each R₂ is independently (C₁-C₆)alkyl, R₁ is (C₁-C₆)alkoxy, halogen, -OH, - (CH₂)₀₋₂NH₂, or CN, R₃ is (C₁-C₆)alkyl substituted with one to three R₄, and each R₄ is independently selected from halogen, -OH, phenyl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the phenyl, heteroaryl, cycloalkyl, and heterocycloalkyl groups are optionally substituted with one to three R₇.

In some embodiments of the formulae above, Rₓ is H, n is 2, each R₂ is independently (C₁-C₆)alkyl, R₁ is (C₁-C₆)alkoxy, halogen, -OH, -(CH₂)₀₋₂NH₂, or CN, R₃ is (C₁-C₆)alkyl optionally substituted with one to three R₄, and each R₄ is independently selected from phenyl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the phenyl, heteroaryl, cycloalkyl, and heterocycloalkyl groups are optionally substituted with one to three R₇. In another embodiment, Rₓ is H, n is 2, each R₂ is independently (C₁-C₆)alkyl, R₁ is (C₁-C₆)alkoxy, halogen, -OH, -(CH₂)₀₋₂NH₂, or CN, R₃ is (C_{I}-C₆)alkyl substituted with one to three R₄, and each R₄ is independently selected from phenyl, 5- or 6-membered heteroaryl comprising 1 to 3 heteroatoms selected from O, N, and S, (C₃-C₈)cycloalkyl, and 5-to 7-membered heterocycloalkyl comprising 1 to 3 heteroatoms selected from O, N, and S, wherein the phenyl, heteroaryl, cycloalkyl, and heterocycloalkyl groups are optionally substituted with one to three R₇.

In some embodiments of the formulae above, Rₓ is H, n is 2, each R₂ is independently (C₁-C₆)alkyl, R₁ is (C₁-C₆)alkoxy, halogen, -OH, -(CH₂)₀₋₂NH₂, or CN, R₃ is (C₁-C₆)alkyl optionally substituted with one to three R₄, and each R₄ is independently selected from phenyl and 5- or 6-membered heteroaryl comprising 1 to 3 heteroatoms selected from O, N, and S, wherein the aryl and heteroaryl groups are optionally substituted with one to three R₇. In another embodiment, Rₓ is H, n is 2, each R₂ is independently (C₁-C₆)alkyl, R₁ is (C₁-C₆)alkoxy, halogen, -OH, -(CH₂)₀₋₂NH₂, or CN, R₃ is (C_{I}-C₆)alkyl substituted with one to three R₄, and each R₄ is independently selected from phenyl and 5- or 6-membered heteroaryl comprising 1 to 3 heteroatoms selected from O, N, and S, wherein the aryl and heteroaryl groups are optionally substituted with one to three R₇.

In some embodiments of the formulae above, Rₓ is H, n is 2, each R₂ is independently (C₁-C₆)alkyl, R₁ is (C₁-C₆)alkoxy, halogen, -OH, -(CH₂)₀₋₂NH₂, or CN, R₃ is (C₁-C₆)alkyl optionally substituted with one to three R₄, and each R₄ is phenyl optionally substituted with one to three R₇. In another embodiment, Rₓ is H, n is 2, each R₂ is independently (C₁-C₆)alkyl, R₁ is (C₁-C₆)alkoxy, halogen, -OH, -(CH₂)₀₋₂NH₂, or CN, R₃ is (C₁-C₆)alkyl substituted with one to three R₄, and each R₄ is phenyl optionally substituted with one to three R₇.

In some embodiments of the formulae above, Rₓ is H, n is 2, each R₂ is independently (C₁-C₆)alkyl, R₁ is (C₁-C₆)alkoxy, halogen, -OH, -(CH₂)₀₋₂NH₂, or CN, R₃ is (C₁-C₆)alkyl optionally substituted with one to three R₄, and each R₄ is 5- or 6-membered heteroaryl comprising 1 to 3 heteroatoms selected from O, N, and S, optionally substituted with one to three R₇. In another embodiment, Rₓ is H, n is 2, each R₂ is independently (C₁-C₆)alkyl, R₁ is (C₁-C₆)alkoxy, halogen, -OH, -(CH₂)₀₋₂NH₂, or CN, R₃ is (C₁-C₆)alkyl substituted with one to three R₄, and each R₄ is 5- or 6-membered heteroaryl comprising 1 to 3 heteroatoms selected from O, N, and S, optionally substituted with one to three R₇.

In some embodiments of the formulae above, Rₓ is H, n is 2, each R₂ is independently (C₁-C₆)alkyl, R₁ is (C₁-C₆)alkoxy, halogen, -OH, -(CH₂)₀₋₂NH₂, or CN, R₃ is (C₁-C₆)alkyl optionally substituted with one to three R₄, and each R₄ is (C₃-C₈)cycloalkyl optionally substituted with one to three R₇. In another embodiment, Rₓ is H, n is 2, each R₂ is independently (C₁-C₆)alkyl, R₁ is (C₁-C₆)alkoxy, halogen, -OH, - (CH₂)₀₋₂NH₂, or CN, R₃ is (C₁-C₆)alkyl substituted with one to three R₄, and each R₄ is (C₃-C₈)cycloalkyl optionally substituted with one to three R₇.

In some embodiments of the formulae above, Rₓ is H, n is 2, each R₂ is independently (C₁-C₆)alkyl, R₁ is (C₁-C₆)alkoxy, halogen, -OH, -(CH₂)₀₋₂NH₂, or CN, R₃ is (C₁-C₆)alkyl optionally substituted with one to three R₄, and each R₄ is 5- to 7-membered heterocycloalkyl comprising 1 to 3 heteroatoms selected from O, N, and S, optionally substituted with one to three R₇. In another embodiment, Rₓ is H, n is 2, each R₂ is independently (C₁-C₆)alkyl, R₁ is (C₁-C₆)alkoxy, halogen, -OH, -(CH₂)₀₋₂NH₂, or CN, R₃ is (C₁-C₆)alkyl substituted with one to three R₄, and each R₄ is 5- to 7-membered heterocycloalkyl comprising 1 to 3 heteroatoms selected from O, N, and S, optionally substituted with one to three R₇.

In some embodiments of the formulae above, Rₓ is H, n is 2, each R₂ is independently (C₁-C₆)alkyl, R₁ is (C₁-C₆)alkoxy, halogen, -OH, -(CH₂)₀₋₂NH₂, or CN, and R₃ is (C₆-C₁₀)aryl, (C₃-C₈)cycloalkyl, or 5- to 7-membered heterocycloalkyl comprising 1 to 3 heteroatoms selected from O, N, and S, wherein the aryl, cycloalkyl, and heterocycloalkyl are optionally substituted with one to three R₅.

In some embodiments of the formulae above, Rₓ is H, n is 2, each R₂ is independently (C₁-C₆)alkyl, R₁ is (C₁-C₆)alkoxy, halogen, -OH, -(CH₂)₀₋₂NH₂, or CN, and R₃ is (C₆-C₁₀)aryl, (C₃-C₈)cycloalkyl, or 5- to 7-membered heterocycloalkyl comprising 1 to 3 heteroatoms selected from O, N, and S.

In some embodiments of the formulae above, Rₓ is H, n is 2, each R₂ is independently (C₁-C₆)alkyl, R₁ is (C₁-C₆)alkoxy, halogen, -OH, -(CH₂)₀₋₂NH₂, or CN, and R₃ is (C₆-C₁₀)aryl, 5- or 6-membered heteroaryl comprising 1 to 3 heteroatoms selected from O, N, and S, or (C₃-C₈)cycloalkyl, wherein the aryl, heteroaryl and cycloalkyl are optionally substituted with one to three R₅.

In some embodiments of the formulae above, Rₓ is H, n is 2, each R₂ is independently (C₁-C₆)alkyl, R₁ is (C₁-C₆)alkoxy, halogen, -OH, -(CH₂)₀₋₂NH₂, or CN, and R₃ is (C₆-C₁₀)aryl, 5- or 6-membered heteroaryl comprising 1 to 3 heteroatoms selected from O, N, and S, or (C₃-C₈)cycloalkyl.

In some embodiments of the formulae above, Rₓ is H, n is 2, each R₂ is independently (C₁-C₆)alkyl, R₁ is (C₁-C₆)alkoxy, halogen, -OH, -(CH₂)₀₋₂NH₂, or CN, and R₃ is (C₆-C₁₀)aryl optionally substituted with one to three R₅.

In some embodiments of the formulae above, Rₓ is H, n is 2, each R₂ is independently (C₁-C₆)alkyl, R₁ is (C₁-C₆)alkoxy, halogen, -OH, -(CH₂)₀₋₂NH₂, or CN, and R₃ is 5- or 6-membered heteroaryl comprising 1 to 3 heteroatoms selected from O, N, and S optionally substituted with one to three R₅.

In some embodiments of the formulae above, Rₓ is H, n is 2, each R₂ is independently (C₁-C₆)alkyl, R₁ is (C₁-C₆)alkoxy, halogen, -OH, -(CH₂)₀₋₂NH₂, or CN, and R₃ is (C₃-C₈)cycloalkyl optionally substituted with one to three R₅.

In some embodiments of the formulae above, Rₓ is H, n is 2, each R₂ is independently (C₁-C₆)alkyl, R₁ is (C₁-C₆)alkoxy, halogen, -OH, -(CH₂)₀₋₂NH₂, or CN, and R₃ is 5- to 7-membered heterocycloalkyl comprising 1 to 3 heteroatoms selected from O, N, and S, optionally substituted with one to three R₅.

In some embodiments of the formulae above, Rₓ is H, n is 2, and each R₂ is independently (C₁-C₆)alkyl, or two R₂ together with the carbon atoms to which they are attached form (C₃-C₇)cycloalkyl or a 4- to 6- membered heterocycloalkyl ring comprising 1 to 3 heteroatoms selected from O, N, and S. In another embodiment, Rₓ is H, n is 2, each R₂ is independently (C₁-C₆)alkyl, or two R₂ together with the carbon atoms to which they are attached form (C₃-C₇)cycloalkyl or a 4- to 6- membered heterocycloalkyl ring comprising 1 to 3 heteroatoms selected from O, N, and S, and R₁ is (C₁-C₆)alkoxy, halogen, -OH, - (CH₂)₀₋₂NH₂, or CN. In another embodiment, Rₓ is H, n is 2, each R₂ is independently (C₁-C₆)alkyl, or two R₂ together with the carbon atoms to which they are attached form (C₃-C₇)cycloalkyl or a 4- to 6- membered heterocycloalkyl ring comprising 1 to 3 heteroatoms selected from O, N, and S, R₁ is (C₁-C₆)alkoxy, halogen, -OH, -(CH₂)₀₋₂NH₂, or CN, and R₃ is (C₁-C₆)alkyl optionally substituted with one to three R₄. In another embodiment, Rₓ is H, n is 2, each R₂ is independently (C₁-C₆)alkyl, or two R₂ together with the carbon atoms to which they are attached form (C₃-C₇)cycloalkyl or a 4- to 6- membered heterocycloalkyl ring comprising 1 to 3 heteroatoms selected from O, N, and S, R₁ is (C₁-C₆)alkoxy, halogen, -OH, -(CH₂)₀₋₂NH₂, or CN, and R₃ is (C_{I}-C₆)alkyl substituted with one to three R₄.

In some embodiments of the formulae above, Rₓ is H, n is 2, each R₂ is independently (C₁-C₆)alkyl, or two R₂ together with the carbon atoms to which they are attached form (C₃-C₇)cycloalkyl or a 4- to 6-membered heterocycloalkyl ring comprising 1 to 3 heteroatoms selected from O, N, and S, R₁ is (C₁-C₆)alkoxy, halogen, -OH, -(CH₂)₀₋₂NH₂, or CN, R₃ is (C₁-C₆)alkyl optionally substituted with one to three R₄, and each R₄ is independently selected from -C(O)OR₆, (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇. In another embodiment, Rₓ is H, n is 2, each R₂ is independently (C₁-C₆)alkyl, or two R₂ together with the carbon atoms to which they are attached form (C₃-C₇)cycloalkyl or a 4- to 6- membered heterocycloalkyl ring comprising 1 to 3 heteroatoms selected from O, N, and S, R₁ is (C₁-C₆)alkoxy, halogen, -OH, -(CH₂)₀₋₂NH₂, or CN, R₃ is (C₁-C₆)alkyl substituted with one to three R₄, and each R₄ is independently selected from - C(O)OR₆, (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇.

In some embodiments of the formulae above, Rₓ is H, n is 2, each R₂ is independently (C₁-C₆)alkyl, or two R₂ together with the carbon atoms to which they are attached form (C₃-C₇)cycloalkyl or a 4- to 6-membered heterocycloalkyl ring comprising 1 to 3 heteroatoms selected from O, N, and S, R₁ is (C₁-C₆)alkoxy, halogen, -OH, -(CH₂)₀₋₂NH₂, or CN, R₃ is (C₁-C₆)alkyl optionally substituted with one to three R₄, and each R₄ is independently selected from (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇. In another embodiment, Rₓ is H, n is 2, each R₂ is independently (C₁-C₆)alkyl, or two R₂ together with the carbon atoms to which they are attached form (C₃-C₇)cycloalkyl or a 4- to 6- membered heterocycloalkyl ring comprising 1 to 3 heteroatoms selected from O, N, and S, R₁ is (C₁-C₆)alkoxy, halogen, -OH, -(CH₂)₀₋₂NH₂, or CN, R₃ is (C₁-C₆)alkyl substituted with one to three R₄, and each R₄ is independently selected from (C₆-C₁₀)aryl, 5- or 6-membered heteroaryl comprising 1 to 3 heteroatoms selected from O, N, and S, (C₃-C₈)cycloalkyl, and 5-to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇.

In some embodiments of the formulae above, Rₓ is H, n is 2, each R₂ is independently (C₁-C₆)alkyl, or two R₂ together with the carbon atoms to which they are attached form (C₃-C₇)cycloalkyl or a 4- to 6-membered heterocycloalkyl ring comprising 1 to 3 heteroatoms selected from O, N, and S, R₁ is (C₁-C₆)alkoxy, halogen, -OH, -(CH₂)₀₋₂NH₂, or CN, R₃ is (C₁-C₆)alkyl optionally substituted with one to three R₄, and each R₄ is independently selected from halogen, -OH, (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇. In another embodiment, Rₓ is H, n is 2, each R₂ is independently (C₁-C₆)alkyl, or two R₂ together with the carbon atoms to which they are attached form (C₃-C₇)cycloalkyl or a 4- to 6- membered heterocycloalkyl ring comprising 1 to 3 heteroatoms selected from O, N, and S, R₁ is (C₁-C₆)alkoxy, halogen, -OH, -(CH₂)₀₋₂NH₂, or CN, R₃ is (C₁-C₆)alkyl substituted with one to three R₄, and each R₄ is independently selected from halogen, -OH, (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇.

In some embodiments of the formulae above, Rₓ is H, n is 2, each R₂ is independently (C₁-C₆)alkyl, or two R₂ together with the carbon atoms to which they are attached form (C₃-C₇)cycloalkyl or a 4- to 6-membered heterocycloalkyl ring comprising 1 to 3 heteroatoms selected from O, N, and S, R₁ is (C₁-C₆)alkoxy, halogen, -OH, -(CH₂)₀₋₂NH₂, or CN, R₃ is (C₁-C₆)alkyl optionally substituted with one to three R₄, and each R₄ is independently selected from halogen, -OH, phenyl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the phenyl, heteroaryl, cycloalkyl, and heterocycloalkyl groups are optionally substituted with one to three R₇. In another embodiment, Rₓ is H, n is 2, each R₂ is independently (C₁-C₆)alkyl, or two R₂ together with the carbon atoms to which they are attached form (C₃-C₇)cycloalkyl or a 4- to 6- membered heterocycloalkyl ring comprising 1 to 3 heteroatoms selected from O, N, and S, R₁ is (C₁-C₆)alkoxy, halogen, -OH, -(CH₂)₀₋₂NH₂, or CN, R₃ is (C₁-C₆)alkyl substituted with one to three R₄, and each R₄ is independently selected from halogen, -OH, phenyl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the phenyl, heteroaryl, cycloalkyl, and heterocycloalkyl groups are optionally substituted with one to three R₇.

In some embodiments of the formulae above, Rₓ is H, n is 2, each R₂ is independently (C₁-C₆)alkyl, or two R₂ together with the carbon atoms to which they are attached form (C₃-C₇)cycloalkyl or a 4- to 6-membered heterocycloalkyl ring comprising 1 to 3 heteroatoms selected from O, N, and S, R₁ is (C₁-C₆)alkoxy, halogen, -OH, -(CH₂)₀₋₂NH₂, or CN, R₃ is (C₁-C₆)alkyl optionally substituted with one to three R₄, and each R₄ is independently selected from phenyl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the phenyl, heteroaryl, cycloalkyl, and heterocycloalkyl groups are optionally substituted with one to three R₇. In another embodiment, Rₓ is H, n is 2, each R₂ is independently (C₁-C₆)alkyl, or two R₂ together with the carbon atoms to which they are attached form (C₃-C₇)cycloalkyl or a 4- to 6- membered heterocycloalkyl ring comprising 1 to 3 heteroatoms selected from O, N, and S, R₁ is (C₁-C₆)alkoxy, halogen, -OH, -(CH₂)₀₋₂NH₂, or CN, R₃ is (C₁-C₆)alkyl substituted with one to three R₄, and each R₄ is independently selected from phenyl, 5- or 6-membered heteroaryl comprising 1 to 3 heteroatoms selected from O, N, and S, (C₃-C₈)cycloalkyl, and 5-to 7-membered heterocycloalkyl comprising 1 to 3 heteroatoms selected from O, N, and S, wherein the phenyl, heteroaryl, cycloalkyl, and heterocycloalkyl groups are optionally substituted with one to three R₇.

In some embodiments of the formulae above, Rₓ is H, n is 2, each R₂ is independently (C₁-C₆)alkyl, or two R₂ together with the carbon atoms to which they are attached form (C₃-C₇)cycloalkyl or a 4- to 6-membered heterocycloalkyl ring comprising 1 to 3 heteroatoms selected from O, N, and S, R₁ is (C₁-C₆)alkoxy, halogen, -OH, -(CH₂)₀₋₂NH₂, or CN, R₃ is (C₁-C₆)alkyl optionally substituted with one to three R₄, and each R₄ is independently selected from phenyl and 5- or 6-membered heteroaryl comprising 1 to 3 heteroatoms selected from O, N, and S, wherein the phenyl and heteroaryl groups are optionally substituted with one to three R₇. In another embodiment, Rₓ is H, n is 2, each R₂ is independently (C₁-C₆)alkyl, or two R₂ together with the carbon atoms to which they are attached form (C₃-C₇)cycloalkyl or a 4- to 6- membered heterocycloalkyl ring comprising 1 to 3 heteroatoms selected from O, N, and S, R₁ is (C₁-C₆)alkoxy, halogen, -OH, -(CH₂)₀₋₂NH₂, or CN, R₃ is (C₁-C₆)alkyl substituted with one to three R₄, and each R₄ is independently selected from phenyl and 5- or 6-membered heteroaryl comprising 1 to 3 heteroatoms selected from O, N, and S, wherein the phenyl and heteroaryl groups are optionally substituted with one to three R₇.

In some embodiments of the formulae above, Rₓ is H, n is 2, each R₂ is independently (C₁-C₆)alkyl, or two R₂ together with the carbon atoms to which they are attached form (C₃-C₇)cycloalkyl or a 4- to 6-membered heterocycloalkyl ring comprising 1 to 3 heteroatoms selected from O, N, and S, R₁ is (C₁-C₆)alkoxy, halogen, -OH, -(CH₂)₀₋₂NH₂, or CN, R₃ is (C₁-C₆)alkyl optionally substituted with one to three R₄, and each R₄ is phenyl optionally substituted with one to three R₇. In another embodiment, Rₓ is H, n is 2, each R₂ is independently (C₁-C₆)alkyl, or two R₂ together with the carbon atoms to which they are attached form (C₃-C₇)cycloalkyl or a 4- to 6- membered heterocycloalkyl ring comprising 1 to 3 heteroatoms selected from O, N, and S, R₁ is (C₁-C₆)alkoxy, halogen, -OH, -(CH₂)₀₋₂NH₂, or CN, R₃ is (C₁-C₆)alkyl substituted with one to three R₄, and each R₄ is phenyl optionally substituted with one to three R₇.

In some embodiments of the formulae above, Rₓ is H, n is 2, each R₂ is independently (C₁-C₆)alkyl, or two R₂ together with the carbon atoms to which they are attached form (C₃-C₇)cycloalkyl or a 4- to 6-membered heterocycloalkyl ring comprising 1 to 3 heteroatoms selected from O, N, and S, R₁ is (C₁-C₆)alkoxy, halogen, -OH, -(CH₂)₀₋₂NH₂, or CN, R₃ is (C₁-C₆)alkyl optionally substituted with one to three R₄, and each R₄ is 5- or 6-membered heteroaryl comprising 1 to 3 heteroatoms selected from O, N, and S, optionally substituted with one to three R₇. In another embodiment, Rₓ is H, n is 2, each R₂ is independently (C₁-C₆)alkyl, or two R₂ together with the carbon atoms to which they are attached form (C₃-C₇)cycloalkyl or a 4- to 6- membered heterocycloalkyl ring comprising 1 to 3 heteroatoms selected from O, N, and S, R₁ is (C₁-C₆)alkoxy, halogen, -OH, -(CH₂)₀₋₂NH₂, or CN, R₃ is (C₁-C₆)alkyl substituted with one to three R₄, and each R₄ is 5- or 6-membered heteroaryl comprising 1 to 3 heteroatoms selected from O, N, and S, optionally substituted with one to three R₇.

In some embodiments of the formulae above, Rₓ is H, n is 2, each R₂ is independently (C₁-C₆)alkyl, or two R₂ together with the carbon atoms to which they are attached form (C₃-C₇)cycloalkyl or a 4- to 6-membered heterocycloalkyl ring comprising 1 to 3 heteroatoms selected from O, N, and S, R₁ is (C₁-C₆)alkoxy, halogen, -OH, -(CH₂)₀₋₂NH₂, or CN, R₃ is (C₁-C₆)alkyl optionally substituted with one to three R₄, and each R₄ is (C₃-C₈)cycloalkyl optionally substituted with one to three R₇. In another embodiment, Rₓ is H, n is 2, each R₂ is independently (C₁-C₆)alkyl, or two R₂ together with the carbon atoms to which they are attached form (C₃-C₇)cycloalkyl or a 4- to 6- membered heterocycloalkyl ring comprising 1 to 3 heteroatoms selected from O, N, and S, R₁ is (C₁-C₆)alkoxy, halogen, -OH, -(CH₂)₀₋₂NH₂, or CN, R₃ is (C₁-C₆)alkyl substituted with one to three R₄, and each R₄ is (C₃-C₈)cycloalkyl optionally substituted with one to three R₇.

In some embodiments of the formulae above, Rₓ is H, n is 2, each R₂ is independently (C₁-C₆)alkyl, or two R₂ together with the carbon atoms to which they are attached form (C₃-C₇)cycloalkyl or a 4- to 6-membered heterocycloalkyl ring comprising 1 to 3 heteroatoms selected from O, N, and S, R₁ is (C₁-C₆)alkoxy, halogen, -OH, -(CH₂)₀₋₂NH₂, or CN, R₃ is (C₁-C₆)alkyl optionally substituted with one to three R₄, and each R₄ is 5- to 7-membered heterocycloalkyl comprising 1 to 3 heteroatoms selected from O, N, and S, optionally substituted with one to three R₇. In another embodiment, Rₓ is H, n is 2, each R₂ is independently (C₁-C₆)alkyl, or two R₂ together with the carbon atoms to which they are attached form (C₃-C₇)cycloalkyl or a 4- to 6- membered heterocycloalkyl ring comprising 1 to 3 heteroatoms selected from O, N, and S, R₁ is (C₁-C₆)alkoxy, halogen, -OH, -(CH₂)₀₋₂NH₂, or CN, R₃ is (C₁-C₆)alkyl substituted with one to three R₄, and each R₄ is 5- to 7-membered heterocycloalkyl comprising 1 to 3 heteroatoms selected from O, N, and S, optionally substituted with one to three R₇.

In some embodiments of the formulae above, Rₓ is H, n is 2, each R₂ is independently (C₁-C₆)alkyl, or two R₂ together with the carbon atoms to which they are attached form (C₃-C₇)cycloalkyl or a 4- to 6-membered heterocycloalkyl ring comprising 1 to 3 heteroatoms selected from O, N, and S, R₁ is (C₁-C₆)alkoxy, halogen, -OH, -(CH₂)₀₋₂NH₂, or CN, and R₃ is (C₆-C₁₀)aryl, (C₃-C₈)cycloalkyl, or 5- to 7-membered heterocycloalkyl comprising 1 to 3 heteroatoms selected from O, N, and S, wherein the aryl, cycloalkyl, and heterocycloalkyl are optionally substituted with one to three R₅.

In some embodiments of the formulae above, Rₓ is H, n is 2, each R₂ is independently (C₁-C₆)alkyl, or two R₂ together with the carbon atoms to which they are attached form (C₃-C₇)cycloalkyl or a 4- to 6-membered heterocycloalkyl ring comprising 1 to 3 heteroatoms selected from O, N, and S, R₁ is (C₁-C₆)alkoxy, halogen, -OH, -(CH₂)₀₋₂NH₂, or CN, and R₃ is (C₆-C₁₀)aryl, (C₃-C₈)cycloalkyl, or 5- to 7-membered heterocycloalkyl comprising 1 to 3 heteroatoms selected from O, N, and S.

In some embodiments of the formulae above, Rₓ is H, n is 2, each R₂ is independently (C₁-C₆)alkyl, or two R₂ together with the carbon atoms to which they are attached form (C₃-C₇)cycloalkyl or a 4- to 6-membered heterocycloalkyl ring comprising 1 to 3 heteroatoms selected from O, N, and S, R₁ is (C₁-C₆)alkoxy, halogen, -OH, -(CH₂)₀₋₂NH₂, or CN, and R₃ is (C₆-C₁₀)aryl, 5- or 6-membered heteroaryl comprising 1 to 3 heteroatoms selected from O, N, and S, or (C₃-C₈)cycloalkyl, wherein the aryl, heteroaryl and cycloalkyl are optionally substituted with one to three R₅.

In some embodiments of the formulae above, Rₓ is H, n is 2, each R₂ is independently (C₁-C₆)alkyl, or two R₂ together with the carbon atoms to which they are attached form (C₃-C₇)cycloalkyl or a 4- to 6-membered heterocycloalkyl ring comprising 1 to 3 heteroatoms selected from O, N, and S, R₁ is (C₁-C₆)alkoxy, halogen, -OH, -(CH₂)₀₋₂NH₂, or CN, and R₃ is (C₆-C₁₀)aryl, 5- or 6-membered heteroaryl comprising 1 to 3 heteroatoms selected from O, N, and S, or (C₃-C₈)cycloalkyl.

In some embodiments of the formulae above, Rₓ is H, n is 2, each R₂ is independently (C₁-C₆)alkyl, or two R₂ together with the carbon atoms to which they are attached form (C₃-C₇)cycloalkyl or a 4- to 6-membered heterocycloalkyl ring comprising 1 to 3 heteroatoms selected from O, N, and S, R₁ is (C₁-C₆)alkoxy, halogen, -OH, -(CH₂)₀₋₂NH₂, or CN, and R₃ is (C₆-C₁₀)aryl optionally substituted with one to three R₅.

In some embodiments of the formulae above, Rₓ is H, n is 2, each R₂ is independently (C₁-C₆)alkyl, or two R₂ together with the carbon atoms to which they are attached form (C₃-C₇)cycloalkyl or a 4- to 6-membered heterocycloalkyl ring comprising 1 to 3 heteroatoms selected from O, N, and S, R₁ is (C₁-C₆)alkoxy, halogen, -OH, -(CH₂)₀₋₂NH₂, or CN, and R₃ is 5- or 6-membered heteroaryl comprising 1 to 3 heteroatoms selected from O, N, and S optionally substituted with one to three R₅.

In some embodiments of the formulae above, Rₓ is H, n is 2, each R₂ is independently (C₁-C₆)alkyl, or two R₂ together with the carbon atoms to which they are attached form (C₃-C₇)cycloalkyl or a 4- to 6-membered heterocycloalkyl ring comprising 1 to 3 heteroatoms selected from O, N, and S, R₁ is (C₁-C₆)alkoxy, halogen, -OH, -(CH₂)₀₋₂NH₂, or CN, and R₃ is (C₃-C₈)cycloalkyl optionally substituted with one to three R₅.

In some embodiments of the formulae above, Rₓ is H, n is 2, each R₂ is independently (C₁-C₆)alkyl, or two R₂ together with the carbon atoms to which they are attached form (C₃-C₇)cycloalkyl or a 4- to 6-membered heterocycloalkyl ring comprising 1 to 3 heteroatoms selected from O, N, and S, R₁ is (C₁-C₆)alkoxy, halogen, -OH, -(CH₂)₀₋₂NH₂, or CN, and R₃ is 5- to 7-membered heterocycloalkyl comprising 1 to 3 heteroatoms selected from O, N, and S, optionally substituted with one to three R₅.

In some embodiments of the formulae above, Rₓ is H, n is 3, and each R₂ is independently (C₁-C₆)alkyl, or two R₂ together with the carbon atoms to which they are attached form (C₃-C₇)cycloalkyl or a 4- to 6- membered heterocycloalkyl ring comprising 1 to 3 heteroatoms selected from O, N, and S. In another embodiment, Rₓ is H, n is 3, each R₂ is independently (C₁-C₆)alkyl, or two R₂ together with the carbon atoms to which they are attached form (C₃-C₇)cycloalkyl or a 4- to 6- membered heterocycloalkyl ring comprising 1 to 3 heteroatoms selected from O, N, and S, and R₁ is (C₁-C₆)alkoxy, halogen, -OH, - (CH₂)₀₋₂NH₂, or CN. In another embodiment, Rₓ is H, n is 3, each R₂ is independently (C₁-C₆)alkyl, or two R₂ together with the carbon atoms to which they are attached form (C₃-C₇)cycloalkyl or a 4- to 6- membered heterocycloalkyl ring comprising 1 to 3 heteroatoms selected from O, N, and S, R₁ is (C₁-C₆)alkoxy, halogen, -OH, -(CH₂)₀₋₂NH₂, or CN, and R₃ is (C₁-C₆)alkyl optionally substituted with one to three R₄. In another embodiment, Rₓ is H, n is 3, each R₂ is independently (C₁-C₆)alkyl, or two R₂ together with the carbon atoms to which they are attached form (C₃-C₇)cycloalkyl or a 4- to 6- membered heterocycloalkyl ring comprising 1 to 3 heteroatoms selected from O, N, and S, R₁ is (C₁-C₆)alkoxy, halogen, -OH, -(CH₂)₀₋₂NH₂, or CN, and R₃ is (C₁-C₆)alkyl substituted with one to three R₄.

In some embodiments of the formulae above, Rₓ is H, n is 3, each R₂ is independently (C₁-C₆)alkyl, or two R₂ together with the carbon atoms to which they are attached form (C₃-C₇)cycloalkyl or a 4- to 6-membered heterocycloalkyl ring comprising 1 to 3 heteroatoms selected from O, N, and S, R₁ is (C₁-C₆)alkoxy, halogen, -OH, -(CH₂)₀₋₂NH₂, or CN, R₃ is (C₁-C₆)alkyl optionally substituted with one to three R₄, and each R₄ is independently selected from -C(O)OR₆, (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇. In another embodiment, Rₓ is H, n is 3, each R₂ is independently (C₁-C₆)alkyl, or two R₂ together with the carbon atoms to which they are attached form (C₃-C₇)cycloalkyl or a 4- to 6- membered heterocycloalkyl ring comprising 1 to 3 heteroatoms selected from O, N, and S, R₁ is (C₁-C₆)alkoxy, halogen, -OH, -(CH₂)₀₋₂NH₂, or CN, R₃ is (C₁-C₆)alkyl substituted with one to three R₄, and each R₄ is independently selected from - C(O)OR₆, (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇.

In some embodiments of the formulae above, Rₓ is H, n is 3, each R₂ is independently (C₁-C₆)alkyl, or two R₂ together with the carbon atoms to which they are attached form (C₃-C₇)cycloalkyl or a 4- to 6-membered heterocycloalkyl ring comprising 1 to 3 heteroatoms selected from O, N, and S, R₁ is (C₁-C₆)alkoxy, halogen, -OH, -(CH₂)₀₋₂NH₂, or CN, R₃ is (C₁-C₆)alkyl optionally substituted with one to three R₄, and each R₄ is independently selected from (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇. In another embodiment, Rₓ is H, n is 3, each R₂ is independently (C₁-C₆)alkyl, or two R₂ together with the carbon atoms to which they are attached form (C₃-C₇)cycloalkyl or a 4- to 6- membered heterocycloalkyl ring comprising 1 to 3 heteroatoms selected from O, N, and S, R₁ is (C₁-C₆)alkoxy, halogen, -OH, -(CH₂)₀₋₂NH₂, or CN, R₃ is (C₁-C₆)alkyl substituted with one to three R₄, and each R₄ is independently selected from (C₆-C₁₀)aryl, 5- or 6-membered heteroaryl comprising 1 to 3 heteroatoms selected from O, N, and S, (C₃-C₈)cycloalkyl, and 5-to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇.

In some embodiments of the formulae above, Rₓ is H, n is 3, each R₂ is independently (C₁-C₆)alkyl, or two R₂ together with the carbon atoms to which they are attached form (C₃-C₇)cycloalkyl or a 4- to 6-membered heterocycloalkyl ring comprising 1 to 3 heteroatoms selected from O, N, and S, R₁ is (C₁-C₆)alkoxy, halogen, -OH, -(CH₂)₀₋₂NH₂, or CN, R₃ is (C₁-C₆)alkyl optionally substituted with one to three R₄, and each R₄ is independently selected from halogen, -OH, (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇. In another embodiment, Rₓ is H, n is 3, each R₂ is independently (C₁-C₆)alkyl, or two R₂ together with the carbon atoms to which they are attached form (C₃-C₇)cycloalkyl or a 4- to 6- membered heterocycloalkyl ring comprising 1 to 3 heteroatoms selected from O, N, and S, R₁ is (C₁-C₆)alkoxy, halogen, -OH, -(CH₂)₀₋₂NH₂, or CN, R₃ is (C₁-C₆)alkyl substituted with one to three R₄, and each R₄ is independently selected from halogen, -OH, (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇.

In some embodiments of the formulae above, Rₓ is H, n is 3, each R₂ is independently (C₁-C₆)alkyl, or two R₂ together with the carbon atoms to which they are attached form (C₃-C₇)cycloalkyl or a 4- to 6-membered heterocycloalkyl ring comprising 1 to 3 heteroatoms selected from O, N, and S, R₁ is (C₁-C₆)alkoxy, halogen, -OH, -(CH₂)₀₋₂NH₂, or CN, R₃ is (C₁-C₆)alkyl optionally substituted with one to three R₄, and each R₄ is independently selected from halogen, -OH, phenyl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the phenyl, heteroaryl, cycloalkyl, and heterocycloalkyl groups are optionally substituted with one to three R₇. In another embodiment, Rₓ is H, n is 3, each R₂ is independently (C₁-C₆)alkyl, or two R₂ together with the carbon atoms to which they are attached form (C₃-C₇)cycloalkyl or a 4- to 6- membered heterocycloalkyl ring comprising 1 to 3 heteroatoms selected from O, N, and S, R₁ is (C₁-C₆)alkoxy, halogen, -OH, -(CH₂)₀₋₂NH₂, or CN, R₃ is (C₁-C₆)alkyl substituted with one to three R₄, and each R₄ is independently selected from halogen, -OH, phenyl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the phenyl, heteroaryl, cycloalkyl, and heterocycloalkyl groups are optionally substituted with one to three R₇.

In some embodiments of the formulae above, Rₓ is H, n is 3, each R₂ is independently (C₁-C₆)alkyl, or two R₂ together with the carbon atoms to which they are attached form (C₃-C₇)cycloalkyl or a 4- to 6-membered heterocycloalkyl ring comprising 1 to 3 heteroatoms selected from O, N, and S, R₁ is (C₁-C₆)alkoxy, halogen, -OH, -(CH₂)₀₋₂NH₂, or CN, R₃ is (C₁-C₆)alkyl optionally substituted with one to three R₄, and each R₄ is independently selected from phenyl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the phenyl, heteroaryl, cycloalkyl, and heterocycloalkyl groups are optionally substituted with one to three R₇. In another embodiment, Rₓ is H, n is 3, each R₂ is independently (C₁-C₆)alkyl, or two R₂ together with the carbon atoms to which they are attached form (C₃-C₇)cycloalkyl or a 4- to 6- membered heterocycloalkyl ring comprising 1 to 3 heteroatoms selected from O, N, and S, R₁ is (C₁-C₆)alkoxy, halogen, -OH, -(CH₂)₀₋₂NH₂, or CN, R₃ is (C₁-C₆)alkyl substituted with one to three R₄, and each R₄ is independently selected from phenyl, 5- or 6-membered heteroaryl comprising 1 to 3 heteroatoms selected from O, N, and S, (C₃-C₈)cycloalkyl, and 5-to 7-membered heterocycloalkyl comprising 1 to 3 heteroatoms selected from O, N, and S, wherein the phenyl, heteroaryl, cycloalkyl, and heterocycloalkyl groups are optionally substituted with one to three R₇.

In some embodiments of the formulae above, Rₓ is H, n is 3, each R₂ is independently (C₁-C₆)alkyl, or two R₂ together with the carbon atoms to which they are attached form (C₃-C₇)cycloalkyl or a 4- to 6-membered heterocycloalkyl ring comprising 1 to 3 heteroatoms selected from O, N, and S, R₁ is (C₁-C₆)alkoxy, halogen, -OH, -(CH₂)₀₋₂NH₂, or CN, R₃ is (C₁-C₆)alkyl optionally substituted with one to three R₄, and each R₄ is independently selected from phenyl and 5- or 6-membered heteroaryl comprising 1 to 3 heteroatoms selected from O, N, and S, wherein the phenyl and heteroaryl groups are optionally substituted with one to three R₇. In another embodiment, Rₓ is H, n is 3, each R₂ is independently (C₁-C₆)alkyl, or two R₂ together with the carbon atoms to which they are attached form (C₃-C₇)cycloalkyl or a 4- to 6- membered heterocycloalkyl ring comprising 1 to 3 heteroatoms selected from O, N, and S, R₁ is (C₁-C₆)alkoxy, halogen, -OH, -(CH₂)₀₋₂NH₂, or CN, R₃ is (C₁-C₆)alkyl substituted with one to three R₄, and each R₄ is independently selected from phenyl and 5- or 6-membered heteroaryl comprising 1 to 3 heteroatoms selected from O, N, and S, wherein the phenyl and heteroaryl groups are optionally substituted with one to three R₇.

In some embodiments of the formulae above, Rₓ is H, n is 3, each R₂ is independently (C₁-C₆)alkyl, or two R₂ together with the carbon atoms to which they are attached form (C₃-C₇)cycloalkyl or a 4- to 6-membered heterocycloalkyl ring comprising 1 to 3 heteroatoms selected from O, N, and S, R₁ is (C₁-C₆)alkoxy, halogen, -OH, -(CH₂)₀₋₂NH₂, or CN, R₃ is (C₁-C₆)alkyl optionally substituted with one to three R₄, and each R₄ is phenyl optionally substituted with one to three R₇. In another embodiment, Rₓ is H, n is 3, each R₂ is independently (C₁-C₆)alkyl, or two R₂ together with the carbon atoms to which they are attached form (C₃-C₇)cycloalkyl or a 4- to 6- membered heterocycloalkyl ring comprising 1 to 3 heteroatoms selected from O, N, and S, R₁ is (C₁-C₆)alkoxy, halogen, -OH, -(CH₂)₀₋₂NH₂, or CN, R₃ is (C₁-C₆)alkyl substituted with one to three R₄, and each R₄ is phenyl optionally substituted with one to three R₇.

In some embodiments of the formulae above, Rₓ is H, n is 3, each R₂ is independently (C₁-C₆)alkyl, or two R₂ together with the carbon atoms to which they are attached form (C₃-C₇)cycloalkyl or a 4- to 6-membered heterocycloalkyl ring comprising 1 to 3 heteroatoms selected from O, N, and S, R₁ is (C₁-C₆)alkoxy, halogen, -OH, -(CH₂)₀₋₂NH₂, or CN, R₃ is (C₁-C₆)alkyl optionally substituted with one to three R₄, and each R₄ is 5- or 6-membered heteroaryl comprising 1 to 3 heteroatoms selected from O, N, and S, optionally substituted with one to three R₇. In another embodiment, Rₓ is H, n is 3, each R₂ is independently (C₁-C₆)alkyl, or two R₂ together with the carbon atoms to which they are attached form (C₃-C₇)cycloalkyl or a 4- to 6- membered heterocycloalkyl ring comprising 1 to 3 heteroatoms selected from O, N, and S, R₁ is (C₁-C₆)alkoxy, halogen, -OH, -(CH₂)₀₋₂NH₂, or CN, R₃ is (C₁-C₆)alkyl substituted with one to three R₄, and each R₄ is 5- or 6-membered heteroaryl comprising 1 to 3 heteroatoms selected from O, N, and S, optionally substituted with one to three R₇.

In some embodiments of the formulae above, Rₓ is H, n is 3, each R₂ is independently (C₁-C₆)alkyl, or two R₂ together with the carbon atoms to which they are attached form (C₃-C₇)cycloalkyl or a 4- to 6-membered heterocycloalkyl ring comprising 1 to 3 heteroatoms selected from O, N, and S, R₁ is (C₁-C₆)alkoxy, halogen, -OH, -(CH₂)₀₋₂NH₂, or CN, R₃ is (C₁-C₆)alkyl optionally substituted with one to three R₄, and each R₄ is (C₃-C₈)cycloalkyl optionally substituted with one to three R₇. In another embodiment, Rₓ is H, n is 3, each R₂ is independently (C₁-C₆)alkyl, or two R₂ together with the carbon atoms to which they are attached form (C₃-C₇)cycloalkyl or a 4- to 6- membered heterocycloalkyl ring comprising 1 to 3 heteroatoms selected from O, N, and S, R₁ is (C₁-C₆)alkoxy, halogen, -OH, -(CH₂)₀₋₂NH₂, or CN, R₃ is (C₁-C₆)alkyl substituted with one to three R₄, and each R₄ is (C₃-C₈)cycloalkyl optionally substituted with one to three R₇.

In some embodiments of the formulae above, Rₓ is H, n is 3, each R₂ is independently (C₁-C₆)alkyl, or two R₂ together with the carbon atoms to which they are attached form (C₃-C₇)cycloalkyl or a 4- to 6-membered heterocycloalkyl ring comprising 1 to 3 heteroatoms selected from O, N, and S, R₁ is (C₁-C₆)alkoxy, halogen, -OH, -(CH₂)₀₋₂NH₂, or CN, R₃ is (C₁-C₆)alkyl optionally substituted with one to three R₄, and each R₄ is 5- to 7-membered heterocycloalkyl comprising 1 to 3 heteroatoms selected from O, N, and S, optionally substituted with one to three R₇. In another embodiment, Rₓ is H, n is 3, each R₂ is independently (C₁-C₆)alkyl, or two R₂ together with the carbon atoms to which they are attached form (C₃-C₇)cycloalkyl or a 4- to 6- membered heterocycloalkyl ring comprising 1 to 3 heteroatoms selected from O, N, and S, R₁ is (C₁-C₆)alkoxy, halogen, -OH, -(CH₂)₀₋₂NH₂, or CN, R₃ is (C₁-C₆)alkyl substituted with one to three R₄, and each R₄ is 5- to 7-membered heterocycloalkyl comprising 1 to 3 heteroatoms selected from O, N, and S, optionally substituted with one to three R₇.

In some embodiments of the formulae above, Rₓ is H, n is 3, each R₂ is independently (C₁-C₆)alkyl, or two R₂ together with the carbon atoms to which they are attached form (C₃-C₇)cycloalkyl or a 4- to 6-membered heterocycloalkyl ring comprising 1 to 3 heteroatoms selected from O, N, and S, R₁ is (C₁-C₆)alkoxy, halogen, -OH, -(CH₂)₀₋₂NH₂, or CN, and R₃ is (C₆-C₁₀)aryl, (C₃-C₈)cycloalkyl, or 5- to 7-membered heterocycloalkyl comprising 1 to 3 heteroatoms selected from O, N, and S, wherein the aryl, cycloalkyl, and heterocycloalkyl are optionally substituted with one to three R₅.

In some embodiments of the formulae above, Rₓ is H, n is 3, each R₂ is independently (C₁-C₆)alkyl, or two R₂ together with the carbon atoms to which they are attached form (C₃-C₇)cycloalkyl or a 4- to 6-membered heterocycloalkyl ring comprising 1 to 3 heteroatoms selected from O, N, and S, R₁ is (C₁-C₆)alkoxy, halogen, -OH, -(CH₂)₀₋₂NH₂, or CN, and R₃ is (C₆-C₁₀)aryl, (C₃-C₈)cycloalkyl, or 5- to 7-membered heterocycloalkyl comprising 1 to 3 heteroatoms selected from O, N, and S.

In some embodiments of the formulae above, Rₓ is H, n is 3, each R₂ is independently (C₁-C₆)alkyl, or two R₂ together with the carbon atoms to which they are attached form (C₃-C₇)cycloalkyl or a 4- to 6-membered heterocycloalkyl ring comprising 1 to 3 heteroatoms selected from O, N, and S, R₁ is (C₁-C₆)alkoxy, halogen, -OH, -(CH₂)₀₋₂NH₂, or CN, and R₃ is (C₆-C₁₀)aryl, 5- or 6-membered heteroaryl comprising 1 to 3 heteroatoms selected from O, N, and S, or (C₃-C₈)cycloalkyl, wherein the aryl, heteroaryl and cycloalkyl are optionally substituted with one to three R₅.

In some embodiments of the formulae above, Rₓ is H, n is 3, each R₂ is independently (C₁-C₆)alkyl, or two R₂ together with the carbon atoms to which they are attached form (C₃-C₇)cycloalkyl or a 4- to 6-membered heterocycloalkyl ring comprising 1 to 3 heteroatoms selected from O, N, and S, R₁ is (C₁-C₆)alkoxy, halogen, -OH, -(CH₂)₀₋₂NH₂, or CN, and R₃ is (C₆-C₁₀)aryl, 5- or 6-membered heteroaryl comprising 1 to 3 heteroatoms selected from O, N, and S, or (C₃-C₈)cycloalkyl.

In some embodiments of the formulae above, Rₓ is H, n is 3, each R₂ is independently (C₁-C₆)alkyl, or two R₂ together with the carbon atoms to which they are attached form (C₃-C₇)cycloalkyl or a 4- to 6-membered heterocycloalkyl ring comprising 1 to 3 heteroatoms selected from O, N, and S, R₁ is (C₁-C₆)alkoxy, halogen, -OH, -(CH₂)₀₋₂NH₂, or CN, and R₃ is (C₆-C₁₀)aryl optionally substituted with one to three R₅.

In some embodiments of the formulae above, Rₓ is H, n is 3, each R₂ is independently (C₁-C₆)alkyl, or two R₂ together with the carbon atoms to which they are attached form (C₃-C₇)cycloalkyl or a 4- to 6-membered heterocycloalkyl ring comprising 1 to 3 heteroatoms selected from O, N, and S, R₁ is (C₁-C₆)alkoxy, halogen, -OH, -(CH₂)₀₋₂NH₂, or CN, and R₃ is 5- or 6-membered heteroaryl comprising 1 to 3 heteroatoms selected from O, N, and S optionally substituted with one to three R₅.

In some embodiments of the formulae above, Rₓ is H, n is 3, each R₂ is independently (C₁-C₆)alkyl, or two R₂ together with the carbon atoms to which they are attached form (C₃-C₇)cycloalkyl or a 4- to 6-membered heterocycloalkyl ring comprising 1 to 3 heteroatoms selected from O, N, and S, R₁ is (C₁-C₆)alkoxy, halogen, -OH, -(CH₂)₀₋₂NH₂, or CN, and R₃ is (C₃-C₈)cycloalkyl optionally substituted with one to three R₅.

In some embodiments of the formulae above, Rₓ is H, n is 3, each R₂ is independently (C₁-C₆)alkyl, or two R₂ together with the carbon atoms to which they are attached form (C₃-C₇)cycloalkyl or a 4- to 6-membered heterocycloalkyl ring comprising 1 to 3 heteroatoms selected from O, N, and S, R₁ is (C₁-C₆)alkoxy, halogen, -OH, -(CH₂)₀₋₂NH₂, or CN, and R₃ is 5- to 7-membered heterocycloalkyl comprising 1 to 3 heteroatoms selected from O, N, and S, optionally substituted with one to three R₅.

In some embodiments of the formulae above, R₃ is (C₁-C₆)alkyl optionally substituted with one to three R₄, and each R₄ is phenyl optionally substituted with one to three R₇.

In some embodiments of the formulae above, R₃ is (C₁-C₆)alkyl substituted with one to three R₄, and each R₄ is phenyl optionally substituted with one to three R₇.

In some embodiments of the formulae above, R₃ is (C₁-C₆)alkyl optionally substituted with one to three R₄, and each R₄ is 5- or 6-membered heteroaryl comprising 1 to 3 heteroatoms selected from O, N, and S, optionally substituted with one to three R₇.

In some embodiments of the formulae above, R₃ is (C₁-C₆)alkyl substituted with one to three R₄, and each R₄ is 5- or 6-membered heteroaryl comprising 1 to 3 heteroatoms selected from O, N, and S, optionally substituted with one to three R₇.

In some embodiments of the formulae above, R₃ is (C₁-C₆)alkyl optionally substituted with one to three R₄, and each R₄ is (C₃-C₈)cycloalkyl optionally substituted with one to three R₇.

In some embodiments of the formulae above, R₃ is (C₁-C₆)alkyl substituted with one to three R₄, and each R₄ is (C₃-C₈)cycloalkyl optionally substituted with one to three R₇.

In some embodiments of the formulae above, R₃ is (C₁-C₆)alkyl optionally substituted with one to three R₄, and each R₄ is 5- to 7-membered heterocycloalkyl comprising 1 to 3 heteroatoms selected from O, N, and S, optionally substituted with one to three R₇.

In some embodiments of the formulae above, R₃ is (C₁-C₆)alkyl substituted with one to three R₄, and each R₄ is 5- to 7-membered heterocycloalkyl comprising 1 to 3 heteroatoms selected from O, N, and S, optionally substituted with one to three R₇.

In some embodiments, there is provided a compound selected from:
3-(5-(1-benzyl-4-hydroxypiperidin-4-yl)-1-oxoisoindolin-2-yl)piperidine-2,6-dione;
3-(5-(1-benzyl-4-methoxypiperidin-4-yl)-1-oxoisoindolin-2-yl)piperidine-2,6-dione;
3-(5-(1-benzyl-4-fluoropiperidin-4-yl)-1-oxoisoindolin-2-yl)piperidine-2,6-dione;
1-benzyl-4-(2-(2,6-dioxopiperidin-3-yl)-1-oxoisoindolin-5-yl)piperidine-4-carbonitrile;
3-(5-(4-amino-1-benzylpiperidin-4-yl)-1-oxoisoindolin-2-yl)piperidine-2,6-dione;
3-(5-(3-benzyl-3-azabicyclo[4.1.0]heptan-6-yl)-1-oxoisoindolin-2-yl)piperidine-2,6-dione;
3-(5-(3-(((1r,4r)-4-methoxycyclohexyl)methyl)-3-azabicyclo[4.1.0]heptan-6-yl)-1-oxoisoindolin-2-yl)piperidine-2,6-dione;
3-(5-(4-fluoro-1-(((1r,4r)-4-methoxycyclohexyl)methyl)piperidin-4-yl)-1-oxoisoindolin-2-yl)piperidine-2,6-dione;
3-(5-(4-hydroxy-1-(((1r,4r)-4-methoxycyclohexyl)methyl)piperidin-4-yl)-1-oxoisoindolin-2-yl)piperidine-2,6-dione;
3-(5-(4-methoxy-1-(((1r,4r)-4-methoxycyclohexyl)methyl)piperidin-4-yl)-1-oxoisoindolin-2-yl)piperidine-2,6-dione;
3-(5-(4-amino-1-(((1r,4r)-4-methoxycyclohexyl)methyl)piperidin-4-yl)-1-oxoisoindolin-2-yl)piperidine-2,6-dione; and
4-(2-(2,6-dioxopiperidin-3-yl)-1-oxoisoindolin-5-yl)-1-(((1r,4r)-4-methoxycyclohexyl)methyl)piperidine-4-carbonitrile;
or a pharmaceutically acceptable salt, hydrate, solvate, stereoisomer, or tautomer thereof.

In some embodiments, there is provide a compound selected from:
3-(1-oxo-5-(1-((6-oxo-1,6-dihydropyridin-3-yl)methyl)piperidin-4-yl)isoindolin-2-yl)piperidine-2,6-dione;
3-(5-(1-benzyl-2,6-dimethylpiperidin-4-yl)-1-oxoisoindolin-2-yl)piperidine-2,6-dione; (1r,4r)-4-((4-(2-(2,6-dioxopiperidin-3-yl)-1-oxoisoindolin-5-yl)piperidin-1-yl)methyl)cyclohexane-1-carbonitrile;
3-(5-(2,6-dimethylpiperidin-4-yl)-1-oxoisoindolin-2-yl)piperidine-2,6-dione;
3-(5-(1-((5-ethoxypyridin-2-yl)methyl)piperidin-4-yl)-1-oxoisoindolin-2-yl)piperidine-2,6-dione;
3-(5-(1-((5-methoxypyridin-2-yl)methyl)piperidin-4-yl)-1-oxoisoindolin-2-yl)piperidine-2,6-dione;
3-(5-(1-((6-methoxypyridin-3-yl)methyl)piperidin-4-yl)-1-oxoisoindolin-2-yl)piperidine-2,6-dione;
3-(5-(1-((6-ethoxypyridin-3-yl)methyl)piperidin-4-yl)-1-oxoisoindolin-2-yl)piperidine-2,6-dione;
3-(5-(1-((5-methyl-1H-imidazol-4-yl)methyl)piperidin-4-yl)-1-oxoisoindolin-2-yl)piperidine-2,6-dione;
3-(5-(1-((4-(fluoromethyl)cyclohexyl)methyl)piperidin-4-yl)-1-oxoisoindolin-2-yl)piperidine-2,6-dione;
3-(5-(1-(2-(1H-pyrazol-1-yl)ethyl)piperidin-4-yl)-1-oxoisoindolin-2-yl)piperidine-2,6-dione
3-(5-(1-((4-methylpyrimidin-5-yl)methyl)piperidin-4-yl)-1-oxoisoindolin-2-yl)piperidine-2,6-dione;
3-(5-(1-((4-methylcyclohex-3-en-1-yl)methyl)piperidin-4-yl)-1-oxoisoindolin-2-yl)piperidine-2,6-dione;
3-(1-oxo-5-(1-(pyrazin-2-ylmethyl)piperidin-4-yl)isoindolin-2-yl)piperidine-2,6-dione;
3-(1-oxo-5-(1-(pyridazin-3-ylmethyl)piperidin-4-yl)isoindolin-2-yl)piperidine-2,6-dione;
3-(1-oxo-5-(1-(pyrimidin-4-ylmethyl)piperidin-4-yl)isoindolin-2-yl)piperidine-2,6-dione;
3-(5-(1-((2-methylpyrimidin-5-yl)methyl)piperidin-4-yl)-1-oxoisoindolin-2-yl)piperidine-2,6-dione;
3-(1-oxo-5-(1-(pyridazin-4-ylmethyl)piperidin-4-yl)isoindolin-2-yl)piperidine-2,6-dione;
3-(5-((1R,4S)-2-benzyl-2-azabicyclo[2.2.2]octan-5-yl)-1-oxoisoindolin-2-yl)piperidine-2,6-dione;
3-(5-((1R,5S)-9-benzyl-3-methyl-3,9-diazabicyclo[3.3.1]nonan-7-yl)-1-oxoisoindolin-2-yl)piperidine-2,6-dione;
3-(5-((1R,5S)-9-benzyl-3-oxa-9-azabicyclo[3.3.1]nonan-7-yl)-1-oxoisoindolin-2-yl)piperidine-2,6-dione;
3-(5-((1R,5S)-9-benzyl-3,9-diazabicyclo[3.3.1]nonan-7-yl)-1-oxoisoindolin-2-yl)piperidine-2,6-dione;
3-(5-((1S,4S)-2-benzyl-2-azabicyclo[2.2.1]heptan-5-yl)-1-oxoisoindolin-2-yl)piperidine-2,6-dione;
3-(5-((1R,5S)-9-ethyl-3-methyl-3,9-diazabicyclo[3.3.1]nonan-7-yl)-1-oxoisoindolin-2-yl)piperidine-2,6-dione;
3-(1-oxo-5-(1-(1-(tetrahydro-2H-pyran-4-yl)ethyl)piperidin-4-yl)isoindolin-2-yl)piperidine-2,6-dione;
3-(5-(1-(1-(1-ethyl-1H-pyrazol-4-yl)ethyl)piperidin-4-yl)-1-oxoisoindolin-2-yl)piperidine-2,6-dione;
3-(5-(1-(1-(1-ethyl-1H-pyrazol-4-yl)propyl)piperidin-4-yl)-1-oxoisoindolin-2-yl)piperidine-2,6-dione;
3-(1-oxo-5-(1-(1-(pyrazin-2-yl)propyl)piperidin-4-yl)isoindolin-2-yl)piperidine-2,6-dione;
3-(1-oxo-5-(1-(1-(pyridazin-4-yl)propyl)piperidin-4-yl)isoindolin-2-yl)piperidine-2,6-dione;
3-(5-(1-(6,7-dihydro-5H-cyclopenta[b]pyridin-5-yl)piperidin-4-yl)-1-oxoisoindolin-2-yl)piperidine-2,6-dione;
3-(5-(1-(4-methoxycyclohexyl)piperidin-4-yl)-1-oxoisoindolin-2-yl)piperidine-2,6-dione;
3-(1-oxo-5-(1-(2-(pyridin-4-yl)propan-2-yl)piperidin-4-yl)isoindolin-2-yl)piperidine-2,6-dione; and
3-(1-oxo-5-(1-((2-oxo-1,2-dihydropyridin-3-yl)methyl)piperidin-4-yl)isoindolin-2-yl)piperidine-2,6-dione;
or a pharmaceutically acceptable salt, hydrate, solvate, stereoisomer, or tautomer thereof.

In some embodiments, there is provided a compound selected from:

| **Cmpd No.** | **Compound Structure** | **Compound Name** |
|---|---|---|
| **I-1** | 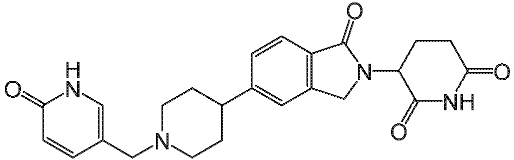 | 3-(1-oxo-5-(1-((6-oxo-1,6-dihydropyridin-3-yl)methyl)piperidin-4-yl)isoindolin-2-yl)piperidine-2,6-dione; |
| **I-2** | 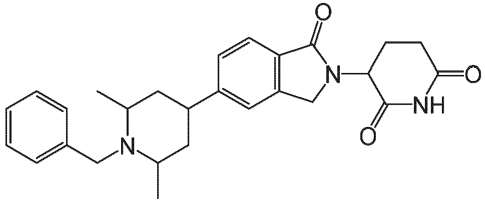 | 3-(5-(1-benzyl-2,6-dimethylpiperidin-4-yl)-1-oxoisoindolin-2-yl)piperidine-2,6-dione; |
| **I-3** | 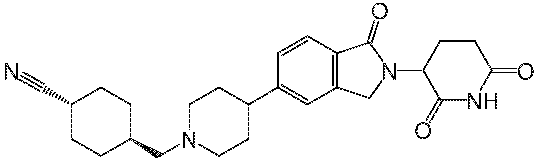 | (1r,4r)-4-((4-(2-(2,6-dioxopiperidin-3-yl)-1-oxoisoindolin-5-yl)piperidin-1-yl)methyl)cyclohexane-1-carbonitrile; |
| **I-4** | 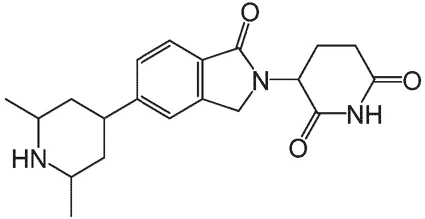 | 3-(5-(2,6-dimethylpiperidin-4-yl)-1-oxoisoindolin-2-yl)piperidine-2,6-dione; |
| **I-5** | 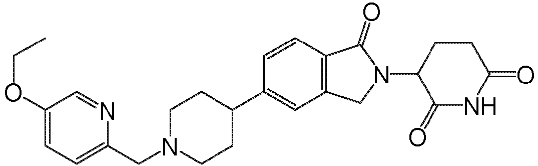 | 3-(5-(1-((5-ethoxypyridin-2-yl)methyl)piperidin-4-yl)-1-oxoisoindolin-2-yl)piperidine-2,6-dione; |
| **I-6** | 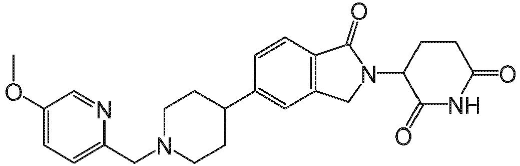 | 3-(5-(1-((5-methoxypyridin-2-yl)methyl)piperidin-4-yl)-1-oxoisoindolin-2-yl)piperidine-2,6-dione; |
| **I-7** | 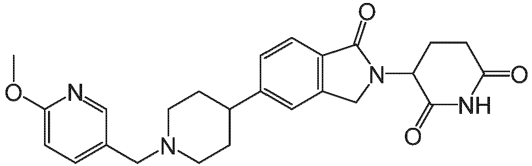 | 3-(5-(1-((6-methoxypyridin-3-yl)methyl)piperidin-4-yl)-1-oxoisoindolin-2-yl)piperidine-2,6-dione; |
| **I-8** | 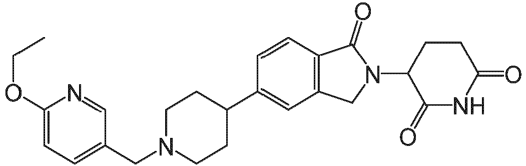 | 3-(5-(1-((6-ethoxypyridin-3-yl)methyl)piperidin-4-yl)-1-oxoisoindolin-2-yl)piperidine-2,6-dione; |
| **I-9** | 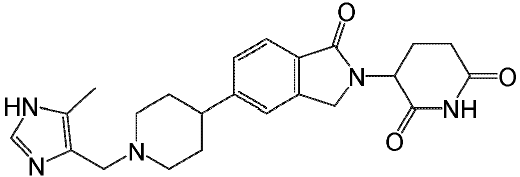 | 3-(5-(1-((5-methyl-1H-imidazol-4-yl)methyl)piperidin-4-yl)-1-oxoisoindolin-2-yl)piperidine-2,6-dione; |
| **I-10** | 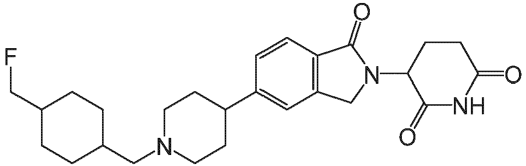 | 3-(5-(1-((4-(fluoromethyl)cyclohexyl)methy 1)piperidin-4-yl)-1-oxoisoindolin-2-yl)piperidine-2,6-dione; |
| **I-11** | 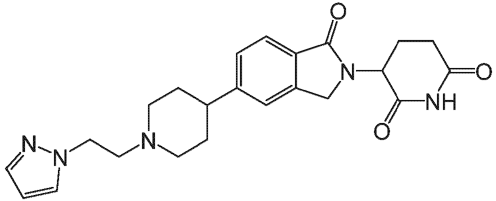 | 3-(5-(1-(2-(1H-pyrazol-1-yl)ethyl)piperidin-4-yl)-1-oxoisoindolin-2-yl)piperidine-2,6-dione; |
| **I-12** | 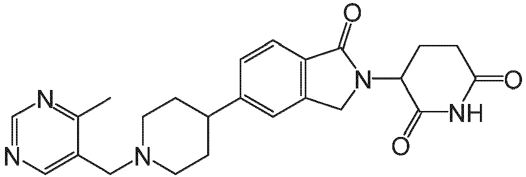 | 3-(5-(1-((4-methylpyrimidin-5-yl)methyl)piperidin-4-yl)-1-oxoisoindolin-2-yl)piperidine-2,6-dione; |
| **I-13** | 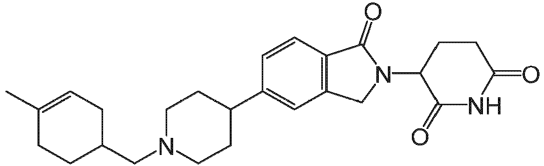 | 3-(5-(1-((4-methylcyclohex-3-en-1-yl)methyl)piperidin-4-yl)-1-oxoisoindolin-2-yl)piperidine-2,6-dione; |
| **I-14** | 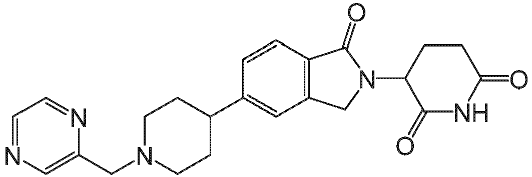 | 3-(1-oxo-5-(1-(pyrazin-2-ylmethyl)piperidin-4-yl)isoindolin-2-yl)piperidine-2,6-dione; |
| **I-15** | 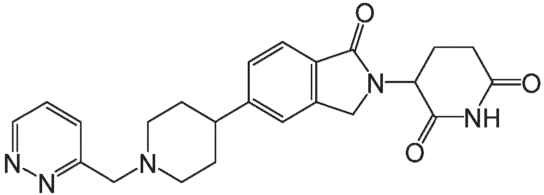 | 3-(1-oxo-5-(1-(pyridazin-3-ylmethyl)piperidin-4-yl)isoindolin-2-yl)piperidine-2,6-dione; |
| **I-16** | 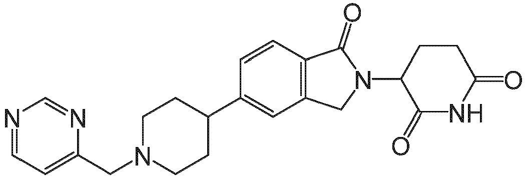 | 3-(1-oxo-5-(1-(pyrimidin-4-ylmethyl)piperidin-4-yl)isoindolin-2-yl)piperidine-2,6-dione; |
| **I-17** | 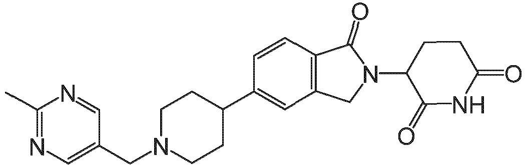 | 3-(5-(1-((2-methylpyrimidin-5-yl)methyl)piperidin-4-yl)-1-oxoisoindolin-2-yl)piperidine-2,6-dione; |
| **I-18** | 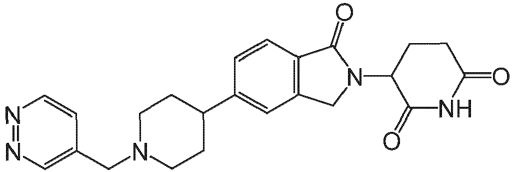 | 3-(1-oxo-5-(1-(pyridazin-4-ylmethyl)piperidin-4-yl)isoindolin-2-yl)piperidine-2,6-dione; |
| **I-19** | 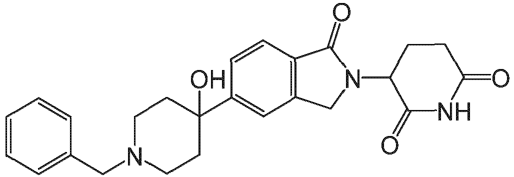 | 3-(5-(1-benzyl-4-hydroxypiperidin-4-yl)-1-oxoisoindolin-2-yl)piperidine-2,6-dione; |
| **I-20** | 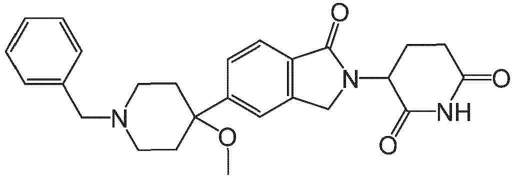 | 3-(5-(1-benzyl-4-methoxypiperidin-4-yl)-1-oxoisoindolin-2-yl)piperidine-2,6-dione; |
| **I-21** | 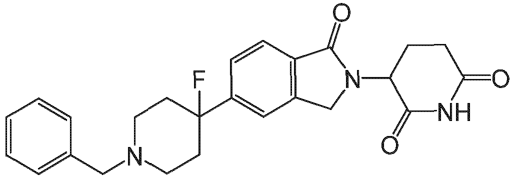 | 3-(5-(1-benzyl-4-fluoropiperidin-4-yl)-1-oxoisoindolin-2-yl)piperidine-2,6-dione; |
| **I-22** | 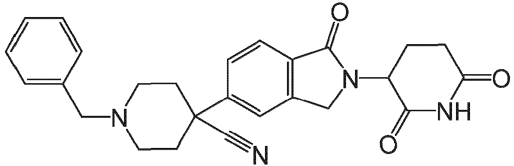 | 1-benzyl-4-(2-(2,6-dioxopiperidin-3-yl)-1-oxoisoindolin-5-yl)piperidine-4-carbonitrile; |
| **I-23** | 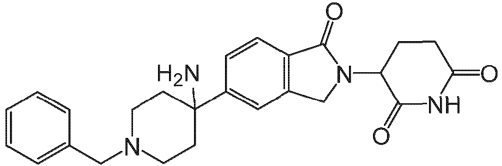 | 3-(5-(4-amino-1-benzylpiperidin-4-yl)-1-oxoisoindolin-2-yl)piperidine-2,6-dione; |
| **I-24** | 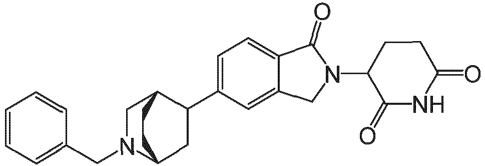 | 3-(5-((1R,4S)-2-benzyl-2-azabicyclo[2.2.2]octan-5-yl)-1-oxoisoindolin-2-yl)piperidine-2,6-dione; |
| **1-25** | 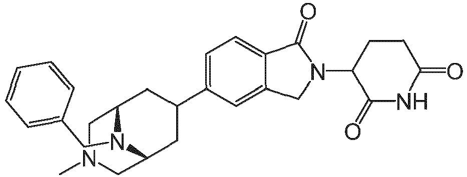 | 3-(5-((1R,5S)-9-benzyl-3-methyl-3,9-diazabicyclo[3.3.1]nonan-7-yl)-1-oxoisoindolin-2-yl)piperidine-2,6-dione; |
| **I-26** | 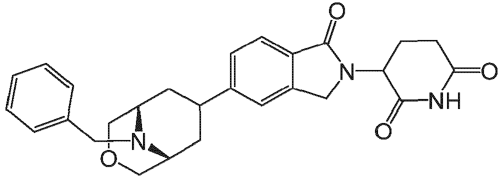 | 3-(5-((1R,5S)-9-benzyl-3-oxa-9-azabicyclo[3.3.1]nonan-7-yl)-1-oxoisoindolin-2-yl)piperidine-2,6-dione |
| **I-27** | 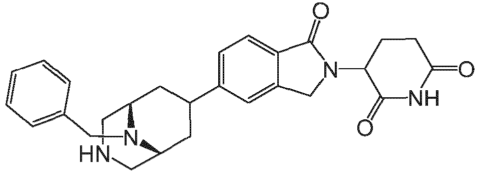 | 3-(5-((1R,5S)-9-benzyl-3,9-diazabicyclo[3.3.1]nonan-7-yl)-1-oxoisoindolin-2-yl)piperidine-2,6-dione; |
| **I-28** | 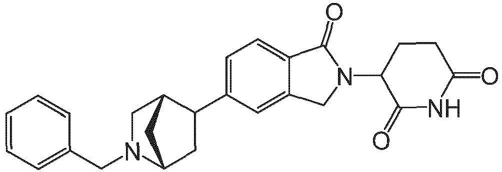 | 3-(5-((1S,4S)-2-benzyl-2-azabicyclo[2.2.1]heptan-5-yl)-1-oxoisoindolin-2-yl)piperidine-2,6-dione; |
| **I-29** | 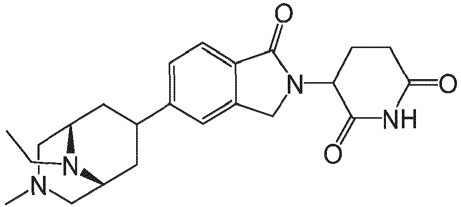 | 3-(5-((1R,5S)-9-ethyl-3-methyl-3,9-diazabicyclo[3.3.1]nonan-7-yl)-1-oxoisoindolin-2-yl)piperidine-2,6-dione; |
| **I-30** | 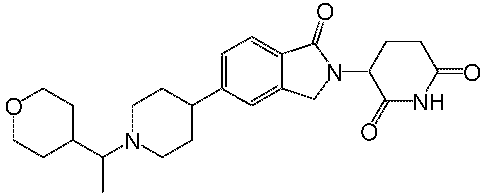 | 3-(1-oxo-5-(1-(1-(tetrahydro-2H-pyran-4-yl)ethyl)piperidin-4-yl)isoindolin-2-yl)piperidine-2,6-dione; |
| **I-31** | 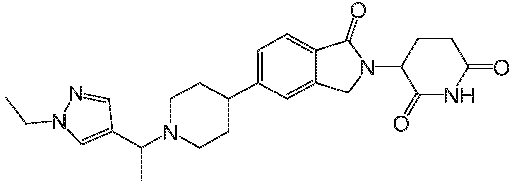 | 3-(5-(1-(1-(1-ethyl-1H-pyrazol-4-yl)ethyl)piperidin-4-yl)-1-oxoisoindolin-2-yl)piperidine-2,6-dione; |
| I-32 | 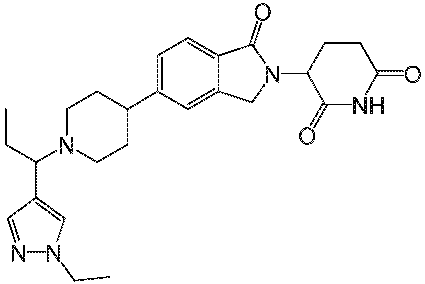 | 3-(5-(1-(1-(1-ethyl-1H-pyrazol-4-yl)propyl)piperidin-4-yl)-1-oxoisoindolin-2-yl)piperidine-2,6-dione; |
| **I-33** | 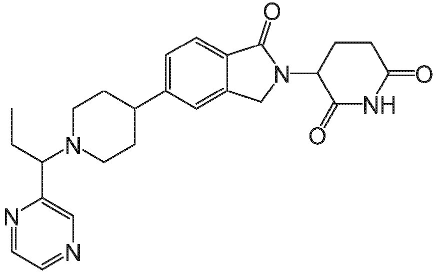 | 3-(1-oxo-5-(1-(1-(pyrazin-2-yl)propyl)piperidin-4-yl)isoindolin-2-yl)piperidine-2,6-dione; |
| **I-34** | 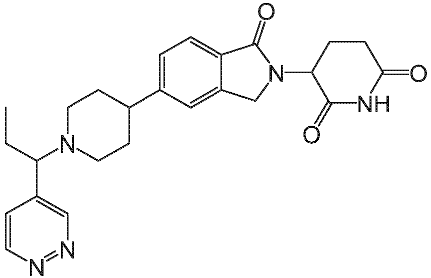 | 3-(1-oxo-5-(1-(1-(pyridazin-4-yl)propyl)piperidin-4-yl)isoindolin-2-yl)piperidine-2,6-dione; |
| **I-35** | 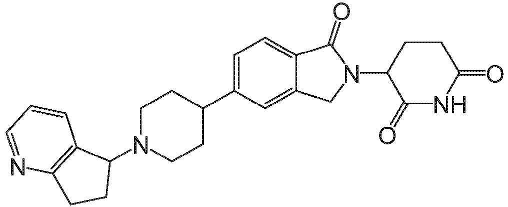 | 3-(5-(1-(6,7-dihydro-5H-cyclopenta[b]pyridin-5-yl)piperidin-4-yl)-1-oxoisoindolin-2-yl)piperidine-2,6-dione; |
| **I-36** | 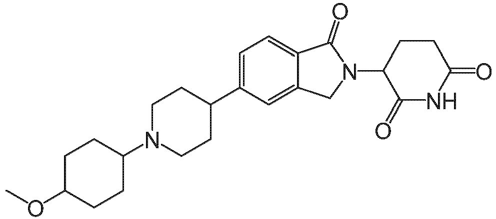 | 3-(5-(1-(4-methoxycyclohexyl)piperidin-4-yl)-1-oxoisoindolin-2-yl)piperidine-2,6-dione; |
| **I-37** | 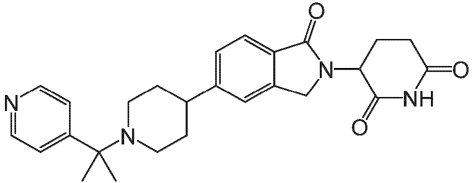 | 3-(1-oxo-5-(1-(2-(pyridin-4-yl)propan-2-yl)piperidin-4-yl)isoindolin-2-yl)piperidine-2,6-dione; |
| **I-38** | 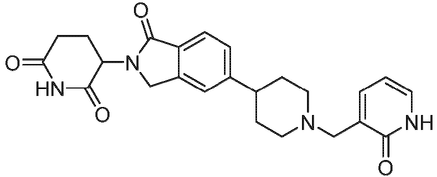 | 3-(1-oxo-5-(1-((2-oxo-1,2-dihydropyridin-3-yl)methyl)piperidin-4-yl)isoindolin-2-yl)piperidine-2,6-dione; |
| **I-39** | 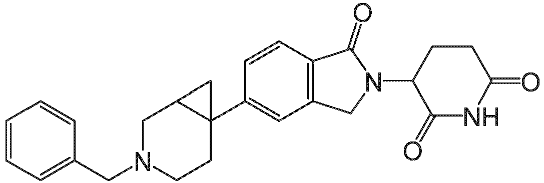 | 3-(5-(3-benzyl-3-azabicyclo[4.1.0]heptan-6-yl)-1-oxoisoindolin-2-yl)piperidine-2,6-dione; |
| **I-40** | 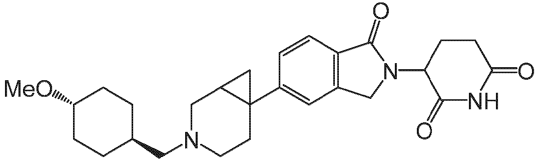 | 3-(5-(3-(((1r,4r)-4-methoxycyclohexyl)methyl)-3-azabicyclo[4.1.0]heptan-6-yl)-1-oxoisoindolin-2-yl)piperidine-2,6-dione; |
| **I-41** | 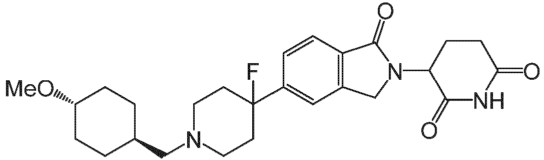 | 3-(5-(4-fluoro-1-(((1r,4r)-4-methoxycyclohexyl)methyl)pipe ridin-4-yl)-1-oxoisoindolin-2-yl)piperidine-2,6-dione; |
| **I-42** | 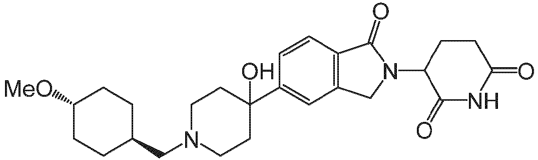 | 3-(5-(4-hydroxy-1-(((1r,4r)-4-methoxycyclohexyl)methyl)pipe ridin-4-yl)-1-oxoisoindolin-2-yl)piperidine-2,6-dione; |
| **I-43** | 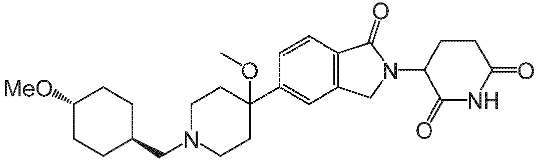 | 3-(5-(4-methoxy-1-(((1r,4r)-4-methoxycyclohexyl)methyl)pipe ridin-4-yl)-1-oxoisoindolin-2-yl)piperidine-2,6-dione; |
| **I-44** | 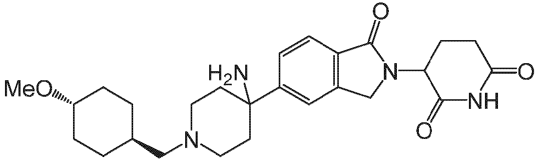 | 3-(5-(4-amino-1-(((1r,4r)-4-methoxycyclohexyl)methyl)pipe ridin-4-yl)-1-oxoisoindolin-2-yl)piperidine-2,6-dione; and |
| **I-45** | 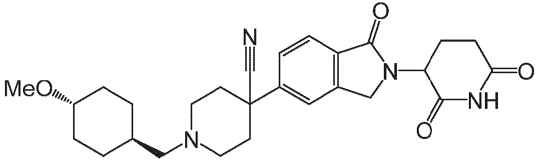 | 4-(2-(2,6-dioxopiperidin-3-yl)-1-oxoisoindolin-5-yl)-1-(((1r,4r)-4-methoxycyclohexyl)methyl)pipe ridine-4-carbonitrile. |

In another embodiment of the invention, the compounds of the present invention are enantiomers. In some embodiments the compounds are the (S)-enantiomer. In other embodiments the compounds are the (R)-enantiomer. In yet other embodiments, the compounds of the present invention may be (+) or (-) enantiomers.

It should be understood that all isomeric forms are included within the present invention, including mixtures thereof. If the compound contains a double bond, the substituent may be in the E or Z configuration. If the compound contains a disubstituted cycloalkyl, the cycloalkyl substituent may have a cis- or trans configuration. All tautomeric forms are also intended to be included.

Compounds of the invention, and pharmaceutically acceptable salts, hydrates, solvates, and stereoisomers, thereof may exist in their tautomeric form (for example, as an amide or imino ether). All such tautomeric forms are contemplated herein as part of the present invention.

The compounds of the invention may contain asymmetric or chiral centers and, therefore, exist in different stereoisomeric forms. It is intended that all stereoisomeric forms of the compounds of the invention as well as mixtures thereof, including racemic mixtures, form part of the present invention. In addition, the present invention embraces all geometric and positional isomers. For example, if a compound of the invention incorporates a double bond or a fused ring, both the cis- and trans-forms, as well as mixtures, are embraced within the scope of the invention. Each compound herein disclosed includes all the enantiomers that conform to the general structure of the compound. The compounds may be in a racemic or enantiomerically pure form, or any other form in terms of stereochemistry. The assay results may reflect the data collected for the racemic form, the enantiomerically pure form, or any other form in terms of stereochemistry.

Diastereomeric mixtures can be separated into their individual diastereomers on the basis of their physical chemical differences by methods well known to those skilled in the art, such as, for example, by chromatography and/or fractional crystallization. Enantiomers can be separated by converting the enantiomeric mixture into a diastereomeric mixture by reaction with an appropriate optically active compound (e.g., chiral auxiliary such as a chiral alcohol or Mosher's acid chloride), separating the diastereomers and converting (e.g., hydrolyzing) the individual diastereomers to the corresponding pure enantiomers. Also, some of the compounds of the invention may be atropisomers (e.g., substituted biaryls) and are considered as part of this invention. Enantiomers can also be separated by use of a chiral HPLC column.

It is also possible that the compounds of the invention may exist in different tautomeric forms, and all such forms are embraced within the scope of the invention and chemical structures and names. Also, for example, all keto-enol and imine-enamine forms of the compounds are included in the invention.

All stereoisomers (for example, geometric isomers, optical isomers, and the like) of the present compounds (including those of the salts, and solvates, of the compounds), such as those which may exist due to asymmetric carbons on various substituents, including enantiomeric forms (which may exist even in the absence of asymmetric carbons), rotameric forms, atropisomers, and diastereomeric forms, are contemplated within the scope of this invention, as are positional isomers (such as, for example, 4-pyridyl and 3-pyridyl). (For example, if a compound of Formula (I) or a compound of Embodiment 17 incorporates a double bond or a fused ring, both the cis- and trans-forms, as well as mixtures, are embraced within the scope of the invention. Also, for example, all keto-enol and imine-enamine forms of the compounds are included in the invention.) Individual stereoisomers of the compounds of the invention may, for example, be substantially free of other isomers, or is admixed, for example, as racemates or with all other, or other selected, stereoisomers.

The chiral centers of the compounds of the invention can have the S or R configuration as defined by the IUPAC 1974 Recommendations. In certain embodiments, each asymmetric atom has at least 50% enantiomeric excess, at least 60% enantiomeric excess, at least 70% enantiomeric excess, at least 80% enantiomeric excess, at least 90% enantiomeric excess, at least 95% enantiomeric excess, or at least 99% enantiomeric excess in the (R)- or (S)- configuration. Substituents at atoms with unsaturated double bonds may, if possible, be present in cis-(Z)- or trans-(E)- form.

The use of the terms "salt", "solvate", and the like, is intended to equally apply to the salt, and solvate, of enantiomers, stereoisomers, rotamers, tautomers, positional isomers, racemates, of the inventive compounds.

The compounds of the invention may form salts which are also within the scope of this invention. Reference to a compound of the Formula herein is generally understood to include reference to salts thereof, unless otherwise indicated.

The compounds and intermediates may be isolated and used as the compound *per se.* Any formula given herein is also intended to represent unlabeled forms as well as isotopically labeled forms of the compounds. Isotopically labeled compounds have structures depicted by the formulas given herein except that one or more atoms are replaced by an atom having a selected atomic mass or mass number. Examples of isotopes that can be incorporated into compounds of the invention include isotopes of hydrogen, carbon, nitrogen, oxygen, phosphorous, fluorine, and, such as ²H, ³H, ¹¹C, ¹³C, ¹⁴C, ¹⁵N, ¹⁸F, ³¹P, ³²P, respectively. The invention includes various isotopically labeled compounds as defined herein, for example those into which radioactive isotopes, such as ³H, ¹³C, and ¹⁴C, are present. Such isotopically labelled compounds are useful in metabolic studies (with ¹⁴C), reaction kinetic studies (with, for example ²H or ³H), detection or imaging techniques, such as positron emission tomography (PET) or single-photon emission computed tomography (SPECT) including drug or substrate tissue distribution assays, or in radioactive treatment of patients. In particular, an ¹⁸F, ¹¹C or labeled compound may be particularly desirable for PET or SPECT studies.

Further, substitution with heavier isotopes, particularly deuterium (i.e., ²H or D) may afford certain therapeutic advantages resulting from greater metabolic stability, for example, increased *in vivo* half-life, reduced dosage requirements, reduced CYP450 inhibition (competitive or time dependent) or an improvement in therapeutic index. For example, substitution with deuterium may modulate undesirable side effects of the undeuterated compound, such as competitive CYP450 inhibition, time dependent CYP450 inactivation, etc. It is understood that deuterium in this context is regarded as a substituent in compounds of the present invention. The concentration of such a heavier isotope, specifically deuterium, may be defined by the isotopic enrichment factor. The term "isotopic enrichment factor" as used herein means the ratio between the isotopic abundance and the natural abundance of a specified isotope. If a substituent in a compound of this invention is denoted deuterium, such compound has an isotopic enrichment factor for each designated deuterium atom of at least 3500 (52.5% deuterium incorporation at each designated deuterium atom), at least 4000 (60% deuterium incorporation), at least 4500 (67.5% deuterium incorporation), at least 5000 (75% deuterium incorporation), at least 5500 (82.5% deuterium incorporation), at least 6000 (90% deuterium incorporation), at least 6333.3 (95% deuterium incorporation), at least 6466.7 (97% deuterium incorporation), at least 6600 (99% deuterium incorporation), or at least 6633.3 (99.5% deuterium incorporation).

Isotopically-labeled compounds of the present invention can generally be prepared by conventional techniques known to those skilled in the art or by carrying out the procedures disclosed in the schemes or in the examples and preparations described below using an appropriate isotopically-labeled reagent in place of the non-isotopically labeled reagent.

Pharmaceutically acceptable solvates in accordance with the invention include those wherein the solvent of crystallization may be isotopically substituted, e.g., D₂O, d₆-acetone, d₆-DMSO.

The present invention relates to compounds which are modulators of IKZF2 protein levels. In one embodiment, the compounds of the present invention decrease IKZF2 protein levels. In yet one embodiment, the compounds of the present invention reduce IKZF2 protein levels. In another embodiment, the compounds of the present invention are degraders of IKZF2.

The present invention relates to compounds, which are modulators of IKZF2 and IKZF4 protein levels. In one embodiment, the compounds of the present invention decrease IKZF2 and IKZF4 protein levels. In yet one embodiment, the compounds of the present invention reduce IKZF2 and IKZF4 protein levels. In another embodiment, the compounds of the present invention are degraders of IKZF2.

In some embodiments, the compounds of the invention are selective over other proteins. As used herein "selective modulator", "selective degrader", or "selective compound" means, for example, a compound of the invention, that effectively modulates, decreases, or reduces the levels of a specific protein or degrades a specific protein to a greater extent than any other protein. A "selective modulator", "selective degrader", or "selective compound" can be identified, for example, by comparing the ability of a compound to modulate, decrease, or reduce the levels of or to degrade a specific protein to its ability to modulate, decrease, or reduce the levels of or to degrade other proteins. In some embodiments, the selectivity can be identified by measuring the AC₅₀, EC₅₀, or IC₅₀ of the compounds.

In some embodiments, the compounds of the present application are selective IKZF2 modulators. As used herein "selective IKZF2 modulator", "selective IKZF2 degrader", or "selective IKZF2 compound" refers to a compound of the application, for example, that effectively modulates, decrease, or reduces the levels of IKZF2 protein or degrades IKZF2 protein to a greater extent than any other protein, particularly any protein (transcription factor) from the Ikaros protein family (e.g., IKZF1, IKZF3, IKZF4, and IKZF5).

A "selective IKZF2 modulator", "selective IKZF2 degrader", or "selective IKZF2 compound" can be identified, for example, by comparing the ability of a compound to modulate IKZF2 protein levels to its ability to modulate levels of other members of the Ikaros protein family or other proteins. For example, a substance may be assayed for its ability to modulate IKZF2 protein levels, as well as IKZF1, IKZF3, IKZF4, IKZF5, and other proteins. In some embodiments, the selectivity can be identified by measuring the EC₅₀ of the compounds. In some embodiments, the selectivity can be identified by measuring the AC₅₀ of the compounds. In some embodiments, a selective IKZF2 degrader is identified by comparing the ability of a compound to degrade IKZF2 to its ability to degrade other members of the Ikaros protein family or other proteins.

In certain embodiments, the compounds of the application are IKZF2 degraders that exhibit at least 2-fold, 3-fold, 5-fold, 10-fold, 25-fold, 50-fold or 100-fold selectivity for the degradation of IKZF2 over other proteins (e.g., IKZF1, IKZF3, IKZF4, and IKZF5). In various embodiments, the compounds of the application exhibit up to 1000-fold selectivity for the degradation of IKZF2 over other proteins.

In certain embodiments, the compounds of the application exhibit at least 2-fold, 3-fold, 5-fold, 10-fold, 25-fold, 50-fold or 100-fold selectivity for the degradation of IKZF2 over the other members of the Ikaros protein family (e.g., IKZF1, IKZF3, IKZF4, and IKZF5). In various embodiments, the compounds of the application exhibit up to 1000-fold selectivity for the degradation of IKZF2 over the other members of the Ikaros protein family (e.g., IKZF1, IKZF3, IKZF4, and IKZF5).

In certain embodiments, the compounds of the application exhibit at least 2-fold, 3-fold, 5-fold, 10-fold, 25-fold, 50-fold or 100-fold selectivity for the degradation of IKZF2 over IKZF1. In various embodiments, the compounds of the application exhibit up to 1000-fold selectivity for the degradation of IKZF2 over IKZF1.

In certain embodiments, the compounds of the application exhibit at least 2-fold, 3-fold, 5-fold, 10-fold, 25-fold, 50-fold or 100-fold selectivity for the degradation of IKZF2 over IKZF3. In various embodiments, the compounds of the application exhibit up to 1000-fold selectivity for the degradation of IKZF2 over IKZF3.

In certain embodiments, the compounds of the application exhibit at least 2-fold, 3-fold, 5-fold, 10-fold, 25-fold, 50-fold or 100-fold selectivity for the degradation of IKZF2 over IKZF4. In various embodiments, the compounds of the application exhibit up to 1000-fold selectivity for the degradation of IKZF2 over IKZF4.

In certain embodiments, the compounds of the application exhibit at least 2-fold, 3-fold, 5-fold, 10-fold, 25-fold, 50-fold or 100-fold selectivity for the degradation of IKZF2 over IKZF5. In various embodiments, the compounds of the application exhibit up to 1000-fold selectivity for the degradation of IKZF2 over IKZF5.

In certain embodiments, the compounds of the application exhibit at least 2-fold, 3-fold, 5-fold, 10-fold, 25-fold, 50-fold or 100-fold selectivity for the degradation of IKZF2 and IKZF4 over the other members of the Ikaros protein family (e.g., IKZF1, IKZF3, and IKZF5). In various embodiments, the compounds of the application exhibit up to 1000-fold selectivity for the degradation of IKZF2 and IKZF4 over the other members of the Ikaros protein family (e.g., IKZF1, IKZF3, and IKZF5).

In certain embodiments, the compounds of the application exhibit at least 2-fold, 3-fold, 5-fold, 10-fold, 25-fold, 50-fold or 100-fold selectivity for the degradation of IKZF2 and IKZF4 over IKZF1. In various embodiments, the compounds of the application exhibit up to 1000-fold selectivity for the degradation of IKZF2 and IKZF4 over IKZF1.

In certain embodiments, the compounds of the application exhibit at least 2-fold, 3-fold, 5-fold, 10-fold, 25-fold, 50-fold or 100-fold selectivity for the degradation of IKZF2 and IKZF4 over IKZF3. In various embodiments, the compounds of the application exhibit up to 1000-fold selectivity for the degradation of IKZF2 and IKZF4 over IKZF3.

In certain embodiments, the compounds of the application exhibit at least 2-fold, 3-fold, 5-fold, 10-fold, 25-fold, 50-fold or 100-fold selectivity for the degradation of IKZF2 and IKZF4 over IKZF5. In various embodiments, the compounds of the application exhibit up to 1000-fold selectivity for the degradation of IKZF2 and IKZF4 over IKZF5.

In some embodiments, the degradation of IKZF2 is measured by AC₅₀.

Potency of can be determined by AC₅₀ value. A compound with a lower AC₅₀ value, as determined under substantially similar degradation conditions, is a more potent degrader relative to a compound with a higher AC₅₀ value. In some embodiments, the substantially similar conditions comprise determining degradation of protein levels in cells expressing the specific protein, or a fragment of any thereof.

The invention is directed to compounds as described herein and pharmaceutically acceptable salts, hydrates, solvates, stereoisomers, or tautomers thereof, and pharmaceutical compositions comprising one or more compounds as described herein, or pharmaceutically acceptable salts, hydrates, solvates, stereoisomers, or tautomers thereof.

### E. Methods of Synthesizing Compounds of the Invention

The compounds of the present invention may be made by a variety of methods, including standard chemistry. Suitable synthetic routes are depicted in the Schemes given below.

The compounds of the present invention may be prepared by methods known in the art of organic synthesis as set forth in part by the following synthetic schemes. In the schemes described below, it is well understood that protecting groups for sensitive or reactive groups are employed where necessary in accordance with general principles or chemistry. Protecting groups are manipulated according to standard methods of organic synthesis (T.W. Greene and P.G.M. Wuts, "Protective Groups in Organic Synthesis", Third edition, Wiley, New York 1999). These groups are removed at a convenient stage of the compound synthesis using methods that are readily apparent to those skilled in the art. The selection processes, as well as the reaction conditions and order of their execution, shall be consistent with the preparation of Compounds of Formula (I) or compounds **(I-1), (I-2), (I-3), (I-4), (I-5), (I-6), (I-7), (I-8), (I-9), (I-10), (I-11), (I-12), (I-13), (I-14), (I-15), (I-16), (I-17), (I-18), (I-24), (I-25), (I-26), (I-27), (I-28), (I-29), (I-30), (I-31), (I-32), (I-33), (I-34), (I-34), (I-36), (I-37),** and **(I-38).** Those skilled in the art will recognize if a stereocenter exists in the compounds of the present invention. Accordingly, the present invention includes both possible stereoisomers (unless specified in the synthesis) and includes not only racemic compounds but the individual enantiomers and/or diastereomers as well. When a compound is desired as a single enantiomer or diastereomer, it may be obtained by stereospecific synthesis or by resolution of the final product or any convenient intermediate. Resolution of the final product, an intermediate, or a starting material may be affected by any suitable method known in the art. See, for example, "Stereochemistry of Organic Compounds" by E.L. Eliel, S.H. Wilen, and L.N. Mander (Wiley-Interscience, 1994).

The compounds described herein may be made from commercially available starting materials or synthesized using known organic, inorganic, and/or enzymatic processes.

### Preparation of Compounds

The compounds of the present invention can be prepared in a number of ways well known to those skilled in the art of organic synthesis. By way of example, compounds of the present invention can be synthesized using the methods described below, together with synthetic methods known in the art of synthetic organic chemistry, or variations thereon as appreciated by those skilled in the art. Preferred methods include but are not limited to those methods described below.

Compounds of the present invention can be synthesized by following the steps outlined in General Schemes **I** to **IV** which comprise different sequences of assembling intermediates **1**-a to **1**-g, **2**-a, **2**-b, **2**-c, **3**-a, **4**-a, and **4**-b. Starting materials are either commercially available or made by known procedures in the reported literature or as illustrated.

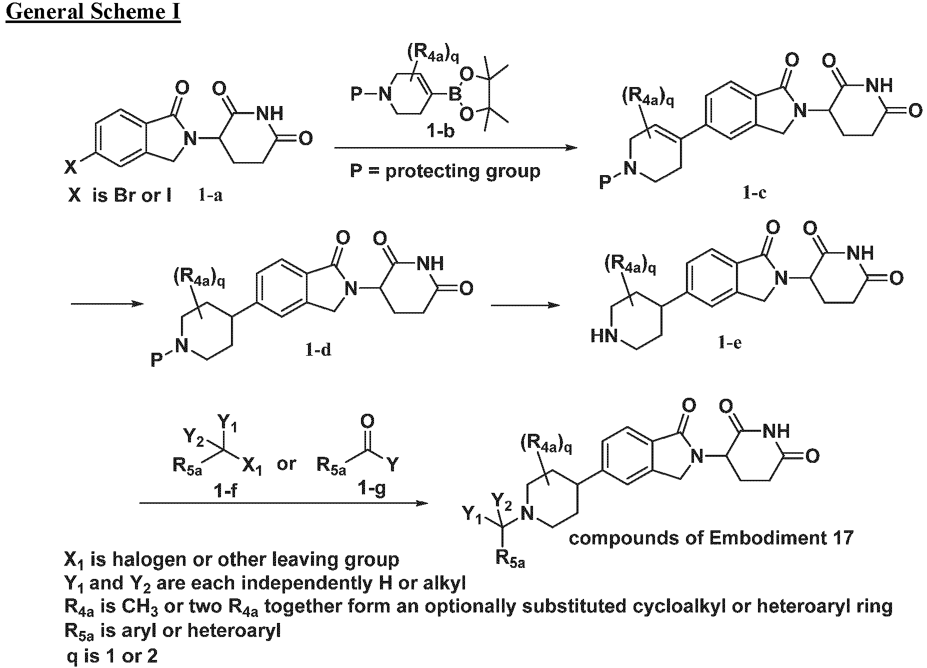

The general way of preparing compounds of Embodiment 17 (e.g., **(I-1)-(I-18)** and( **(I-24)-(I-38)),** by using intermediates **1**-a, **1**-b, **1**-c, **1**-d, **1**-e, **1**-f, and **1**-g is outlined in General Scheme I. Coupling of 1-a with boronic ester 1-b using a catalyst (e.g., Pd(dppf)Cl₂•DCM), and a base (e.g., cesium carbonate (Cs₂CO₃)), in a solvent (e.g., *N,N*-dimethylformamide (DMF)) at elevated temperature yields 1-c. Hydrogenation of 1-c in the presence of a suitable catalyst (e.g., Pd/C or PtO₂) in a solvent (e.g., DMF) and under an atmosphere of hydrogen gas provides **1-d.** Removal of the amine protecting group (e.g., *tert-*butyloxycarbonyl (Boc)) on intermediate **1-d** can be accomplished using a strong acid such as trifluoroacetic acid (TFA) or hydrochloric acid (HCl) in a solvent (e.g., tetrahydrofuran (THF), 1,2-dichloroethane, dioxane or dichloromethane (DCM)) optionally at elevated temperature to provide **I-e.** Reductive amination of **1-e** with aldehyde or ketone **1-g** provides the desired product. Alternatively, compounds of Embodiment 17 where X₁ is CH and R₂ is a substituted alkyl can be obtained by alkylation of **I-e** with an alkyl halide **1-f** in the presence of a base (e.g., triethyl amine (TEA), cesium carbonate (Cs₂CO₃), etc.), in a solvent (e.g., DCM, DMF, etc.), and optionally at elevated temperature. 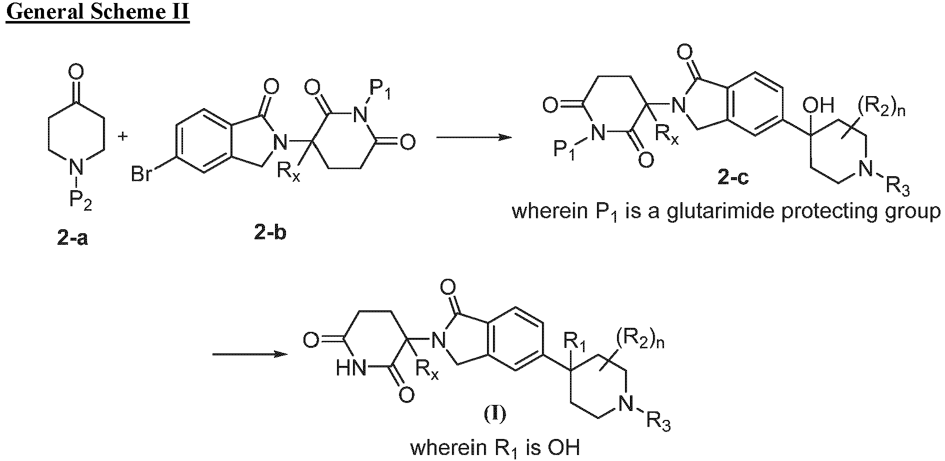 wherein Rₓ, R₂, R₃ and n are as defined herein above.

The general way of preparing compounds of Formula (I) wherein R₁ is OH by using intermediates **2-a, 2-b,** and **2-c** is outlined in General Scheme II. Alkylation of ketone **2-a** with **2-b** in the presence of a strong base (e.g., n-butyl lithium (n-BuLi), tert-butyl lithium (t-BuLi), sec-butyl lithium (s-BuLi)) in a solvent (e.g., tetrahydrofuran (THF), diethyl ether (Et₂O)), optionally at cold temperatures provides **2-c.** Removal of the glutarimide protecting group (e.g., para-methoxybenzyl (PMB) or [2-(Trimethylsilyl)ethoxy]methyl acetal (SEM)) can be accomplished in the presence of strong acid (e.g., HCl or TFA) optionally in a solvent (e.g., THF, 1,2-dichloroethane, dioxane, or dichloromethane (DCM)) and optionally followed by treatment with a base (e.g., TEA) in a solvent and in the presence of N1,N2-dimethylethane-1,2-diamine (when P₁ is SEM) provides the desired compound of Formula (I) wherein R₁ is OH. 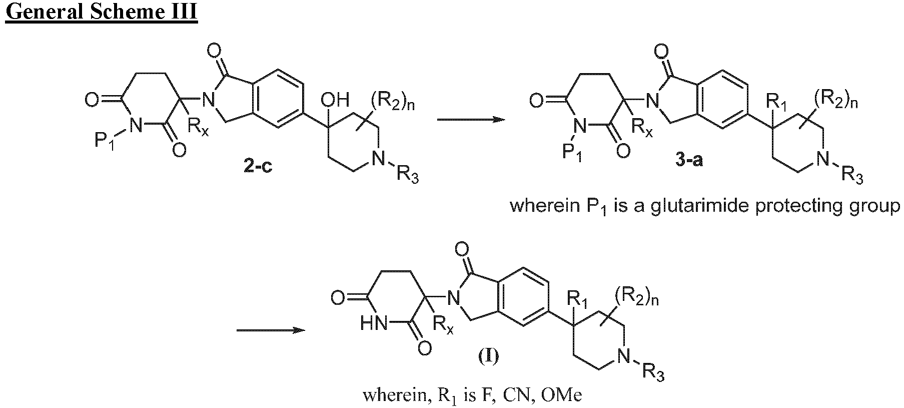 wherein Rₓ, R₂, R₃ and n are as defined herein above.

The general way of preparing compounds of Formula (I) wherein R₁ is F, CN or OMe by using intermediates 2-c and 3-a is outlined in General Scheme III. Treatment of 2-c with a fluorinating agent (e.g., diethylaminosulfur trifluoride (DAST)) in a solvent (e.g., DCM) optionally at cold temperatures followed by removal of the glutarimide protecting group as described above for General Scheme II provides the desired compound of Formula (I) wherein R₁ is F. Alternatively, treatment of 2-c with trimethylsilyl cyanide (TMSCN) and a Lewis Acid (e.g., zinc(II) iodide (ZnI₂), scandium(III) triflate (Sc(OTf)₃), tin(IV) chloride (SnCl₄), indium(III) chloride (InCl₃), or titanium montmorillonite) in a solvent (e.g., DCM) optionally at cold temperatures followed by removal of the glutarimide protecting group as described above for General Scheme II provides the desired compound of Formula (I) wherein R₁ is CN. Furthermore, compounds of Formula (I) wherein R₁ is OMe can be obtained by the treatment of **2-c** with a methylating agent (e.g., diazomethane (CH₂N₂), trimethyloxonium tetrafluoroborate ([Me₃O]⁺[BF₄]⁻, methyl triflate (MeOTf)) in a solvent (e.g., DCM, MeCN, THF) optionally in the presence of base (N,N-diisopropylethylamine (*i*-Pr₂NEt), sodium hydride (NaH)) and optionally at cold temperatures followed by removal of the glutarimide protecting group. 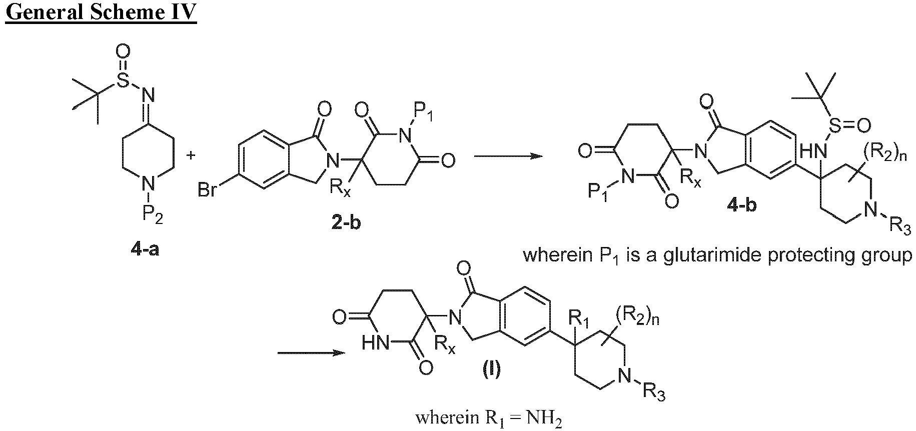 wherein Rₓ, R₂, R₃ and n are as defined herein above.

The general way of preparing compounds of Formula (I) wherein R₁ is NH₂ by using intermediates **4-a, 2-b,** and **4-b** is outlined in General Scheme IV. Alkylation of ketone **4-a** with **2-b** in the presence of a strong base (e.g., n-butyl lithium (n-BuLi), tert-butyl lithium (t-BuLi), sec-butyl lithium (s-BuLi)) in a solvent (e.g., tetrahydrofuran (THF), diethyl ether (Et₂O)), optionally at cold temperatures provides **4-b.** Removal of the glutarimide protecting group (e.g., *para*-methoxybenzyl (PMB) or [2-(Trimethylsilyl)ethoxy]methyl acetal (SEM)) can be accomplished in the presence of strong acid (e.g., HCl or TFA) optionally in a solvent (e.g., THF, 1,2-dichloroethane, dioxane or dichloromethane (DCM)) and optionally followed by treatment with a base (e.g., TEA) in a solvent and in the presence of N1,N2-dimethylethane-1,2-diamine (when P₁ is SEM) provides the desired compound of Formula (I) wherein R₁ is NH₂.

A mixture of enantiomers, diastereomers, and cis/trans isomers resulting from the process described above can be separated into their single components by chiral salt technique, chromatography using normal phase, reverse phase or chiral column, depending on the nature of the separation.

Any resulting racemates of compounds of the present invention or of intermediates can be resolved into the optical antipodes by known methods, e.g., by separation of the diastereomeric salts thereof, obtained with an optically active acid or base, and liberating the optically active acidic or basic compound. In particular, a basic moiety may thus be employed to resolve the compounds of the present invention into their optical antipodes, e.g., by fractional crystallization of a salt formed with an optically active acid, e.g., tartaric acid, dibenzoyl tartaric acid, diacetyl tartaric acid, di-O,O'-p-toluoyl tartaric acid, mandelic acid, malic acid, or camphor-10-sulfonic acid. Racemic compounds of the present invention or racemic intermediates can also be resolved by chiral chromatography, e.g., high pressure liquid chromatography (HPLC) using a chiral adsorbent.

Any resulting mixtures of stereoisomers can be separated on the basis of the physicochemical differences of the constituents, into the pure or substantially pure geometric or optical isomers, diastereomers, racemates, for example, by chromatography and/or fractional crystallization.

It should be understood that in the description and formula shown above, the various groups Rₓ, R₂, R₃ and n and other variables are as defined above, except where otherwise indicated. Furthermore, for synthetic purposes, the compounds of General Schemes **I** to **IV** are merely representative with elected radicals to illustrate the general synthetic methodology of the Compounds of Formula (I) as defined herein.

### F. Methods of Using Compounds of the Invention

In another aspect, the present invention relates to a compound of Formula (I), or a pharmaceutically acceptable salt, hydrate, solvate, stereoisomer, or tautomer thereof, or a composition comprising a compound of Formula (I), or a compound of formula **(I-1), (I-2), (I-3), (I-4), (I-5), (I-6), (I-7), (I-8), (I-9), (I-10), (I-11), (I-12), (I-13), (I-14), (I-15), (I-16), (I-17), (I-18), (I-24), (I-25), (I-26), (I-27), (I-28), (I-29), (I-30), (I-31), (I-32), (I-33), (I-34), (I-34), (I-36), (I-37),** or **(I-38)**, or a pharmaceutically acceptable salt, hydrate, solvate, stereoisomer, or tautomer thereof, for use in the treatment of cancer.

In another aspect, the present invention relates to a compound of Formula (I), or a pharmaceutically acceptable salt, hydrate, solvate, stereoisomer, or tautomer thereof, or a composition comprising a compound of Formula (I), or a compound of formula **(I-1), (I-2), (I-3), (I-4), (I-5), (I-6), (I-7), (I-8), (I-9), (I-10), (I-11), (I-12), (I-13), (I-14), (I-15), (I-16), (I-17), (I-18), (I-24), (I-25), (I-26), (I-27), (I-28), (I-29), (I-30), (I-31), (I-32), (I-33), (I-34), (I-34), (I-36), (I-37),** or **(I-38),** for use in the treatment of an IKZF2-dependent cancer.

Another aspect of the invention relates to a compound of Formula (I), or a pharmaceutically acceptable salt, hydrate, solvate, stereoisomer, or tautomer thereof, or a composition comprising a compound of Formula (I), or a compound of formula **(I-1), (I-2), (I-3), (I-4), (I-5), (I-6), (I-7), (I-8), (I-9), (I-10), (I-11), (I-12), (I-13), (I-14), (I-15), (I-16), (I-17), (I-18), (I-24), (I-25), (I-26), (I-27), (I-28), (I-29), (I-30), (I-31), (I-32), (I-33), (I-34), (I-34), (I-36), (I-37),** or **(I-38),** for use in the treatment of an IKZF2-dependent disease or disorder.

Another aspect of the invention relates to a compound of Formula (I), or a pharmaceutically acceptable salt, hydrate, solvate, stereoisomer, or tautomer thereof, or a composition comprising a compound of Formula (I), or a compound of formula **(I-1), (I-2), (I-3), (I-4), (I-5), (I-6), (I-7), (I-8), (I-9), (I-10), (I-11), (I-12), (I-13), (I-14), (I-15), (I-16), (I-17), (I-18), (I-24), (I-25), (I-26), (I-27), (I-28), (I-29), (I-30), (I-31), (I-32), (I-33), (I-34), (I-34), (I-36), (I-37),** or **(I-38),** for use in the treatment of a disease or disorder that is affected by the reduction of IKZF2 protein levels.

The compounds of the present invention can be used for the treatment, of a disease or disorder selected from liposarcoma, neuroblastoma, glioblastoma, bladder cancer, adrenocortical cancer, multiple myeloma, colorectal cancer, non-small cell lung cancer, Human Papilloma Virus-associated cervical, oropharyngeal, penis, anal, thyroid, or vaginal cancer or Epstein-Barr Virus-associated nasopharyngeal carcinoma, gastric cancer, rectal cancer, thyroid cancer, Hodgkin lymphoma or diffuse large B-cell lymphoma. the cancer is selected from prostate cancer, breast carcinoma, lymphomas, leukaemia, myeloma, bladder carcinoma, colon cancer, cutaneous melanoma, hepatocellular carcinoma, endometrial cancer, ovarian cancer, cervical cancer, lung cancer, renal cancer, glioblastoma multiform, glioma, thyroid cancer, parathyroid tumor, nasopharyngeal cancer, tongue cancer, pancreatic cancer, esophageal cancer, cholangiocarcinoma, gastric cancer, soft tissue sarcomas, rhabdomyosarcoma (RMS), synovial sarcoma, osteosarcoma, rhabdoid cancers, cancer for which the immune response is deficient, an immunogenic cancer, and Ewing's sarcoma. In one embodiment, the IKZF2-dependent disease or disorder is a disease or disorder is selected from non-small cell lung cancer (NSCLC), melanoma, triple-negative breast cancer (TNBC), nasopharyngeal cancer (NPC), microsatellite stable colorectal cancer (mssCRC), thymoma, carcinoid, and gastrointestinal stromal tumor (GIST). In another embodiment, the cancer is selected from non-small cell lung cancer (NSCLC), melanoma, triple-negative breast cancer (TNBC), nasopharyngeal cancer (NPC), microsatellite stable colorectal cancer (mssCRC), thymoma, carcinoid, acute myelogenous leukemia, and gastrointestinal stromal tumor (GIST). In another embodiment, the IKZF2-dependent disease or disorder is a disease or disorder is selected from non-small cell lung cancer (NSCLC), melanoma, triple-negative breast cancer (TNBC), nasopharyngeal cancer (NPC), and microsatellite stable colorectal cancer (mssCRC).

The compounds of the invention can be administered in effective amounts to treat or prevent a disorder and/or prevent the development thereof in subjects.

### G. Administration, Pharmaceutical Compositions, and Dosing of Compounds of the Invention

Administration of the disclosed compounds can be accomplished via any mode of administration for therapeutic agents. These modes include systemic or local administration such as oral, nasal, parenteral, transdermal, subcutaneous, vaginal, buccal, rectal or topical administration modes.

Depending on the intended mode of administration, the disclosed compositions can be in solid, semi-solid or liquid dosage form, such as, for example, injectables, tablets, suppositories, pills, time-release capsules, elixirs, tinctures, emulsions, syrups, powders, liquids, suspensions, or the like, sometimes in unit dosages and consistent with conventional pharmaceutical practices. Likewise, they can also be administered in intravenous (both bolus and infusion), intraperitoneal, subcutaneous or intramuscular form, and all using forms well known to those skilled in the pharmaceutical arts.

Illustrative pharmaceutical compositions are tablets and gelatin capsules comprising a compound of the invention and a pharmaceutically acceptable carrier, such as a) a diluent, e.g., purified water, triglyceride oils, such as hydrogenated or partially hydrogenated vegetable oil, or mixtures thereof, com oil, olive oil, sunflower oil, safflower oil, fish oils, such as EPA or DHA, or their esters or triglycerides or mixtures thereof, omega-3 fatty acids or derivatives thereof, lactose, dextrose, sucrose, mannitol, sorbitol, cellulose, sodium, saccharin, glucose and/or glycine; b) a lubricant, e.g., silica, talcum, stearic acid, its magnesium or calcium salt, sodium oleate, sodium stearate, magnesium stearate, sodium benzoate, sodium acetate, sodium chloride, and/or polyethylene glycol; for tablets also; c) a binder, e.g., magnesium aluminum silicate, starch paste, gelatin, tragacanth, methylcellulose, sodium carboxymethylcellulose, magnesium carbonate, natural sugars such as glucose or beta-lactose, corn sweeteners, natural and synthetic gums such as acacia, tragacanth or sodium alginate, waxes, and/or polyvinylpyrrolidone, if desired; d) a disintegrant, e.g., starches, agar, methyl cellulose, bentonite, xanthan gum, algic acid or its sodium salt, or effervescent mixtures; e) absorbent, colorant, flavorant and sweetener; f) an emulsifier or dispersing agent, such as Tween 80, Labrasol, HPMC, DOSS, caproyl 909, labrafac, labrafil, peceol, transcutol, capmul MCM, capmul PG-12, captex 355, gelucire, vitamin E TGPS or other acceptable emulsifier; and/or g) an agent that enhances absorption of the compound such as cyclodextrin, hydroxypropyl-cyclodextrin, PEG400, PEG200.

Liquid, particularly injectable, compositions can, for example, be prepared by dissolution, dispersion, etc. For example, the disclosed compound is dissolved in or mixed with a pharmaceutically acceptable solvent such as, for example, water, saline, aqueous dextrose, glycerol, ethanol, and the like, to thereby form an injectable isotonic solution or suspension. Proteins such as albumin, chylomicron particles, or serum proteins can be used to solubilize the disclosed compounds.

The disclosed compounds can be also formulated as a suppository that can be prepared from fatty emulsions or suspensions; using polyalkylene glycols such as propylene glycol, as the carrier.

The disclosed compounds can also be administered in the form of liposome delivery systems, such as small unilamellar vesicles, large unilamellar vesicles, and multilamellar vesicles. Liposomes can be formed from a variety of phospholipids, containing cholesterol, stearylamine or phosphatidylcholines.

In some embodiments, a film of lipid components is hydrated with an aqueous solution of drug to a form lipid layer encapsulating the drug, as described in U.S. Pat. No. 5,262,564.

Disclosed compounds can also be delivered by the use of monoclonal antibodies as individual carriers to which the disclosed compounds are coupled. The disclosed compounds can also be coupled with soluble polymers as targetable drug carriers. Such polymers can include polyvinylpyrrolidone, pyran copolymer, polyhydroxypropylmethacrylamide-phenol, polyhydroxyethylaspanamidephenol, or polyethyleneoxidepolylysine substituted with palmitoyl residues. Furthermore, the disclosed compounds can be coupled to a class of biodegradable polymers useful in achieving controlled release of a drug, for example, polylactic acid, polyepsilon caprolactone, polyhydroxy butyric acid, polyorthoesters, polyacetals, polydihydropyrans, polycyanoacrylates, and cross-linked or amphipathic block copolymers of hydrogels. In one embodiment, disclosed compounds are not covalently bound to a polymer, e.g., a polycarboxylic acid polymer, or a polyacrylate.

Parental injectable administration is generally used for subcutaneous, intramuscular or intravenous injections and infusions. Injectables can be prepared in conventional forms, either as liquid solutions or suspensions or solid forms suitable for dissolving in liquid prior to injection.

Another aspect of the invention is directed to pharmaceutical compositions comprising a compound of Formula (I), and a pharmaceutically acceptable carrier. The pharmaceutical acceptable carrier may further include an excipient, diluent, or surfactant.

Another aspect of the invention is directed to pharmaceutical compositions comprising a compound of **(I-1), (I-2), (I-3), (I-4), (I-5), (I-6), (I-7), (I-8), (I-9), (I-10), (I-11), (I-12), (I-13), (I-14), (I-15), (I-16), (I-17), (I-18), (I-24), (I-25), (I-26), (I-27), (I-28), (I-29), (I-30), (I-31), (I-32), (I-33), (I-34), (I-34), (I-36), (I-37),** or **(I-38),** and a pharmaceutically acceptable carrier. The pharmaceutical acceptable carrier may further include an excipient, diluent, or surfactant.

Compositions can be prepared according to conventional mixing, granulating or coating methods, respectively, and the present pharmaceutical compositions can contain from about 0.1% to about 99%, from about 5% to about 90%, or from about 1% to about 20% of the disclosed compound by weight or volume.

In one embodiment, the invention provides a kit comprising two or more separate pharmaceutical compositions, at least one of which contains a compound of the present invention. In one embodiment, the kit comprises means for separately retaining said compositions, such as a container, divided bottle, or divided foil packet. An example of such a kit is a blister pack, as typically used for the packaging of tablets, capsules and the like.

The kit of the invention may be used for administering different dosage forms, for example, oral and parenteral, for administering the separate compositions at different dosage intervals, or for titrating the separate compositions against one another. To assist compliance, the kit of the invention typically comprises directions for administration.

The dosage regimen utilizing the disclosed compound is selected in accordance with a variety of factors including type, species, age, weight, sex, and medical condition of the patient; the severity of the condition to be treated; the route of administration; the renal or hepatic function of the patient; and the particular disclosed compound employed. A physician or veterinarian of ordinary skill in the art can readily determine and prescribe the effective amount of the drug required to prevent, counter or arrest the progress of the condition.

Effective dosage amounts of the disclosed compounds, when used for the indicated effects, range from about 0.5 mg to about 5000 mg of the disclosed compound as needed to treat the condition. Compositions for *in vivo* or *in vitro* use can contain about 0.5, 5, 20, 50, 75, 100, 150, 250, 500, 750, 1000, 1250, 2500, 3500, or 5000 mg of the disclosed compound, or, in a range of from one amount to another amount in the list of doses. In one embodiment, the compositions are in the form of a tablet that can be scored.

### H. Combination Therapy

The compounds of the invention can be administered in therapeutically effective amounts in a combinational therapy with one or more therapeutic agents (pharmaceutical combinations) or modalities, e.g., non-drug therapies. For example, synergistic effects can occur with other cancer agents. Where the compounds of the application are administered in conjunction with other therapies, dosages of the co-administered compounds will of course vary depending on the type of co-drug employed, on the specific drug employed, on the condition being treated and so forth.

The compounds can be administered simultaneously (as a single preparation or separate preparation), sequentially, separately, or over a period of time to the other drug therapy or treatment modality. In general, a combination therapy envisions administration of two or more drugs during a single cycle or course of therapy. A therapeutic agent is, for example, a chemical compound, peptide, antibody, antibody fragment or nucleic acid, which is therapeutically active or enhances the therapeutic activity when administered to a patient in combination with a compound of the present invention.

In one aspect, a compound of formula (I), or **(I-1), (I-2), (I-3), (I-4), (I-5), (I-6), (I-7), (I-8), (I-9), (I-10), (I-11), (I-12), (I-13), (I-14), (I-15), (I-16), (I-17), (I-18), (I-24), (I-25), (I-26), (I-27), (I-28), (I-29), (I-30), (I-31), (I-32), (I-33), (I-34), (I-34), (I-36), (I-37),** or **(I-38),** or a pharmaceutically acceptable salt, hydrate, solvate, stereoisomer, or tautomer thereof, can be combined with other therapeutic agents, such as other anti-cancer agents, anti-allergic agents, anti-nausea agents (or anti-emetics), pain relievers, cytoprotective agents, and combinations thereof.

In some embodiments, the compounds of the invention, or a pharmaceutically acceptable salt, hydrate, solvate, stereoisomer, or tautomer thereof of the present invention are administered in combination with one or more second agent(s) selected from a PD-1 inhibitor, a PD-L1 inhibitor, a LAG-3 inhibitor, a cytokine, an A2A antagonist, a GITR agonist, a TIM-3 inhibitor, a STING agonist, and a TLR7 agonist, to treat a disease, e.g., cancer.

In other embodiments, the compounds of the invention, or a pharmaceutically acceptable salt, hydrate, solvate, stereoisomer, or tautomer thereof, are used in combination with one or more other anti-HER2 antibodies, e.g., trastuzumab, pertuzumab, margetuximab, or HT-19 described above, or with other anti-HER2 conjugates, e.g., ado-trastuzumab emtansine (also known as Kadcyla^{®}, or T-DM1).

In other embodiments, the compounds of the invention, or a pharmaceutically acceptable salt, hydrate, solvate, stereoisomer, or tautomer thereof, are used in combination with one or more tyrosine kinase inhibitors, including but not limited to, EGFR inhibitors, Her3 inhibitors, IGFR inhibitors, and Met inhibitors, for treating a disease, e.g., cancer.

In another embodiment, the compounds of the invention are used in combination with one or more proliferation signaling pathway inhibitors, including but not limited to, MEK inhibitors, BRAF inhibitors, PI3K/Akt inhibitors, SHP2 inhibitors, and also mTOR inhibitors, and CDK inhibitors, for treating a disease, e.g., cancer.

In yet another embodiment, the compounds of the invention, or a pharmaceutically acceptable salt, hydrate, solvate, stereoisomer, or tautomer thereof, are used in combination with one or more pro-apoptotics, including but not limited to, IAP inhibitors, BCL2 inhibitors, MCL1 inhibitors, TRAIL agents, CHK inhibitors, for treating a disease, e.g., cancer.

In a further embodiment, the compounds of the invention, or a pharmaceutically acceptable salt, hydrate, solvate, stereoisomer, or tautomer thereof, are used in combination with one or more immunomodulators (*e.g.,* one or more of an activator of a costimulatory molecule or an inhibitor of an immune checkpoint molecule), for treating a disease, e.g., cancer..

In one embodiment, the combination disclosed herein is suitable for the treatment of cancer *in vivo.* For example, the combination can be used to inhibit the growth of cancerous tumors. The combination can also be used in combination with one or more of: a standard of care treatment (*e.g.,* for cancers or infectious disorders), a vaccine (*e.g.,* a therapeutic cancer vaccine), a cell therapy, a radiation therapy, surgery, or any other therapeutic agent or modality, to treat a disorder herein. For example, to achieve antigen-specific enhancement of immunity, the combination can be administered together with an antigen of interest.

### EXAMPLES

The invention is further illustrated by the following examples and synthesis schemes, which are not to be construed as limiting this invention in scope to the specific procedures herein described. It is to be understood that the examples are provided to illustrate certain embodiments and that no limitation to the scope of the invention is intended thereby.

Compounds of the present invention may be prepared by methods known in the art of organic synthesis. In all of the methods it is understood that protecting groups for sensitive or reactive groups may be employed where necessary in accordance with general principles of chemistry. Protecting groups are manipulated according to standard methods of organic synthesis (T.W. Green and P.G.M. Wuts (1999) Protective Groups in Organic Synthesis, 3rd edition, John Wiley & Sons). These groups are removed at a convenient stage of the compound synthesis using methods that are readily apparent to those skilled in the art.

### Analytical Methods, Materials, and Instrumentation

Unless otherwise noted, reagents and solvents were used as received from commercial suppliers. Proton nuclear magnetic resonance (NMR) spectra were obtained on either Bruker Avance spectrometer or Varian Oxford 400 MHz spectrometer unless otherwise noted. Spectra are given in ppm (δ) and coupling constants, *J*, are reported in Hertz. Tetramethylsilane (TMS) was used as an internal standard. Chemical shifts are reported in ppm relative to dimethyl sulfoxide (δ 2.50), methanol (δ 3.31), chloroform (δ 7.26) or other solvent as indicated in NMR spectral data. A small amount of the dry sample (2-5 mg) is dissolved in an appropriate deuterated solvent (1 mL). The chemical names were generated using ChemBioDraw Ultra v12 or v17 from Cambridge Soft.

Mass spectra (ESI-MS) were collected using a Waters System (Acquity UPLC and a Micromass ZQ mass spectrometer) or Agilent-1260 Infinity (6120 Quadrupole); all masses reported are the m/z of the protonated parent ions unless recorded otherwise. The sample was dissolved in a suitable solvent such as MeCN, DMSO, or MeOH and was injected directly into the column using an automated sample handler. The analysis is performed on Waters Acquity UPLC system (Column: Waters Acquity UPLC BEH C18 1.7µm, 2.1 x 30mm; Flow rate: 1 mL/min; 55°C (column temperature); Solvent A: 0.05% formic acid in water, Solvent B: 0.04% formic acid in MeOH; gradient 95% Solvent A from 0 to 0.10 min; 95% Solvent A to 20% Solvent A from 0.10 to 0.50 min; 20% Solvent A to 5% Solvent A from 0.50 to 0.60 min; hold at 5% Solvent A from 0.6 min to 0.8 min; 5% Solvent A to 95% Solvent A from 0.80 to 0.90 min; and hold 95% Solvent A from 0.90 to 1.15 min.

### Abbreviations used in the following examples and elsewhere herein are:

- AC₅₀: half maximal active concentration
- aq.: aqueous
- br: broad
- Cs₂CO₃: cesium carbonate
- cat.: catalyst
- d: doublet
- DAST: Diethylaminosulfur trifluoride
- di-tBu-bipy: 4,4'-di-*tert*-butyl-2,2'-dipyridyl
- dd: doublet of doublets
- ddd: doublet of doublet of doublets
- ddq: doublet of doublet of quartets
- ddt: doublet of doublet of triplets
- dq: doublet of quartets
- dt: doublet of triplets
- dtd: doublet of triplet of doublets
- DCM: dichloromethane
- DMA: *N,N*-dimethylacetamide
- DME: 1,2-Dimethoxyethane
- DMF: *N,N*-dimethylformamide
- DMSO: dimethylsulfoxide
- dppf: 1,1'-Bis(diphenylphosphino)ferrocene
- EC₅₀: half maximal effective concentration
- Et₂O: diethyl ether
- Et₃N: triethylamine
- EtOAc: ethyl acetate
- HCl: hydrogen chloride
- hept: heptet
- HPLC: high performance liquid chromatography
- h or hr: hour
- HRMS: high resolution mass spectrometry
- g: gram
- IC₅₀: half maximal inhibitory concentration
- K₂CO₃: potassium carbonate
- KI: potassium iodide
- K₃PO₄: tripotassium phosphate
- LCMS: liquid chromatography mass spectrometry
- LiHMDS: Lithium bis(trimethylsilyl)amide
- m: multiplet
- MeCN: acetonitrile
- MeOH: methanol
- mg: milligram
- MgCl₂: magnesium chloride
- MHz: megahertz
- min: minutes
- mL: milliliter
- mmol: millimole
- M: molar
- MS: mass spectrometry
- NaBH(OAc)₃: sodium triacetoxyborohydride
- NaHCO₃: sodium bicarbonate
- Na₂SO₄: sodium sulfate
- NiBr₂·DME: nickel (II) bromide ethylene glycol dimethyl ether complex
- NiI₂: nickel (II) iodide
- NMR: Nuclear magnetic resonance
- Pd(dppf)Cl₂•DCM: [1,1' -Bis(diphenylphosphino)ferrocene]dichloropalladium(II), complex with dichloromethane
- PtO₂: platinum (IV) oxide
- q: quartet
- qd: quartet of doublets
- quint: quintet
- quintd: quintet of doublets
- rt: room temperature
- Rt: retention time
- s: singlet
- t: triplet
- TEA: triethylamine
- td: triplet of doublets
- tdd: triplet of doublet of doublets
- THF: tetrahydrofuran
- Ts: tosyl
- tt: triplet of triplets
- ttd: triplet of triplet of doublets
- TLC: thin-layer chromatography
- UPLC: ultra-Performance Liquid Chromatography
- XPhos Pd G2: chloro(2-dicyclohexylphosphino-2',4',6'-triisopropyl-1,1'-biphenyl)[2-(2'-amino-1,1'-biphenyl)]palladium(II)

### Example 1: 3-(1-oxo-5-(piperidin-4-yl)isoindolin-2-yl)piperidine-2,6-dione (INT-G)

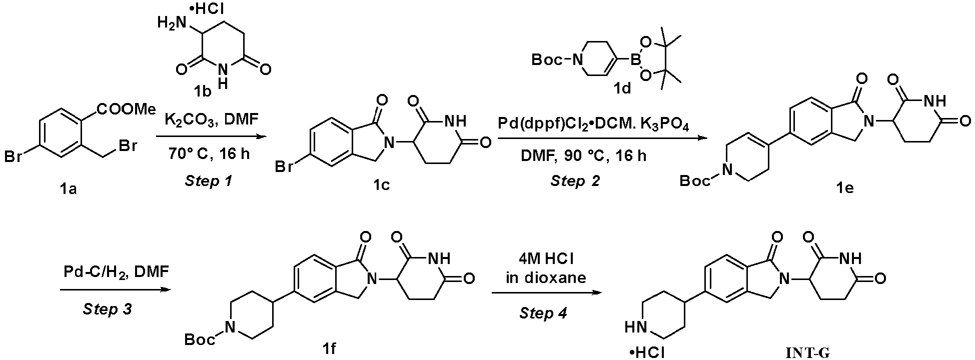

### Step 1. 3-(5-bromo-1-oxoisoindolin-2-yl)piperidine-2,6-dione (1c)

Intermediate **1a** was prepared as reported in U.S. Patent Application US 2009/0142297.

To a stirred solution of methyl 4-bromo-2-(bromomethyl)benzoate **(1a,** 15 g, 48.7 mmol) in DMF (150 mL) was added 3-aminopiperidine-2,6-dioneHCl salt **(1b,** 6.9 g, 53.6 mmol) and K₂CO₃ (20.2 g, 146.1 mmol). The resulting mixture was stirred at 70 °C for 16 h after which time the reaction mixture was cooled to rt and then concentrated to dryness. To the resulting residue, water was added and the mixture stirred at rt for 30 min. The resultant solid was filtered and washed with ether and ethyl acetate. The solid was dried under vacuum filtration to afford **1c** (10.6 g, 32.9 mmol, 67% yield). MS [M+H]⁺ = 323.0. ¹H NMR (400 MHz, DMSO-*d₆*) δ 10.99 (s, 1H), 7.91-7.88 (m, 1H), 7.72 (dd, *J* = 8.1, 1.6 Hz, 1H), 7.67 (d, *J* = 8.0 Hz, 1H), 5.11 (dd, *J* = 13.3, 5.1 Hz, 1H), 4.47 (d, *J* = 17.7 Hz, 1H), 4.34 (d, *J* = 17.7 Hz, 1H), 2.98-2.83 (m, 1H), 2.65-2.55 (m, 1H), 2.45-2.29 (m, 1H), 2.01 (dtd, *J =* 12.7, 5.3, 2.3 Hz, 1H).

### Step 2. tert-Butyl-4-(2-(2,6-dioxopiperidin-3-yl)-1-oxoisoindolin-5-yl)-3,6-dihydropyridine-1(2H)-carboxylate (1e)

A solution of **1c** (1.80 g, 5.60 mmol) in DMF (10 mL) in a sealed tube was purged with argon for 5 min prior to addition of 3,6-dihydro-2H-pyridine-1-*tert*-butoxycarbonyl-4-boronic acid pinacol ester **(1d,** 2.2 g, 7.2 mmol), K₃PO₄ (1.42 g, 6.7 mmol) and Pd(dppf)Cl₂•DCM (227 mg, 0.28 mmol). The reaction mixture was again purged with argon for 5 min and then stirred at 90 °C for 16 h. After this time the reaction mixture was cooled to rt and then concentrated under reduced pressure. Water was added to the residue, which was then extracted with EtOAc. The organic layer was washed with brine, dried over Na₂SO₄, filtered, and then concentrated under a reduced pressure. The crude compound was purified by silica gel chromatography, eluting with 70-80% of EtOAc in hexanes, to afford **1e** as a light brown solid (1.0 g, 2.4 mmol, 42% yield). MS [M+H]⁺ = 426.3.

### Step 3. tert-Butyl 4-(2-(2,6-dioxopiperidin-3-yl)-1-oxoisoindolin-5-yl)piperidine-1-carboxylate (1f):

To a stirred solution of **1e** (1.0 g, 2.35 mmol) in DMF (20 mL) was added 10% Pd/C (150 mg) and the mixture was stirred under a hydrogen atmosphere (balloon) at rt for 6 h. The reaction mixture was then filtered through a bed of Celite^{®} filter aid. The filtrate was concentrated under reduced pressure affording **1f** as an off-white solid (0.85 g, 1.97 mmol, 84% yield). MS [M-tBu+H]⁺ = 372.3. ¹H NMR (400 MHz, CDCl₃) δ 8.40 (s, 1H), 7.81 (d, *J* = 7.9 Hz, 1H), 7.32 (d, *J =* 8.0 Hz, 1H), 7.29 (s, 1H), 5.22 (dd, *J =* 13.3, 5.1 Hz, 1H), 4.46 (d, *J* = 16.0 Hz, 1H), 4.31 (d, *J =* 16.1 Hz, 1H), 4.27 (d, *J =* 16.2 Hz, 2H), 2.97-2.67 (m, 5H), 2.41-2.26 (m, 1H), 2.23-2.13 (m, 1H), 1.83 (d, *J =* 12.6 Hz, 2H), 1.71-1.55 (m, 2H), 1.48 (s, 9H).

### Step 4. 3-(1-oxo-5-(piperidin-4-yl)isoindolin-2-yl)piperidine-2,6-dione hydrochloride (INT-G):

To a stirred solution of **1f** (0.85 g, 2.0 mmol) in dioxane (10 mL) was added 4M HCl in dioxane (5.0 mL). The reaction mixture was then stirred at rt for 2 h. The reaction mixture was concentrated under reduced pressure to afford the HCl salt of desired compound **INT-G** as an off-white solid (0.65 g, 1.8 mmol, 90% yield, hydrochloride salt). MS [M+H]⁺ = 328.3. ¹H NMR (400 MHz, DMSO-*d₆*): δ 10.99 (s, 1H), 9.28 (s, 1H), 7.70 (d, *J =* 7.8 Hz, 1H), 7.46 (s, 1H), 7.39 (d, *J* = 8.0 Hz, 1H), 5.74 (s, 1H), 5.11 (dd, *J* = 13.3, 5.2 Hz, 1H), 4.46 (d, *J* = 17.3 Hz, 1H), 4.32 (d, *J =* 17.3 Hz, 1H), 3.36 (d, *J =* 11.5 Hz, 2H), 3.10-2.86 (m, 4H), 2.61 (d, *J =* 14.8 Hz, 1H), 2.39 (qd, *J =* 13.2, 4.3 Hz, 1H), 2.14-1.79 (m, 5H).

### Conversion of 1c to 1f was also achieved in a single step via Negishi coupling using the following procedure:

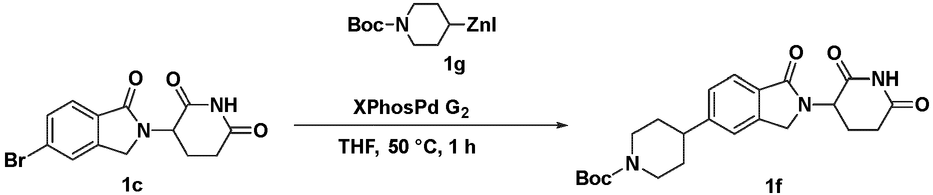

### 1-(tert-butoxycarbonyl)piperidin-4-yl)zinc(II) iodide (1g) was prepared as reported in Corley, E. G., et al., J Org. Chem. 2004, 69, 5120.

A mixture of **1c** (41 mg, 0.125 mmol) and XPhos Pd cycle G2 (15 mg, 0.019 mmol) in THF (1.5 mL) was purged with nitrogen prior to addition of (1-(*tert*-butoxycarbonyl)piperidin-4-yl)zinc(II) iodide (**1g**, 142 mg, 0.376 mmol) in THF (0.7 mL). The resulting mixture was stirred at 50 °C for 1 h after which time the reaction was cooled to rt, quenched with brine, and extracted with EtOAc. The organic layer was passed through a phase separator and concentrated. The crude material was purified by silica gel chromatography (eluting with 0-100% EtOAc in heptane) to afford **1f** as a white solid (30 mg, 0.070 mmol, 56% yield).

### Conversion of 1c to 1f was also achieved in a single step via an alternative reductive cross-coupling procedure:

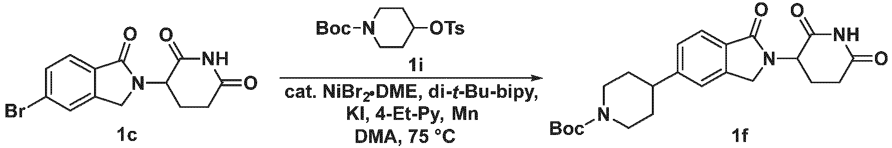

To crude **1c** (84% pure, 34 mg, 0.088 mmol), *tert-*butyl 4-(tosyloxy)piperidine-1-carboxylate **(1i,** 38 mg, 0.11 mmol), NiBr₂·DME (2.7 mg, 8.8 µmol), di-t-Bu-bipy (2.4 mg, 8.8 µmol), KI (15 mg, 0.09 mmol) and manganese powder (10 mg, 0.18 mmol) in DMA (0.50 mL) was added 4-ethylpyridine (10 µL, 0.088 mmol) and the reaction mixture was stirred vigorously at 75 °C for 5 h. The reaction mixture was filtered through a short pad of Celite^{®} filter aid and eluted with MeCN. The obtained solution was concentrated by azeotroping with heptane. The crude product was purified via chromatography on silica gel eluting with MeOH in DCM to afford **1f** (21.7 mg, 0.051 mmol, 57% yield) as a white solid.

### In a similar fashion, intermediate 1f could be obtained from intermediate 29d, (the route for synthesis of 29d is outlined in Example 29):

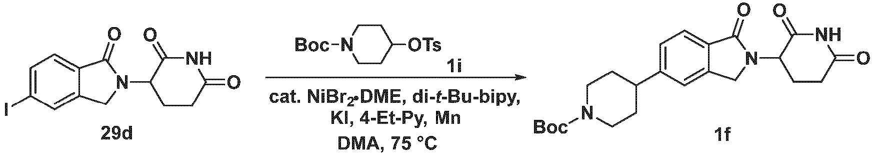

To 29d (48 mg, 0.13 mmol), *tert-*butyl 4-(tosyloxy)piperidine-1-carboxylate **(1i,** 55 mg, 0.16 mmol), NiBr₂•DME (4.0 mg, 0.013 mmol), di-t-Bu-bipy (3.5 mg, 0.013 mmol), KI (22 mg, 0.13 mmol) and manganese powder (14 mg, 0.26 mmol) in DMA (0.67 mL) was added 4-ethylpyridine (0.015 mL, 0.14 mmol) and the reaction mixture was stirred vigorously at 75 °C for 5 h. The reaction mixture was filtered through a short pad of Celite^{®} filter aid and eluted with MeCN. The obtained solution was concentrated by azeotroping with heptane. The crude product was purified via chromatography on silica gel eluting with MeOH in DCM to afford **1f** (33.3 mg, 0.078 mmol, 60% yield) as a white solid.

### Example 2: 3-(1-oxo-5-(1-(1-(tetrahydro-2H-pyran-4-yl)ethyl)piperidin-4-yl)isoindolin-2-yl)piperidine-2,6-dione (I-30)

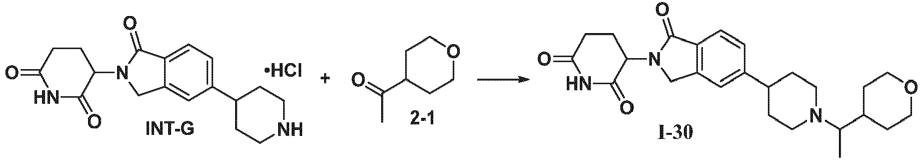

To a stirred solution of **INT-G** (200 mg, 0.55 mmol) and 1-(tetrahydro-2H-pyran-4-yl)ethan-1-one (2-1, 210 mg, 1.64 mmol) in DMSO (10 mL), was added titanium isopropoxide (310 mg, 1.09 mmol). The resulting mixture was stirred at rt for 16 h and then NaBH₃(CN) (69 mg, 2.0 mmol) was added and stirring was continued at rt for an additional 16 h. Upon complete consumption of the starting materials, the reaction mixture was quenched with aq. NaHCO₃ and extracted with EtOAc (2 x 50 mL). The combined organic extracts were washed with brine, dried over Na₂SO₄, filtered, and concentrated under reduced pressure. The obtained crude material was purified by silica gel column chromatography eluting with 7% MeOH in DCM to afford **I-30** as an off-white solid (110 mg, 0.25 mmol, 46% yield). MS [M+H]⁺ = 440.1. ¹H NMR (300 MHz, DMSO-*d₆*): δ 10.99 (s, 1H), 7.63 (d, *J* = 7.2 Hz, 1H), 7.48 (s, 1H), 7.38 *(d, J =* 7.8 Hz, 1H), 5.08 (dd, *J =* 13.2, 4.8 Hz, 1H), 4.34 (d, *J=* 17.2 Hz, 1H), 4.19 (d, *J =* 17.2 Hz, 1H), 3.86-3.83 (m, 2H), 3.45-3.25 (m, 2H), 2.98-2.85 (m, 1H), 2.78-2.55 (m, 5H), 2.45-2.15 (m, 3H), 2.08-1.82 (m, 2H), 1.72-1.51 (m, 6H), 1.18-1.04 (m, 2H), 0.89 (d*, J =* 6.9 Hz, 3H).

### Example 3: 3-(5-(1-(1-(1-ethyl-1H-pyrazol-4-yl)ethyl)piperidin-4-yl)-1-oxoisoindolin-2-yl)piperidine-2,6-dione (I-31)

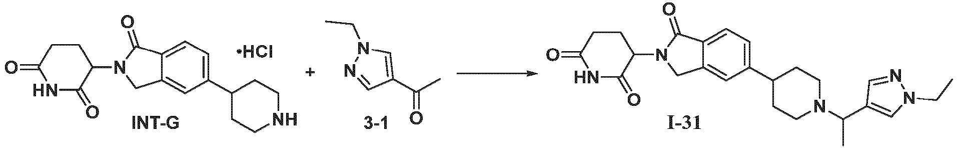

To a stirred solution of **INT-G** (400 mg, 0.09 mmol) and **3-1** (303 mg, 2.20 mmol) in DMSO (20 mL), was added titanium isopropoxide (1.24 g, 4.40 mmol). The resulting mixture was stirred at rt for 16 h and then NaBH₃(CN) (138 mg, 2.20 mmol) was added and stirring was continued at rt for an additional 16 h. Upon complete consumption of the starting materials, the reaction mixture was quenched with aq. NaHCO₃ and extracted with EtOAc (2 x 75 mL). The combined organic extracts were washed with brine, dried over Na₂SO₄, filtered, and concentrated under reduced pressure. The obtained crude material was purified by silica gel column chromatography eluting with 7% MeOH in DCM to afford **I-31** as an off-white solid (170 mg, 0.38 mmol, 34% yield). MS [M+H]⁺ = 450.5. ¹H NMR (400 MHz, DMSO-*d₆*): δ 10.99 (s, 1H), 7.63 (d, *J =* 7.6 Hz, 1H), 7.58 (br s, 1H), 7.46 (s, 1H), 7.36 (d, *J =* 8.0 HZ, 1H), 7.32 (br s, 1H), 5.09 (dd, *J =* 13.2, 4.8 Hz, 1H), 4.42 (d, *J =* 17.2 Hz, 1H), 4.27 (d, *J =* 17.2 Hz, 1H), 4.09-4.06 (m, 2H), 3.70 (br s, 1H), 2.90-267 (m, 3H), 2.60-2.32 (m, 4H), 1.99-1.96 (m, 3H), 1.79-1.62 (m, 3H), 1.37-1.23 (m, 6H).

### Example 4: 3-(5-(1-(1-(1-ethyl-1H-pyrazol-4-yl)propyl)piperidin-4-yl)-1-oxoisoindolin-2-yl)piperidine-2,6-dione (I-32)

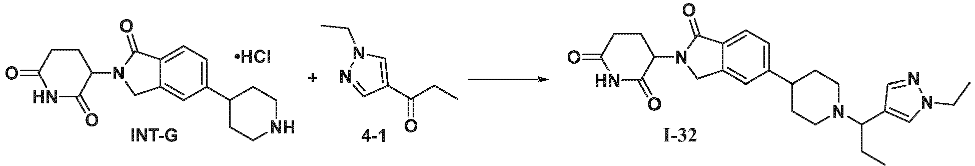

To a stirred solution of **INT-G** (400 mg, 0.09 mmol) and **4-1** (333 mg, 2.19 mmol) in DMSO (10 mL), was added titanium isopropoxide (993 mg, 3.29 mmol). The resulting mixture was stirred at rt for 16 h and then NaBH₃(CN) (138 mg, 2.20 mmol) was added and stirring was continued at rt for an additional 16 h. Upon complete consumption of the starting materials, the reaction mixture was quenched with aq. NaHCO₃ and extracted with EtOAc (2 x 75 mL). The combined organic extracts were washed with brine, dried over Na₂SO₄, filtered, and concentrated under reduced pressure. The obtained crude material was purified by silica gel column chromatography eluting with 7% MeOH in DCM to afford **I-32** as an off-white solid (170 mg, 0.36 mmol, 34% yield). MS [M+H]⁺ = 464.3. ¹H NMR (400 MHz, DMSO-*d₆*): δ 10.75 (s, 1H), 7.65-7.62 (m, 2H), 7.44 (s, 1H), 7.37-7.35 (m, 2H), 5.06 (dd, *J =* 13.2, 4.8 Hz, 1H), 4.42 (d, *J =* 17.2 Hz, 1H), 4.32 (d, *J =* 17.2 Hz, 1H), 4.15-4.10 (m, 2H), 3.68-3.85 (m, 1H), 3.20-3.19 (m, 3H), 2.93-2.84 (m, 2H), 2.67-2.09 (m, 4H), 2.04-1.82 (m, 2H), 1.90-1.65 (m, 4H), 1.39 (t, *J* = 7.6 Hz, 3H), 0.86 (t, *J* = 7.2 Hz, 3H).

### Example 5: 3-(1-oxo-5-(1-(1-(pyrazin-2-yl)propyl)piperidin-4-yl)isoindolin-2-yl)piperidine-2,6-dione (I-33)

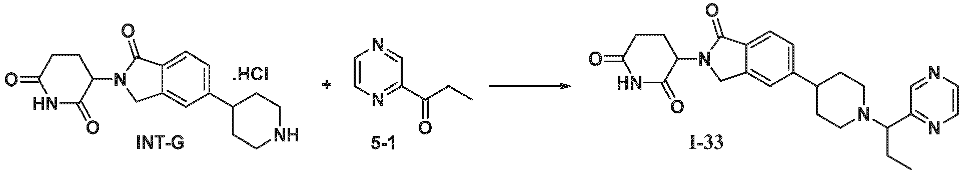

To a stirred solution of **INT-G** (400 mg, 0.09 mmol) and **5-1** (298 mg, 1.34 mmol) in DMSO (15 mL), titanium isopropoxide (1.24 g, 4.38 mmol) was added. The resulting mixture was stirred at rt for 16 h and then NaBH₃(CN) (137 mg, 2.20 mmol) was added and stirring was continued at rt for an additional 16 h. Upon complete consumption of the starting materials, the reaction mixture was quenched with aq. NaHCO₃ and extracted with EtOAc (2 x 75 mL). The combined organic extracts were washed with brine, dried over Na₂SO₄, filtered, and concentrated under reduced pressure. The obtained crude material was purified by silica gel column chromatography eluting with 7% MeOH in DCM to afford **I-33** as an off-white solid (100 mg, 0.22 mmol, 20% yield). MS [M+H]⁺ = 448.1. ¹H NMR (400 MHz, DMSO-*d₆*): δ 10.98 (s, 1H), 8.65-8.63 (m, 2H), 8.54 (d, *J =* 2.4 Hz, 1H), 7.61 (d, *J* = 7.6 Hz, 1H), 7.45 (s, 1H), 7.35 *(d, J=* 8.4 Hz, 1H), 5.09 (dd, *J=* 13.2, 4.8 Hz, 1H), 4.40 *(d, J=* 17.2 Hz, 1H), 4.26 (d, *J =* 17.2 Hz, 1H), 3.69-3.65 (m, 1H), 3.06-2.87 (m, 3H), 3.60-2.35 (m, 4H), 2.08-1.92 (m, 5H), 1.73-1.61 (m, 3H), 0.78 (t, *J=* 7.6 Hz, 3H).

### Example 6: 3-(5-(1-(6,7-dihydro-SH-cyclopenta[b]pyridin-5-yl)piperidin-4-yl)-1-oxoisoindolin-2-yl)piperidine-2,6-dione (I-35)

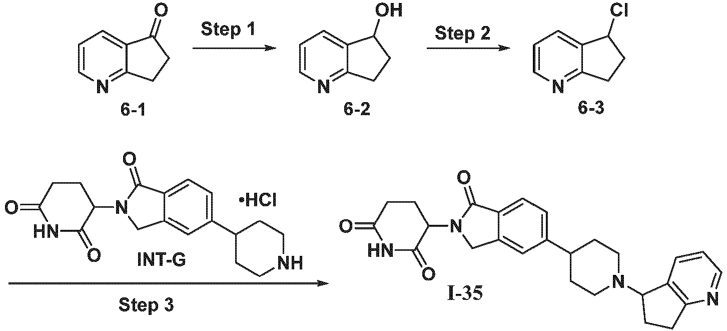

### Step 1. 6,7-dihydro-5H-cyclopenta[b]pyridin-5-ol (6-2)

To a solution of **6-1** (1.0 g, 7.5 mmol) in MeOH (20 mL) was added NaBH₄ (541 mg, 14.3 mmol) in small portions at 0 °C and the resulting mixture was stirred at rt for 2 h. Upon complete consumption of the starting materials, the reaction mixture was quenched with water and extracted with 10% MeOH in DCM (3 x 75 mL). The combined organic extracts were dried over Na₂SO₄, filtered, and concentrated under reduced pressure. The obtained crude material was purified by silica gel column chromatography eluting with 7% MeOH in DCM to afford compound **6-2** (750 mg, 5.55 mmol, 74% yield). ¹H NMR (400 MHz, CDCl₃): δ 8.33-8.31 (m, 1H), 7.69-7.66 (m, 1H), 7.11-7.07 (m, 1H), 5.27-5.21 (m, 1H), 3.05-2.98 (m, 1H), 2.86-2.81 (m, 1H), 2.51-2.48 (m, 1H), 1.97-1.93 (m, 1H).

### Step 2. 5-chloro-6,7-dihydro-5H-cyclopenta[b]pyridine (6-3)

To a solution of **6-2** (750 mg, 5.55 mmol) in DCM (30 mL) was added SOCl₂ (5 mL) drop wise at 0 °C and the resulting mixture was stirred at 50 °C for 4 h. Upon complete consumption of the starting materials, the reaction mixture was cooled to rt, diluted with DCM (20 mL), and washed with aq. NaHCO₃ (2 x 50 mL). The combined organic extracts were dried over Na₂SO₄, filtered, and concentrated under reduced pressure to afford crude 6-3 (750 mg), which was used in the next step without further purification. MS [M+H]⁺ = 153.95.

### Step 3. 3-(5-(1-(6,7-dihydro-SH-cyclopenta[b]pyridin-5-yl)piperidin-4-yl)-1-oxoisoindolin-2-yl)piperidine-2,6-dione (I-35)

To a solution of **INT-G** (500 mg, 1.52 mmol) in DMF (10 mL) was added Et₃N (0.64 mL, 4.6 mmol) followed by crude **6-3** (350 mg) in DCM (2 mL) at 0 °C and the resulting mixture was stirred at 80 °C for 16 h. Upon complete consumption of the starting materials, the reaction mixture was quenched with water and extracted with DCM (3 x 75 mL). The combined organic extracts were washed with brine, dried over Na₂SO₄, filtered, and concentrated under reduced pressure. The obtained crude material was purified by silica gel column chromatography eluting with 8% MeOH in DCM to afford **I-35** as brown colored solid (200 mg, 0.45 mmol, 30% yield). MS [M+H]⁺ = 445.2. ¹H NMR (400 MHz, DMSO-*d₆*): δ 10.75 (s, 1H), 10.05 br s, 1H), 8.48 (br s, 1H), 7.66 (d, *J =* 7.6 Hz, 1H), 7.46 (s, 1H), 7.39 (d, *J =* 7.6 Hz, 1H), 7.26 (br s, 1H), 5.05 (dd, *J =* 13.2, 4.8 Hz, 1H), 4.44 *(d, J=* 17.2 Hz, 1H), 4.33 (d, *J =* 17.2 Hz, 1H), 3.1-3.02 (m, 5H), 2.94-2.85 (m, 3H), 2.67-2.34 (m, 2H), 2.05-2.00 (m, 3H), 1.91-1.21 (m, 5H).

### Example 7: 3-(5-(1-(4-methoxycyclohexyl)piperidin-4-yl)-1-oxoisoindolin-2-yl)piperidine-2,6-dione (I-36)

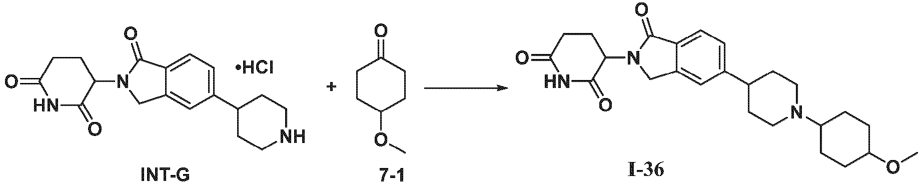

To a stirred solution of **INT-G** (450 mg, 1.23 mmol) and **7-1** (517 mg, 3.69 mmol) in DMSO (15 mL) was added titanium isopropoxide (698 mg, 2.46 mmol) was added. The resulting mixture was stirred at rt for 16 h and then NaBH(OAc)₃ (1.3 g, 6.15 mmol) was added and stirring was continued at rt for an additional 16 h. Upon complete consumption of the starting materials, the reaction mixture was quenched with aq. NaHCO₃ and extracted with EtOAc (2 x 75 mL). The combined organic extracts were washed with brine, dried over Na₂SO₄, filtered, and concentrated under reduced pressure. The obtained crude material was purified by silica gel column chromatography eluting with 7% MeOH in DCM to afford **I-36** as an off-white solid (100 mg, 0.23 mmol, 19% yield). MS [M+H]⁺ = 440.3. ¹H NMR (400 MHz, DMSO-*d₆*): δ 10.99 (s, 1H), 7.63 (d, *J =* 8.0 Hz, 1H), 7.48 (s, 1H), 7.39 (d, *J =* 7.6 Hz, 1H), 5.09 (dd, *J =* 13.2, 4.8 Hz, 1H), 4.42 (d, *J=* 17.2 Hz, 1H), 4.27 (d, *J =* 17.2 Hz, 1H), 3.20 (s, 3H), 2.94-2.88 (m, 4H), 2.67-2.37 (m, 6H), 2.05-1.72 (m, 6H), 1.68-1.23 (m, 7H).

### Example 8: 3-(5-(1-(((1r,4r)-4-(fluoromethyl)cyclohexyl)methyl)piperidin-4-yl)-1-oxoisoindolin-2-yl)piperidine-2,6-dione trifluoroacetate (I-10) and 3-(5-(1-(((S)-4-methylcyclohex-3-en-1-yl)methyl)piperidin-4-yl)-1-oxoisoindolin-2-yl)piperidine-2,6-dione trifluoroacetate (I-14)

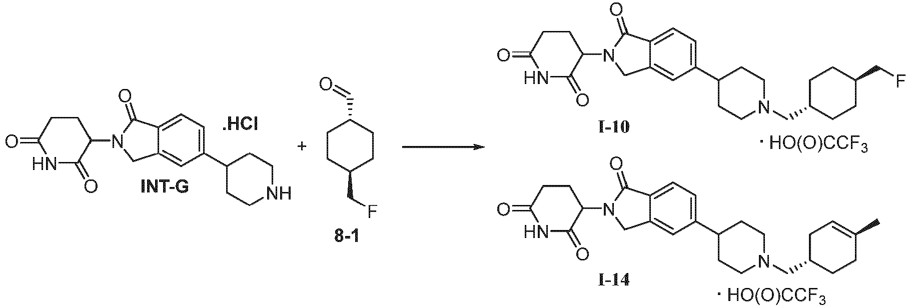

To a solution of **INT-G** (150 mg, 0.41 mmol) and **8-1** (89 mg, 0.62 mmol) in DMF (7.5 mL), was added NaBH(OAc)₃ (261 mg, 1.23 mmol) at 0 °C and the resulting mixture was stirred at rt for 16 h. Upon complete consumption of the starting materials, the reaction mixture was quenched with ice-cold water and extracted with EtOAc (2 x 50 mL). The combined organic extracts were washed with brine, dried over Na₂SO₄ and concentrated under reduced pressure. The obtained crude material was purified by silica gel column chromatography eluting with 5% MeOH in DCM. The obtained material was further purified by prep HPLC (ZORBAX XDB C18, (21.2mm x 150mm), Mobile phase: 0.1% TFA in H₂O (A): MeCN (B), Flow: 20 mL/min, Time/%B: 0/20, 2/30, 9/40) to afford **I-10** as an off-white solid (5 mg, 0.01 mmol, 6% yield, TFA salt) and **I-14** as an off-white solid (22 mg, 0.05 mmol, 23% yield, TFA salt). **I-10:** MS [M+H]⁺ = 436.4. ¹H NMR (400 MHz, DMSO-*d₆*): δ 10.98 (s, 1H), 7.68 (d, *J* = 7.6 Hz, 1H), 7.44 (s, 1H), 7.36 (d, *J* = 7.6 Hz, 1H), 5.08 (dd, *J =* 13.2, 4.8 Hz, 1H), 4.44-4.40 (m, 2H), 4.30-4.26 (m, 2H), 4.18-4.16 (m, 1H), 3.59-3.56 (m, 2H), 3.16-2.85 (m, 6H), 2.64-2.46 (m, 2H), 1.98-1.60 (m, 10H), 1.02-0.96 (m, 4H). **I-14:** MS [M+H]⁺ = 456.4. ¹H NMR (400 MHz, DMSO-*d₆*): δ 10.99 (s, 1H), 7.63 (d, *J =* 7.6 Hz, 1H), 7.49 (s, 1H), 7.39 (d, *J =* 8.0 Hz, 1H), 5.34 (br s, 1H), 5.08 (dd, *J =* 13.2, 4.8 Hz, 1H), 4.42 (d, *J=* 17.2 Hz, 1H), 4.28 (d, *J* = 17.2 Hz, 1H), 2.95-2.91 (m, 3H), 2.61-2.18 (m, 5H), 1.99-1.71 (m, 13H), 1.60 (s, 3H), 1.21-1.19 (m, 1H).

### Example 9: 3-(5-(1-((4-methylpyrimidin-5-yl)methyl)piperidin-4-yl)-1-oxoisoindolin-2-yl)piperidine-2,6-dione (I-12)

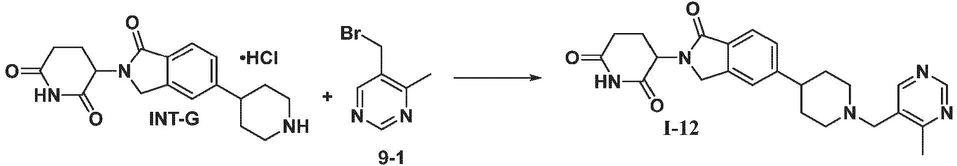

To a stirred solution of **INT-G** 150 mg, 0.41 mmol) and Et₃N (0.18 mL, 2.06 mmol) in DMF (5 mL), was added 9-1 (85 mg, 0.45 mmol) in DMF (5 mL) was added at 0 °C and the resulting mixture was stirred at rt for 16 h. Upon complete consumption of the starting materials, the reaction mixture was quenched with ice-cold water and extracted with EtOAc (2 x 50 mL). The combined organic extracts were washed with brine, dried over Na₂SO₄, filtered, and concentrated under reduced pressure. The obtained crude material was purified by silica gel column chromatography eluting with 8% MeOH in DCM to afford **I-12** as an off-white solid (75 mg, 0.17 mmol, 42% yield). MS [M+H]⁺ = 434.4. ¹H NMR (400 MHz, DMSO-*d₆*): δ 10.98 (s, 1H), 8.93 (s, 1H), 8.55 (s, 1H), 7.63 (d, *J =* 7.6 Hz, 1H), 7.50 (s, 1H), 7.40 (d, *J =* 8.0 Hz, 1H), 5.09 (dd, *J =* 13.2, 4.8 Hz, 1H), 4.42 (d, *J =* 17.2 Hz, 1H), 4.27 (d, *J =* 17.2 Hz, 1H), 3.53 (s, 2H), 2.94-2.87 (m, 3H), 2.69-2.32 (m, 6H), 2.19-2.13 (m, 2H), 2.00-1.96 (m, 1H), 1.80-1.66 (m, 4H).

### Example 10: 3-(1-oxo-5-(1-(pyridazin-3-ylmethyl)piperidin-4-yl)isoindolin-2-yl)piperidine-2,6-dione (I-15)

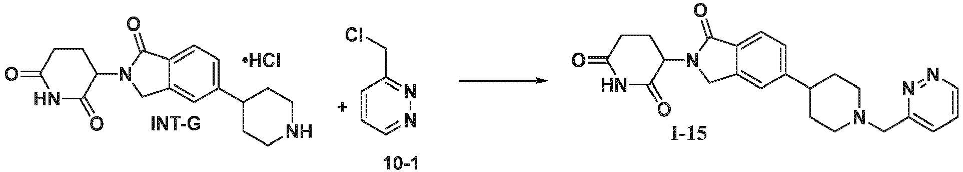

To a stirred solution of **INT-G** (250 mg, 0.68 mmol) and Et₃N (0.18 mL, 2.06 mmol) in DMF (7 mL) was added **10-1** (106 mg, 0.82 mmol), dissolved in DMF (5 mL), at 0 °C and the resulting mixture was stirred at rt for 16 h. Upon complete consumption of the starting materials, the reaction mixture was quenched with ice-cold water and extracted with EtOAc (2 x 75 mL). The combined organic extracts were washed with brine, dried over Na₂SO₄, filtered, and concentrated under reduced pressure. The obtained crude material was purified by silica gel column chromatography eluting with 10% MeOH in DCM to afford **I-15** as pale brown solid (110 mg, 0.26 mmol, 36% yield). MS [M+H]⁺ = 420.1. ¹H NMR (400 MHz, DMSO-*d₆*): δ 10.99 (s, 1H), 9.15-9.13 (m, 1H), 7.76-7.63 (m, 3H), 7.49 (s, 1H), 7.40 (d, *J=* 8.0 Hz, 1H), 5.10 (dd, *J =* 13.2, 4.8 Hz, 1H), 4.42 (d, *J =* 17.2 Hz, 1H), 4.28 *(d, J =* 17.2 Hz, 1H), 3.84 (s, 2H), 2.93-2.90 (m, 3H), 2.67-2.32 (m, 3H), 2.24-2.18 (m, 2H), 2.01-1.98 (m, 1H), 1.71-1.65 (m, 4H).

### Example 11: 3-(1-oxo-5-(1-(pyrazin-2-ylmethyl)piperidin-4-yl)isoindolin-2-yl)piperidine-2,6-dione (I-14)

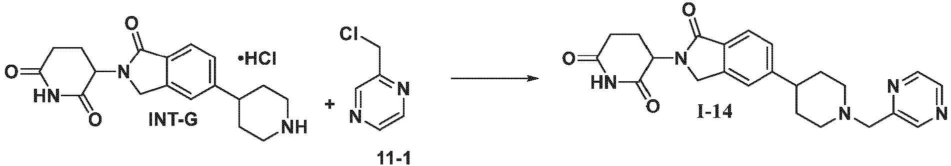

To a stirred solution of **INT-G** (250 mg, 0.68 mmol) and Et₃N (0.18 mL, 2.06 mmol) in DMF (7 mL) was added **11-1** (140 mg, 0.82 mmol), dissolved in DMF (5 mL), at 0 °C and the resulting mixture was stirred at rt for 16 h. Upon complete consumption of the starting materials, the reaction mixture was quenched with ice-cold water and extracted with EtOAc (2 x 75 mL). The combined organic extracts were washed with brine, dried over Na₂SO₄, filtered, and concentrated under reduced pressure. The obtained crude material was purified by silica gel column chromatography eluting with 10% MeOH in DCM to afford **I-14** as an off-white solid (120 mg, 0.28 mmol, 42% yield). MS [M+H]⁺ = 420.3. ¹H NMR (400 MHz, DMSO-*d₆*): δ 10.99 (s, 1H), 8.72 (d, *J =* 1.2 Hz, 1H), 8.60-8.58 (m, 1H), 8.54 (d, *J =* 2.4 Hz, 1H), 7.64 (d, *J =* 8.0 Hz, 1H), 7.50 (s, 1H), 7.41 (d, *J =* 8.4 Hz, 1H), 5.10 (dd, *J =* 13.2, 4.8 Hz, 1H), 4.41 (d, *J =* 17.2 Hz, 1H), 4.28 (d, *J =* 17.2 Hz, 1H), 3.71 (s, 2H), 2.98-2.92 (m, 3H), 2.67-2.32 (m, 3H), 2.21-2.19 (m, 2H), 2.01-1.98 (m, 1H), 1.78-1.75 (m, 4H).

### Example 12: 3-(1-oxo-5-(1-(pyrimidin-4-ylmethyl)piperidin-4-yl)isoindolin-2-yl)piperidine-2,6-dione (I-16)

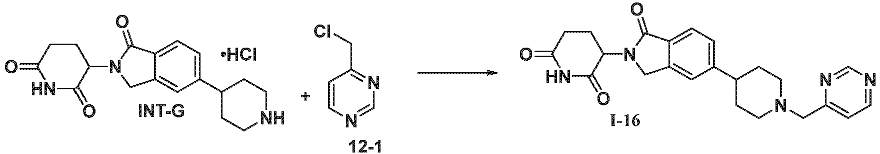

To a stirred solution of **INT-G** (250 mg, 0.68 mmol) and Et₃N (0.18 mL, 2.06 mmol) in DMF (7 mL) was added **12-1** (89 mg, 0.68 mmol), dissolved in DMF (5 mL), at 0 °C and the resulting mixture was stirred at rt for 16 h. Upon complete consumption of the starting materials, the reaction mixture was quenched with ice-cold water and extracted with EtOAc (2 x 75 mL). The combined organic extracts were washed with brine, dried over Na₂SO₄, filtered, and concentrated under reduced pressure. The obtained crude material was purified by silica gel column chromatography eluting with 8% MeOH in DCM to afford **I-16** as an off-white solid (150 mg, 0.36 mmol, 52% yield). MS [M+H]⁺ = 420.1. ¹H NMR (400 MHz, DMSO-*d₆*): δ 10.99 (s, 1H), 9.10 (d, *J =* 1.2 Hz, 1H), 8.77-8.76 (m, 1H), 7.66-7.60 (m, 2H), 7.51 (s, 1H), 7.42 (d, *J =* 8.0 Hz, 1H), 5.10 (dd, *J =* 13.2, 4.8 Hz, 1H), 4.43 (d, *J =* 17.2 Hz, 1H), 4.29 (d, *J =* 17.2 Hz, 1H), 3.65 (s, 2H), 2.96-2.87 (m, 3H), 2.68-235 (m, 3H), 2.25-2.19 (m, 2H), 2.05-1.98 (m, 1H), 1.80-1.75 (m, 4H).

### Example 13: 3-(1-oxo-5-(1-(pyridazin-4-ylmethyl)piperidin-4-yl)isoindolin-2-yl)piperidine-2,6-dione (I-18)

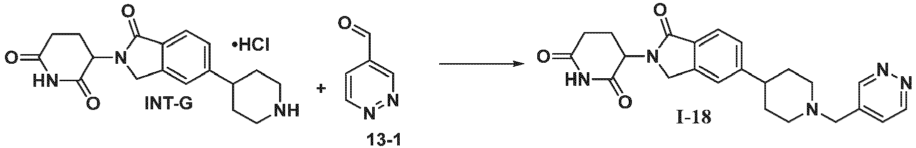

To a stirred solution of **INT-G** (250 mg, 0.68 mmol) and **13-1** (147 mg, 1.36 mmol) in DMF (10 mL), was added NaBH(OAc)₃ (432 mg, 2.04 mmol) at 0 °C and the resulting mixture was stirred at 60 °C for 16 h. Upon complete consumption of the starting materials, the reaction mixture was cooled to rt, quenched with ice-cold water and extracted with EtOAc (2 x 75 mL). The combined organic extracts were washed with brine, dried over Na₂SO₄, filtered, and concentrated under reduced pressure. The obtained crude material was purified by silica gel column chromatography eluting with 5% MeOH in DCM to afford **I-18** as an off-white solid (74 mg, 0.18 mmol, 27% yield). MS [M+H]⁺ = 420.2. ¹H NMR (400 MHz, DMSO-*d₆*): δ 10.98 (s, 1H), 9.21 (s, 1H), 9.18-9.15 (m, 1H), 7.65-7.62 (m, 2H), 7.50 (s, 1H), 7.40 (d, *J =* 8.0 Hz, 1H), 5.10 (dd, *J =* 13.2, 4.8 Hz, 1H), 4.42 (d, *J* = 17.2 Hz, 1H), 4.28 (d, *J =* 17.2 Hz, 1H), 3.61 (s, 2H), 2.88-2.87 (m, 3H), 2.67-2.32 (m, 3H), 2.16-2.12 (m, 2H), 1.99-1.95 (m, 1H), 1.78-1.76 (m, 4H).

### Example 14: 3-(5-(1-((2-methylpyrimidin-5-yl)methyl)piperidin-4-yl)-1-oxoisoindolin-2-yl)piperidine-2,6-dione (I-17)

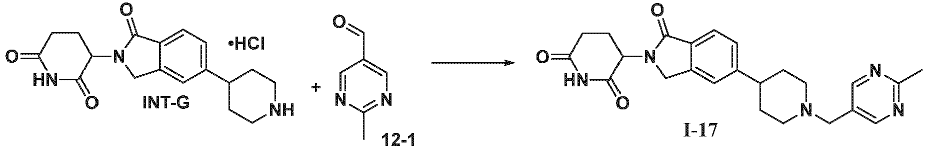

To a stirred solution of **INT-G** (250 mg, 0.68 mmol) and **12-1** (147 mg, 1.36 mmol) in DMF (10 mL), was added NaBH(OAc)₃ (432 mg, 2.04 mmol) at 0 °C and the resulting mixture was stirred at 60 °C for 16 h. Upon complete consumption of the starting materials, the reaction mixture was cooled to rt, quenched with ice-cold water and extracted with EtOAc (2 x 75 mL). The combined organic extracts were washed with brine, dried over Na₂SO₄, filtered, and concentrated under reduced pressure. The obtained crude material was purified by silica gel column chromatography eluting with 5% MeOH in DCM to afford **I-17** as an off-white solid (74 mg, 0.18 mmol, 27% yield). MS [M+H]⁺ = 434.1. ¹H NMR (400 MHz, DMSO-*d₆*): δ 10.98 (s, 1H), 8.62 (s, 2H), 7.63 (d, *J =* 8.0 Hz, 1H), 7.49 (s, 1H), 7.38 (d, *J =* 8.0 Hz, 1H), 5.10 (dd, *J=* 13.2, 4.8 Hz, 1H), 4.42 (d, *J* = 17.2 Hz, 1H), 4.28 (d, *J* = 17.2 Hz, 1H), 3.52 (s, 2H), 2.92-2.85 (m, 3H), 2.65-2.58 (m, 2H), 2.60 (s, 3H), 2.48-2.38 (m, 1H), 2.12-1.96 (m, 3H), 1.75-1.65 (m, 4H).

### Example 15: 3-(1-oxo-5-(1-((6-oxo-1,6-dihydropyridin-3-yl)methyl)piperidin-4-yl)isoindolin-2-yl)piperidine-2,6-dione (I-1)

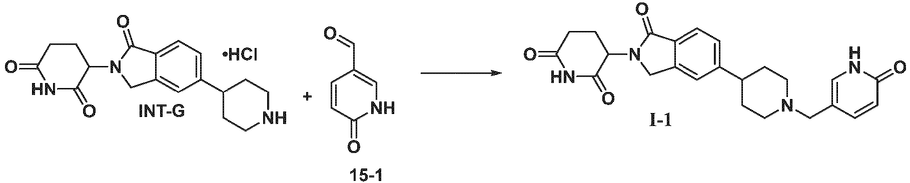

To a stirred solution of INT-G (150 mg, 0.41 mmol) and 15-1 (76 mg, 0.62 mmol) in DMF (10 mL) was added NaBH(OAc)₃ (262 mg, 1.23 mmol) at 0 °C and the resulting mixture was stirred at 60 °C for 16 h. Upon complete consumption of the starting materials, the reaction mixture was cooled to rt, quenched with ice-cold water and extracted with EtOAc (2 x 75 mL). The combined organic extracts were washed with brine, dried over Na₂SO₄, filtered, and concentrated under reduced pressure. The obtained crude material was purified by silica gel column chromatography eluting with 5% MeOH in DCM to afford **I-1** as an off-white solid (70 mg, 0.16 mmol, 39% yield). MS [M+H]⁺ = 435.2. ¹H NMR (400 MHz, DMSO-*d₆*): δ 11.45 (s, 1H), 10.99 (s, 1H), 7.63 (d, *J =* 8.0 Hz, 1H), 7.49 (s, 1H), 7.42-7.38 (m, 2H), 7.22 (s, 1H), 6.31 (d, *J=* 9.2 Hz, 1H), 5.10 (dd, *J =* 13.2, 4.8 Hz, 1H), 4.41 (d, *J =* 17.2 Hz, 1H), 4.27 (d, *J =* 17.2 Hz, 1H), 3.23 (s, 2H), 2.91-2.89 (m, 3H), 2.65-2.55 (m, 3H), 2.41-2.38 (m, 1H), 2.05-1.98 (m, 2H), 1.78-1.65 (m, 4H).

### Example 16: 3-(1-oxo-5-(1-((2-oxo-1,2-dihydropyridin-3-yl)methyl)piperidin-4-yl)isoindolin-2-yl)piperidine-2,6-dione (I-38)

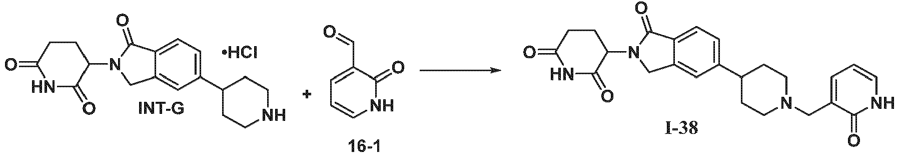

To a stirred solution of **INT-G** (150 mg, 0.41 mmol) and **16-1** (152 mg, 1.23 mmol) in DMF (10 mL), was added NaBH(OAc)₃ (261 mg, 1.23 mmol) at 0 °C and the resulting mixture was stirred at 60 °C for 16 h. Upon complete consumption of the starting materials, the reaction mixture was cooled to rt, quenched with ice-cold water and extracted with EtOAc (2 x 75 mL). The combined organic extracts were washed with brine, dried over Na₂SO₄, filtered, and concentrated under reduced pressure. The obtained crude material was purified by silica gel column chromatography eluting with 5% MeOH in DCM to afford **I-38** as an off-white solid (50 mg, 0.11 mmol, 28% yield). MS [M+H]⁺ = 435.3. ¹H NMR (400 MHz, DMSO-*d₆*): δ 11.55 (s, 1H), 10.99 (s, 1H), 7.64 (d, *J =* 8.0 Hz, 1H), 7.50 (s, 1H), 7.47-7.38 (m, 2H), 2.28-7.22 (m, 1H), 6.21-6.18 (m, 1H), 5.10 (dd, *J =* 13.2, 4.8 Hz, 1H), 4.41 (d, *J = 17.2* Hz, 1H), 4.28 (d, *J=* 17.2 Hz, 1H), 2.98-2.85 (m, 3H), 2.68-2.55 (m, 2H), 2.48-2.32 (m, 2H), 2.15-1.98 (m, 4H), 1.70-1.65 (m, 4H).

### Example 17: 3-(5-(2,6-dimethylpiperidin-4-yl)-1-oxoisoindolin-2-yl)piperidine-2,6-dione hydrochloride (I-4)

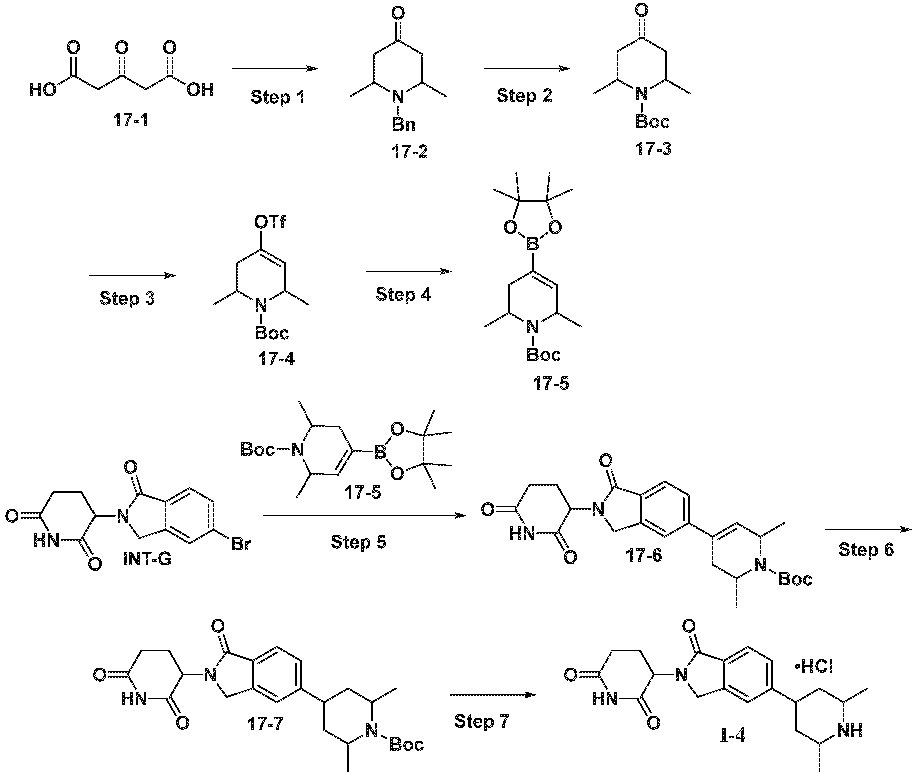

### Step 1. 1-benzyl-2,6-dimethylpiperidin-4-one (17-2)

To a solution of **17-1** (10.0 g, 68.5 mmol) and acetaldehyde (7.73 mL, 137 mmol) in water (50 mL) was added benzyl amine (7.48 mL, 68.5 mmol) dropwise at 10 °C (vigorous gas evolution was observed) and stirred at rt for 72 h. After completion of the reaction, pH was adjusted to 2 by adding 1M aq. HCl and stirred at rt. After 1 h, pH was neutralized with sat. aq. NaHCO₃ and extracted with DCM (3x150 mL). The combined organic extracts were washed with brine, dried over Na₂SO₄ and concentrated under reduced pressure. The obtained crude material was purified by silica gel column chromatography eluting with 20% EtOAc in hexanes to afford 17-2 (4.1 g, 18.87 mmol, 27% yield) as an off-white solid. MS [M+H]⁺ = 218.1.

### Step 2. tert-butyl 2,6-dimethyl-4-oxopiperidine-1-carboxylate (17-3)

To a solution of 17-2 (1.2 g, 5.5 mmol) and Boc₂O (2.43 mL, 11.0 mmol) in EtOH (50 mL) was added 10% Pd/C (300 mg) and stirred under hydrogen atmosphere for 24 h in parr apparatus. After completion of the reaction, the reaction mixture was filtered through a small pad of Celite^{®} was washed with EtOH. The combined filtrates were concentrated to dryness and the obtained crude material was purified by silica gel column chromatography eluting with 15% EtOAc in hexanes to afford 17-3 (850 mg, 3.74 mmol, 68% yield) as white solid. ¹H NMR (300 MHz, CDCl₃): δ 4.40-4.35 (m, 2H), 2.88-2.80 (m, 2H), 2.40-2.33 (m, 2H), 1.49 (s, 9H), 1.24 (d, *J =* 6.6 Hz, 6H).

### Step 3. tert-butyl 2,6-dimethyl-4-(((trifluoromethyl)sulfonyl)oxy)-3,6-dihydropyridine-1(2H)-carboxylate (17-4)

To a solution of 17-3 (600 mg, 2.64 mmol) in THF (25 mL) was added LiHMDS (3.17 mL, 3.17 mmol, 1M in THF) dropwise at -78 °C and the obtained reaction mixture was stirred at the same temperature for 1 h. N-(5-chloropyridin-2-yl)-1,1,1-trifluoro-N-((trifluoromethyl)sulfonyl)methanesulfonamide (1.34 g, 3.43 mmol), dissolved in THF (10 mL), was added dropwise and the reaction mixture was stirred at 0 °C for 3 h. The reaction mixture was quenched with sat. aq. NH₄Cl and extracted with EtOAc (3x50 mL). The combined organic extracts were washed with brine, dried over Na₂SO₄ and concentrated to dryness. The obtained crude material was purified by silica gel column chromatography eluting with 10% EtOAc in hexanes to afford **17-4** (500 mg, 1.39 mmol, 53% yield) as pale-yellow liquid. ¹H NMR (300 MHz, CDCl₃): δ 5.80-5.78 (m, 1H), 4.39-4.30 (m, 2H), 2.88-2.80 (m, 1H), 2.20-2.13 (m, 1H), 1.48 (s, 9H), 1.36 (d, *J=* 5.8 Hz, 3H), 1.23 (d, *J=* 6.3 Hz, 3H).

### Step 4. tert-butyl 2,6-dimethyl-4-(4,4,5,5-tetramethyl-1,3,2-dioxaborolan-2-yl)-3,6-dihydropyridine-1(2H)-carboxylate (17-5)

To a solution of **17-4** (500 mg, 1.39 mmol) in dioxane (20 mL) were added bis(pinacolato)diboron (389 mg, 1.53 mmol) and KOAc (409 mg, 4.17 mmol) and degassed for 10 min with argon. Then, PdCl₂(dppf)•DCM (56 mg, 0.07 mmol) and dppf (38 mg, 0.07 mmol) were added and the reaction mixture was stirred at 90 ^{e}C for 16 h. The reaction mixture was allowed to cool to rt, filtered through a small pad of Celite^{®} was washed with dioxane. The combined filtrates were evaporated under reduced pressure to afford crude **17-5** (950 mg) as an off-white solid, which was used in the next step without further purification. ¹H NMR (400 MHz, CDCl₃): δ 6.58-6.55 (m, 1H), 4.25-4.15 (m, 2H), 2.40-2.35 (m, 1H), 2.19-2.14 (m, 1H), 1.47 (s, 6H), 1.27-1.24 (m, 21H).

### Step 5. tert-butyl 4-(2-(2,6-dioxopiperidin-3-yl)-1-oxoisoindolin-5-yl)-2,6-dimethyl-3,6-dihydropyridine-1(2H)-carboxylate (17-6)

To a solution of **INT-G** (500 mg, 1.54 mmol) and crude **17-5** (940 mg) in DMF (10 mL), was added K₂CO₃ (640 mg, 4.64 mmol) and the resulting mixture was degassed for 15 min with argon. Then, PdCl₂(dppf)•DCM (94 mg, 0.11 mmol) was then added and the reaction mixture was stirred at 100 °C for 16 h. The reaction mixture was cooled to rt and filtered through a small pad of Celite^{®} and washed with DMF (20 mL). Ice-cold water was added to the filtrate and the obtained solid was filtered. The obtained crude material was purified by silica gel column chromatography eluting with 5% MeOH in DCM to afford **17-6** as an off-white solid (530, 1.17 mmol, 75% yield). MS [M+H]⁺ = 454.4.

### Step 6. tert-butyl 4-(2-(2,6-dioxopiperidin-3-yl)-1-oxoisoindolin-5-yl)-2,6-dimethylpiperidine-1-carboxylate (17-7)

To a solution of **17-6** (530 mg, 1.17 mmol) in DMF (20 mL) was added Pd/C (10 wt%, 200 mg) and the resulting mixture was stirred at rt under an atmosphere of hydrogen (in parr apparatus, 60 psi) for 16 h. Upon complete consumption of the starting material, the reaction mixture was filtered through a pad of Celite^{®} and washed with DMF. The filtrate was quenched with water and extracted with EtOAc (2 x 100 mL). The combined organic extracts were washed with brine (50 mL), dried over Na₂SO₄, filtered, and concentrated under reduced pressure to afford **17-7** as an off-white solid (290 mg, 0.64 mmol, 55% yield). MS [M-tBu+H]⁺ = 400.1.

### Step 7. 3-(5-(2,6-dimethylpiperidin-4-yl)-1-oxoisoindolin-2-yl)piperidine-2,6-dione hydrochloride (I-4)

To a solution of **17-7** (290 mg, 0.64 mmol) in dioxane (10 mL) was added drop wise 4M HCl in dioxane (3 ml) at 0 °C and the resulting mixture was stirred at rt for 16 h. Upon complete consumption of the starting materials, the solvent was evaporated under reduced pressure. The crude material was triturated with diethyl ether and dried under reduced pressure to afford **I-4** as an off-white solid (150 mg, 0.38 mmol, 60% yield). MS [M+H]⁺ = 356.2. ¹H NMR (400 MHz, DMSO-*d₆*): δ 11.00 (s, 1H), 8.82 (br s, 1H), 7.70 (d, *J =* 8.0 Hz, 1H), 7.47 (s, 1H), 7.38 (d, *J =* 8.8 Hz, 1H), 5.11 (dd, *J =* 13.2, 4.8 Hz, 1H), 4.44 (d*, J* = 17.2 Hz, 1H), 4.31 (d, *J =* 17.2 Hz, 1H), 3.79-3.78 (m, 1H), 3.50-3.47 (m, 1H), 3.27-3.21 (m, 1H), 2.96-2.87 (m, 1H), 2.67-2.50 (m, 1H), 2.41-2.34 (m, 1H), 2.07-1.94 (m, 3H), 1.80-1.77 (m, 1H), 1.66-1.60 (m, 1H), 1.42 (d, *J =* 6.8 Hz, 3H), 1.27 (d, *J =* 6.8 Hz, 3H).

### Example 18: 3-(5-(1-benzyl-2,6-dimethylpiperidin-4-yl)-1-oxoisoindolin-2-yl)piperidine-2,6-dione hydrochloride (I-2)

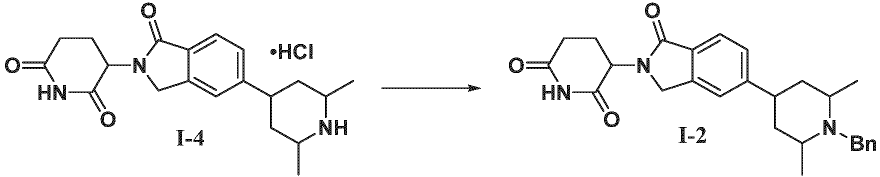

To a stirred solution of **I-4** (100 mg, 0.25 mmol) and Et₃N (0.11 mL, 0.76 mmol) in DMF (5 mL) was added benzyl bromide (0.04 mL, 0.30 mmol) at 0 °C and the resulting mixture was stirred at rt for 16 h. Upon complete consumption of the starting materials, the reaction mixture was quenched with ice-cold water and extracted with EtOAc (2 x 50 mL). The combined organic extracts were washed with brine, dried over Na₂SO₄, filtered, and concentrated under reduced pressure. The obtained crude material was purified by silica gel column chromatography eluting with 6% MeOH in DCM to afford **I-2** as an off-white solid (30 mg, 0.07 mmol, 26% yield). MS [M+H]⁺ = 446.1. ¹H NMR (400 MHz, DMSO-*d₆*): δ 10.99 (s, 1H), 7.65 (d, *J =* 7.6 Hz, 1H), 7.51 (s, 1H), 7.41-7.20 (m, 6H), 5.11 (dd, *J* = 13.4, 4.8 Hz, 1H), 4.45 (d, *J=* 17.2 Hz, 1H), 4.28 (d, *J =* 17.2 Hz, 1H), 3.93-3.89 (m, 1H), 3.48-3.45 (m, 1H), 3.10-2.85 (m, 4H), 2.65-2.35 (m, 2H), 2.03-1.45 (m, 5H), 1.08-1.05 (m, 6H).

### Example 19: 3-(5-(1-((5-methoxypyridin-2-yl)methyl)piperidin-4-yl)-1-oxoisoindolin-2-yl)piperidine-2,6-dione (I-6)

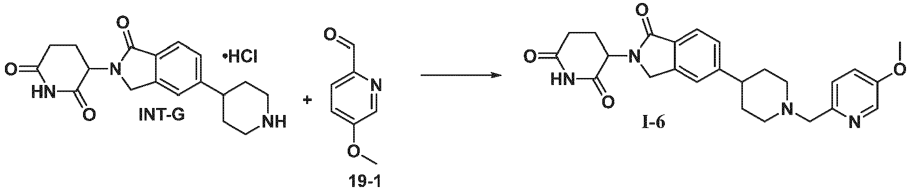

To a stirred solution of **INT-G** (150 mg, 0.41 mmol) and **19-1** (85 mg, 0.62 mmol) in DMF (10 mL), was added NaBH(OAc)₃ (262 mg, 1.23 mmol) at 0 °C and the resulting mixture was stirred at 60 °C for 16 h. After completion of the reaction, the mixture was allowed to cool to rt, quenched with ice-cold water and extracted with EtOAc (2 x 75 mL). The combined organic extracts were washed with brine, dried over Na₂SO₄, filtered, and concentrated under reduced pressure. The obtained crude material was purified by silica gel column chromatography eluting with 7% MeOH in DCM to afford **I-6** as an off-white solid (40 mg, 0.09 mmol, 22% yield). MS [M+H]⁺ = 449.2. ¹H NMR (400 MHz, DMSO-*d₆*): δ 10.99 (s, 1H), 8.20-8.19 (m, 1H), 7.63 (d, *J =* 8.0 Hz, 1H), 7.49 (s, 1H), 7.41-7.35 (m, 3H), 5.10 (dd, *J =* 13.2, 4.8 Hz, 1H), 4.42 (d, *J =* 17.2 Hz, 1H), 4.28 (d, *J =* 17.2 Hz, 1H), 3.81 (s, 3H), 3.56 (s, 2H), 2.94-2.75 (m, 3H), 2.66-2.33 (m, 3H), 2.14-1.96 (m, 3H), 1.77-1.66 (m, 4H).

### Example 20: 3-(5-(1-((5-ethoxypyridin-2-yl)methyl)piperidin-4-yl)-1-oxoisoindolin-2-yl)piperidine-2,6-dione (I-5)

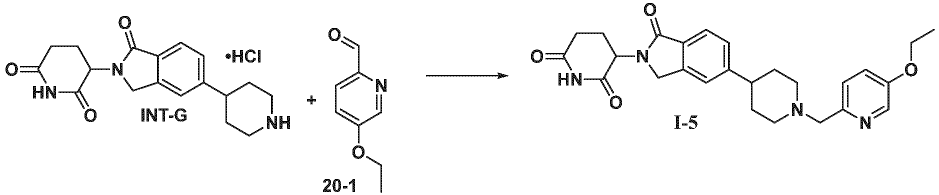

To a stirred solution of **INT-G** (150 mg, 0.41 mmol) and **20-1** (93 mg, 0.62 mmol) in DMF (10 mL) was added NaBH(OAc)₃ (262 mg, 1.23 mmol) at 0 °C and the resulting mixture was stirred at 60 °C for 16 h. After completion of the reaction, the mixture was allowed to cool to rt, quenched with ice-cold water and extracted with EtOAc (2 x 75 mL). The combined organic extracts were washed with brine, dried over Na₂SO₄, filtered, and concentrated under reduced pressure. The obtained crude material was purified by silica gel column chromatography eluting with 7% MeOH in DCM to afford **I-5** as an off-white solid (60 mg, 0.13 mmol, 32% yield). MS [M+H]⁺ = 463.2. ¹H NMR (400 MHz, DMSO-*d₆*): δ 10.99 (s, 1H), 8.18-8.17 (m, 1H), 7.63 (d, *J =* 8.0 Hz, 1H), 7.49 (s, 1H), 7.41-7.33 (m, 3H), 5.09 (dd, *J =* 13.2, 4.8 Hz, 1H), 4.42 (d, *J=* 17.2 Hz, 1H), 4.26 (d, *J =* 17.2 Hz, 1H), 4.08 (q, *J =* 6.8 Hz, 2H), 3.55 (s, 2H), 3.04-2.86 (m, 3H), 2.66-2.33 (m, 3H), 2.14-1.96 (m, 3H), 1.77-1.66 (m, 4H), 1.33 (t, *J =* 6.8 Hz, 3H).

### Example 21: 3-(5-(1-((6-methoxypyridin-3-yl)methyl)piperidin-4-yl)-1-oxoisoindolin-2-yl)piperidine-2,6-dione (I-7)

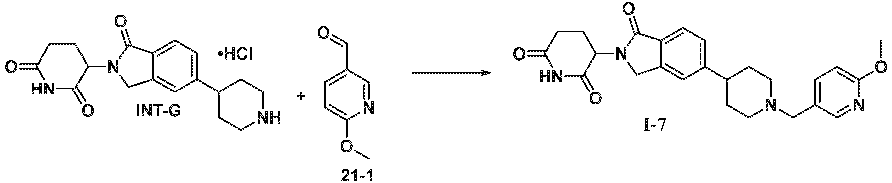

To a stirred solution of **INT-G** (150 mg, 0.41 mmol) and **21-1** (147 mg, 0.82 mmol) in DMF (10 mL) was added NaBH(OAc)₃ (262 mg, 1.23 mmol) at 0 °C and the resulting mixture was stirred at 60 °C for 16 h. After completion of the reaction, the mixture was allowed to cool to rt, quenched with ice-cold water and extracted with EtOAc (2 x 50 mL). The combined organic extracts were washed with brine, dried over Na₂SO₄, filtered, and concentrated under reduced pressure. The obtained crude material was purified by silica gel column chromatography eluting with 4% MeOH in DCM to afford **I-7** as pale brown solid (70 mg, 0.15 mmol, 34% yield). MS [M+H]⁺ = 449.1. ¹H NMR (400 MHz, DMSO-*d₆*): δ 10.99 (s, 1H), 8.07 (s, 1H), 7.66-7.62 (m, 2H), 7.48 (s, 1H), 7.39 (d, *J =* 8.0 Hz, 1H), 6.80 (d, *J =* 8.0 Hz, 1H), 5.09 (dd, *J =* 13.2, 4.8 Hz, 1H), 4.42 (d, *J =* 17.2 Hz, 1H), 4.27 (d, *J =* 17.2 Hz, 1H), 3.83 (s, 3H), 3.45 (br s, 2H), 2.94-2.87 (m, 3H), 2.61-2.32 (m, 3H), 2.05-1.98 (m, 3H), 1.79-1.67 (m, 4H).

### Example 22: 3-(5-(1-((6-ethoxypyridin-3-yl)methyl)piperidin-4-yl)-1-oxoisoindolin-2-yl)piperidine-2,6-dione (I-8)

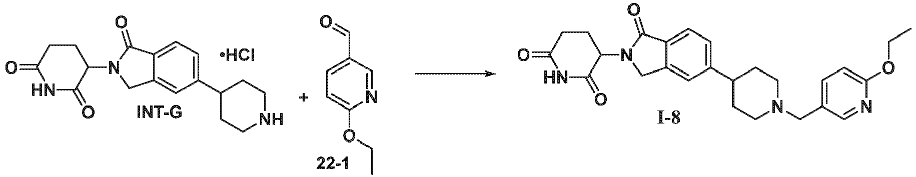

To a stirred solution of **INT-G** (150 mg, 0.41 mmol) and **22-1** (93 mg, 0.62 mmol) in DMF (10 mL) was added NaBH(OAc)₃ (262 mg, 1.23 mmol) at 0 °C and the resulting mixture was stirred at 60 °C for 16 h. After completion of the reaction, the mixture was allowed to cool to rt, quenched with ice-cold water and extracted with EtOAc (2 x 50 mL). The combined organic extracts were washed with brine, dried over Na₂SO₄, filtered, and concentrated under reduced pressure. The obtained crude material was purified by silica gel column chromatography eluting with 8% MeOH in DCM to afford **I-8** as an off-white solid (40 mg, 0.08 mmol, 21% yield). MS [M+H]⁺ = 463.1. ¹H NMR (400 MHz, DMSO-*d₆*): δ 10.97 (s, 1H), 8.03 (d, *J =* 2.0 Hz, 1H), 7.65-7.62 (m, 2H), 7.48 (s, 1H), 7.39 (d, *J =* 8.0 Hz, 1H), 6.76 (d, *J =* 8.4 Hz, 1H), 5.09 (dd *J =* 13.2, 4.8 Hz, 1H), 4.41 (d, *J =* 17.2 Hz, 1H), 4.30-4.24 (m, 3H), 3.44 (s, 2H), 2.94-2.87 (m, 3H), 2.67-2.32 (m, 3H), 2.07-1.95 (m, 3H), 1.77-1.65 (m, 4H), 1.30 (t, *J =* 7.2 Hz, 3H).

### Example 23: 3-(5-(1-(2-(1H-pyrazol-1-yl)ethyl)piperidin-4-yl)-1-oxoisoindolin-2-yl)piperidine-2,6-dione (I-11)

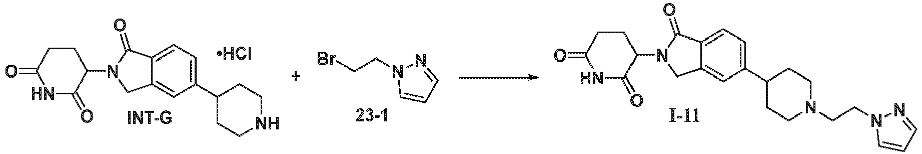

To a stirred solution of **INT-G** (150 mg, 0.41 mmol) and Et₃N (0.17 mL, 1.23 mmol) in DMF (10 mL) was added **23-1** (142 mg, 0.82 mmol) at 0 °C and the resulting mixture was stirred at rt for 16 h. Upon complete consumption of the starting materials, the reaction mixture was quenched with ice-cold water and extracted with EtOAc (2 x 75 mL). The combined organic extracts were washed with brine, dried over Na₂SO₄, filtered, and concentrated under reduced pressure. The obtained crude material was purified by silica gel column chromatography eluting with 6% MeOH in DCM to afford **I-11** as an off-white solid (110 mg, 0.26 mmol, 63% yield). MS [M+H]⁺ = 422.3. ¹H NMR (400 MHz, DMSO-*d₆*): δ 10.99 (s, 1H), 7.75 (d, *J =* 2.0 Hz, 1H), 7.63 (d, *J =* 8.0 Hz, 1H), 7.48 (s, 1H), 7.42-7.38 (m, 2H), 6.22-6.21 (m, 1H), 5.09 (dd, *J* = 13.2, 4.8 Hz, 1H), 4.42 (d, *J =* 17.2 Hz, 1H), 4.30-4.22 (m, 3H), 2.99-2.86 (m, 3H), 2.75-2.55 (m, 3H), 2.42-2.31 (m, 2H), 2.15-1.92 (m, 3H), 1.78-1.62 (m, 4H).

### Example 24: 4-((4-(2-(2,6-dioxopiperidin-3-yl)-1-oxoisoindolin-5-yl)piperidin-1-yl)methyl)cyclohexane-1-carbonitrile (I-3)

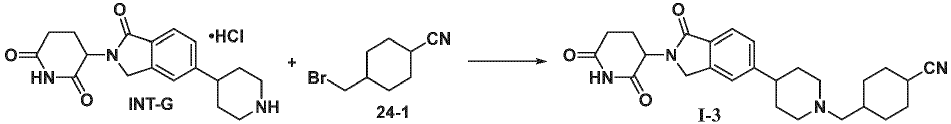

To a stirred solution of **INT-G** (150 mg, 0.41 mmol) and Et₃N (0.17 mL, 1.23 mmol) in DMF (10 mL) was added **24-1** (99 mg, 0.49 mmol) at 0 °C and the resulting mixture was stirred at rt for 16 h. Upon complete consumption of the starting materials, the reaction mixture was quenched with ice-cold water and extracted with EtOAc (2 x 75 mL). The combined organic extracts were washed with brine, dried over Na₂SO₄, filtered, and concentrated under reduced pressure. The obtained crude material was purified by silica gel column chromatography eluting with 6% MeOH in DCM to afford **I-3** as an off-white solid (30 mg, 0.07 mmol, 16% yield). MS [M+H]⁺ = 449.2. ¹H NMR (400 MHz, DMSO-*d₆*): δ 10.99 (s, 1H), 7.63 (d, *J =* 8.0 Hz, 1H), 7.49 (s, 1H), 7.39 (d, *J = 8.0* Hz, 1H), 5.09 (dd, *J =* 13.2, 4.8 Hz, 1H), 4.41 (d, *J=* 17.2 Hz, 1H), 4.27 (d, *J =* 17.2 Hz, 1H), 3.34-3.28 (m, 2H), 3.29-2.87 (m, 3H), 2.67-2.32 (m, 3H), 2.10-2.09 (m, 2H), 2.08-1.93 (m, 5H), 1.80-1.66 (m, 5H), 1.53-1.45 (m, 3H), 0.90-0.86 (m, 2H).

### Example 25: 3-(1-oxo-5-(1-(2-(pyridin-4-yl)propan-2-yl)piperidin-4-yl)isoindolin-2-yl)piperidine-2,6-dione HCOOH salt (I-37)

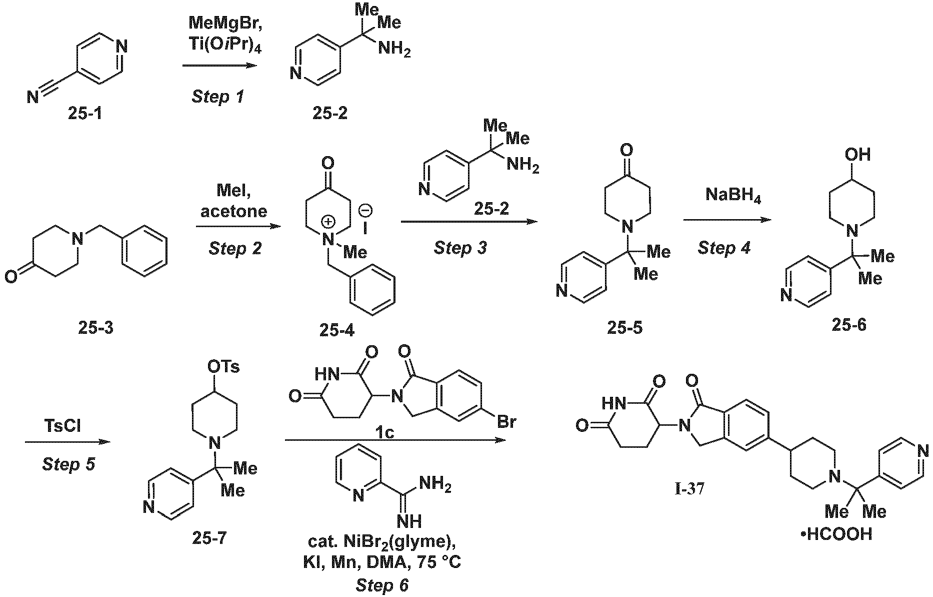

### Step 1: 2-(pyridin-4-yl)propan-2-amine (25-2)

To a solution of compound **25-1** (5.00 g, 48.0 mmol) in Et₂0 (100 mL) was added MeMgBr (3M in THF, 48.0 mL, 144 mmol) at 0 °C and the reaction mixture was stirred at room temperature for 30 minutes. To the reaction mixture was added Ti(O*i*Pr)₄ (14.2 mL, 48.0 mmol) and stirred at 50 °C for 16 h. After completion of the reaction, the reaction mixture was quenched with 1M aq. NaOH and extracted with Et₂O (3 x 100 mL). The combined organic extracts were washed with brine, dried over Na₂SO₄ and concentrated under reduced pressure. The obtained crude material was purified by silica gel column chromatography eluting with 5-10% MeOH in DCM to afford compound **25-2** (1.80 g, 13.2 mmol, 28%) as a viscous yellow liquid. ¹H NMR (400 MHz, DMSO-*d₆*): δ 8.46-8.45 (m, 2H), 7.50-7.49 (m, 2H), 1.96 (brs, 2H), 1.33 (s, 6H)

### Step 2: 1-benzyl-1-methyl-4-oxopiperidin-1-ium (25-4)

To a solution of compound 25-3 (5.0 g, 26 mmol) in acetone (100 mL), MeI (2.0 mL, 32 mmol) was added. The reaction mixture was stirred at room temperature for 16 h. After completion of the reaction, the reaction mixture was filtered, washed with acetone and dried reduced pressure to afford compound **25-4** (4.0 g, 12 mmol, 46%) as a white solid. ¹H NMR (300 MHz, DMSO-*d₆*): δ 7.56-7.55 (m, 5H), 4.73 (s, 2H), 3.85-3.62 (m, 4H), 3.14 (s, 3H), 2.89-2.75 (m, 2H), 2.60-2.72 (m, 2H).

### Step 3: 1-(2-(pyridin-4-yl)propan-2-yl)piperidin-4-one (25-5)

To a solution of compound **25-2** (2.0 g, 6.0 mmol) in EtOH:H₂O (v/v = 3:2) (30 mL) was added compound **25-4** (1.20 g, 9.05 mmol) and K₂CO₃ (125 mg, 0.90 mmol). The reaction mixture was stirred at 100 °C for 12 h. After completion of the reaction, the reaction mixture was diluted with water and extracted with EtOAc (3 x 50 mL). The combined organic extracts were washed with brine, dried over Na₂SO₄ and concentrated under reduced pressure. The obtained crude material was purified by silica gel column chromatography eluting with 50% EtOAc in Hexane to afford compound **25-5** (0.60 g, 2.7 mmol, 46%) as brown liquid. ¹H NMR (600 MHz, CDCl₃): δ 8.56-8.55 (d, *J =* 6.0 Hz, 2H), 7.50-7.49 (d, *J =* 4.8 Hz, 2H), 2.75-2.73 (t, *J =* 6.0 Hz, 4H), 2.42-2.40 (t, *J =* 6.0 Hz, 4H), 1.38 (s, 6H).

### Step 4: 1-(2-(pyridin-4-yl)propan-2-yl)piperidin-4-ol (25-6)

To a solution of **25-5** (232 mg, 1.06 mmol) in MeOH (3 mL) was added NaBH₄ (48 mg, 1.3 mmol) portion wise at 0 °C. The reaction mixture was stirred for 30 min at rt. The reaction mixture was quenched with sat. aq. NaHCO₃ and extracted with DCM (x3). Combined organic phases were dried over Na₂SO₄, filtered and concentrated to dryness to afford compound **25-6** (233 mg, 1.06 mmol, 100 % yield) as a colorless oil. The product was used in the next step without further purification. MS [M+H]⁺ = 221.4. ¹H NMR (400 MHz, Chloroform-*d*) δ 8.54 (d, *J* = 5.3 Hz, 2H), 7.47 (d, *J =* 5.2 Hz, 2H), 3.79 - 3.57 (m, 1H), 2.77 - 2.63 (m, 2H), 2.25 (t, *J =* 10.2 Hz, 2H), 1.94 - 1.81 (m, 2H), 1.61 - 1.51 (m, 2H), 1.34 (s, 6H).

### Step 5: 1-(2-(pyridin-4-yl)propan-2-yl)piperidin-4-yl 4-methylbenzenesulfonate (25-7)

To a solution of 25-6 (233 mg, 1.06 mmol), DIPEA (0.28 mL, 1.6 mmol), DMAP (13 mg, 0.11 mmol) in DCM (10 mL) was added TsCl (121 mg, 1.06 mmol) and reaction mixture was stirred for 1 h at 0 °C and then overnight at rt. The reaction mixture was quenched with sat. aq. NaHCO₃ and extracted with DCM (x3). The combined organic phases were dried over Na₂SO₄, filtered, and concentrated to dryness to afford crude **25-7** (390 mg, assumed quantitative yield) as a brown oil. The obtained crude product was used in the next step without further purification.

### Step 6: 3-(1-oxo-5-(1-(2-(pyridin-4-yl)propan-2-yl)piperidin-4-yl)isoindolin-2-yl)piperidine-2,6-dione HCOOH salt (I-37).

To a suspension of NiBr₂(glyme) (7.2 mg, 0.023 mmol), picolinimidamide HCl salt (3.7 mg, 0.023 mmol), KI (77 mg, 0.46 mmol) and manganese powder (43 mg, 0.77 mmol) in DMA (0.2 mL) was added a mixture of **1c** (50 mg, 0.16 mmol) and crude **25-7** (174 mg, 0.465 mmol), dissolved in DMA (1 mL) under nitrogen atmosphere. The reaction mixture was then stirred vigorously at 75 °C for 7 hours under atmosphere of nitrogen. The reaction was filtered and the filter was washed with MeCN. The reaction mixture was then concentrated. The crude product was purified by RP HPLC (eluting with MeCN/H₂O with 0.1% NH₃ as a modifier) using collecting into tubes containing ~2 drops of HCOOH. Fractions containing desired product were concentrated by lyophilizer to afford the formate salt of compound **I-37** (6.9 mg, 0.014 mmol, 9% yield) was obtained as a white solid. MS [M+H]⁺ = 447.5. ¹H NMR (400 MHz, CD₃CN:D₂O (v/v = 1:1)) δ 8.61 (d, *J =* 5.9 Hz, 2H), 8.33 (s, 1H), 7.74 - 7.68 (m, 1H), 7.64 (d, *J =* 5.3 Hz, 2H), 7.43 (d, *J = 3.8* Hz, 1H), 7.38 (d, *J =* 8.3 Hz, 1H), 5.12 - 4.94 (m, 1H), 4.50 - 4.27 (m, 2H), 3.32 (d, *J* = 11.7 Hz, 2H), 2.93 - 2.69 (m, 5H), 2.47 - 2.30 (m, 1H), 2.18 - 2.08 (m, 1H), 1.72 (s, 6H). Missing protons are overlapping with residual acetonitrile or H₂O solvent peaks.

### Biological Assays and Data

The activity of a compound according to the present invention can be assessed by the following in vitro methods.

### Example 26: Prolabel Quantification of IKZF1 or IKZF2 protein levels in 293GT cells

The Prolabel system from DiscoverX was used to develop high-throughput and quantitative assays to measure changes in IKZF1 and IKZF2 protein levels in response to compounds. The prolabel tag was derived from the alpha fragment of beta galactosidase and has the following protein sequence: mssnslavvlqrrdwenpgvtqlnrlaahppfaswrnseeartdrpsqqlrslnge. The complementary fragment of beta-galactosidase (from DiscoverX), is added to the prolabel tag to form an active beta galactosidase enzyme whose activity can be precisely measured. In this way, the levels of a fusion protein with the prolabel tag can be quantified in cell lysates.

Lentiviral vectors, based on the Invitrogen pLenti6.2/V5 DEST backbone, were constructed that placed the prolabel tag upstream of IKZF1, IKZF2 or GSPT1 and expressed the fusion protein from a CMV promoter.

To ensure moderate and consistent expression of the prolabel fusion proteins across all cells in the population, stable cell lines were constructed from cells expressing a single copy of the construct. Lentivirus packaged with the constructs was made using the Virapower kit from Invitrogen. Strongly adherent 293GT cell, GripTite 293 MSR cells from Thermo Fisher Scientific (Catalog number: R79507), were infected with the virus at low multiplicity of infection and selected by 5 µg/mL blasticidin for 2 weeks.

The levels of prolabel tagged fusion proteins in compound treated cell lines were measured as follows:
Day 1, Cells were diluted to 1.0 x 10⁶ cells/ml in normal growth medium. 17.5 µL of cells were plated in each well of a solid white 384 well plate. Plates were incubated overnight in a 37 °C tissue culture incubator.
Day 2, Serial dilutions of compounds were made in 384 well plates from 10 mM stocks. 15 µL of DMSO was added to each well of a 384 well plate. In the first column, 15µL of stock compound was added. The solution was mixed and 15 µL was transferred to the next column. This was repeated until 20 two-fold dilutions were prepared. 2.5 µL of diluted compounds were transferred into 60 µL of cell culture medium in another 384 well plate, and mixed well. 2.5 µL of this mixture was added to the plated cells. The final DMSO concentration was 0.5% and the highest concentration of compound was 50 µM. Plates were incubated overnight (e.g., about 14 h, 18 h, or 24 h) in a 37 °C tissue culture incubator.
Day 3, Plates were removed from the incubator and allowed to equilibrate at rt for 30 minutes. Prolabel substrate (DiscoverX PathHunter Prolabel Detection Kit, User manual: 93-0180) was added as described by the manufacturers protocols. Plates were incubated at rt for three hours and luminescence was read using an Envision reader (Perkin Elmer) Data was analyzed and visualized using the Spotfire software package.

Table 14 shows Helios (IKZF2), Ikaros (IKZF1) and G1 to S phase transition 1 protein (GSPT1) degradation activity of compounds of the invention in Pro-label assays in 293GT cells, (% degradation is at 10 µM). Pomalidomide was tested as the control.

**TABLE 14:**

| **IKZF2 and IKZF1 Activity** | | | |
|---|---|---|---|
| **Cmpd No.** | **IKZF2** | | **IKZF1 AC₅₀ (µM)** |
| | **AC₅₀ (µM)** | **% protein reduction at 10 µM, 24 h** | |
| **I-1** | 2.2 | 70% | >30 |
| **I-2** | - | 35% at 30uM | >30 |
| **I-3** | 0.089 | 60% | >30 |
| **I-4** | - | 30% at 30uM | >30 |
| **I-5** | 0.025 | 60% | >30 |
| **I-6** | 0.009 | 50% | >30 |
| **I-7** | 0.024 | 80% | >30 |
| **I-8** | 0.014 | 70% | >30 |
| **I-10** | 0.017 | 75% | >30 |
| **I-11** | 0.14 | 60% | >30 |
| **I-12** | - | - | >30 |
| **I-13** | 0.012 | 80% | >30 |
| **I-14** | 2.86 | 55% | >30 |
| **I-15** | 1.87 | 40% | >30 |
| **I-16** | 13.1 | 50% | >30 |
| **I-17** | 1.73 | 50% | >30 |
| **I-18** | 10.0 | 40% | >30 |
| **I-30** | 0.020 | 80% | >30 |
| **I-31** | 0.079 | 70% | >30 |
| **I-32** | 0.092 | 62% | >30 |
| **I-33** | 0.064 | 62% | >30 |
| **I-35** | 0.019 | 80% | >30 |
| **I-36** | 0.038 | 74% | >30 |
| **Control** | >50 | | 0.05 (80% degradation at 10 µM) |

### Example 26: Quantification of in vitro Suppressive Potency of Primary Human Regulatory T cells Expanded in the Presence of Compounds

### Materials and methods

### Treg cell sorting:

Human buffy coats are obtained from BioreclamationIVT, in the USA. CD4+ T cells are isolated from said buffy coats using the RosetteSep Human CD4+ T cell enrichment Cocktail (Stemcell technologies, USA) and gradient centrifugation over Ficoll Paque Plus (GE HealthCare LifeSciences, USA) as per manufacturer's recommendations. Cells are resuspended in RPMI medium supplemented with 1% penicillin-Streptomycin solution, 10% Fetal Bovine Serum, HEPES (10 mM), MEM NEAA (100 nM), sodium pyruvate (1 mM) (all supplements from Thermo Fisher Scientific, USA), thereafter referred to as complete RPMI (cRPMI), and rested overnight at 37 °C, 5% CO₂ in the presence of 2U/mL rhIL-2 (Proleukin, Novartis). Cells are collected and resuspended in autoMACS Running Buffer supplemented with BSA (Miltenyi Biotec, USA) and labelled using CD4-FITC antibody (clone RPA-T4), CD25-APC antibody (clone M-A251) (Biolegend) and CD25 Microbeads (Miltenyi Biotec, USA). CD25-enriched cells are then isolated using the autoMACS Pro Separator. A highly purified population of Treg cells is then obtained by further sorting CD4+ CD25Hi cells using a Sony SH800 cell sorter. The resulting Treg cell population is routinely above 90% pure according to FOXP3 expression.

### Treg cell expansion:

Purified Treg cells are plated in cRPMI in 96-well, round-bottom plates at a density of 25000-50000 cells per well and activated in the presence of 500 U/mL rhIL2, and Treg expander Dynabeads (Thermo Fisher Scientific, USA) according to manufacturer's recommendations, in the presence or absence of 100 µM rapamycin (Thermo Fisher Scientific, USA). The compounds of the present invention are then added at a final concentration of 10 µM and DMSO was added as a vehicle control. Cells are incubated at 37 °C, 5% CO₂ for a total of 12-14 days. The compound and rhIL2 are replenished every 48h during the entirety of the culture.

### Phenotypic analysis of expanded Treg cells:

Cell are collected and counted and the fold expansion is calculated as (number of cells recovered)/(number of cells plated). A fraction of the cells is fixed and permeabilized using the eBioscience Foxp3 staining Buffer kit (eBioscience, Thermo Fisher Scientific, USA) and stained with Helios-PECyanine7 antibody (Clone 22F6). To determine IL2-expression, expanded Treg cells are further incubated in the presence of the eBioscience Cell Stimulation Cocktail with Protein inhibitors (Thermo Fisher Scientific) for 4 hours, followed by fixation and staining with IL2-BV711 antibody (clone MQ1-17H12) (Biolegend, USA). Cells are acquired on an LSRFortessa (Becton Dickinson, USA) and analysis was performed using the FlowJo software (TreeStar, USA).

### Functional analysis of expanded Treg cells:

Primary human PBMCs are obtained from freshly prepared buffy coats (BioReclamationIVT) using gradient centrifugation over Ficoll Paque Plus as per manufacturer's recommendations. Cells are then labelled with CFSE (5(6)-Carboxyfluorescein diacetate N-succinimidyl ester, Sigma-Aldrich, USA) and plated in triplicates cRPMI in round bottom 96-well plates, alone or with expanded Treg cells at a 1:2 PBMC:Treg ratio. The compounds of the present invention are then added at a final concentration of 10 µM and DMSO is added as a vehicle control. Cells are activated using soluble anti-CD3 antibody (clone OKT3) (eBioscience, ThermoFisher Scientific, USA) at a final concentration of 100 ng/ml. Cells are incubated at 37 °C, 5% CO₂ for a total of 4-5 days. At the end of the culture, cells are stained using the Live/dead Blue viability stain (Thermo Fisher Scientific, USA) as per manufacturer's instructions, followed by staining with CD4-BUV737 (Clone SK3) (BDBiosciences, USA) and CD8-BV711 (clone RPA-T8) (Biolegend, USA). Cells are acquired on an LSRFortessa (Becton Dickinson, USA) and analysis is performed using the FlowJo software (TreeStar, USA). Proliferation is assessed in each population as the proportion of cells having diluted CFSE. Suppression is assessed for each condition in comparison to the responders plated alone.

## Claims

1. A compound of Formula (I): 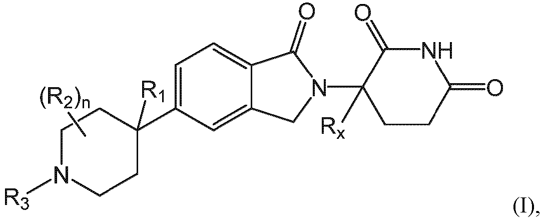 wherein:
R₁ is (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -(CH₂)₀₋₂NH₂, -(CH₂)₀₋₂NH(C₁-C₆)alkyl, -(CH₂)₀₋₂N((C₁-C₆)alkyl)₂, -C(O)NH₂, -C(O)OH or CN;
each R₂ is independently (C₁-C₆)alkyl, (C₁-C₆)haloalkyl, (C₁-C₆)hydroxyalkyl, CN, or halogen, or
R₁ and R₂ together with the carbon atoms to which they are attached form a (C₃-C₇)cycloalkyl or a 4- to 6-membered heterocycloalkyl ring comprising 1 to 3 heteroatoms selected from O, N, and S, or
two R₂ together with the carbon atoms to which they are attached form (C₃-C₇)cycloalkyl or a 4- to 6-membered heterocycloalkyl ring comprising 1 to 3 heteroatoms selected from O, N, and S;
R₃ is (C₁-C₆)alkyl, (C₆-C₁₀)aryl, 5- or 6-membered heteroaryl comprising 1 to 3 heteroatoms selected from O, N, and S, (C₃-C₈)cycloalkyl, or 4- to 7-membered heterocycloalkyl comprising 1 to 3 heteroatoms selected from O, N, and S, wherein the alkyl is optionally substituted with one or more R₄; and the aryl, heteroaryl, cycloalkyl, and heterocycloalkyl are optionally substituted with one or more R₅, or
R₂ and R₃, when on adjacent atoms, together with the atoms to which they are attached form a 5- or 6-membered heterocycloalkyl ring;
each R₄ is independently selected from -C(O)OR₆, -C(O)NR₆R_{6'}, -NR₆C(O)R_{6'}, halogen, -OH, -NH₂, CN, (C₆-C₁₀)aryl, 5- or 6-membered heteroaryl comprising 1 to 4 heteroatoms selected from O, N, and S, (C₃-C₈)cycloalkyl, and 4- to 7-membered heterocycloalkyl ring comprising 1 to 3 heteroatoms selected from O, N, and S, wherein the aryl, heteroaryl, cycloalkyl, and heterocycloalkyl groups are optionally substituted with one or more R₇;
each R₅ is independently selected from (C₁-C₆)alkyl, (C₂-C₆)alkenyl, (C₂-C₆)alkynyl, (C₁-C₆)alkoxy, (C₁-C₆)haloalkyl, (C₁-C₆)haloalkoxy, (C₁-C₆)hydroxyalkyl, halogen, -OH, -NH₂, CN, (C₃-C₇)cycloalkyl, 5- to 7-membered heterocycloalkyl comprising 1 to 3 heteroatoms selected from O, N, and S, (C₆-C₁₀)aryl, and 5- or 6-membered heteroaryl comprising 1 to 3 heteroatoms selected from O, N, and S, or
two R₅, when on adjacent atoms, together with the atoms to which they are attached form a (C₆-C₁₀)aryl ring or a 5- or 6-membered heteroaryl ring comprising 1 to 3 heteroatoms selected from O, N, and S, optionally substituted with one or more R₁₀, or
two R₅ together with the atoms to which they are attached form a (C₃-C₇)cycloalkyl ring or a 4- to 7-membered heterocycloalkyl ring comprising 1 to 3 heteroatoms selected from O, N, and S optionally substituted with one or more R₁₀;
R₆ and R_{6'} are each independently H, (C₁-C₆)alkyl, or (C₆-C₁₀)aryl;
each R₇ is independently selected from (C₁-C₆)alkyl, (C₂-C₆)alkenyl, (C₂-C₆)alkynyl, (C₁-C₆)alkoxy, (C₁-C₆)haloalkyl, (C₁-C₆)haloalkoxy, -C(O)R₈, -(CH₂)₀₋₃C(O)OR₈, -C(O)NR₈R₉, -NR₈C(O)R₉, - NR₈C(O)OR₉, -S(O)ₚNR₈R₉, -S(O)ₚR₁₂, (C₁-C₆)hydroxyalkyl, halogen, -OH, -O(CH₂)₁₋₃CN, -NH₂, CN, -O(CH₂)₀₋₃(C₆-C₁₀)aryl, adamantyl, -O(CH₂)₀₋₃-5- or 6-membered heteroaryl comprising 1 to 3 heteroatoms selected from O, N, and S, (C₆-C₁₀)aryl, monocyclic or bicyclic 5- to 10-membered heteroaryl comprising 1 to 3 heteroatoms selected from O, N, and S, (C₃-C₇)cycloalkyl, and 5- to 7-membered heterocycloalkyl comprising 1 to 3 heteroatoms selected from O, N, and S, wherein the alkyl is optionally substituted with one or more R₁₁, and the aryl, heteroaryl, and heterocycloalkyl are optionally substituted with one or more substituents each independently selected from halogen, (C₁-C₆)alkyl, (C₁-C₆)haloalkyl, and (C₁-C₆)alkoxy, or
two R₇ together with the carbon atom to which they are attached form a =(O), or
two R₇, when on adjacent atoms, together with the atoms to which they are attached form a (C₆-C₁₀)aryl ring or a 5- or 6-membered heteroaryl ring comprising 1 to 3 heteroatoms selected from O, N, and S, optionally substituted with one or more R₁₀, or
two R₇ together with the atoms to which they are attached form a (C₅-C₇) cycloalkyl ring or a 5- to 7-membered heterocycloalkyl ring comprising 1 to 3 heteroatoms selected from O, N, and S, optionally substituted with one or more R₁₀;
R₈ and R₉ are each independently H or (C₁-C₆)alkyl;
each R₁₀ is independently selected from (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -NH₂, and CN, or
two R₁₀ together with the carbon atom to which they are attached form a =(O);
each R₁₁ is independently selected from CN, (C₁-C₆)alkoxy, (C₆-C₁₀)aryl, and 5- to 7-membered heterocycloalkyl comprising 1 to 3 heteroatoms selected from O, N, and S, wherein the aryl and heterocycloalkyl are optionally substituted with one or more substituents each independently selected from (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -NH₂, and CN;
R₁₂ is (C₁-C₆)alkyl, (C₁-C₆)haloalkyl, (C₆-C₁₀)aryl, or 5- to 7-membered heterocycloalkyl comprising 1 to 3 heteroatoms selected from O, N, and S;
Rₓ is H or D; and
n is 0, 1, 2, or 3;
or a pharmaceutically acceptable salt, hydrate, solvate, stereoisomer, or tautomer thereof.

2. The compound according to claim 1, wherein Rₓ is H.

3. The compound according to claim 1 or 2, wherein R₁ is (C₁-C₆)alkoxy, halogen, -OH, -(CH₂)₀₋₂NH₂, or CN.

4. The compound according to any one of claims 1-3, wherein R₃ is (C₁-C₆)alkyl optionally substituted with one to three R₄, optionally wherein R₃ is (C₁-C₆)alkyl substituted with one to three R₄.

5. The compound according to any one of claims 1-4, wherein R₄ is selected from (C₆-C₁₀)aryl and 5- or 6-membered heteroaryl comprising 1 to 4 heteroatoms selected from O, N, and S, wherein the aryl and heteroaryl are optionally substituted with one to three R₆, optionally wherein R₄ is phenyl optionally substituted with one to three R₆.

6. The compound according to any one of claims 1-5, wherein n is 0.

7. The compound of claim 1, having a Formula (Ia) or Formula (Ib): 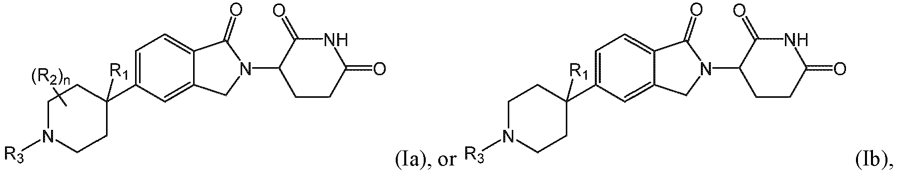 or a pharmaceutically acceptable salt, hydrate, solvate, stereoisomer, or tautomer thereof.

8. The compound according to claim 7, wherein R₃ is (C₁-C₆)alkyl optionally substituted with one to three R₄, optionally wherein R₃ is (C₁-C₆)alkyl substituted with one to three R₄.

9. The compound according to any one of claims 7-8, wherein R₄ is selected from (C₆-C₁₀)aryl and 5- or 6-membered heteroaryl comprising 1 to 4 heteroatoms selected from O, N, and S, wherein the aryl and heteroaryl are optionally substituted with one to three R₆, optionally wherein R₄ is phenyl optionally substituted with one to three R₆.

10. The compound according to claim 1 selected from: 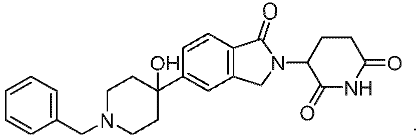 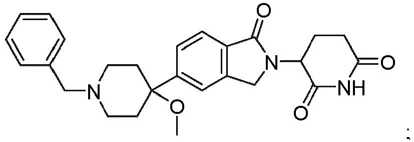 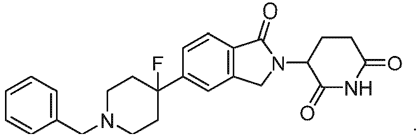 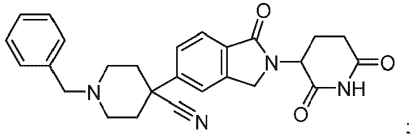 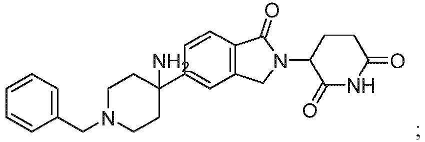 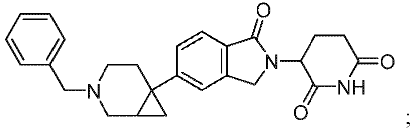 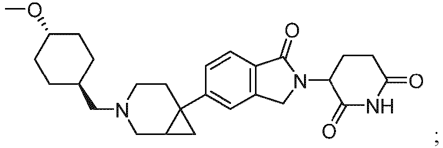 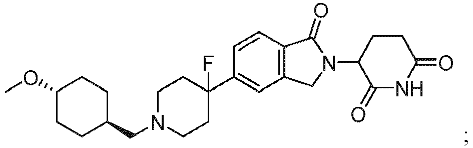 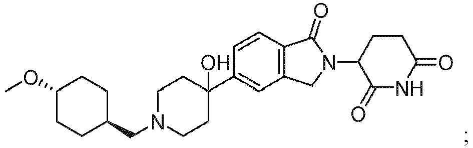 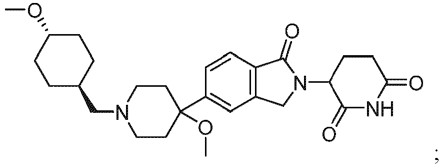 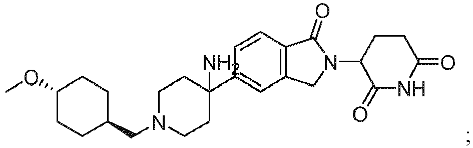 and 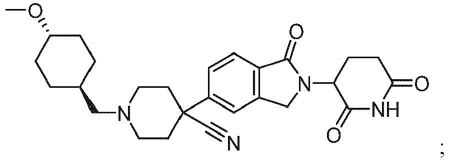 or a pharmaceutically acceptable salt, hydrate, solvate, stereoisomer, or tautomer thereof.

11. A compound selected from: 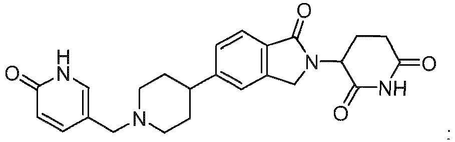 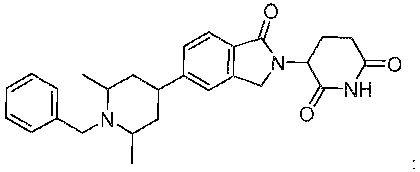 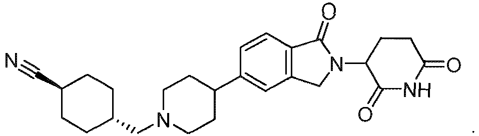 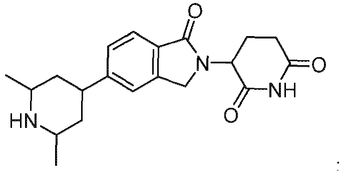 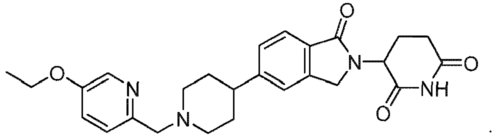 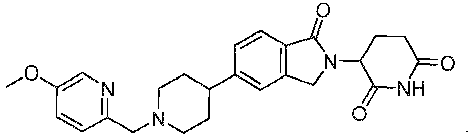 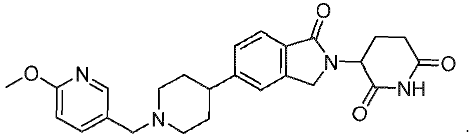 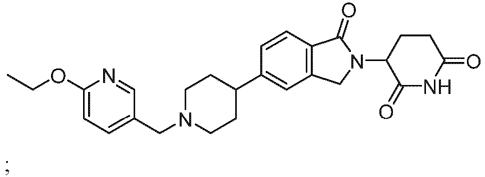 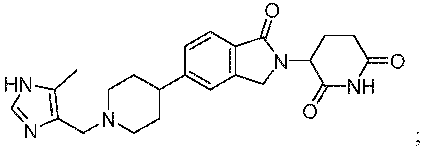 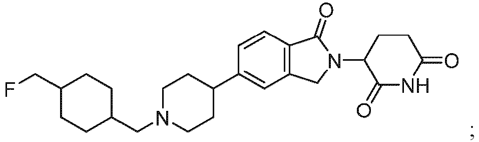 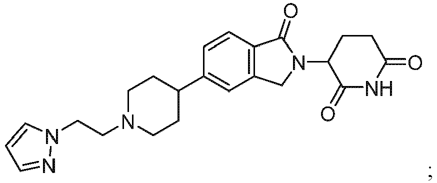 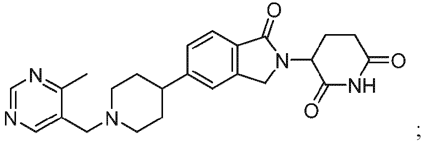 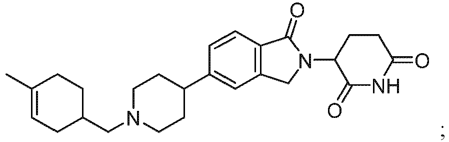 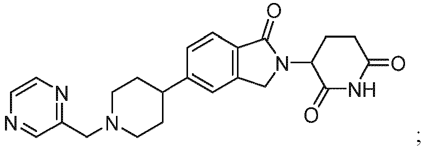 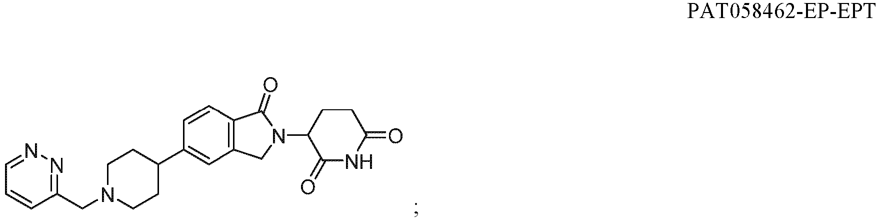 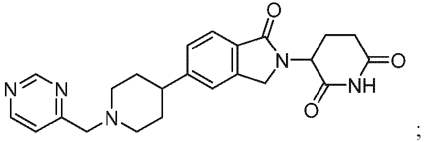 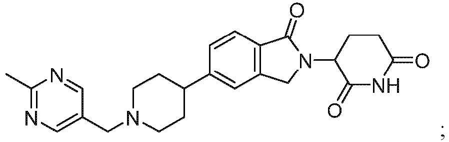 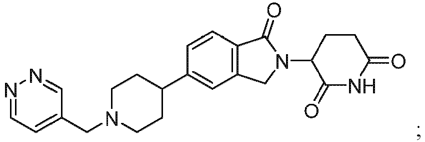 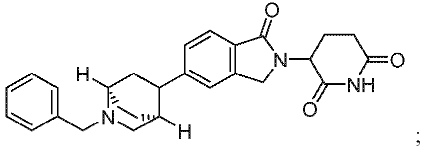 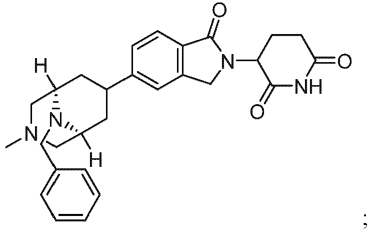 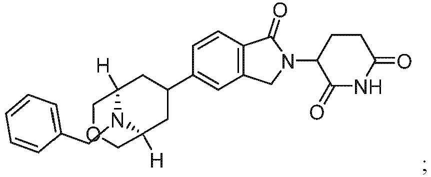 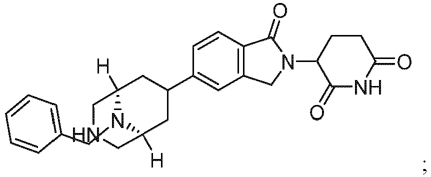 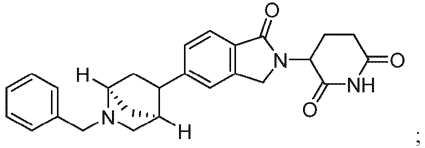 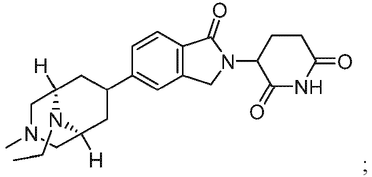 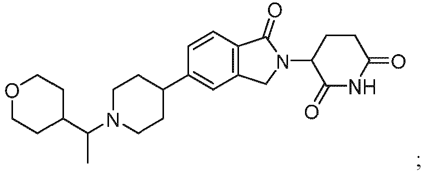 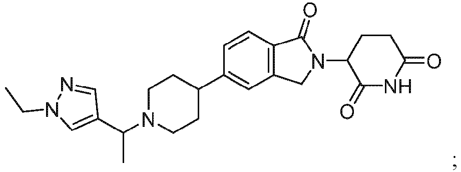 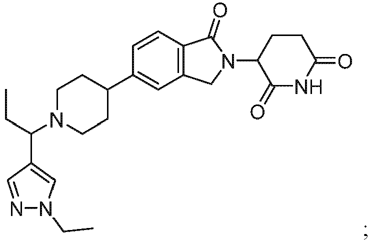 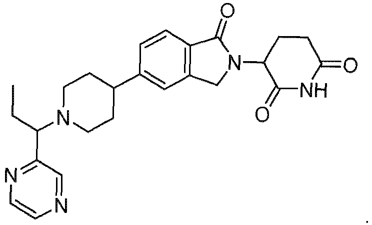 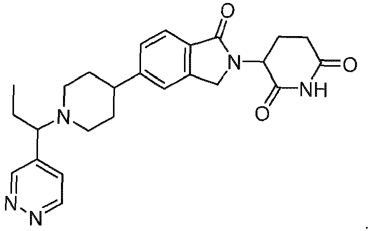 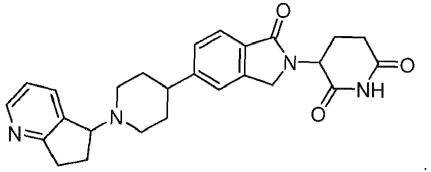 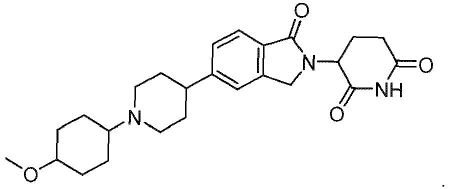 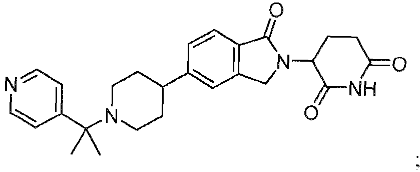 and 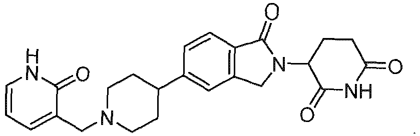 or a pharmaceutically acceptable salt, hydrate, solvate, stereoisomer, or tautomer thereof.

12. A pharmaceutical composition comprising a therapeutically effective amount of a compound according to any one of the claims 1-11, or a pharmaceutically acceptable salt, hydrate, solvate, stereoisomer, or tautomer thereof, and a pharmaceutically acceptable carrier or excipient, optionally further comprising at least one additional pharmaceutical agent.

13. The pharmaceutical composition according to claim 12 for use in the treatment of a disease or disorder that is affected by the reduction of IKZF2 protein levels.

14. A compound according to any one of claims 1-11, or a pharmaceutically acceptable salt, hydrate, solvate, stereoisomer, or tautomer thereof, for use in the treatment of a disease or disorder that is affected by the reduction of IKZF2 protein levels.

15. The compound for use according to claim 14, wherein the disease or disorder is selected from non-small cell lung cancer (NSCLC), melanoma, triple-negative breast cancer (TNBC), nasopharyngeal cancer (NPC), microsatellite stable colorectal cancer (mssCRC), thymoma, carcinoid, acute myelogenous leukemia, and gastrointestinal stromal tumor (GIST).

## Patentansprüche

1. Verbindung der Formel (I): 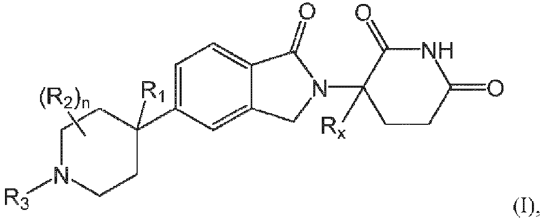 wobei:
R₁ für (C₁-C₆)Alkyl, (C₁-C₆)Alkoxy, (C₁-C₆)Halogenalkyl, (C₁-C₆)Halogenalkoxy, (C₁-C₆)Hydroxyalkyl, Halogen, -OH, - (CH₂)₀₋₂NH₂, - (CH₂)₀₋₂NH(C₁-C₆)Alkyl, - (CH₂)₀₋₂N((C₁-C₆)Alkyl)₂, -C(O)NH₂, -C(O)OH oder CN steht;
R₂ jeweils unabhängig für (C₁-C₆)Alkyl, (C₁-C₆) Halogenalkyl, (C₁-C₆)Hydroxyalkyl, CN oder Halogen steht oder
R₁ and R₂ zusammen mit den Kohlenstoffatomen, an die sie gebunden sind, ein (C₃-C₇)-Cycloalkyl oder einen 4- bis 6-gliedrigen Heterocycloalkylring mit 1-3 Heteroatomen, die aus O, N und S ausgewählt sind, bilden oder
zwei R₂ zusammen mit den Kohlenstoffatomen, an die sie gebunden sind, ein (C₃-C₇)-Cycloalkyl oder einen 4- bis 6-gliedrigen Heterocycloalkylring mit 1-3 Heteroatomen, die aus O, N und S ausgewählt sind, bilden;
R₃ für (C₁-C₆)Alkyl, (C₆-C₁₀)Aryl, 5- oder 6-gliedriges Heteroaryl mit 1 bis 3 Heteroatomen, die aus O, N und S ausgewählt sind, (C₃-C₈) Cycloalkyl oder 4- bis 7-gliedriges Heterocycloalkyl mit 1 bis 3 Heteroatomen, die aus O, N und S ausgewählt sind, steht, wobei das Alkyl gegebenenfalls durch ein oder mehrere R₄ substituiert ist und das Aryl, Heteroaryl, Cycloalkyl und Heterocycloalkyl gegebenenfalls durch ein oder mehrere R₅ substituiert sind, oder
R₂ und R₃, wenn sie sich an benachbarten Atomen befinden, zusammen mit den Atomen, an die sie gebunden sind, einen 5- oder 6-gliedrigen Heterocycloalkylring bilden;
R₄ jeweils unabhängig aus -C(O)OR₆, -C(O)NR₆R_{6'}, - NR₆C(O)R_{6'}, Halogen, -OH, -NH₂, CN, (C₆-C₁₀)Aryl, 5- oder 6-gliedrigem Heteroaryl mit 1 bis 4 Heteroatomen, die aus O, N und S ausgewählt sind, (C₃-C₈) Cycloalkyl und einem 4- bis 7-gliedrigen Heterocycloalkylring mit 1 bis 3 Heteroatomen, die aus O, N und S ausgewählt sind, ausgewählt ist, wobei die Aryl-, Heteroaryl-, Cycloalkyl- und Heterocycloalkylgruppen gegebenenfalls durch ein oder mehrere R₇ substituiert sind;
R₅ jeweils unabhängig aus (C₁-C₆)Alkyl, (C₂-C₆)Alkenyl, (C₂-C₆)Alkinyl, (C₁-C₆)Alkoxy, (C₁-C₆)Halogenalkyl, (C₁-C₆)Halogenalkoxy, (C₁-C₆)Hydroxyalkyl, Halogen, -OH, -NH₂, CN, (C₃-C₇)Cycloalkyl, 5- bis 7-gliedrigem Heterocycloalkyl mit 1 bis 3 Heteroatomen, die aus O, N und S ausgewählt sind, (C₆-C₁₀)Aryl und 5- oder 6-gliedrigem Heteroaryl mit 1 bis 3 Heteroatomen, die aus O, N und S ausgewählt sind, ausgewählt ist oder
zwei R₅, wenn sie sich an benachbarten Atomen befinden, zusammen mit den Atomen, an die sie gebunden sind, einen (C₆-C₁₀) Arylring oder einen 5- oder 6-gliedrigen Heteroarylring mit 1 bis 3 Heteroatomen, die aus O, N und S ausgewählt sind, der gegebenenfalls durch ein oder mehrere R₁₀ substituiert ist, bilden oder
zwei R₅ zusammen mit den Atomen, an die sie gebunden sind, einen (C₃-C₇)Cycloalkylring oder einen 4- bis 7-gliedrigen Heterocycloalkylring mit 1 bis 3 Heteroatomen, die aus O, N und S ausgewählt sind, der gegebenenfalls durch ein oder mehrere R₁₀ substituiert ist, bilden;
R₆ und R_{6'} jeweils unabhängig für H, (C₁-C₆)Alkyl oder (C₆-C₁₀)Aryl stehen;
R₇ jeweils unabhängig aus (C₁-C₆)Alkyl, (C₂-C₆)Alkenyl, (C₂-C₆)Alkinyl, (C₁-C₆)Alkoxy, (C₁-C₆)Halogenalkyl, (C₁-C₆) Halogenalkoxy, -C(O)R₈, -(CH₂)₀₋₃C(O)OR₈, -C(O)NR₈R₉, - NR₈C(O)R₉, -NR₈C(O)OR₉, -S(O)ₚNR₈R₉, -S(O)ₚR₁₂, (C₁-C₆) Hydroxyalkyl, Halogen, -OH, -O (CH₂)₁₋₃CN, -NH₂, CN, - O (CH₂)₀₋₃(C₆-C₁₀) Aryl, Adamantyl, -O(CH₂)₀₋₃-5- oder 6-gliedrigem Heteroaryl mit 1 bis 3 Heteroatomen, die aus O, N und S ausgewählt sind, (C₆-C₁₀)Aryl, monocyclischen oder bicyclischen 5- bis 10-gliedrigem Heteroaryl mit 1 bis 3 Heteroatomen, die aus O, N und S ausgewählt sind, (C₃-C₇)Cycloalkyl und 5- bis 7-gliedrigem Heterocycloalkyl mit 1 bis 3 Heteroatomen, die aus O, N und S ausgewählt sind, ausgewählt ist, wobei das Alkyl gegebenenfalls durch ein oder mehrere R₁₁ substituiert ist und das Aryl, Heteroaryl und Heterocycloalkyl gegebenenfalls durch einen oder mehrere Substituenten, die jeweils unabhängig aus Halogen, (C₁-C₆)Alkyl, (C₁-C₆) Halogenalkyl und (C₁-C₆)Alkoxy ausgewählt sind, substituiert sind, oder
zwei R₇ zusammen mit dem Kohlenstoffatom, an das sie gebunden sind, ein =(O) bilden oder
zwei R₇, wenn sie sich an benachbarten Atomen befinden, zusammen mit den Atomen, an die sie gebunden sind, einen (C₆-C₁₀) Arylring oder einen 5- oder 6-gliedrigen Heteroarylring mit 1 bis 3 Heteroatomen, die aus O, N und S ausgewählt sind, der gegebenenfalls durch ein oder mehrere R₁₀ substituiert ist, bilden oder
zwei R₇ zusammen mit den Atomen, an die sie gebunden sind, einen (C₅-C₇) Cycloalkylring oder einen 5- bis 7-gliedrigen Heterocycloalkylring mit 1 bis 3 Heteroatomen, die aus O, N und S ausgewählt sind, der gegebenenfalls durch ein oder mehrere R₁₀ substituiert ist, bilden;
R₈ und R₉ jeweils unabhängig für H oder (C₁-C₆)Alkyl stehen;
R₁₀ jeweils unabhängig aus (C₁-C₆)Alkyl, (C₁-C₆)Alkoxy, (C₁-C₆) Halogenalkyl, (C₁-C₆) Halogenalkoxy, (C₁-C₆)Hydroxyalkyl, Halogen, -OH, -NH₂ und CN ausgewählt ist oder
zwei R₁₀ zusammen mit dem Kohlenstoffatom, an das sie gebunden sind, ein =(O) bilden;
R₁₁ jeweils unabhängig aus CN, (C₁-C₆)Alkoxy, (C₆-C₁₀)Aryl und 5- bis 7-gliedrigem Heterocycloalkyl mit 1 bis 3 Heteroatomen, die aus O, N und S ausgewählt sind, ausgewählt ist, wobei das Aryl und Heterocycloalkyl gegebenenfalls durch einen oder mehrere Substituenten, die jeweils unabhängig aus (C₁-C₆)Alkyl, (C₁-C₆)Alkoxy, (C₁-C₆) Halogenalkyl, (C₁-C₆) Halogenalkoxy, (C₁-C₆)Hydroxyalkyl, Halogen, -OH, -NH₂ und CN ausgewählt sind, substituiert sind;
R₁₂ für (C₁-C₆)Alkyl, (C₁-C₆)Halogenalkyl, (C₆-C₁₀)Aryl oder 5- bis 7-gliedriges Heterocycloalkyl mit 1 bis 3 Heteroatomen, die aus O, N und S ausgewählt sind, steht; Rₓ für H oder D steht; und
n für 0, 1, 2 oder 3 steht;
oder ein pharmazeutisch unbedenkliches Salz, Hydrat, Solvat, Stereoisomer oder Tautomer davon.

2. Verbindung nach Anspruch 1, wobei Rₓ für H steht.

3. Verbindung nach Anspruch 1 oder 2, wobei R₁ für (C₁-C₆)Alkoxy, Halogen, -OH, -(CH₂)₀₋₂NH₂ oder CN steht.

4. Verbindung nach einem der Ansprüche 1-3, wobei R₃ für (C₁-C₆)Alkyl, das gegebenenfalls durch ein bis drei R₄ substituiert ist, wobei R₃ gegebenenfalls für (C₁-C₆)Alkyl, das durch ein bis drei R₄ substituiert ist, steht.

5. Verbindung nach einem der Ansprüche 1-4, wobei R₄ aus (C₆-C₁₀)Aryl und 5- oder 6-gliedrigem Heteroaryl mit 1 bis 4 Heteroatomen, die aus O, N und S ausgewählt sind, ausgewählt ist, wobei das Aryl und Heteroaryl gegebenenfalls durch ein bis drei R₆ substituiert sind, gegebenenfalls wobei R₄ für Phenyl, das gegebenenfalls durch ein bis drei R₆ substituiert ist, steht.

6. Verbindung nach einem der Ansprüche 1 bis 5, wobei n für 0 steht.

7. Verbindung nach Anspruch 1 mit einer Formel (Ia) oder Formel (Ib): 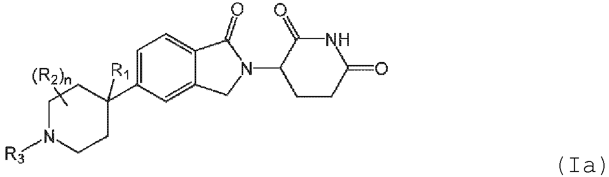 oder 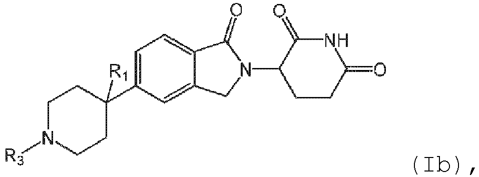 oder ein pharmazeutisch unbedenkliches Salz, Hydrat, Solvat, Stereoisomer oder Tautomer davon.

8. Verbindung nach Anspruch 7, wobei R₃ für (C₁-C₆)Alkyl, das gegebenenfalls durch ein bis drei R₄ substituiert ist, steht, gegebenenfalls wobei R₃ für (C₁-C₆)Alkyl, das durch ein bis drei R₄ substituiert ist, steht.

9. Verbindung nach einem der Ansprüche 7-8, wobei R₄ aus (C₆-C₁₀)Aryl und 5- oder 6-gliedrigem Heteroaryl mit 1 bis 4 Heteroatomen, die aus O, N und S ausgewählt sind, ausgewählt ist, wobei das Aryl und Heteroaryl gegebenenfalls durch ein bis drei R₆ substituiert sind, gegebenenfalls wobei R₄ für Phenyl, das gegebenenfalls durch ein bis drei R₆ substituiert ist, steht.

10. Verbindung nach Anspruch 1, ausgewählt aus: 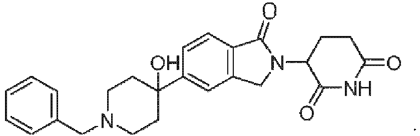 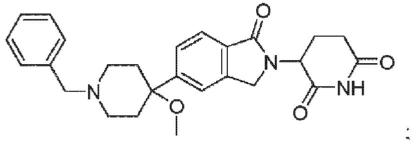 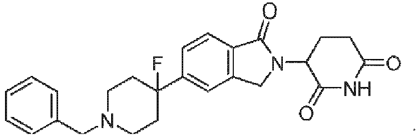 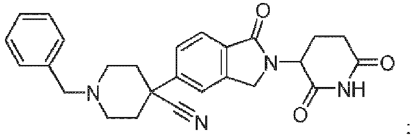 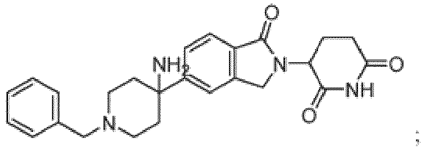 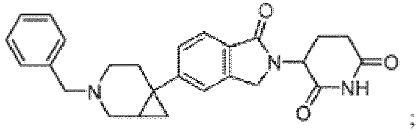 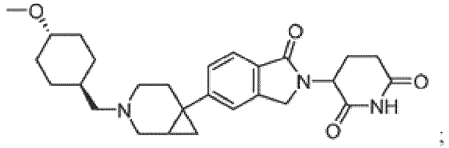 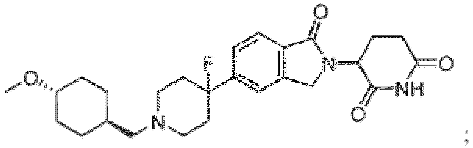 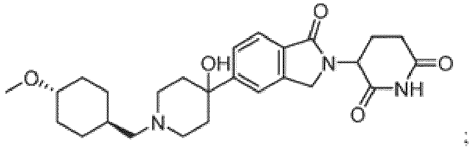 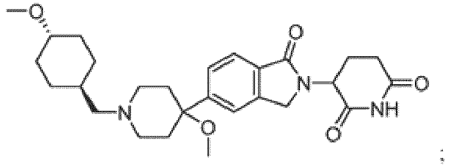 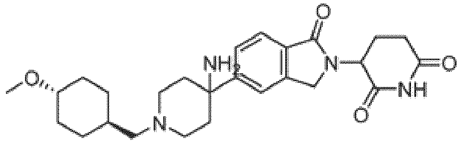 und 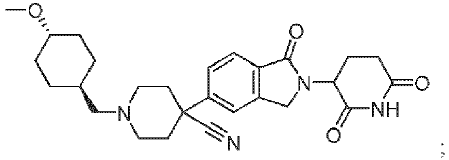 oder ein pharmazeutisch unbedenkliches Salz, Hydrat, Solvat, Stereoisomer oder Tautomer davon.

11. Verbindung, ausgewählt aus: 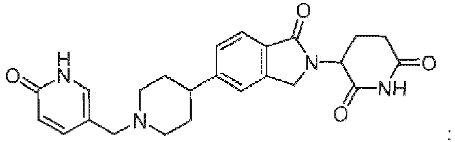 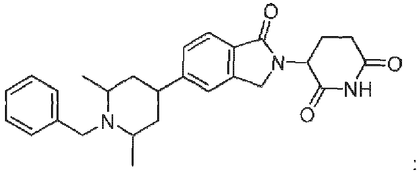 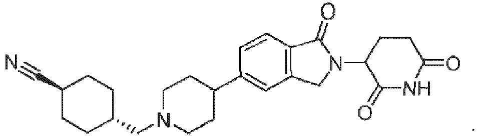 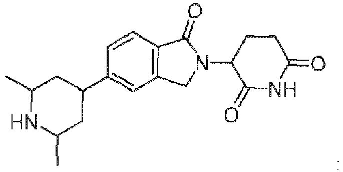 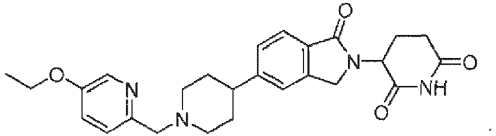 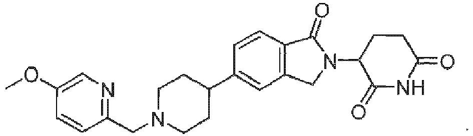 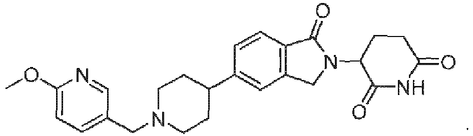 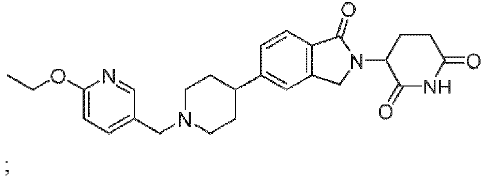 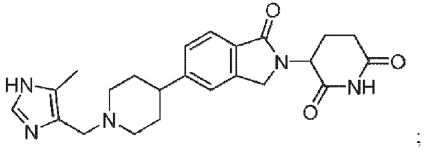 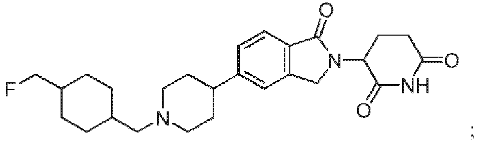 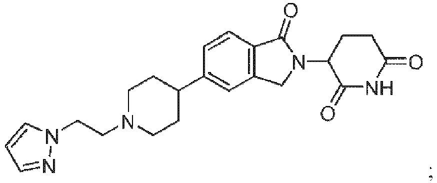 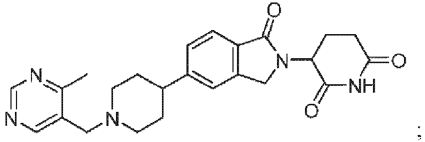 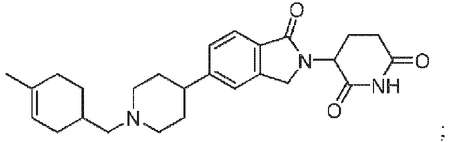 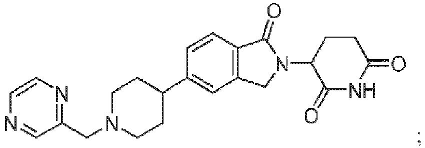 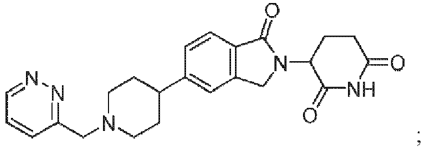 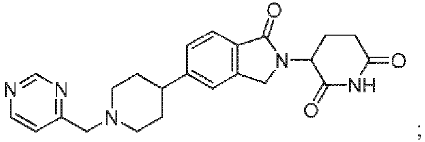 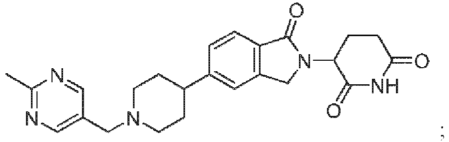 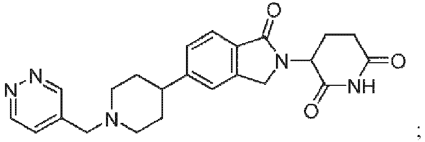 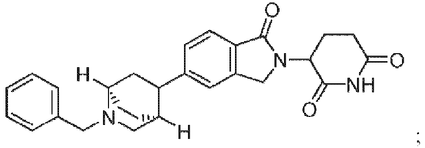 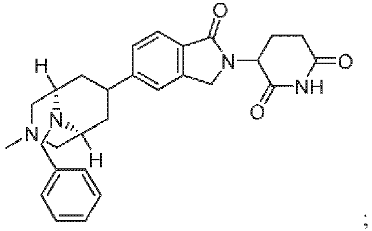 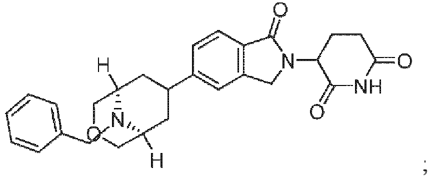 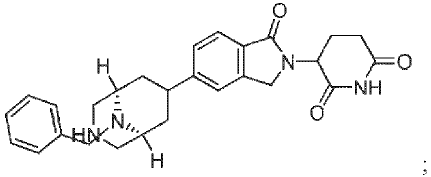 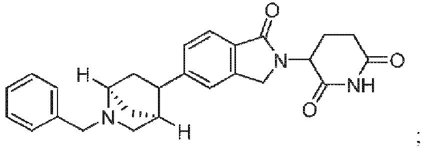 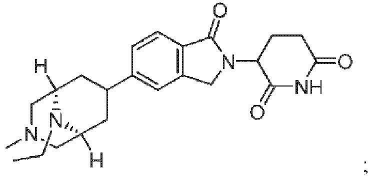 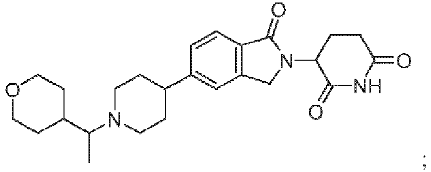 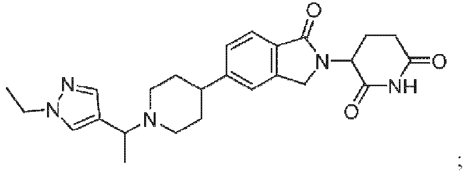 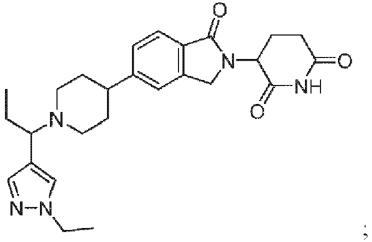 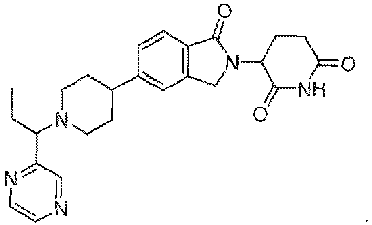 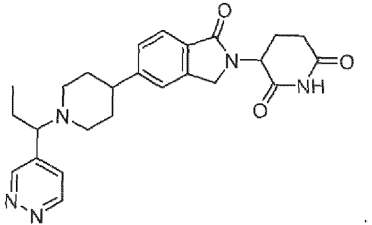 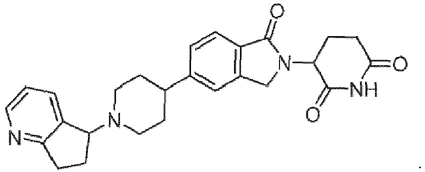 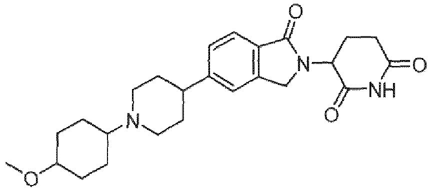 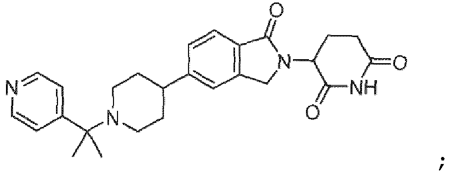 und 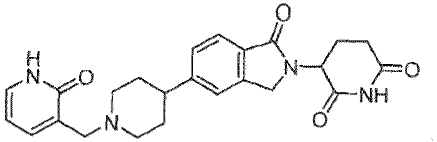 oder ein pharmazeutisch unbedenkliches Salz, Hydrat, Solvat, Stereoisomer oder Tautomer davon.

12. Pharmazeutische Zusammensetzung, umfassend eine therapeutisch wirksame Menge einer Verbindung nach einem der Ansprüche 1-11 oder eines pharmazeutisch unbedenklichen Salzes, Hydrats, Solvats, Stereoisomers oder Tautomers davon und einen pharmazeutisch unbedenklichen Träger oder Hilfsstoff und gegebenenfalls ferner mindestens ein zusätzliches pharmazeutisches Mittel.

13. Pharmazeutische Zusammensetzung nach Anspruch 12 zur Verwendung bei der Behandlung einer Erkrankung oder Störung, die durch die Verringerung von IKZF2-Proteinniveaus beeinflusst wird.

14. Verbindung nach einem der Ansprüche 1-11 oder ein pharmazeutisch unbedenkliches Salz, Hydrat, Solvat, Stereoisomer oder Tautomer davon zur Verwendung bei der Behandlung einer Erkrankung oder Störung, die durch die Verringerung von IKZF2-Proteinniveaus beeinflusst wird.

15. Verbindung zur Verwendung nach Anspruch 14, wobei die Erkrankung oder Störung aus nichtkleinzelligem Lungenkrebs (NSCLC), Melanom, dreifach-negativem Brustkrebs (TNBC), Nasopharyngealkrebs (NPC), mikrosatellitenstabilem Kolorektalkarzinom (mssCRC), Thymom, Karzinoid, akuter myeloischer Leukämie und gastrointestinalem Stromatumor (GIST) ausgewählt ist.

## Revendications

1. Composé de formule (I) : 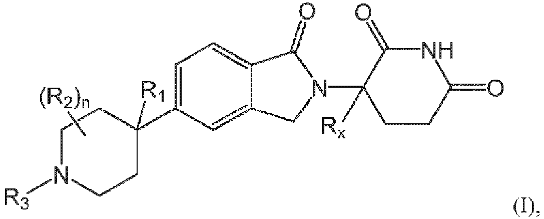
R₁ étant (C₁-C₆) alkyle, (C₁-C₆) alcoxy, (C₁-C₆) halogénoalkyle, (C₁-C₆) halogénoalcoxy, (C₁-C₆) hydroxyalkyle, halogène, -OH, - (CH₂)₀₋₂NH₂, -(CH₂)₀₋₂NH(C₁-C₆)alkyle, -(CH₂)₀₋₂N((C₁-C₆)alkyl)₂, -C(O)NH₂, - C(O)OH ou CN ;
chaque R₂ étant indépendamment (C₁-C₆) alkyle, (C₁-C₆) halogénoalkyle, (C₁-C₆) hydroxyalkyle, CN, ou halogène, ou
R₁ et R₂ conjointement avec les atomes de carbone auxquels ils sont fixés formant un (C₃-C₇)cycloalkyle ou un cycle hétérocycloalkyle à 4 à 6 chaînons comprenant 1 à 3 hétéroatomes choisis parmi O, N, et S, ou
deux R₂ conjointement avec les atomes de carbone auxquels ils sont fixés formant (C₃-C₇)cycloalkyle ou un cycle hétérocycloalkyle à 4 à 6 chaînons comprenant 1 à 3 hétéroatomes choisis parmi O, N, et S ;
R₃ étant (C₁-C₆) alkyle, (C₆-C₁₀) aryle, hétéroaryle à 5 ou 6 chaînons comprenant 1 à 3 hétéroatomes choisis parmi O, N, et S, (C₃-C₈)cycloalkyle, ou hétérocycloalkyle à 4 à 7 chaînons comprenant 1 à 3 hétéroatomes choisis parmi O, N, et S, l'alkyle étant éventuellement substitué par un ou plusieurs R₄; et l'aryle, hétéroaryle, cycloalkyle, et hétérocycloalkyle étant éventuellement substitués par un ou plusieurs R₅, ou
R₂ et R₃, lorsqu'ils sont sur des atomes adjacents, conjointement avec les atomes auxquels ils sont fixés formant un cycle hétérocycloalkyle à 5 ou 6 chaînons ;
chaque R₄ étant indépendamment choisi parmi -C(O)OR₆, - C(O)NR₆R_{6'}, -NR₆C(O)R_{6'}, halogène, -OH, -NH₂, CN, (C₆-C₁₀)aryle, hétéroaryle à 5 ou 6 chaînons comprenant 1 à 4 hétéroatomes choisis parmi O, N, et S, (C₃-C₈)cycloalkyle, et un cycle hétérocycloalkyle à 4 à 7 chaînons comprenant 1 à 3 hétéroatomes choisis parmi O, N, et S, les groupes aryle, hétéroaryle, cycloalkyle et hétérocycloalkyle étant éventuellement substitués par un ou plusieurs R₇ ;
chaque R₅ étant indépendamment choisi parmi (C₁-C₆) alkyle, (C₂-C₆) alcényle, (C₂-C₆) alcynyle, (C₁-C₆)alcoxy, (C₁-C₆) halogénoalkyle, (C₁-C₆) halogénoalcoxy, (C₁-C₆)hydroxyalkyle, halogène, -OH, -NH₂, CN, (C₃-C₇)cycloalkyle, hétérocycloalkyle à 5 à 7 chaînons comprenant 1 à 3 hétéroatomes choisis parmi O, N, et S, (C₆-C₁₀) aryle, et hétéroaryle à 5 ou 6 chaînons comprenant 1 à 3 hétéroatomes choisis parmi O, N, et S, ou
deux R₅, lorsqu'ils sont sur des atomes adjacents, conjointement avec les atomes auxquels ils sont fixés formant un cycle (C₆-C₁₀)aryle ou un cycle hétéroaryle à 5 ou 6 chaînons comprenant 1 à 3 hétéroatomes choisis parmi O, N, et S, éventuellement substitué par un ou plusieurs R₁₀, ou
deux R₅ conjointement avec les atomes auxquels ils sont fixés formant un cycle (C₃-C₇)cycloalkyle ou un cycle hétérocycloalkyle à 4 à 7 chaînons comprenant 1 à 3 hétéroatomes choisis parmi O, N, et S éventuellement substitué par un ou plusieurs R₁₀ ;
R₆ et R₆, étant chacun indépendamment H, (C₁-C₆)alkyle, ou (C₆-C₁₀) aryle ;
chaque R₇ étant indépendamment choisi parmi (C₁-C₆) alkyle, (C₂-C₆) alcényle, (C₂-C₆) alcynyle, (C₁-C₆)alcoxy, (C₁-C₆) halogénoalkyle, (C₁-C₆)halogénoalcoxy, -C(O)R₈, - (CH₂)₀₋₃C(O)OR₈, -C(O)NR₈R₉, -NR₈C(O)R₉, -NR₈C(O)OR₉, - S(O)ₚNR₈R₉, -S(O)ₚR₁₂, (C₁-C₆)hydroxyalkyle, halogène, -OH, -O(CH₂)₁₋₃CN, -NH₂, CN, -O(CH₂)₀₋₃(C₆-C₁₀) aryle, adamantyle, -O(CH₂)₀₋₃-hétéroaryle à 5 ou 6 chaînons comprenant 1 à 3 hétéroatomes choisis parmi O, N, et S, (C₆-C₁₀) aryle, hétéroaryle monocyclique ou bicyclique à 5 à 10 chaînons comprenant 1 à 3 hétéroatomes choisis parmi O, N, et S, (C₃-C₇)cycloalkyle, et hétérocycloalkyle à 5 à 7 chaînons comprenant 1 à 3 hétéroatomes choisis parmi O, N, et S, l'alkyle étant éventuellement substitué par un ou plusieurs R₁₁, et l'aryle, hétéroaryle, et hétérocycloalkyle étant éventuellement substitués par un ou plusieurs substituants chacun indépendamment choisi parmi halogène, (C₁-C₆) alkyle, (C₁-C₆)halogénoalkyle, et (C₁-C₆) alcoxy, ou
deux R₇ conjointement avec l'atome de carbone auquel ils sont fixés formant un =(O), ou
deux R₇, lorsqu'ils sont sur des atomes adjacents, conjointement avec les atomes auxquels ils sont fixés formant un cycle (C₆-C₁₀)aryle ou un cycle hétéroaryle à 5 ou 6 chaînons comprenant 1 à 3 hétéroatomes choisis parmi O, N, et S, éventuellement substitué par un ou plusieurs R₁₀, ou
deux R₇ conjointement avec les atomes auxquels ils sont fixés formant un cycle (C₅-C₇) cycloalkyle ou un cycle hétérocycloalkyle à 5 à 7 chaînons comprenant 1 à 3 hétéroatomes choisis parmi O, N, et S, éventuellement substitué par un ou plusieurs R₁₀ ;
R₈ et R₉ étant chacun indépendamment H ou (C₁-C₆)alkyle ; chaque R₁₀ étant indépendamment choisi parmi (C₁-C₆) alkyle, (C₁-C₆) alcoxy, (C₁-C₆)halogénoalkyle, (C₁-C₆)halogénoalcoxy, (C₁-C₆)hydroxyalkyle, halogène, -OH, - NH₂, et CN, ou
deux R₁₀ conjointement avec l'atome de carbone auquel ils sont fixés formant un =(O) ;
chaque R₁₁ étant indépendamment choisi parmi CN, (C₁-C₆) alcoxy, (C₆-C₁₀) aryle, et hétérocycloalkyle à 5 à 7 chaînons comprenant 1 à 3 hétéroatomes choisis parmi O, N, et S, l'aryle et hétérocycloalkyle étant éventuellement substitués par un ou plusieurs substituants chacun indépendamment choisi parmi (C₁-C₆) alkyle, (C₁-C₆) alcoxy, (C₁-C₆) halogénoalkyle, (C₁-C₆) halogénoalcoxy, (C₁-C₆)hydroxyalkyle, halogène, -OH, - NH₂, et CN ;
R₁₂ étant (C₁-C₆) alkyle, (C₁-C₆) halogénoalkyle, (C₆-C₁₀)aryle, ou hétérocycloalkyle à 5 à 7 chaînons comprenant 1 à 3 hétéroatomes choisis parmi O, N, et S ; Rₓ étant H ou D ; et
n étant 0, 1, 2, ou 3 ;
ou sel, hydrate, solvate, stéréoisomère ou forme tautomère pharmaceutiquement acceptable correspondant(e).

2. Composé selon la revendication 1, Rₓ étant H.

3. Composé selon la revendication 1 ou 2, R₁ étant (C₁-C₆) alcoxy, halogène, -OH, -(CH₂)₀₋₂NH₂, ou CN.

4. Composé selon l'une quelconque des revendications 1-3, R₃ étant (C₁-C₆)alkyle éventuellement substitué par un à trois R₄, éventuellement, R₃ étant (C₁-C₆)alkyle substitué par un à trois R₄.

5. Composé selon l'une quelconque des revendications 1-4, R₄ étant choisi parmi (C₆-C₁₀)aryle et hétéroaryle à 5 ou 6 chaînons comprenant 1 à 4 hétéroatomes choisis parmi O, N, et S, l'aryle et hétéroaryle étant éventuellement substitués par un à trois R₆, éventuellement, R₄ étant phényle éventuellement substitué par un à trois R₆.

6. Composé selon l'une quelconque des revendications 1-5, n étant 0.

7. Composé selon la revendication 1, ayant une formule (Ia) ou une formule (Ib) : 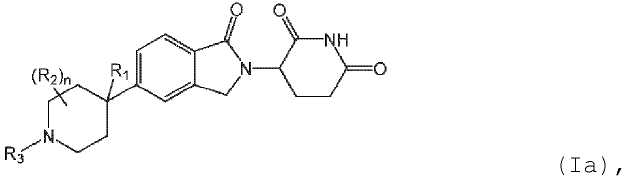 ou 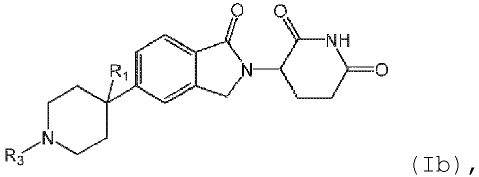 ou sel, hydrate, solvate, stéréoisomère ou forme tautomère pharmaceutiquement acceptable correspondant(e).

8. Composé selon la revendication 7, R₃ étant (C₁-C₆)alkyle éventuellement substitué par un à trois R₄, éventuellement, R₃ étant (C₁-C₆)alkyle substitué par un à trois R₄.

9. Composé selon l'une quelconque des revendications 7-8, R₄ étant choisi parmi (C₆-C₁₀)aryle et hétéroaryle à 5 ou 6 chaînons comprenant 1 à 4 hétéroatomes choisis parmi O, N, et S, l'aryle et hétéroaryle étant éventuellement substitués par un à trois R₆, éventuellement, R₄ étant phényle éventuellement substitué par un à trois R₆.

10. Composé selon la revendication 1 choisi parmi : 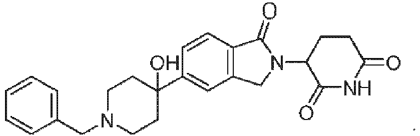 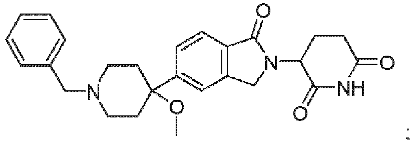 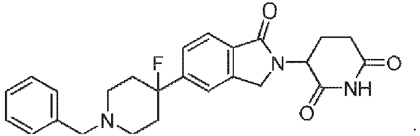 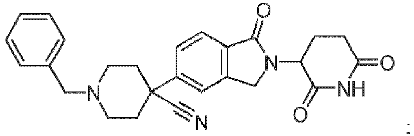 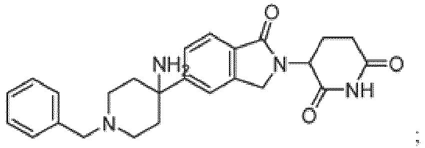 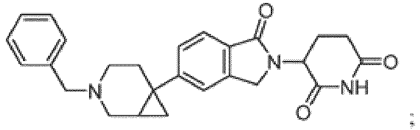 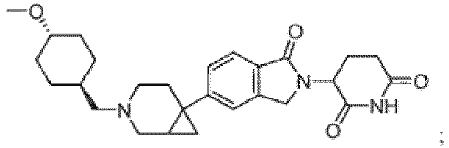 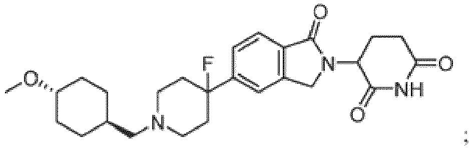 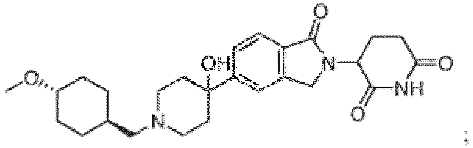 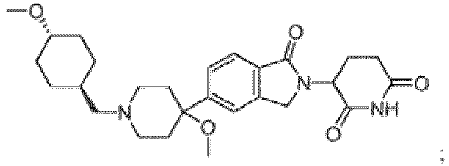 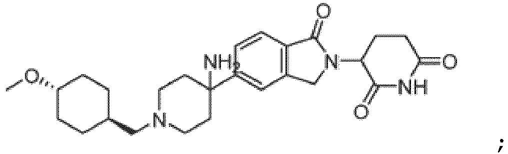 et 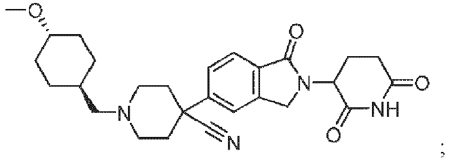 ou sel, hydrate, solvate, stéréoisomère ou forme tautomère pharmaceutiquement acceptable correspondant(e).

11. Composé choisi parmi : 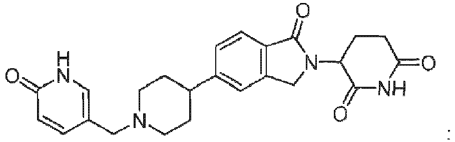 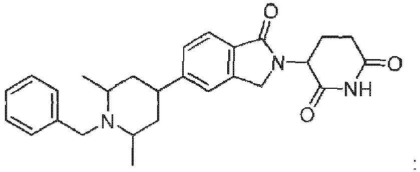 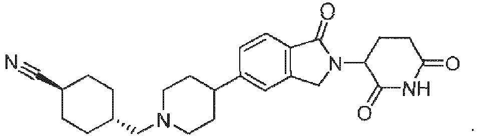 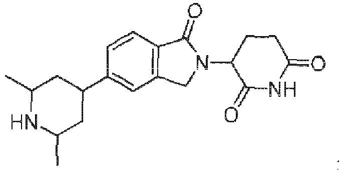 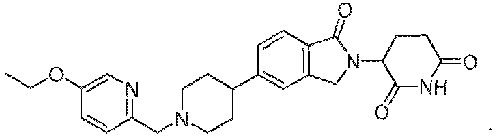 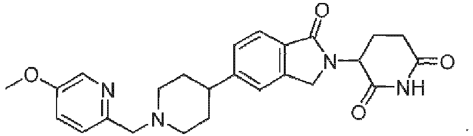 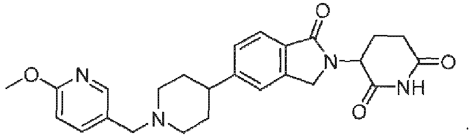 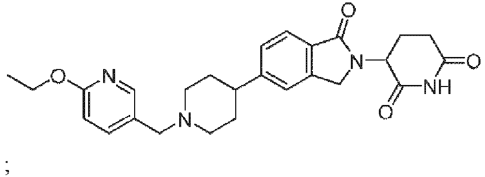 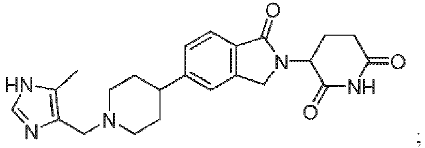 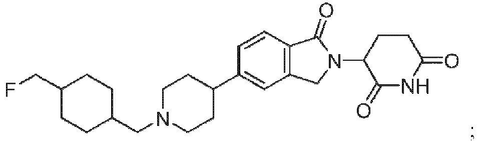 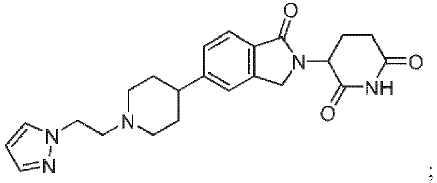 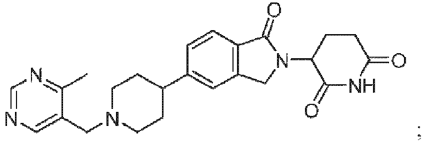 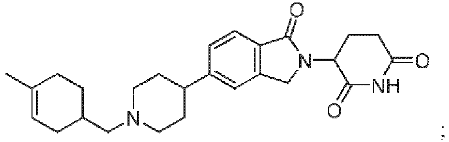 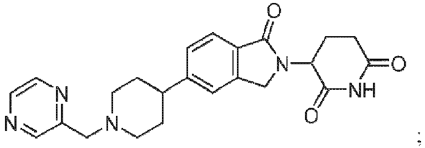 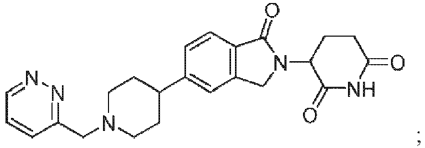 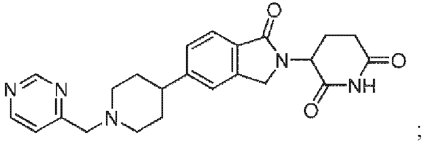 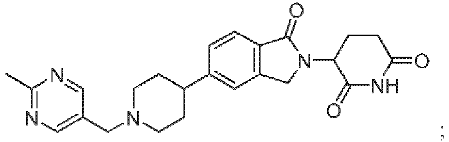 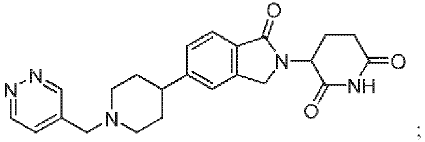 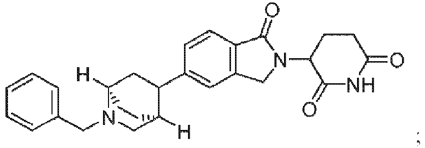 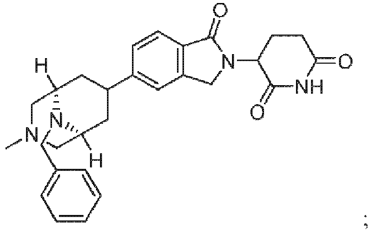 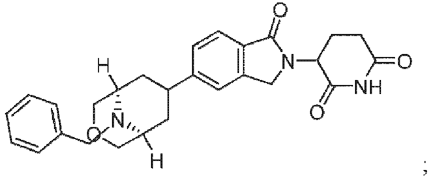 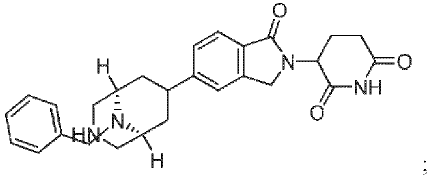 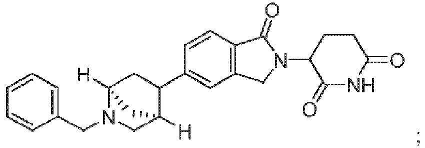 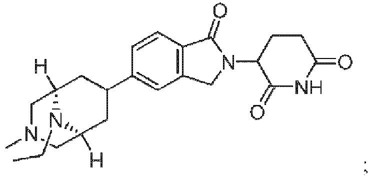 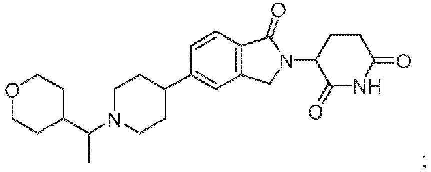 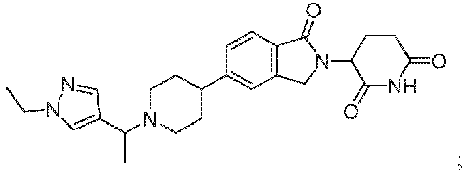 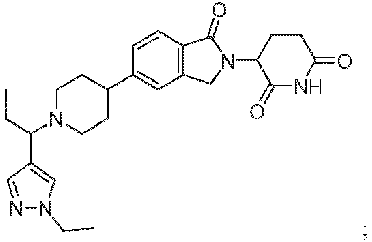 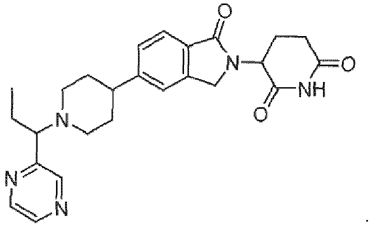 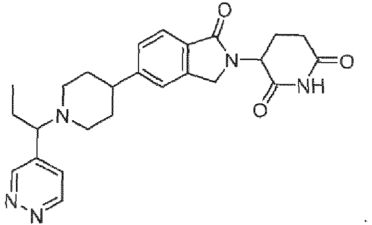 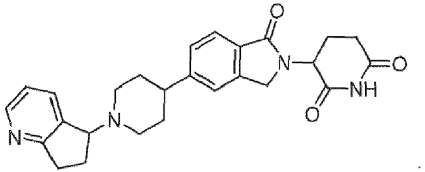 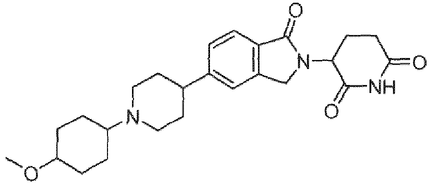 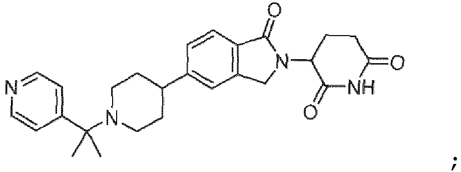 et 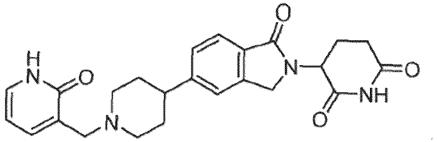 ou sel, hydrate, solvate, stéréoisomère ou forme tautomère pharmaceutiquement acceptable correspondant(e).

12. Composition pharmaceutique comprenant une quantité thérapeutiquement efficace d'un composé selon l'une quelconque des revendications 1 à 11, ou d'un sel, d'un hydrate, d'un solvate, d'un stéréoisomère ou d'une forme tautomère pharmaceutiquement acceptable correspondant(e), et un excipient ou support pharmaceutiquement acceptable, comprenant éventuellement en outre au moins un agent pharmaceutique supplémentaire.

13. Composition pharmaceutique selon la revendication 12, destinée à être utilisée dans le traitement d'une maladie ou d'un trouble qui est affecté(e) par la réduction des taux de protéine IKZF2.

14. Composé selon l'une quelconque des revendications 1 à 11, ou sel, hydrate, solvate, stéréoisomère ou forme tautomère pharmaceutiquement acceptable correspondant(e), pour une utilisation dans le traitement d'une maladie ou d'un trouble qui est affecté(e) par la réduction des taux de protéine IKZF2.

15. Composé pour une utilisation selon la revendication 14, dans lequel la maladie ou le trouble est choisi(e) parmi le cancer du poumon non à petites cellules (NSCLC), le mélanome, le cancer du sein triple négatif (TNBC), le cancer du nasopharynx (NPC), le cancer colorectal microsatellite stable (mssCRC), le thymome, le carcinoïde, la leucémie myéloïde aiguë et la tumeur stromale gastro-intestinale (GIST).
